# EUROPÄISCHE PATENTSCHRIFT

(11) **EP 0 880 568 B1**
(45) Veröffentlichungstag und Bekanntmachung des Hinweises auf die Patenterteilung: **06.05.2004**
(21) Anmeldenummer: 97904377.5
(22) Anmeldetag: 04.02.1997
(51) Int. Cl.: C09K 9/02, G02F 1/15

(54) **ELEKTROCHROMES SYSTEM**
ELECTROCHROMIC SYSTEM
SYSTEME ELECTROCHROME

(30) Priorität: 15.02.1996 DE 19605451
(43) Veröffentlichungstag der Anmeldung: 02.12.1998
(73) Patentinhaber: Bayer Chemicals AG, 51368 Leverkusen (DE)
(72) Erfinder: CLAUSSEN, Uwe, D-51379 Leverkusen (DE); BERNETH, Horst, D-51373 Leverkusen (DE); HAARER, Dietrich, D-95448 Bayreuth (DE); SIMMERER, Jürgen, D-91052 Erlangen (DE); SCHALLER, Jochen, D-95126 Schwarzenbach (DE)
(86) Internationale Anmeldenummer: PCT/EP1997/000498
(87) Internationale Veröffentlichungsnummer: WO 1997/030134

(56) Entgegenhaltungen:
- EP-A- 0 240 226
- EP-A- 0 431 547
- EP-A- 0 491 364
- EP-A- 0 613 039
- WO-A-98/05736
- GB-A- 2 021 277

## Beschreibung

Die vorliegende Erfindung betrifft ein elektrochromes System, eine elektrochrome Flüssigkeit enthaltend dieses elektrochrome System, eine elektrochrome Vorrichtung sowie neue elektrochrome Substanzen und Verfahren zu deren Herstellung.

Elektrochrome Vorrichtungen, die ein elektrochromes System enthalten, sind bereits bekannt.

Solche Vorrichtungen enthalten als elektrochromes System üblicherweise Paare von Redoxsubstanzen, die in einem inerten Lösungsmittel gelöst sind. Zusätzlich können Leitsalze, Lichtstabilisatoren und Substanzen, die die Viskosität beeinflussen, enthalten sein.

Als Paar von Redoxsubstanzen wird je eine reduzierbare und eine oxidierbare Substanz verwendet. Beide sind farblos oder nur schwach gefärbt. Unter Einfluß einer elektrischen Spannung wird die eine Substanz reduziert, die andere oxidiert, wobei wenigstens eine farbig wird. Nach Abschalten der Spannung bilden sich die beiden ursprünglichen Redoxsubstanzen wieder zurück, wobei Entfärbung bzw. Farbaufhellung auftritt.

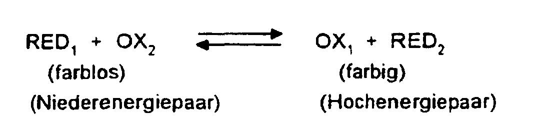

Aus US-4.902.108 ist bekannt, daß solche Paare von Redoxsubstanzen geeignet sind, bei denen die reduzierbare Substanz wenigstens zwei chemisch reversible Reduktionswellen im cyclischen Voltammogramm und die oxidierbare Substanz entsprechend wenigstens zwei chemisch reversible Oxidationswellen besitzt.

Elektrochrome Vorrichtungen können auf vielfältige Weise Anwendung finden. So können sie z.B. als Automobilrückspiegel ausgebildet sein, der bei Nachtfahrt durch Anlegen einer Spannung abgedunkelt werden kann und somit das Blenden durch Scheinwerfer fremder Fahrzeuge verhindert wird (vgl. z.B. US-3.280.701, US-4.902.108, EP-A-0.435.689). Weiterhin können solche Vorrichtungen auch in Fensterscheiben oder Autosonnendächern eingesetzt werden, wo sie nach Anlegen einer Spannung das Sonnenlicht abdunkeln. Letztlich kann mit solchen Vorrichtungen auch eine Matrixanzeige aufgebaut werden zur bildlichen Darstellung von Informationen wie Buchstaben, Zahlen und Zeichen.

Elektrochrome Vorrichtungen bestehen normalerweise aus einem Paar Glas- oder Kunststoffscheiben, von denen im Falle eines Autospiegels eine verspiegelt ist. Eine Seite dieser Scheiben ist mit einer lichtdurchlässigen, elektrisch leitfähigen Schicht, z.B. Indium-Zinn-Oxid (ITO), beschichtet. Aus diesen Scheiben wird nun eine Zelle aufgebaut, indem sie mit ihrer einander zugewandten elektrisch leitfähig beschichteten Seite mit einem ringförmigen oder rechteckigen Dichtungsring verbunden, vorzugsweise verklebt werden. Der Dichtungsring stellt einen gleichmäßigen Abstand zwischen den Scheiben her, beispielsweise 0,1 bis 0,5 mm. In diese Zelle wird nun über eine Öffnung eine elektrochrome Lösung eingefüllt und die Zelle dicht verschlossen. Über die ITO-Schicht lassen sich die beiden Scheiben getrennt kontaktieren.

Bei den aus dem Stand der Technik bekannten elektrochromen Systemen sind solche Paare von Redoxsubstanzen enthalten, die nach Reduktion bzw. Oxidation farbige Radikale, Kationradikale oder Anionradikale bilden, die chemisch reaktiv sind. Wie beispielsweise aus Topics in Current Chemistry, Vol. 92, S. 1-44 (1980) bekannt ist, können solche Radikal(ionen) empfindlich gegenüber Elektrophilen oder Nukleophilen oder auch Radikalen sein. Es muß deshalb zum Erreichen einer hohen Stabilität einer elektrochromen Vorrichtung, die ein solches elektrochromes System enthält, das mehrere tausend Schaltcyclen überstehen soll, dafür gesorgt werden, daß das verwendete Lösungsmittel absolut frei von Elektrophilen, z.B. Protonen, Nukleophilen und Sauerstoff ist. Weiterhin muß dafür gesorgt werden, daß sich solche reaktiven Spezies nicht durch elektrochemische Prozesse an den Elektroden während des Betriebs der elektrochromen Vorrichtung bilden.

Die gemäß obiger Reaktionsgleichung formulierte Rückreaktion zu RED₁ und OX₂ erfolgt auch während des Betriebs der elektrochromen Vorrichtung kontinuierlich abseits der Elektroden innerhalb des Lösungsvolumens. Wegen der beschriebenen Gefahren von Abbaureaktionen der Radikal-(ionen) durch Elektrophile, Nukleophile oder Radikale ist es für die Langzeitstabilität des Displays wichtig, daß die Rückreaktion gemäß obiger Reaktionsgleichung möglichst rasch und ohne Nebenreaktionen erfolgen kann.

Es wurde gefunden, daß durch eine Kopplung von RED₁ und OX₂ über eine kovalente chemische Bindung die Elektronenübertragung erleichtert wird und somit die Rückreaktion gemäß obiger Reaktionsgleichung beschleunigt und Nebenreaktionen vermieden werden können.

Die vorliegende Erfindung betrifft demnach ein elektrochromes System, enthaltend mindestens eine oxidierbare Substanz RED₁, die durch Elektronenabgabe an einer Anode und mindestens eine reduzierbare Substanz OX₂, die durch Elektronenaufnahme an einer Kathode, jeweils unter Zunahme der Extinktion im sichtbaren Bereich des Spektrums von einer schwach gefärbten oder farblosen Form in eine gefärbte Form OX₁ bzw. RED₂ übergeht, wobei nach Ladungsausgleich jeweils die schwach gefärbte bzw. farblose Form zurückgebildet wird, dadurch gekennzeichnet, daß mindestens eine der enthaltenen Substanzen RED₁ und OX₂ über eine Brücke kovalent miteinander verknüpft sind.

Die Reduktions- und Oxidationsprozesse in dem erfindungsgemäßen elektrochromen System erfolgen im allgemeinen durch Elektronenaufnahme bzw. -abgabe an einer Kathode bzw. Anode, wobei zwischen den Elektroden vorzugsweise eine Potentialdifferenz von 0,3 bis 3 V herrscht. Nach Abschalten des elektrischen Potentials erfolgt im allgemeinen spontan ein Ladungsausgleich zwischen den Substanzen RED₂ und OX₁, wobei eine Entfärbung bzw. Farbaufhellung eintritt. Ein solcher Ladungsausgleich erfolgt auch bereits während des Stromflusses im Innern des Eiektrolytvolumens.

Das erfindungsgemäße elektrochrome System enthält bevorzugt mindestens eine elektrochrome Substanz der Formel (I) 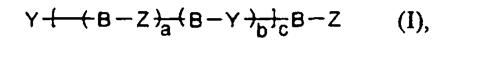 worin
- Y und Z: unabhängig voneinander für einen Rest OX₂ oder RED₁ stehen, wobei aber mindestens ein Y für OX₂ und mindestens ein Z für RED₁ steht,
wobei
OX₂ für den Rest eines reversibel elektrochemisch reduzierbaren Redoxsystems steht, und
RED₁ für den Rest eines reversibel elektrochemisch oxidierbaren Redoxsystems steht,
- B: für ein Brückenglied steht,
- c: für eine ganze Zahl von 0 bis 5 steht, und
- a und b: unabhängig voneinander für eine ganze Zahl von 0 bis 5 stehen, vorzugsweise für eine ganze Zahl von 0 bis 3 stehen.

Bevorzugt enthält das elektrochrome System mindestens eine elektrochrome Substanz der Formel (I), worin
Y für OX₂ und Z für RED₁ steht und Y und Z in ihrer Reihenfolge alternieren.

Insbesondere bevorzugt enthält das erfindungsgemäße elektrochrome System mindestens eine elektrochrome Substanz der Formeln

OX₂-B-RED₁ (Ia),

OX₂-B-RED₁-B-OX₂ (Ib),

RED₁-B-OX₂-B-RED₁ (Ic),

oder

OX₂-(B-RED₁-B-OX₂)_{d}-B-RED₁ (Id),

worin
- OX₂, RED₁ und B: die oben angegebene Bedeutung haben, und
- d: für eine ganze Zahl von 1 bis 5 steht.

Ganz besonders bevorzugt enthält das erfindungsgemäße elektrochrome System mindestens eine elektrochrome Substanz der Formeln (Ia)-(Id),
worin
- OX₂: für den Rest einer kathodisch reduzierbaren Substanz steht, die im cyclischen Voltammogramm, aufgenommen in einem inerten Lösungsmittel bei Raumtemperatur, wenigstens zwei chemisch reversible Reduktionswellen zeigt, wobei die erste dieser Reduktionswellen zu einer Zunahme der Extinktion bei wenigstens einer Wellenlänge im sichtbaren Bereich des elektromagnetischen Spektrums führt,
- RED₁: für den Rest der anodisch reversibel oxidierbaren Substanz steht, die im cyclischen Voltammogramm, aufgenommen in einem inerten Lösungsmittel bei Raumtemperatur, wenigstens zwei chemisch reversible Oxidationswellen zeigt, wobei die erste dieser Oxidationswellen zu einer Zunahme der Extinktion bei wenigstens einer Wellenlänge im sichtbaren Bereich des elektromagnetischen Spektrums führt, und
- B: für ein Brückenglied steht.

Besonders bevorzugt ist ein erfindungsgemäßes elektrochromes System, welches mindestens eine Substanz der Formel (Ia)-(Id) enthält, worin
- OX₂: für einen Rest der Formeln 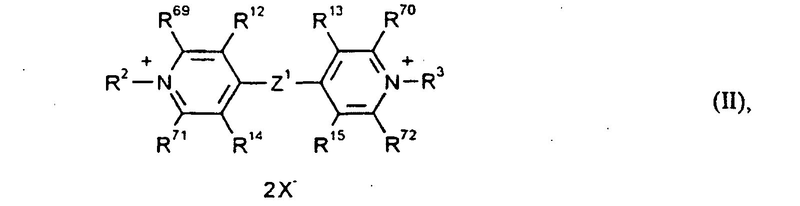 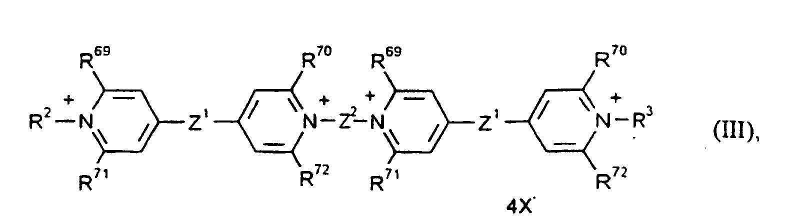 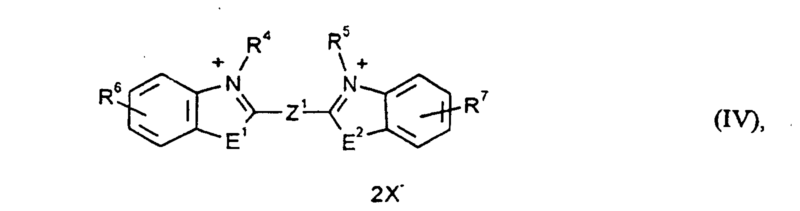 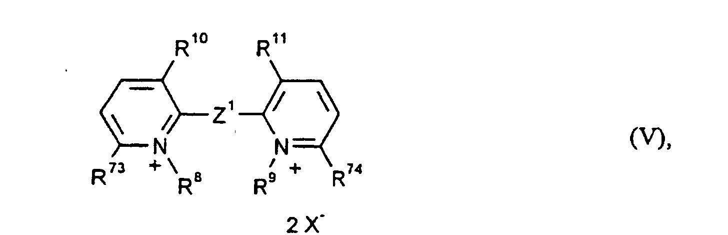 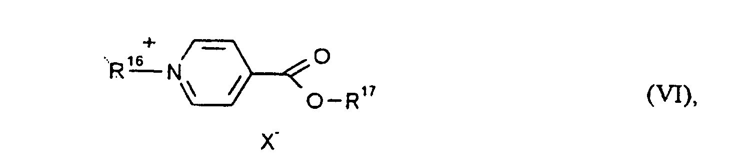 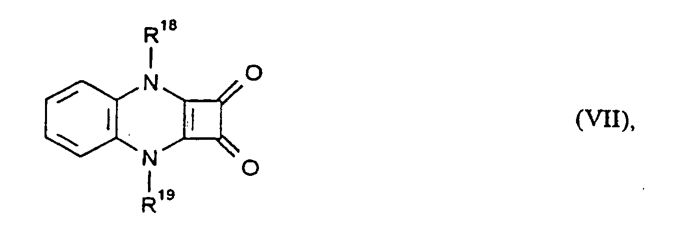 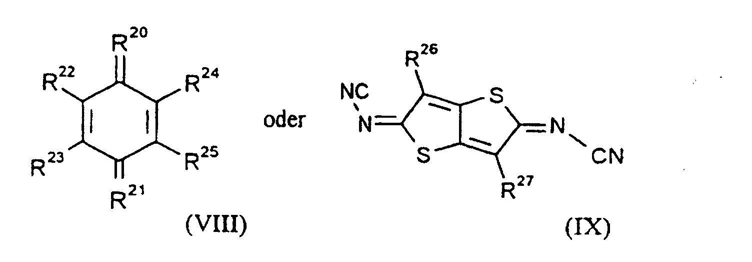
steht, wobei
- R² bis R⁵, R⁸, R⁹, R¹⁶ bis R¹⁹: unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten oder
- R⁴ und R⁵ oder R⁸ und R⁹: gemeinsam eine -(CH₂)₂- oder -(CH)₃-Brücke bilden,
- R⁶, R⁷ und R²² bis R²⁵: unabhängig voneinander Wasserstoff, C₁- bis C₄-Alkyl, C₁- bis C₄-Alkoxy, Halogen, Cyano, Nitro oder C₁- bis C₄-Alkoxycarbonyl bedeuten oder
- R²² und R²³ und/oder R²⁴ und R²⁵: eine -CH=CH-CH=CH-Brücke bilden,
- R¹⁰ und R¹¹; R¹² und R¹³; R¹⁴ und R¹⁵: unabhängig voneinander Wasserstoff oder paarweise eine -(CH₂)₂-, -(CH₂)₃- oder -CH=CH-Brücke bedeuten,
- R²⁰ und R²¹: unabhängig voneinander O, N-CN, C(CN)₂ oder N-C₆- bis C₁₀-Aryl bedeuten,
- R²⁶: Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeutet,
- R⁶⁹ bis R⁷⁴: unabhängig voneinander Wasserstoff oder C₁-C₆-Alkyl bedeuten, oder R⁶⁹; R¹² und/oder R⁷⁰; R¹³ eine -CH=CH-CH=CH-Brücke bilden,
- E¹ und E²: unabhängig voneinander O, S, NR¹ oder C(CH₃)₂ bedeuten oder
- E¹ und E²: gemeinsam eine -N-(CH₂)₂-N-Brücke bilden,
- R¹: C₁- bis C₁₈-Alkyl, C₂- bis C₁₂-Alkenyl, C₄- bis C₇-Cycloalkyl, C₇- bis C₁₅-Aralkyl, C₆- bis C₁₀-Aryl bedeutet,
- Z¹: eine direkte Bindung, -CH=CH-, -C(CH₃)=CH-, -C(CN)=CH-, -CCl=CCl-, -C(OH)=CH-, -CCl=CH-, -C≡C-, -CH=N-N=CH-, -C(CH₃)=N-N=C(CH₃)- oder -CCl=N-N=CCl- bedeutet,
- Z²: -(CH₂)ᵣ- oder -CH₂-C₆H₄-CH₂- bedeutet,
- r: eine ganze Zahl von 1 bis 10 bedeutet,
- X⁻: ein unter den Bedingungen redox-inertes Anion bedeutet,
wobei die Bindung zum Brückenglied B über einen der Reste R²-R¹⁹, R²²-R²⁷ oder im Falle, daß E¹ oder E² für NR¹ steht über R¹ erfolgt und die genannten Reste dann für eine direkte Bindung stehen,
- RED₁: für einen der folgenden Reste 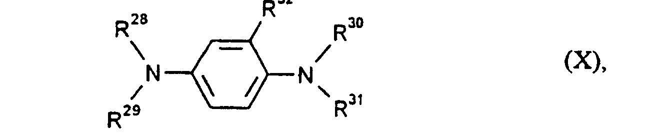 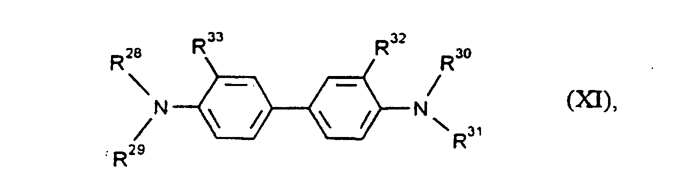 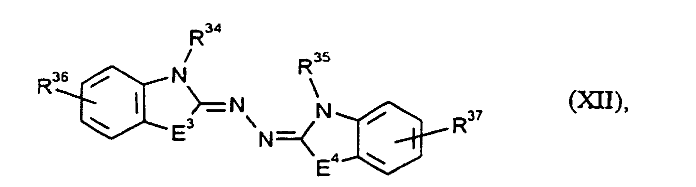 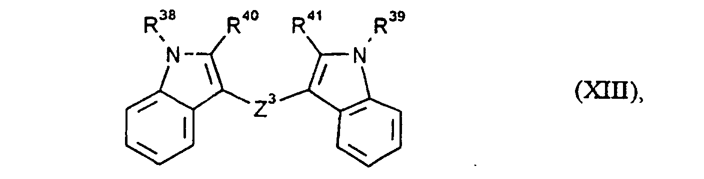 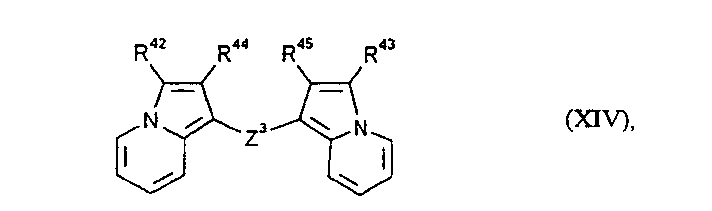 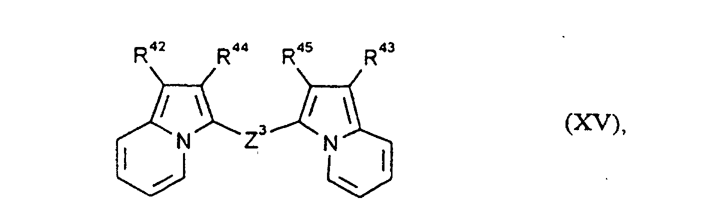 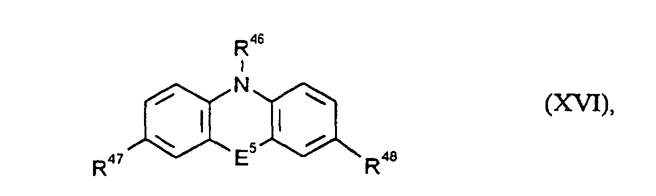 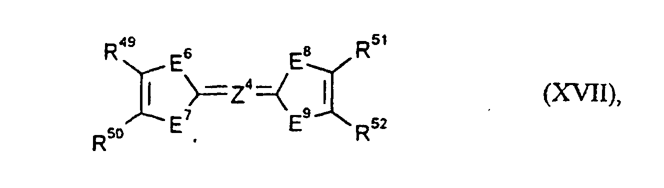 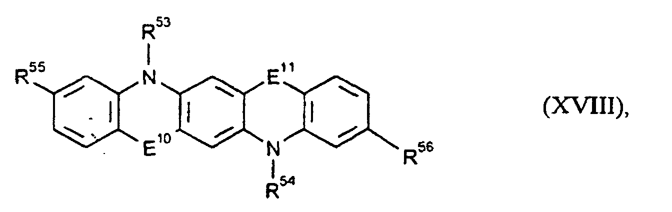 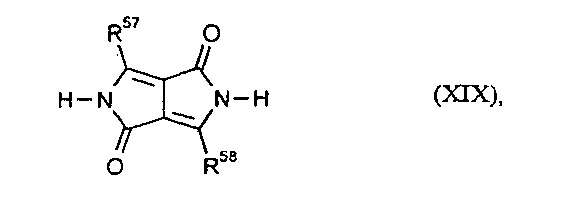 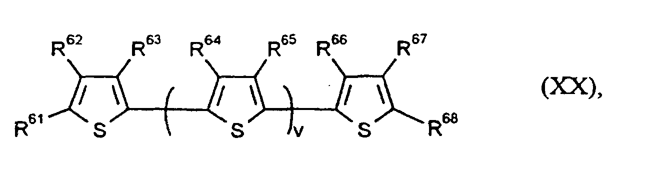
steht, worin
- R²⁸ bis R³¹, R³⁴, R³⁵, R³⁸, R³⁹, R⁴⁶, R⁵³ und R⁵⁴: unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten, und R⁴⁶, R⁵³ und R⁵⁴ zusätzlich Wasserstoff bedeuten,
- R³², R³³, R³⁶, R³⁷, R⁴⁰, R⁴¹, R⁴² bis R⁴⁵, R⁴⁷, R⁴⁸, R⁴⁹ bis R⁵² und R⁵⁵ bis R⁵⁷: unabhängig voneinander Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeuten und R⁵⁷ und R⁵⁸ zusätzlich einen gegebenenfalls benzanellierten aromatischen oder quasiaromatischen fünf- oder sechsgliedrigen heterocyclischen Ring bedeuten und R⁴⁸ zusätzlich NR⁷⁵R⁷⁶ bedeutet,
- R⁴⁹ und R⁵⁰ und/oder R⁵¹ und R⁵²: eine -(CH₂)₃-, -(CH₂)₄-, -(CH₂)₅- oder -CH=CH-CH=CH-Brücke bilden,
- Z³: eine direkte Bindung, eine -CH=CH- oder -N=N-Brücke bedeutet,
- =Z⁴=: eine direkte Doppelbindung, eine =CH-CH= oder =N-N=-Brücke bedeutet,
- E³ bis E⁵, E¹⁰ und E¹¹: unabhängig voneinander O, S, NR⁵⁹ oder C(CH₃)₂ bedeuten und E⁵ zusätzlich C = O oder SO₂ bedeutet, oder
- E³ und E⁴: unabhängig voneinander -CH=CH- bedeuten,
- E⁶ bis E⁹: unabhängig voneinander S, Se oder NR⁵⁹ bedeuten,
- R⁵⁹, R⁷⁵ und R⁷⁶: unabhängig voneinander C₁- bis C₁₂-Alkyl, C₂- bis C₈-Alkenyl, C₃- bis C₇-Cycloalkyl, C₇- bis C₁₅-Aralkyl oder C₆- bis C₁₀-Aryl bedeuten und R⁷³ zusätzlich Wasserstoff bedeutet, oder
- R⁷³ und R⁷⁴: in der Bedeutung von NR⁷³R⁷⁴ gemeinsam mit dem N-Atom, an das sie gebunden sind, einen fünf- oder sechsgliedrigen, gesättigten Ring bilden, der weitere Heteroatome enthalten kann,
- R⁶¹ bis R⁶⁸: unabhängig voneinander Wasserstoff, C₁- bis C₆-Alkyl, C₁- bis C₄-Alkoxy, Cyano, C₁- bis C₄-Alkoxycarbonyl oder C₆- bis C₁₀-Aryl bedeuten, oder
- R⁶¹; R⁶² und R⁶⁷; R⁶⁸: unabhängig voneinander gemeinsam eine -(CH₂)₃-, -(CH₂)₄- oder -CH=CH-CH=CH-Brücke bilden,
- v: eine ganze Zahl zwischen 0 und 10 bedeutet,
wobei die Bindung zum Brückenglied B über einen der Reste R²⁸-R⁵⁸, R⁶¹, R⁶², R⁶⁷, R⁶⁸ oder im Falle, daß einer der Reste E³-E¹¹ für NR⁵⁹ steht über R⁵⁹ erfolgt und die genannten Reste dann für eine direkte Bindung stehen, und
- B: für ein Brückenglied der Formeln -(CH₂)ₙ- oder -[Y¹ₛ(CH₂)ₘ-Y²]ₒ-(CH₂)ₚ-Y³_{q}- steht, welche jeweils gegebenenfalls durch C₁- bis C₄-Alkoxy, Halogen oder Phenyl substituiert sind,
- Y¹ bis Y³: unabhängig voneinander für O, S, NR⁶⁰, COO, CONH, NHCONH, Cyclopentandiyl, Cyclohexandiyl, Phenylen oder Naphthylen stehen,
- R⁶⁰: C₁- bis C₆-Alkyl, C₂- bis C₆-Alkenyl, C₄- bis C₇-Cycloalkyl, C₇- bis C₁₅-Aralkyl oder C₆- bis C₁₀-Aryl bedeutet,
- n: eine ganze Zahl von 1 bis 12 bedeutet,
- m und p: unabhängig voneinander eine ganze Zahl von 0 bis 8 bedeuten,
- o: eine ganze Zahl von 0 bis 6 bedeutet und
- q und s: unabhängig voneinander 0 oder 1 bedeuten.

Ganz besonders bevorzugt ist ein erfindungsgemäßes elektrochromes System, welches mindestens eine Substanz der Formel (Ia)-(Id) enthält,
worin
- OX₂: für einen Rest der Formeln (II), (III), (IV) oder (V) steht,
wobei
- R², R³, R⁴, R⁵, R⁸ und R⁹: unabhängig voneinander für C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl stehen,
- R⁶ und R⁷: unabhängig voneinander für Wasserstoff, Methyl, Ethyl, Methoxy, Ethoxy, Fluor, Chlor, Brom, Cyano, Nitro, Methoxycarbonyl oder Ethoxycarbonyl stehen,
- R¹⁰, R¹¹; R¹², R¹³ und R¹⁴, R¹⁵: unabhängig voneinander für Wasserstoff oder, falls Z¹ eine direkte Bindung bedeutet, jeweils gemeinsam für eine -(CH₂)₂-, -(CH₂)₃- oder -CH=CH-Brücke stehen,
oder
- R⁴, R⁵ und R⁸, R⁹: unabhängig voneinander paarweise gemeinsam für -(CH₂)₂- oder -(CH₃)₃-Brücke stehen, falls Z¹ eine direkte Bindung bedeutet,
- R⁶⁹ bis R⁷⁴: unabhängig voneinander Wasserstoff oder C₁-C₄-Alkyl bedeuten,
- E¹ und E²: gleich sind und für O, S, NR¹ oder C(CH₃)₂ stehen oder gemeinsam eine -N-(CH₂)₂-N-Brücke bilden,
- R¹: für C₁- bis C₁₂-Alkyl, C₂- bis C₄-Alkenyl, C₅- bis C₇-Cycloalkyl, C₇- bis C₁₅-Aralkyl oder C₆- bis C₁₀-Aryl steht,
- Z¹: für eine direkte Bindung, -CH=CH-, -C(CH₃)=CH-, -C(CN)=CH-, -C≡C- oder -CH=N-N=CH- steht,
- Z²: für -(CH)ᵣ- oder -CH₂-C₆H₄-CH₂- steht,
- r: für eine ganze Zahl zwischen 1 und 6 steht,
- X⁻: für ein unter den Bedingungen redox-inertes, farbloses Anion steht,
wobei die Bindung zum Brückenglied B über einen der Reste R²-R¹¹ oder im Falle, daß E¹ oder E² für NR¹ steht über R¹ erfolgt und die genannten Reste dann für eine direkte Bindung stehen,
- RED₁: für einen Rest der Formeln (X), (XI), (XII), (XIII), (XVI), (XVII), (XVIII) oder (XX) steht,
wobei
- R²⁸ bis R³¹, R³⁴, R³⁵, R³⁸, R³⁹, R⁴⁶, R⁵³ und R⁵⁴: unabhängig voneinander C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl bedeuten und
- R⁴⁶, R⁵³ und R⁵⁴: zusätzlich Wasserstoff bedeuten,
- R³², R³³, R³⁶, R³⁷, R⁴⁰, R⁴¹, R⁴⁷ bis R⁵², R⁵⁵ und R⁵⁶: unabhängig voneinander Wasserstoff, Methyl, Ethyl, Methoxy, Ethoxy, Fluor, Chlor, Brom, Cyan, Nitro, Methoxycarbonyl, Ethoxycarbonyl oder Phenyl bedeuten und
- R⁵⁷ und R⁵⁸: zusätzlich 2- oder 4-Pyridyl bedeuten und
- R⁴⁸: zusätzlich NR⁷⁵R⁷⁶ bedeutet,
- Z³: eine direkte Bindung, eine -CH=CH- oder -N=N-Brücke bedeutet,
- =Z⁴=: eine direkte Doppelbindung, eine =CH-CH= oder =N-N=-Brücke bedeutet,
- E³ bis E⁵, E¹⁰ und E¹¹: unabhängig voneinander O, S, NR⁵⁹ oder C(CH₃)₂ bedeuten, E³ und E⁴ aber die gleiche Bedeutung haben,
- E⁶ bis E⁹: untereinander gleich sind und S, Se oder NR⁵⁹ bedeuten und
- E⁵: zusätzlich C = O bedeutet,
- E⁶: für NR⁵⁹ steht, wobei R⁵⁹ eine direkte Bindung zur Brücke B bedeutet und
- E⁷ bis E⁹: die oben angegebene Bedeutung besitzen, aber untereinander nicht gleich sein müssen,
- R⁵⁹, R⁷⁵ und R⁷⁶: unabhängig voneinander C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl bedeuten, und R⁷³ zusätzlich Wasserstoff bedeutet oder
- R⁷³ und R⁷⁴: in der Bedeutung NR⁷³R⁷⁴ gemeinsam mit dem N-Atom, an das sie gebunden sind, Pyrrolidino, Piperidino oder Morpholino bedeuten,
- R⁶¹, R⁶² und R⁶⁷, R⁶⁸: unabhängig voneinander für Wasserstoff, C₁- bis C₄-Alkyl, Methoxycarbonyl, Ethoxycarbonyl oder Phenyl oder paarweise gemeinsam für eine -(CH₂)₃- oder -(CH₂)₄-Brücke stehen,
- R⁶³ bis R⁶⁶: für Wasserstoff stehen und
- v: für eine ganze Zahl von 1 bis 6 steht,
wobei die Bindung zum Brückenglied B über einen der Reste R²⁸-R⁴¹, R⁴⁶-R⁵⁶, R⁶¹, R⁶², R⁶⁷, R⁶⁸ oder im Falle, daß einer der Reste E³-E¹¹ für NR⁵⁹ steht, über R⁵⁹ erfolgt und die genannten Reste dann für eine direkte Bindung stehen,
- B: für ein Brückenglied der Formeln -(CH₂)ₙ-, -(CH₂)ₘ-O-(CH₂)ₚ-, -(CH)ₘ-NR⁶⁰-(CH₂)ₚ-, -(CH₂)ₘ-C₆H₄-(CH₂)ₚ-, -[O-(CH₂)ₚ]ₒ-O-, -[NR⁶⁰-(CH₂)ₚ]ₒ-NR ⁶⁰-, -[C₆H₄-(CH₂)ₚ]ₒ-C₆H₄-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)-, -(CH₂)ₘ-NHCO-(CH₂)ₜ-CONH-(CH)ₚ-, -(CH₂)ₘ-NHCONH-(CH₂)ₜ-NHCONH-(CH₂)ₚ- steht,
- R⁶⁰: für Methyl, Ethyl, Benzyl oder Phenyl steht,
- n: für eine ganze Zahl von 1 bis 10 steht,
- m und p: unabhängig voneinander für eine ganze Zahl von 0 bis 4 stehen,
- o: für eine ganze Zahl von 0 bis 2 steht und
- t: für eine ganze Zahl von 1 bis 6 steht.

Insbesondere bevorzugt ist ein erfindungsgemäßes elektrochromes System, welches mindestens eine Substanz der Formel (Ia)-(Id) enthält,
worin
- OX₂: für einen Rest der Formeln (II), (IV) oder (V) steht,
worin
- R², R⁴ und R⁸: für eine direkte Bindung zum Brückenglied B stehen,
- R³, R⁵ und R⁹: unabhängig voneinander für Methyl, Ethyl, Propyl, Butyl, Pentyl, Hexyl, Heptyl, Octyl, Benzyl oder Phenyl stehen, oder im Falle der Formeln Ic oder Id ebenfalls für eine direkte Bindung zum Brückenglied B stehen,
- R⁶ und R⁷: gleich sind und für Wasserstoff, Methyl, Methoxy, Chlor, Cyano oder Methoxycarbonyl stehen,
- R¹⁰, R¹¹; R¹², R¹³ und R¹⁴, R¹⁵: unabhängig voneinander für Wasserstoff oder, falls Z¹ eine direkte Bindung bedeutet, jeweils paarweise zusammen für eine -CH=CH-Brücke stehen,
- R⁶⁹ bis R⁷²: gleich sind und Wasserstoff, Methyl oder Ethyl bedeuten,
- R⁷³ und R⁷⁴: Wasserstoff bedeuten,
- E¹ und E²: gleich sind und für O oder S stehen,
- Z¹: für eine direkte Bindung oder -CH=CH- steht,
- X⁻: für ein unter den Bedingungen redox-inertes, farbloses Anion steht,
- RED₁: für einen Rest der Formeln (X), (XII), (XIII), (XVI) oder (XVII) steht,
- R²⁸, R³⁴, R³⁸, R⁴⁶ und R⁴⁹: für eine direkte Bindung zum Brückenglied B stehen,
- R²⁹ bis R³¹, R³⁵ und R³⁹: unabhängig voneinander für Methyl, Ethyl, Propyl, Butyl, Pentyl, Hexyl, Heptyl, Octyl, Benzyl oder Phenyl stehen, oder im Falle der Formeln Ib oder Id R³⁰, R³⁵ und R³⁹ ebenfalls für die direkte Bindung zum Brückenglied B stehen,
- R³², R⁴⁷ und R⁴⁸: für Wasserstoff stehen,
- R³⁶, R³⁷, R⁴⁰, R⁴¹ und R⁵⁰ bis R⁵²: unabhängig voneinander für Wasserstoff, Methyl, Methoxy, Chlor, Cyan, Methoxycarbonyl oder Phenyl stehen, oder im Falle der Formeln Ib oder Id R⁵¹ ebenfalls für eine direkte Bindung zum Brückenglied B steht,
- Z³: für eine direkte Bindung, eine -CH=CH- oder -N=N-Brücke steht,
- =Z⁴=: für eine direkte Doppelbindung, eine =CH-CH= oder =N-N=Brücke steht,
- E³ bis E⁵: unabhängig voneinander für O, S oder NR⁵⁹ stehen, E³ und E⁴ aber die gleiche Bedeutung haben,
- E⁶ bis E⁹: untereinander gleich sind und für S, Se oder NR⁵⁹ stehen,
- R⁵⁹: für Methyl, Ethyl, Propyl, Butyl, Pentyl, Hexyl, Heptyl, Octyl, Benzyl oder Phenyl steht, oder im Falle der Formel XVI in Ib oder Id ebenfalls für eine direkte Bindung zum Brückenglied B steht,
- B: für ein Brückenglied der Formeln -(CH₂)ₙ-, -(CH₂)ₘ-O-(CH₂)ₚ-, -(CH₂)ₘ-NR⁶⁰-(CH₂)ₚ-, -(CH₂)ₘ-C₆H₄-(CH₂)ₚ-, -O-(CH₂)ₚ-O-, -NR⁶⁰-(CH₂)ₚ-NR⁶⁰-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)ₚ-,-(CH₂)ₘ-NHCO-(CH₂)ₜ-CONH-(CH₂)ₚ-, -(CH₂)ₘ-NHCONH-(CH₂)ₜ-NHCONH-(CH₂)ₚ- steht,
- R⁶⁰: für Methyl steht,
- n: für eine ganze Zahl von 1 bis 10 steht,
- m und p: gleich sind und für eine ganze Zahl von 0 bis 2 stehen und
- t: für eine ganze Zahl von 1 bis 6 steht.

Ganz besonders bevorzugt ist ein erfindungsgemäßes elektrochromes System, welches mindestens eine Substanz der Formel (Ia) entsprechend einer der Formeln 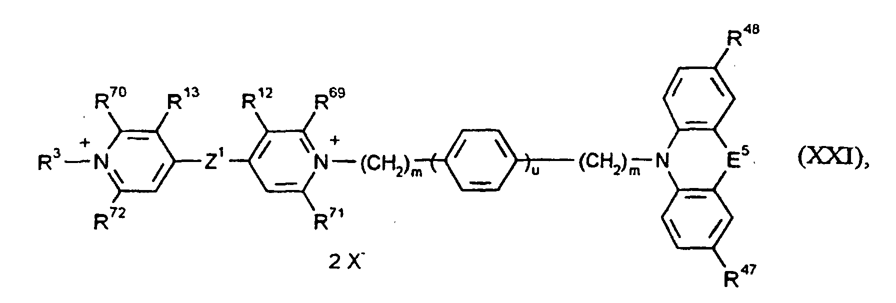 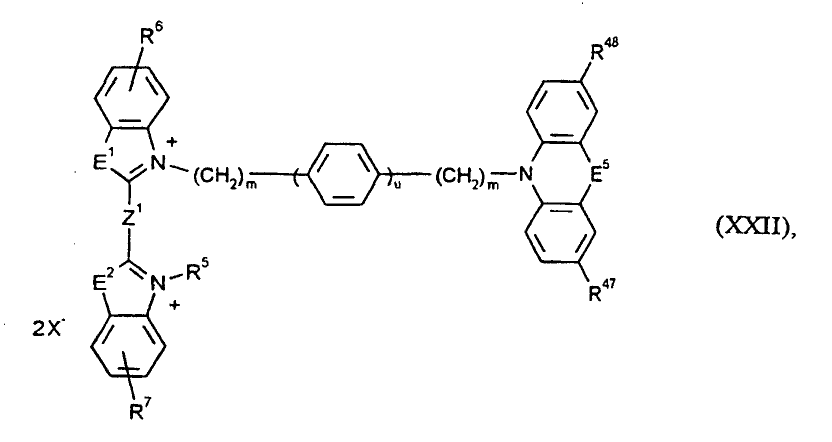 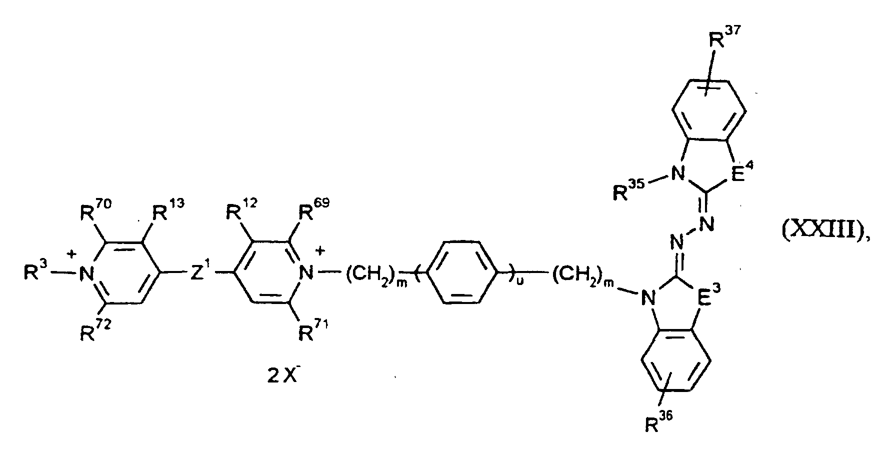 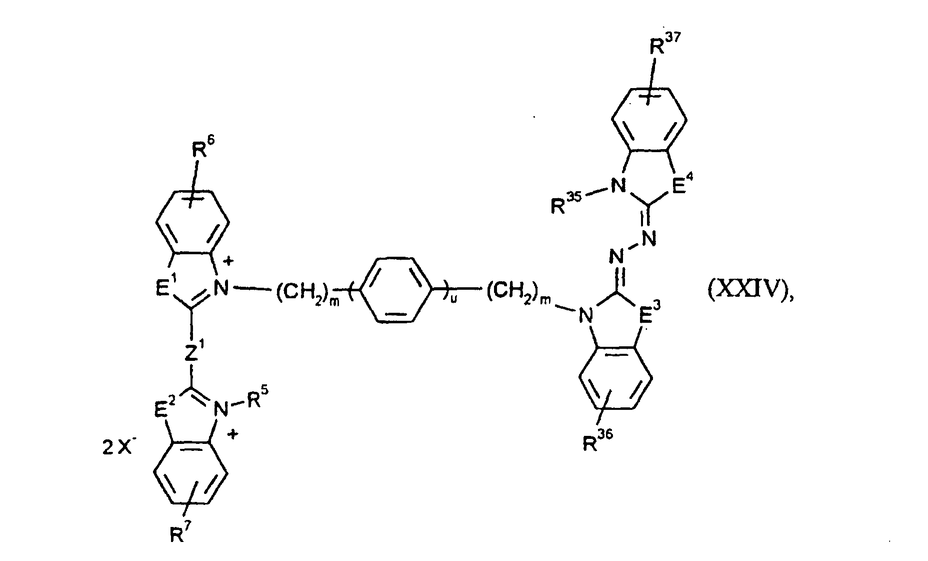 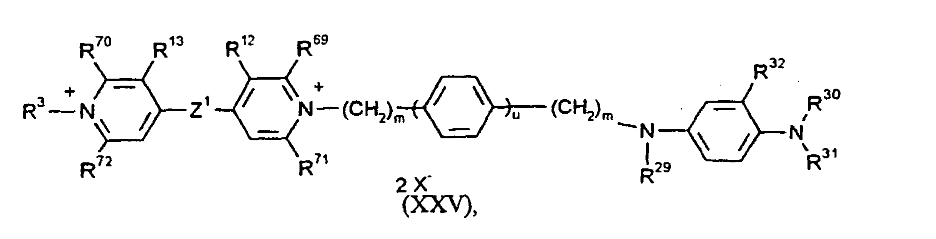 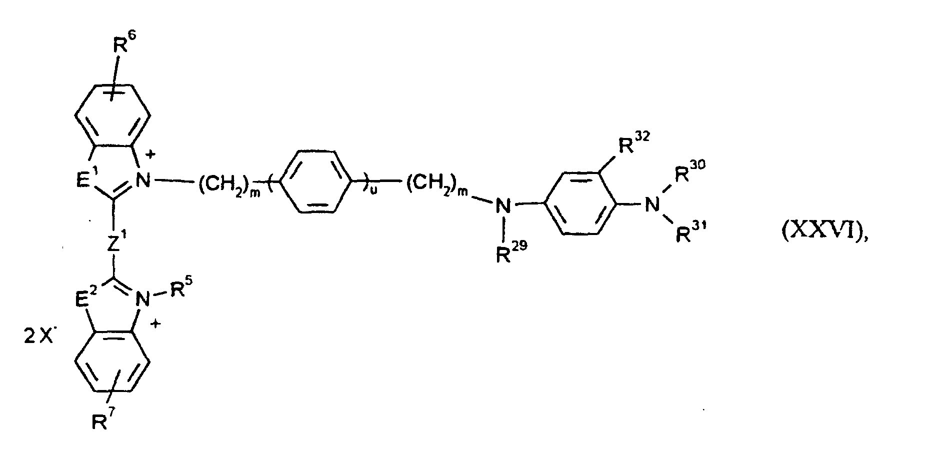 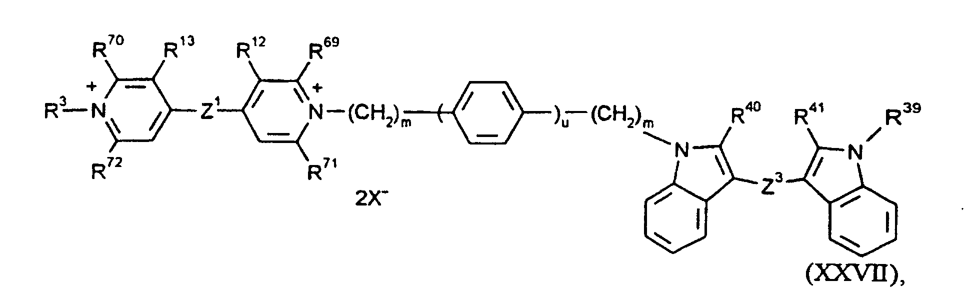 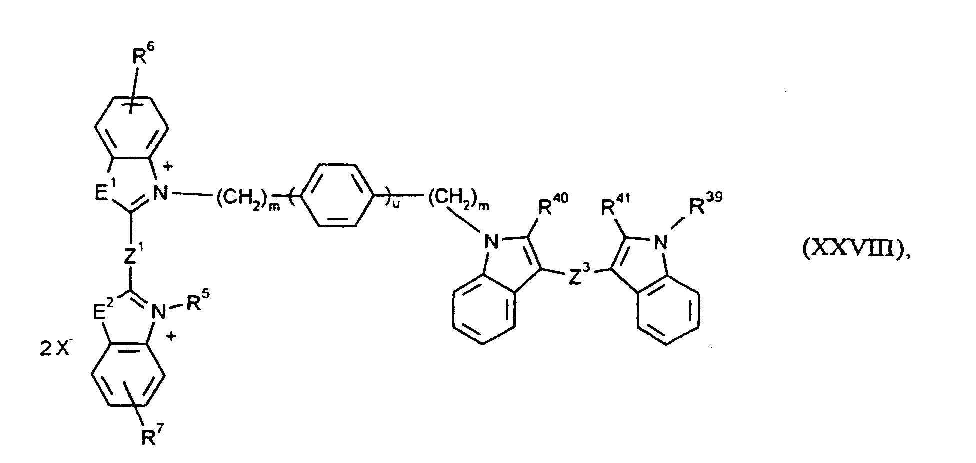 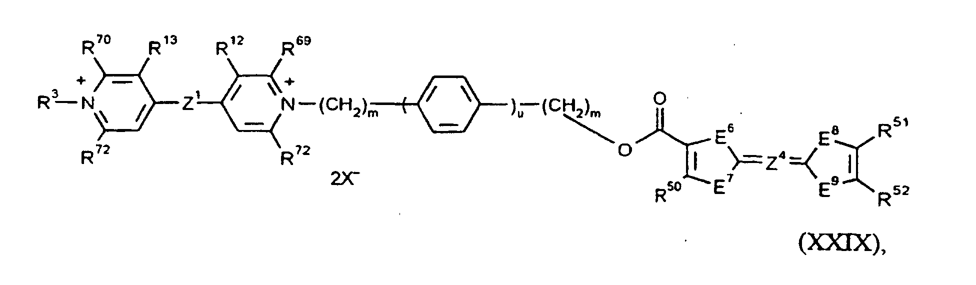 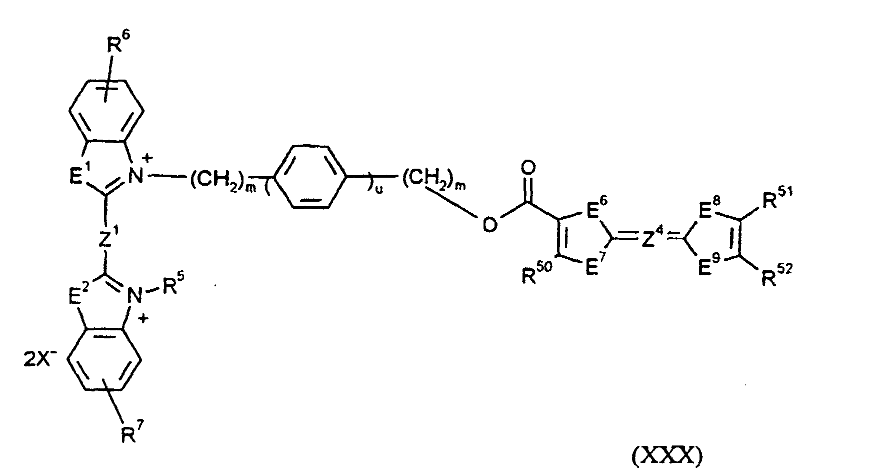 enthält, oder mindestens eine Substanz der Formel (Ib) entsprechend einer der Formeln 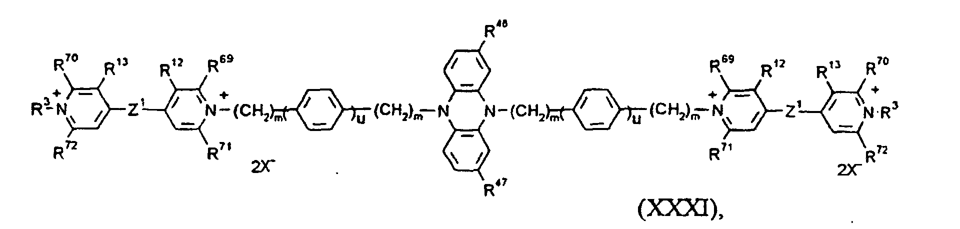 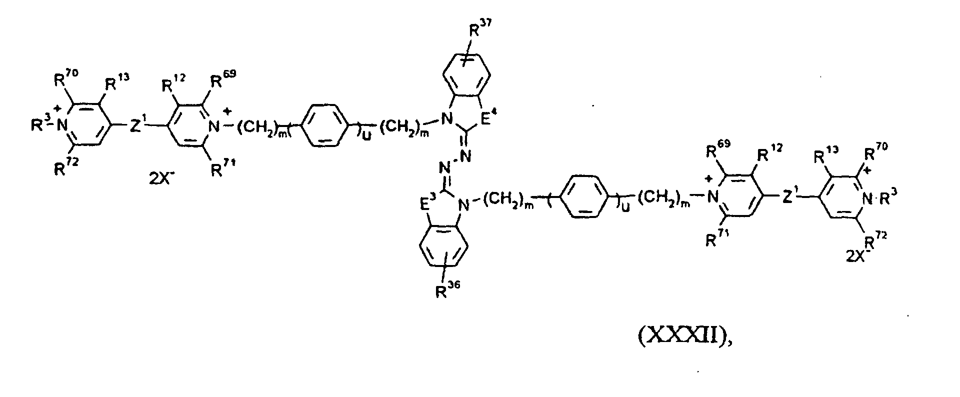 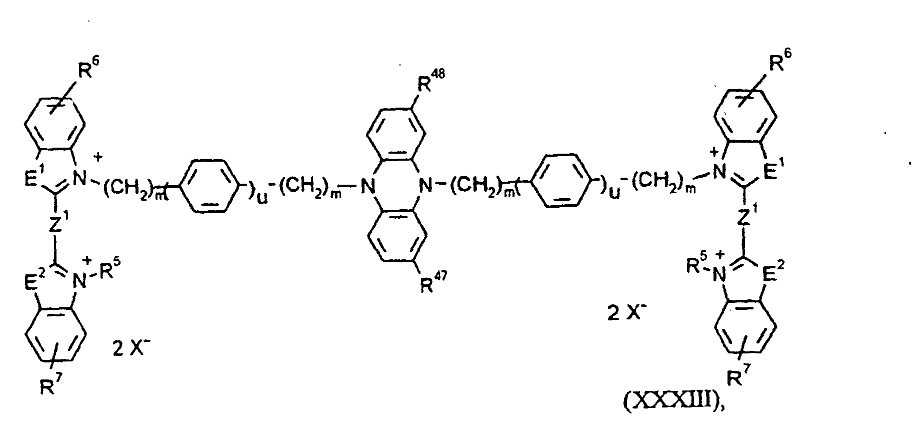 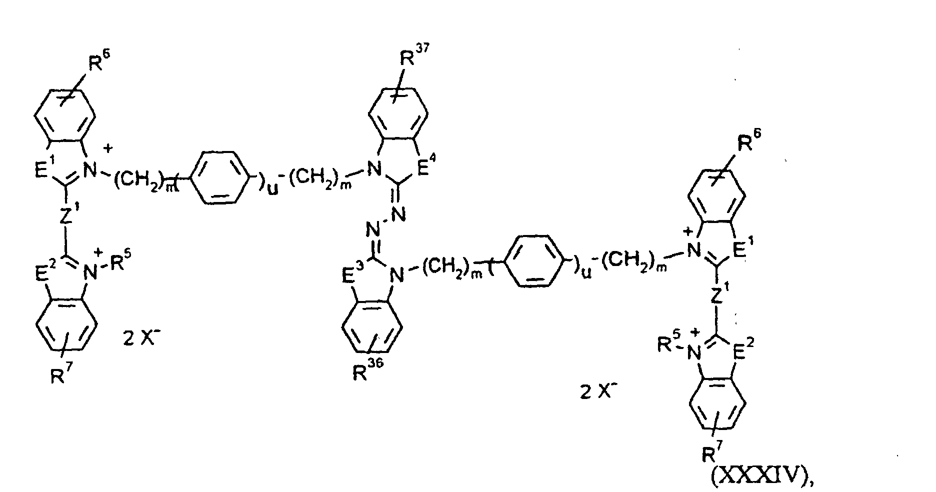 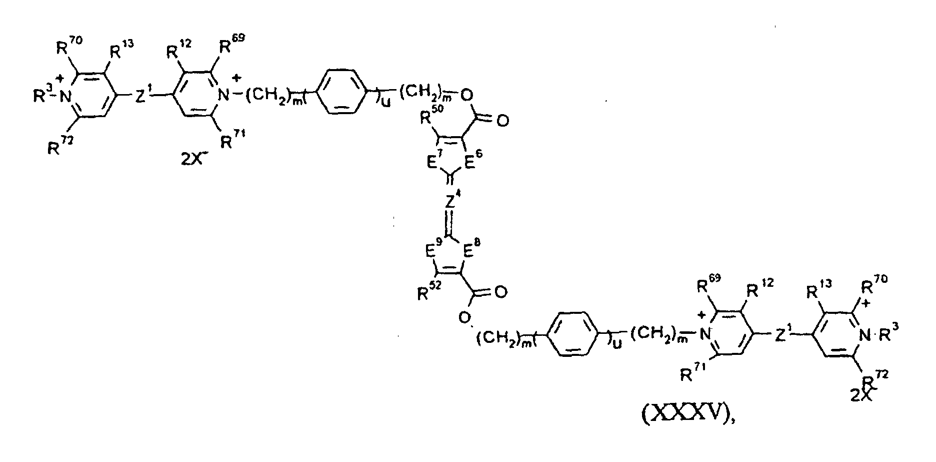 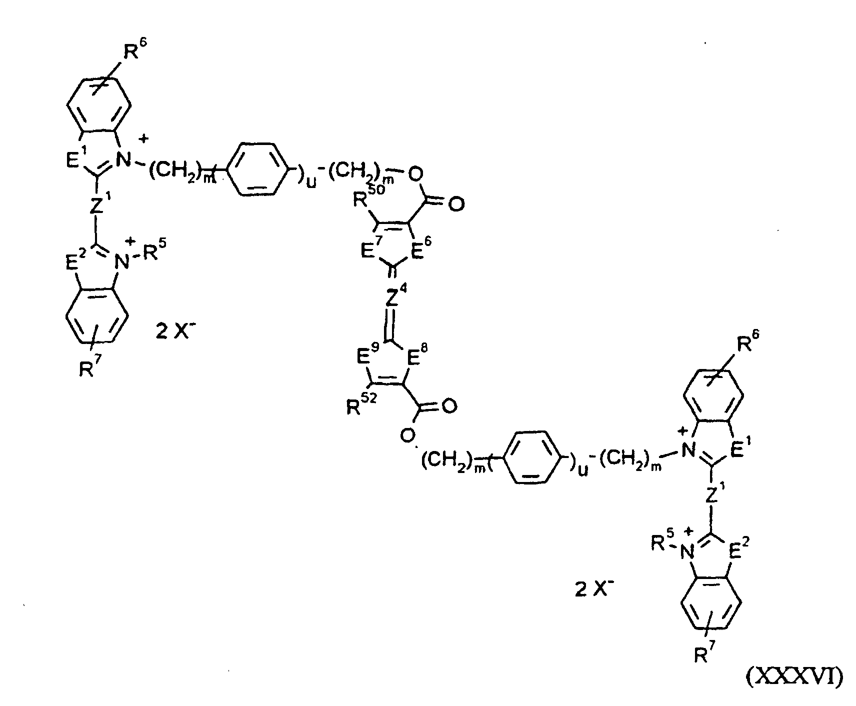 oder mindestens eine Substanz der Formel (Ic) entsprechend einer der Formeln 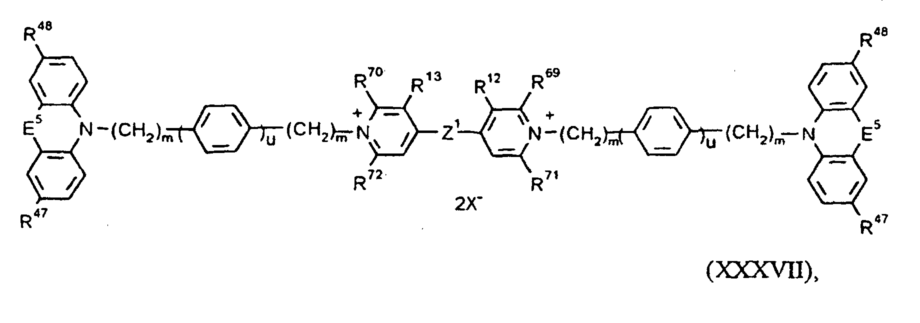 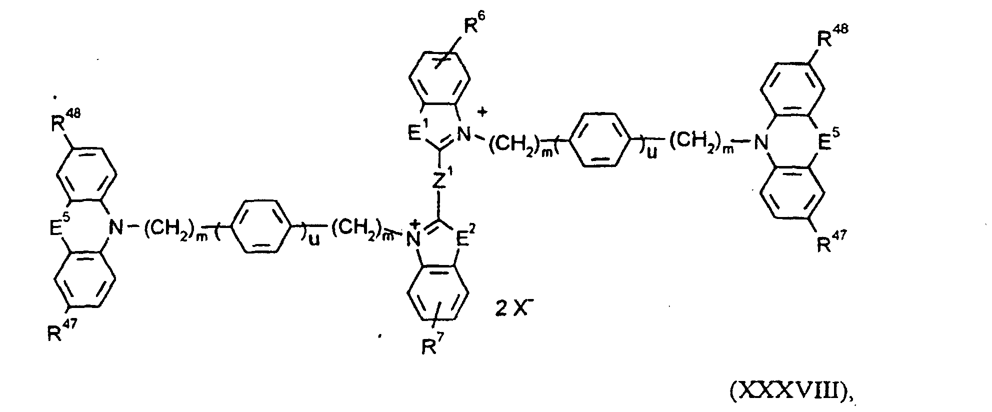 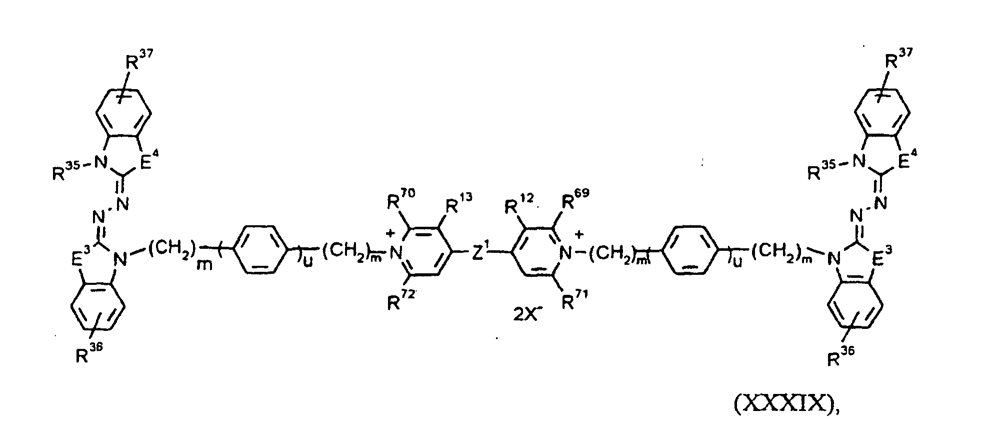 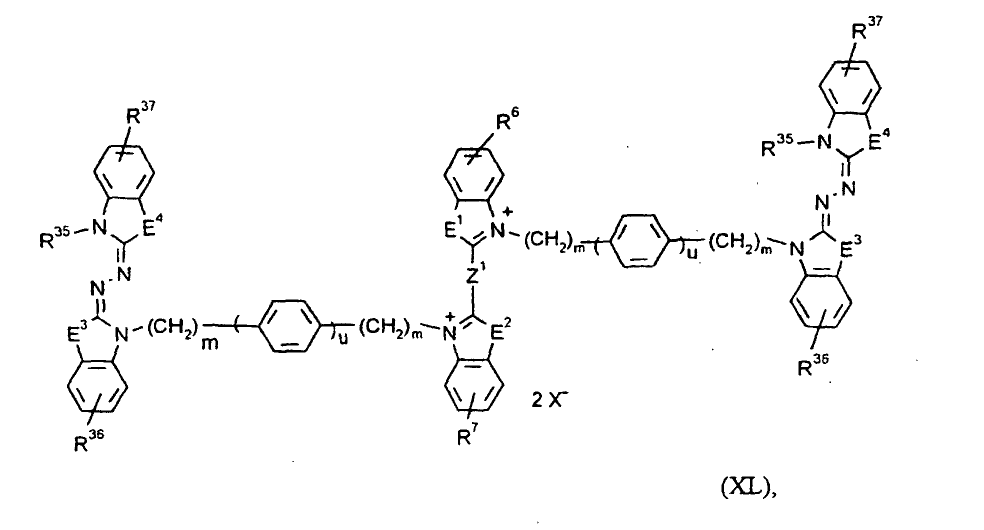 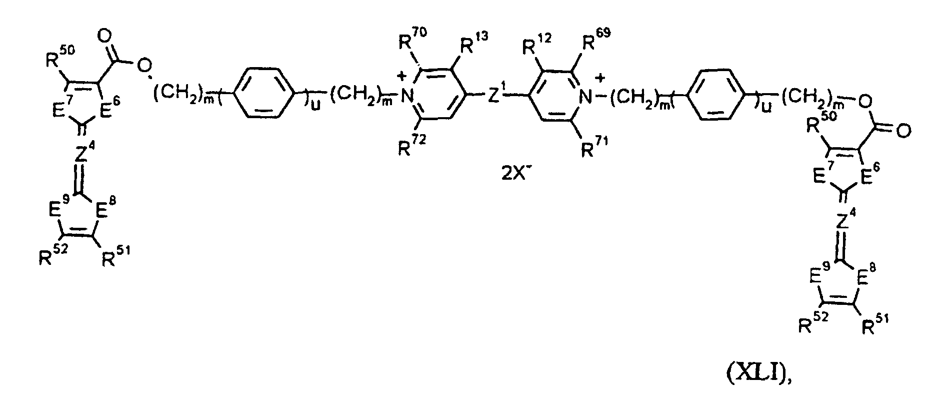 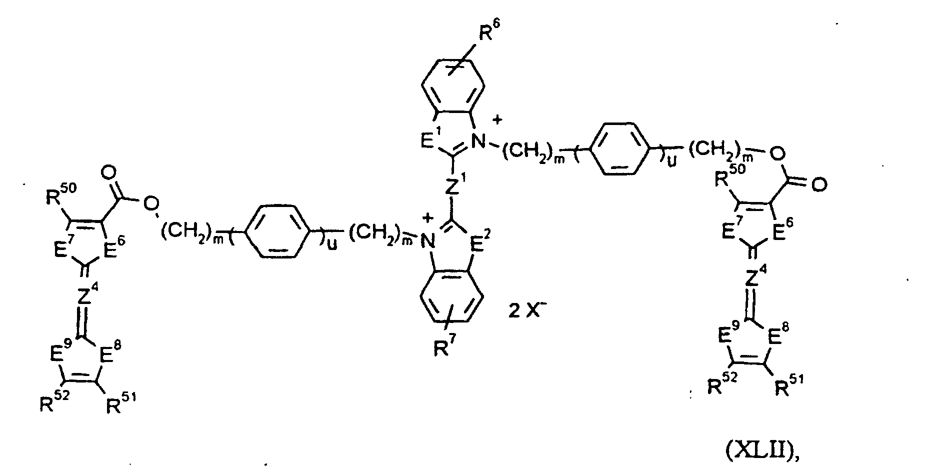 worin
- R³, R⁵, R³⁵ und R³⁹: unabhängig voneinander für Methyl, Ethyl, Propyl, Butyl, Pentyl, Hexyl oder Benzyl stehen,
- R⁶, R⁷ und R³⁶, R³⁷: paarweise gleich sind und für Wasserstoff, Methyl, Methoxy, Chlor, Cyano oder Methoxycarbonyl stehen,
- R¹² und R¹³: für Wasserstoff oder, wenn Z¹ eine direkte Bindung bedeutet, gemeinsam für eine CH=CH-Brücke stehen,
- R⁶⁹ bis R⁷²: gleich sind und für Wasserstoff oder Methyl stehen,
- E¹ und R²: gleich sind und für O oder S stehen,

- Z¹: für eine direkte Bindung oder -CH=CH- steht,
- R³², R⁴⁷ und R⁴⁸: für Wasserstoff stehen,
- E³ bis E⁵: unabhängig voneinander für O, S oder NR⁵⁹ stehen, wobei E³ und E⁴ aber gleich sind,
- R²⁹ bis R³¹ und R⁵⁹: unabhängig voneinander für Methyl, Ethyl, Propyl, Butyl, Pentyl, Hexyl oder Benzyl stehen, wobei R²⁹ bis R³¹ vorzugsweise gleich sind,
- R⁴⁰ und R⁴¹: gleich sind und für Wasserstoff, Methyl, Ethyl, Propyl, Butyl oder Phenyl stehen.
- Z³: für eine direkte Bindung, -CH=CH- oder -N=N- steht,
- R⁵⁰ bis R⁵²: unabhängig voneinander für Wasserstoff, Methyl, Methoxy, Chlor, Cyano, Methoxycarbonyl, Ethoxycarbonyl oder Phenyl stehen, vorzugsweise jedoch gleich sind, E⁶ bis E⁹ untereinander gleich sind und für S, Se oder NR⁵⁹ stehen,
- Z⁴: für eine direkte Doppelbindung, eine =CH-CH= oder =N-N=-Brücke steht,
- m: für eine ganze Zahl von 1 bis 5 steht,
- u: für 0 oder 1 steht und
- X⁻: für ein unter den Bedingungen redox-inertes, farbloses Anion steht.
Elektrochrome Substanzen der Formel (I) 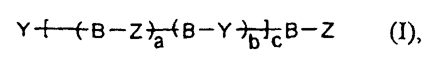 worin
- Y und Z: unabhängig voneinander für einen Rest OX₂ oder RED₁ stehen, wobei aber mindestens ein Y für OX₂ und mindestens ein Z für RED₁ steht,
wobei
OX₂ für den Rest eines reversibel elektrochemisch reduzierbaren Redoxsystems steht, und
RED₁ für den Rest eines reversibel elektrochemisch oxidierbaren Redoxsystems steht, aber nicht für einen Rest der Formel (XX),
- B: für ein Brückenglied steht,
- c: für eine ganze Zahl von 0 bis 5 steht, und
- a und b: unabhängig voneinander für eine ganze Zahl von 0 bis 5, vorzugsweise für eine ganze Zahl von 0-3, stehen,
mit Ausnahme von Verbindungen der Formel 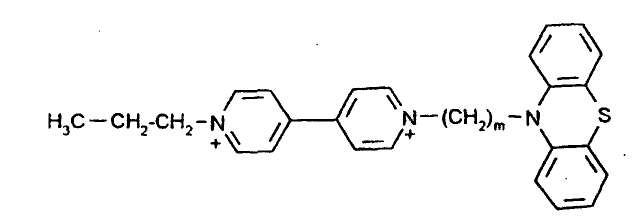 worin
- m: für eine ganze Zahl von 2 bis 16 steht, und
- X⁻: die oben angegebene Bedeutung hat,
sind neu und ebenfalls Gegenstand der vorliegenden Erfindung.

Von den elektrochromen Substanzen der Formel (I) sind diejenigen bevorzugt, welche den Formeln (Ia) bis (Id)

OX₂-B-RED₁ (Ia),

OX₂-B-RED₁-B-OX₂ (Ib),

RED₁-B-OX₂-B-RED₁ (Ic),

OX₂-(B-RED₁-B-OX₂)_{d}-B-RED₁ (Id),

worin OX₂, RED₁ und B die oben angegebenen allgemeinen und bevorzugten Bedeutungen haben und
- d: für eine ganze Zahl von 1 bis 5 steht,
entsprechen.

Insbesondere bevorzugt sind elektrochrome Substanzen der Formel (Ia) entsprechend den Formeln 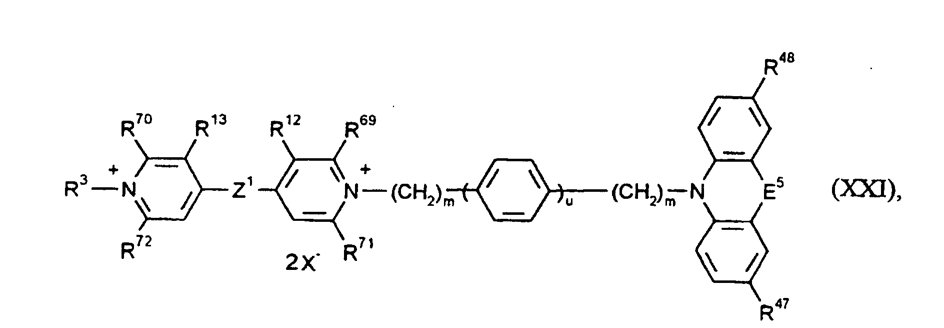 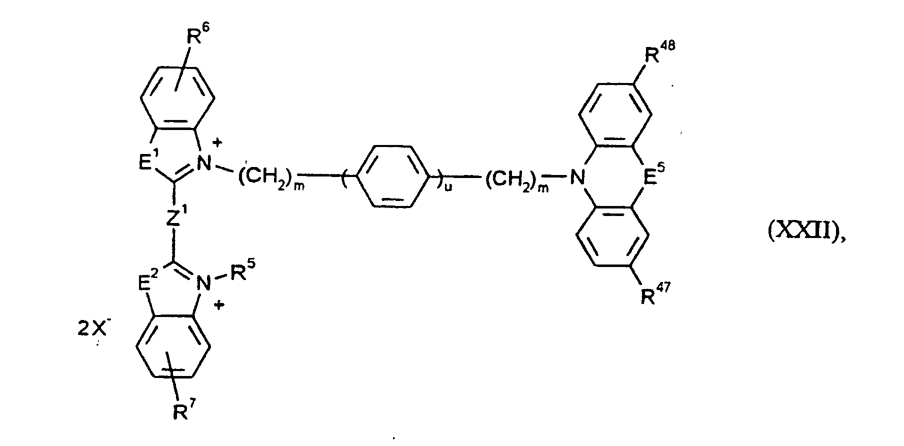 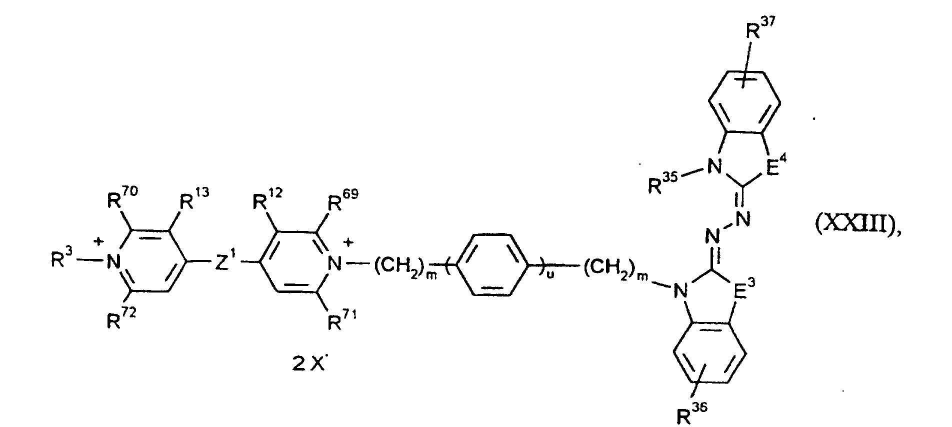 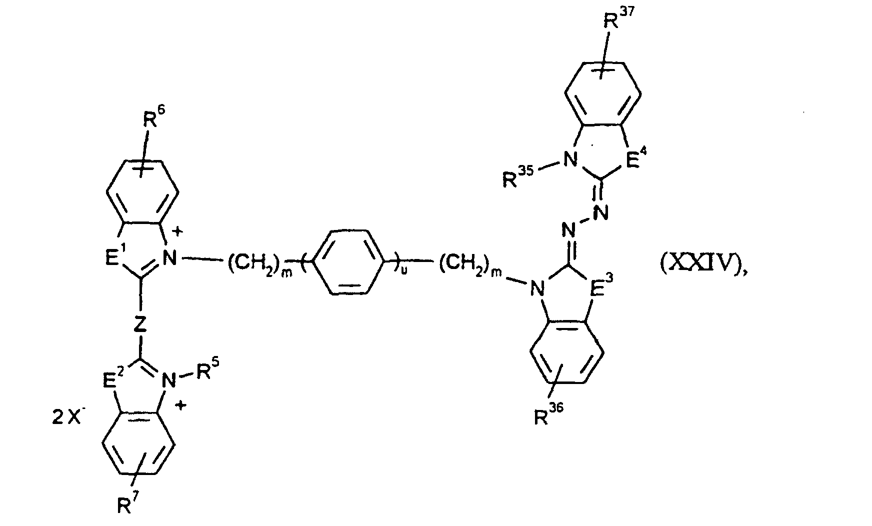 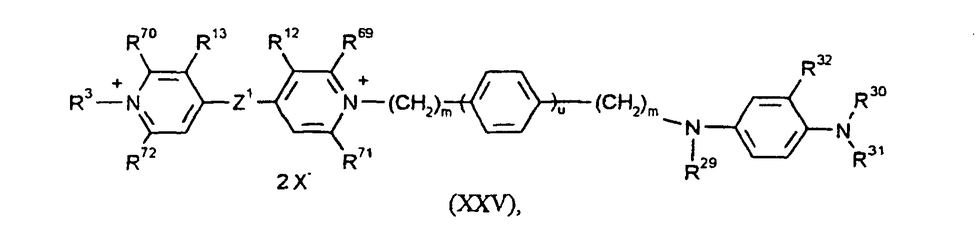 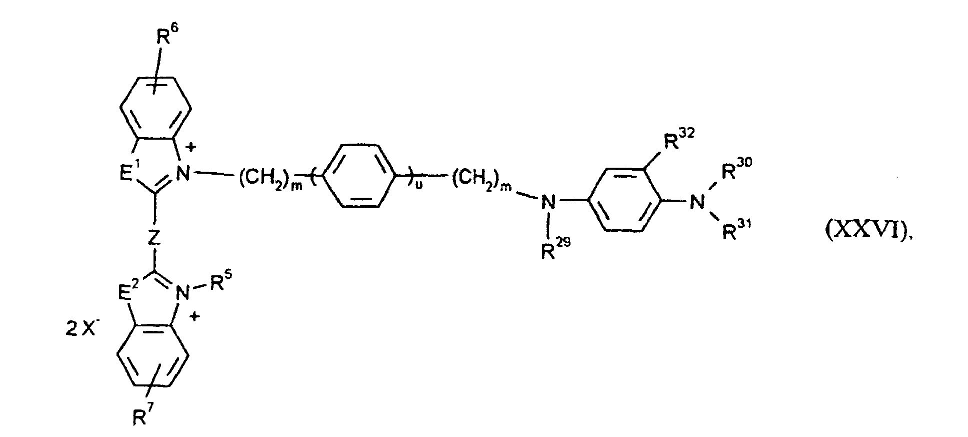 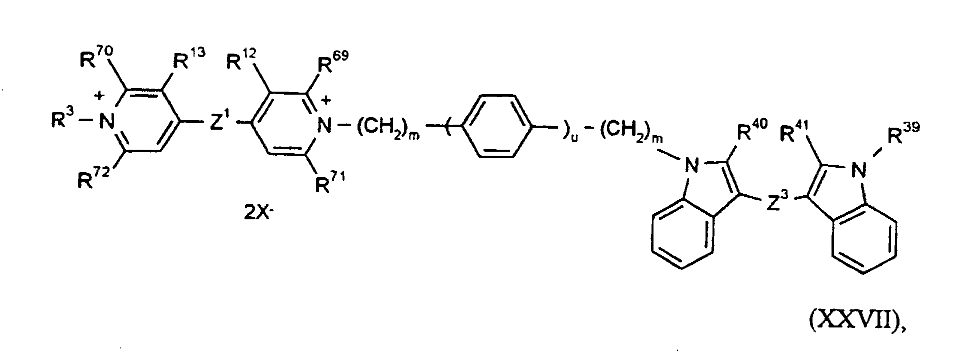 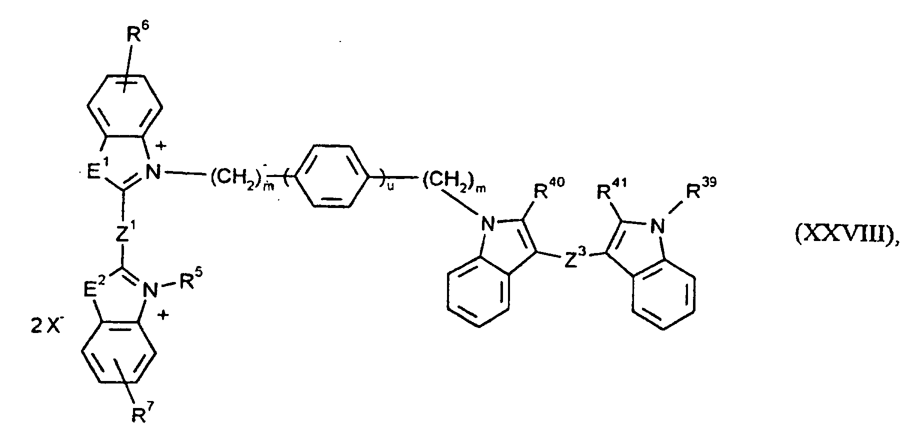 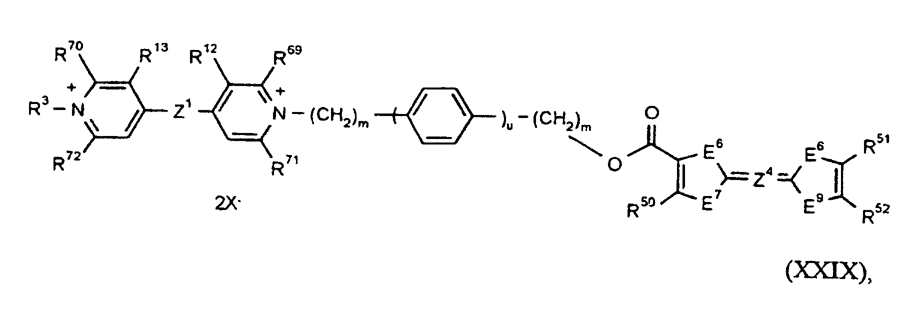 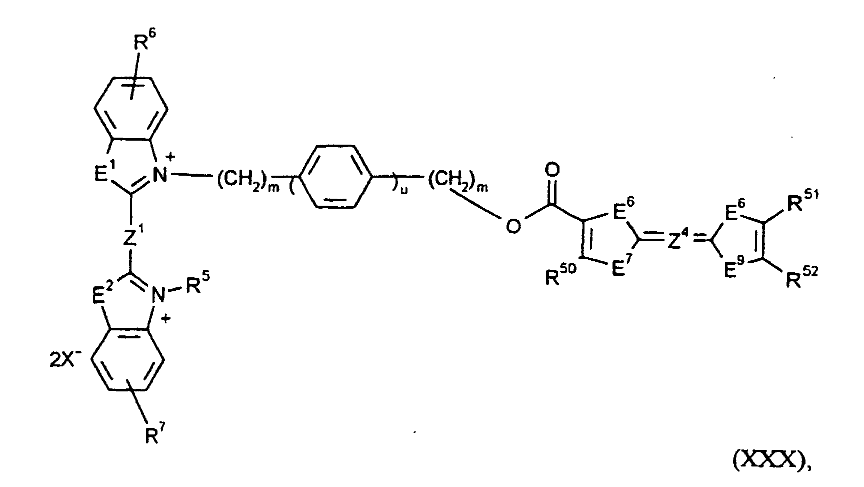 oder elektrochrome Substanzen der Formel (1b) 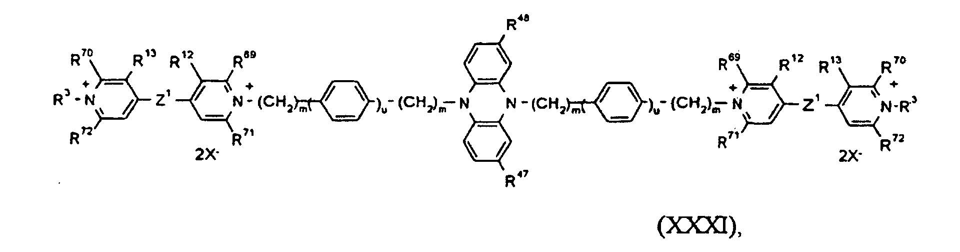 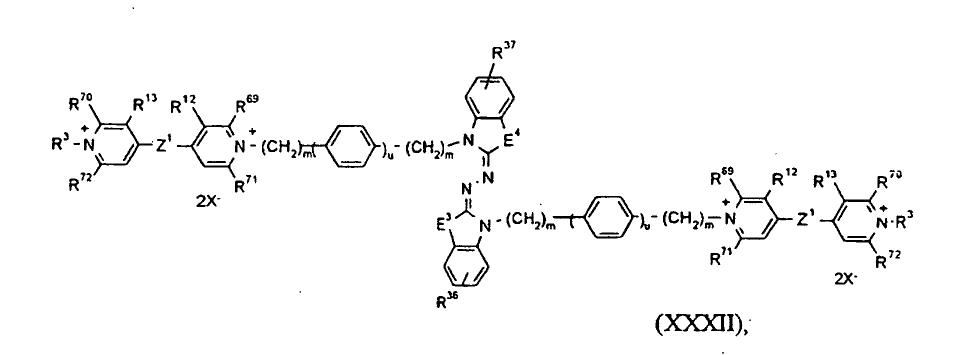 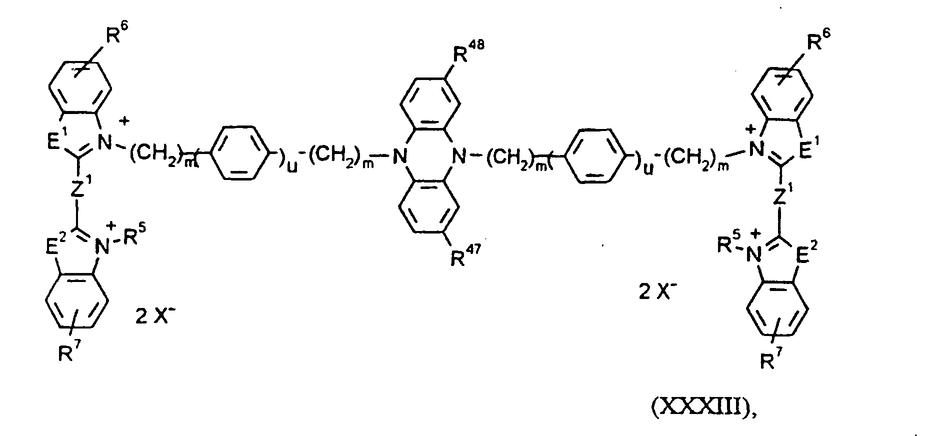 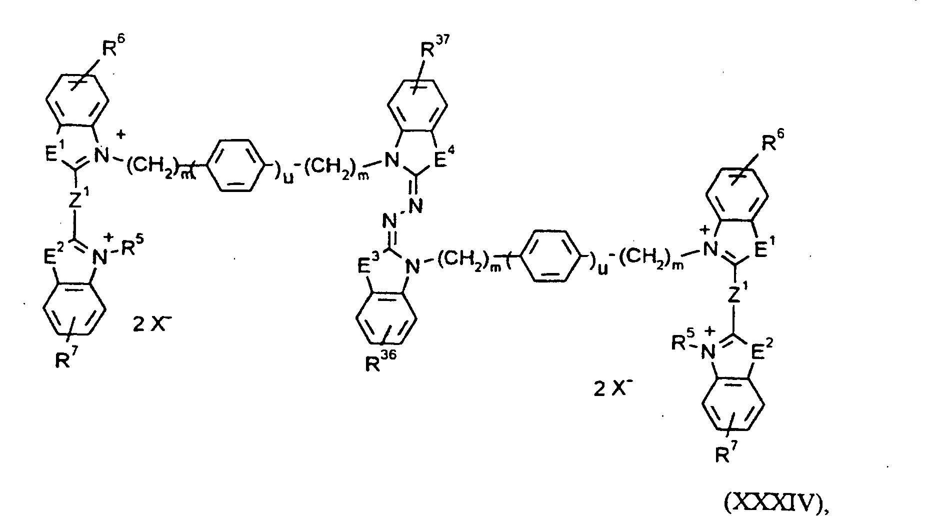 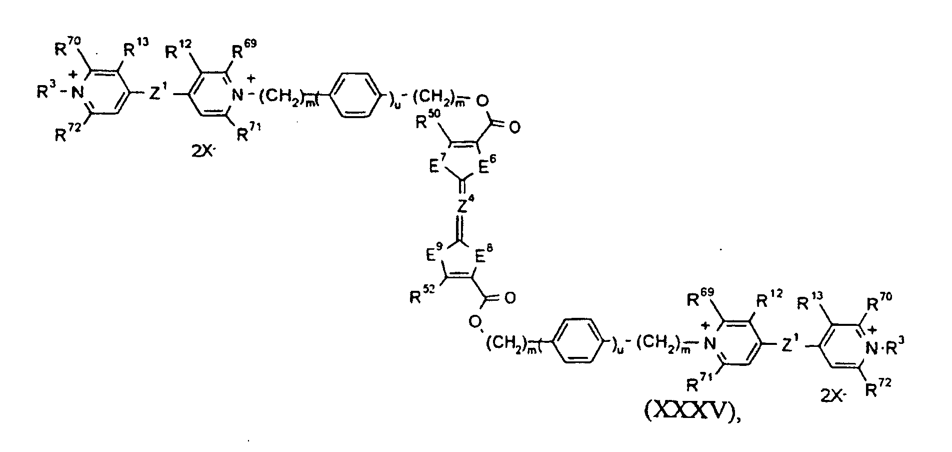 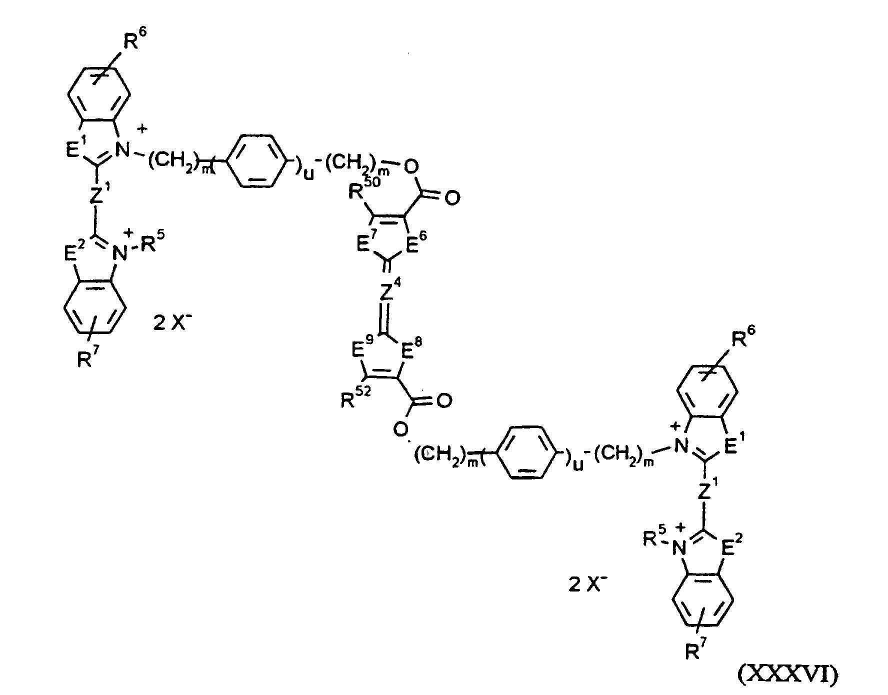 oder elektrochrome Substanzen der Formel (1c) 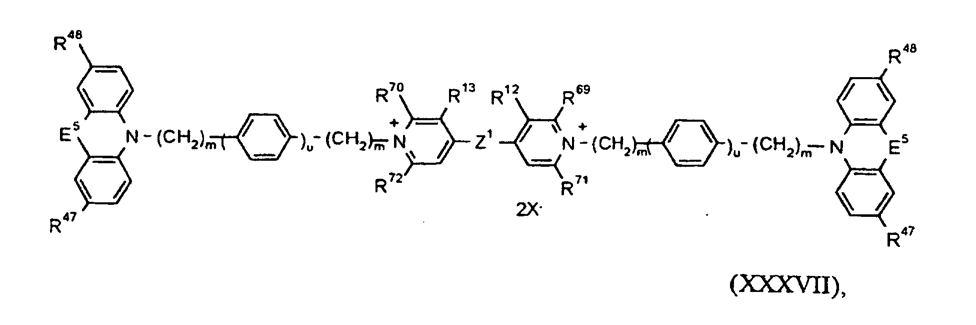 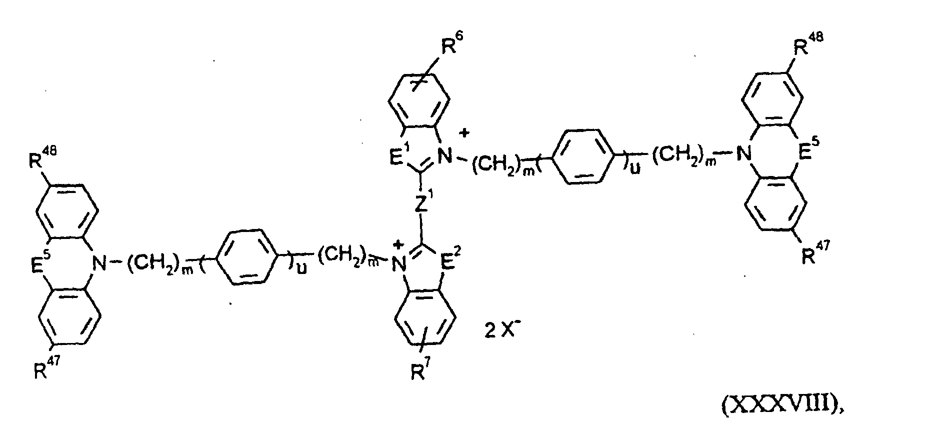 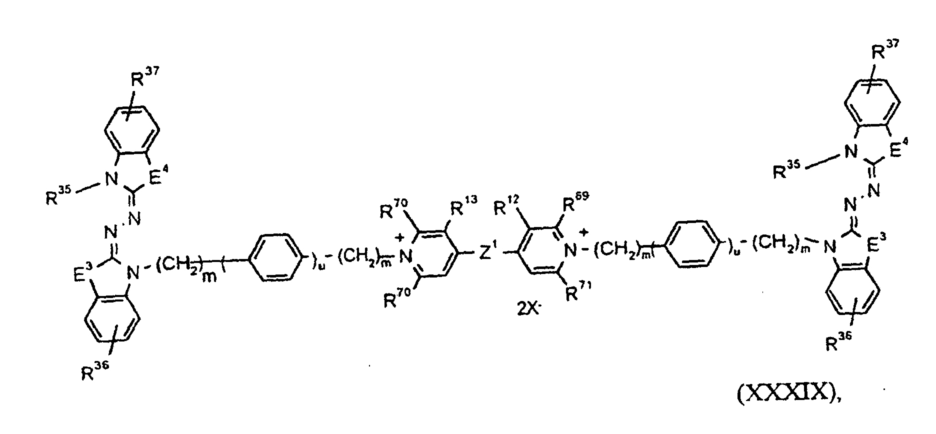 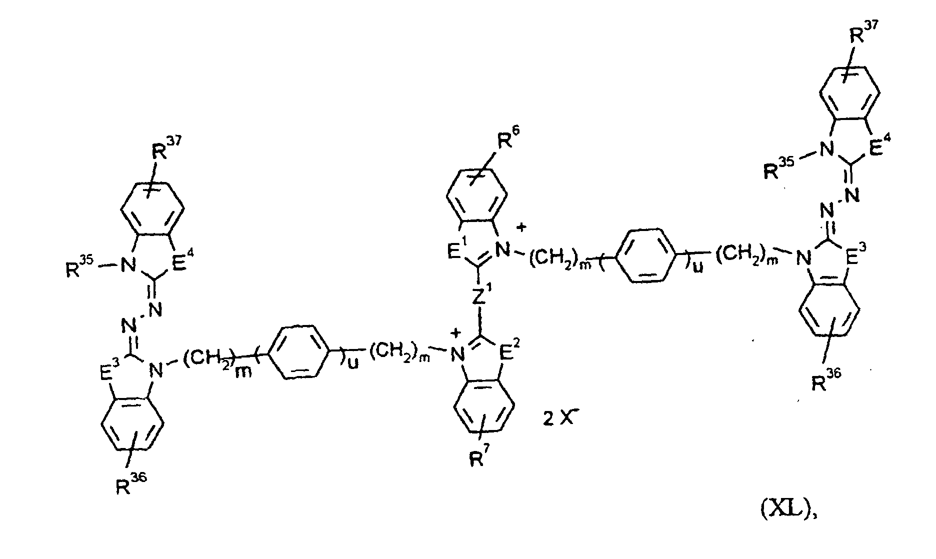 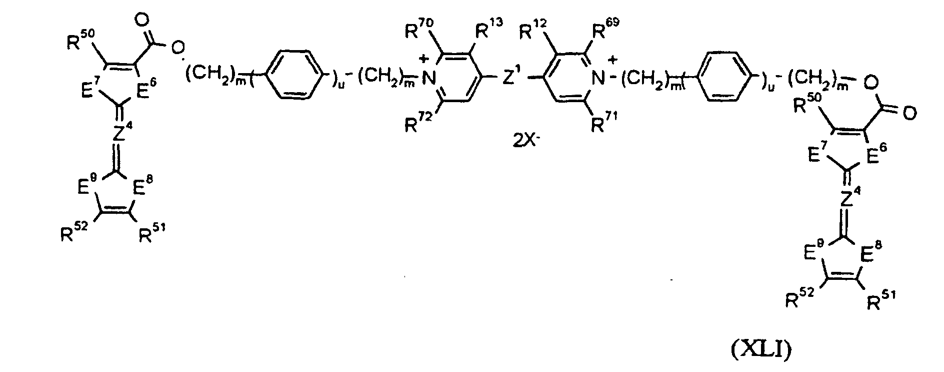 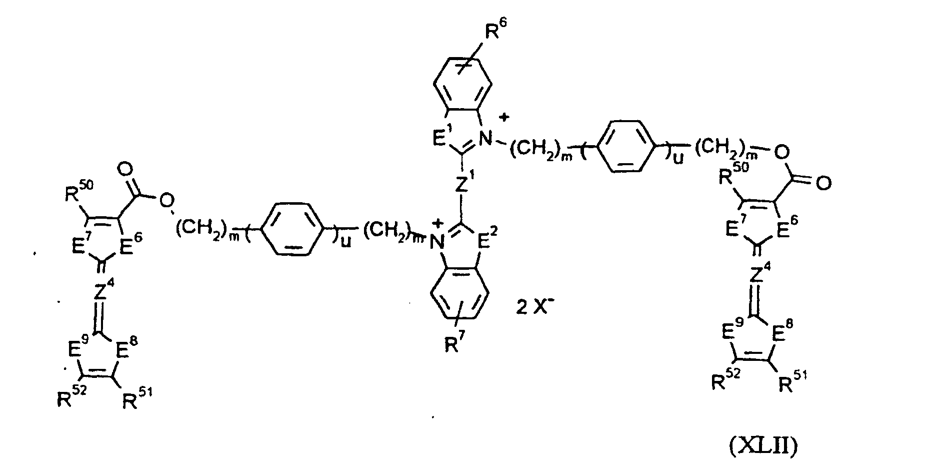 worin
- R³, R⁵, R³⁵ und R³⁹: unabhängig voneinander für Methyl, Ethyl, Propyl, Butyl, Pentyl, Hexyl oder Benzyl stehen,
- R⁶, R⁷ und R³⁶, R³⁷: paarweise gleich sind und für Wasserstoff, Methyl, Methoxy, Chlor, Cyano oder Methoxycarbonyl stehen,
- R¹² und R¹³: für Wasserstoff oder, wenn Z¹ eine direkte Bindung bedeutet, gemeinsam für eine CH=CH-Brücke stehen,
- R⁶⁹ bis R⁷²: gleich sind und für Wasserstoff oder Methyl stehen,
- E¹ und R²: gleich sind und für O oder S stehen,
- Z¹: für eine direkte Bindung oder -CH=CH steht,
- R³², R⁴⁷ und R⁴⁸: für Wasserstoff stehen,
- E³ bis E⁵: unabhängig voneinander für O, S oder NR⁵⁹ stehen, wobei E³ und E⁴ aber gleich sind,
- R²⁹ bis R³¹ und R⁵⁹: unabhängig voneinander für Methyl, Ethyl, Propyl, Butyl, Pentyl, Hexyl oder Benzyl stehen, wobei R²⁹ bis R³¹ vorzugsweise gleich sind,
- R⁴⁰ und R⁴¹: gleich sind und für Wasserstoff, Methyl, Ethyl, Propyl, Butyl oder Phenyl stehen,
- Z³: für eine direkte Bindung, -CH=CH- oder -N=N- steht,
- R⁵⁰ bis R⁵²: unabhängig voneinander für Wasserstoff, Methyl, Methoxy, Chlor, Cyano, Methoxycarbonyl, Ethoxycarbonyl oder Phenyl stehen, vorzugsweise jedoch gleich sind,
- E⁶ bis E⁹: untereinander gleich sind und für S, Se oder NR⁵⁹ stehen,
- Z⁴: für eine direkte Doppelbindung, eine =CH-CH= oder =N-N=-Brücke steht,
- m: für eine ganze Zahl von 1 bis 5 steht,
- u: für 0 oder 1 steht und
- X⁻: für ein unter den Bedingungen redox-inertes, farbloses Anion steht.

Die neuen Substanzen der Formel (Ia)

OX₂-B-RED₁ (Ia),

vorzugsweise solche Substanzen, die den Formeln (XXI) bis (XXX) entsprechen, können hergestellt werden durch Umsetzung von Verbindungen der Formel

OX₂-B-A (XLIII)

mit Verbindungen der Formel

RED₁ (XLIV)

oder durch Umsetzung von Verbindungen der Formel

OX₂ (XLV)

mit Verbindungen der Formel

A-B-RED₁ (XLVI),

wobei
- A: eine Abgangsgruppe wie Chlor, Brom, Iod, OSO₂-Alkyl, OSO₂-Perfluoralkyl oder OSO₂-Aryl bedeutet
und OX₂, RED₁ und B die oben angegebene allgemeine und bevorzugte Bedeutung besitzen.

Insbesondere ist mit OX₂-B-A eine der folgenden Verbindungen gemeint: 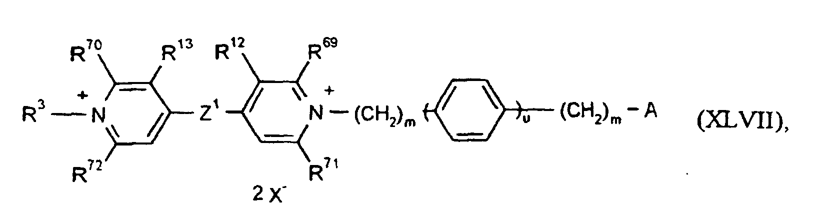 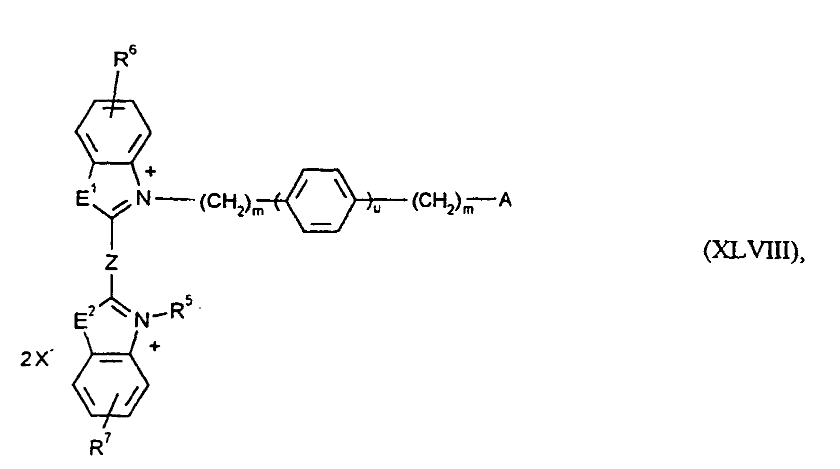 worin
A die oben angegebene Bedeutung hat, insbesondere für Brom steht und die übrigen Reste die oben unter der Definition der Formeln (XXI) und (XXII) angegebene Bedeutung haben.

Insbesondere ist mit RED₁-B-A eine der folgenden Verbindungen gemeint: 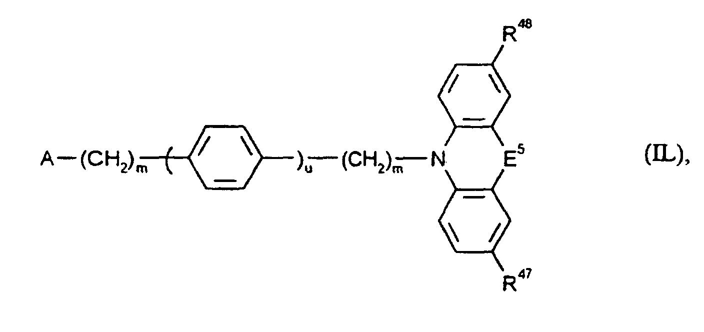 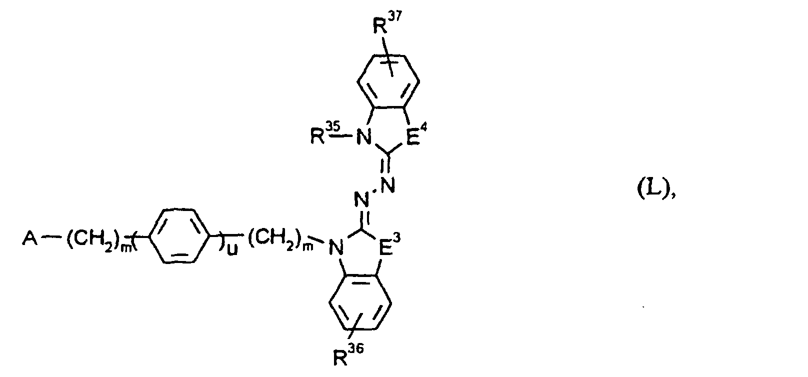 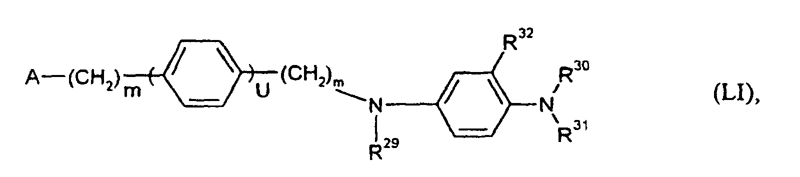 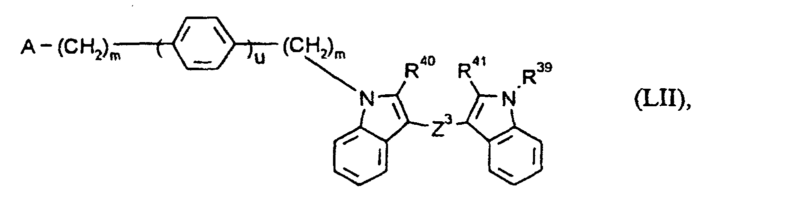 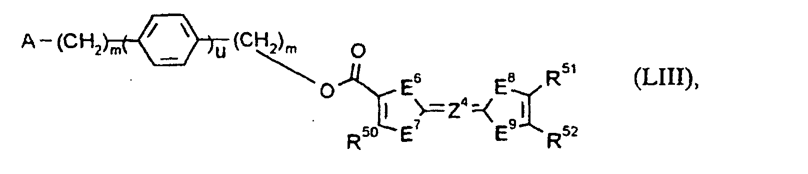 worin
A die oben angegebene Bedeutung hat, insbesondere für Brom steht und die übrigen Reste die oben in der Definition der Formeln (XXI), (XXIII), (XXV), (XXVII) und (XXIX) angegebene Bedeutung haben.

Insbesondere ist dann mit RED, eine der folgenden Verbindungen gemeint: 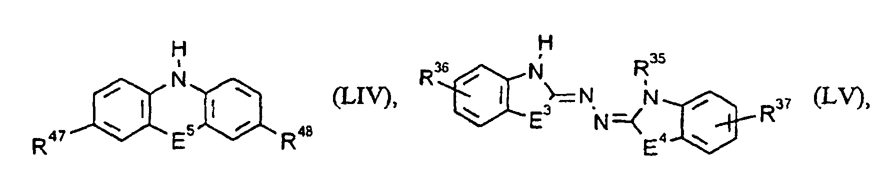 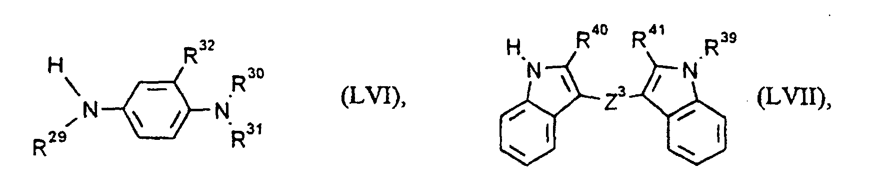 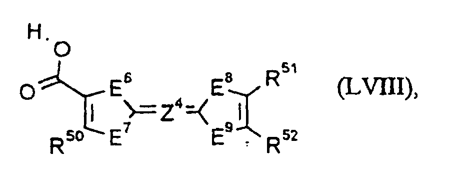 worin die Reste die oben angegebene Bedeutung haben.

Insbesondere ist dann mit OX₂ eine der folgenden Verbindungen gemeint: 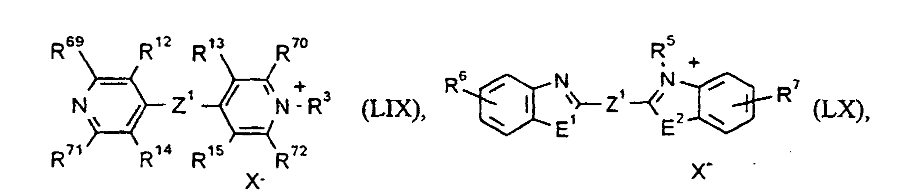 worin die Reste die oben angegebene Bedeutung besitzen.

Die Umsetzung der Komponenten (XLIII) und (XLIV) miteinander erfolgt vorzugsweise in polaren Lösungsmitteln wie Alkoholen - z.B. Methanol, Ethanol, Propanol, tert.-Butanol - , Nitrilen - z.B. Acetonitril, Propionitril - , Amiden - z.B. Dimethylformamid, N-Methylpyrrolidon - , Dimethylsulfoxid oder Sulfolan in Gegenwart basischer Verbindungen wie Hydroxide - z.B. Natrium- oder Kaliumhydroxid - , Oxiden - , z.B. Magnesiumoxid - , Alkoholaten - , z.B. Natriummethylat, Kalium-tert.-butylat - , Amiden - z.B. Natriumamid - oder basischen Ionenaustauschern bei Temperaturen zwischen 0°C und dem Siedepunkt des Mediums, vorzugsweise bei 5 bis 70°C. Die Produkte der Formel (I) fallen je nach Art des Anions X⁻ entweder direkt aus dem Medium aus oder können durch Zusatz von beispielsweise Wasser oder Alkoholen ausgefällt werden oder das Lösungsmittel wird im Vakuum abdestilliert.

Die Umsetzung der Komponenten (XLV) und (XLVI) erfolgt unter den oben angegebenen Bedingungen, jedoch in Abwesenheit einer basischen Verbindung.

In beiden Fällen kann es notwendig sein, die Anionen der so erhaltenen elektrochromen Substanzen der Formel (I) auszutauschen. Dies kann erfolgen mittels Ionenaustauschern oder durch Umfällen mittels Natrium-, Kalium- oder Tetraalkylammoniumsalzen der entsprechenden gewünschten, redox-inerten Anionen. Geeignete Lösungsmittel für diese Operation sind die oben beschriebenen Alkohole und Nitrile sowie Wasser. Geeignete Salze sind beispielsweise Natriumtetrafluoroborat, Natriumperchlorat, Natrium-hexafluorosilikat, Natriumhexafluorophosphat, Tetrabutylammoniumtetrafluoroborat.

Die Grundkörper OX₂ und RED₁ sind bekannt, beispielsweise die Substanzen der Formeln (II) bis (IX) aus Topics in Current Chemistry, Vol. 92, S. 1-44 (1980), Angew. Chem. 90, 927 (1978), Adv. Mater. 3, 225 (1991), DE-OS 3,917,323 und beispielsweise die Substanzen der Formeln (X) bis (XIX) aus Topics in Current Chemistry, Vol. 92, S. 1-44 (1980), Angew. Chem. 90, 927 (1978), J. Am. Chem. Soc. 117, 8528 (1995), J. C. S. Perkin II, 1990, 1777 und DE-OS 4,435,211 oder aus der dort jeweils zitierten Literatur oder lassen sich analog herstellen.

Die Komponenten OX₂-B-A (XLIII), speziell (XLVII) und (XLVIII), und A-B-RED₁ (XLVI), speziell (IL) bis (LIII), lassen sich dann aus OX₂ und RED₁ in einfacher Weise herstellen durch Umsetzung mit beispielsweise

A-B-A (LXI)

in einem der oben genannten Lösungsmittel, im Fall RED₁ in Gegenwart, im Falle OX₂ in Abwesenheit einer der oben genannten basischen Verbindungen unter den oben genannten Reaktionsbedingungen.

Ganz analog lassen sich die neuen Substanzen der Formeln (Ib) bis (Id) herstellen.

Die Substanzen der Formel (Ib)

OX₂-B-RED₁-B-OX₂ (Ib),

vorzugsweise solche Substanzen, die den Formeln (XXXI) bis (XXXVI) entsprechen, können hergestellt werden durch Umsetzung von 2 Äquivalenten von Verbindungen der Formel

OX₂-B-A (XLIII)

mit einem Äquivalent Verbindungen der Formel

RED₁ (XLIV),

wobei OX₂, RED₁, A und B die oben angegebene Bedeutung besitzen.

Insbesondere sind mit OX₂-B-A die oben genannten Verbindungen der Formeln (XLVII) und (XLVIII) gemeint. Mit RED₁ sind insbesondere die oben genannten Verbindungen der Formeln (LIV), (LV) und (LVIII) gemeint,
worin
- E⁵: für NH steht,
- R³⁵: für Wasserstoff steht,
- R⁵¹: für -COOH steht und
die anderen Reste die oben angegebene Bedeutung besitzen.

Die Substanzen der Formel (Ic)

RED₁-B-OX₂-B-RED₁ (Ic),

vorzugsweise solche Substanzen, die den Formeln (XXXVII) bis (XLII) entsprechen, können hergestellt werden durch Umsetzung von 2 Äquivalenten Verbindungen der Formel

A-B-RED₁ (XLVI)

mit einem Äquivalent Verbindungen der Formel

OX₂ (XLV),

wobei OX₂, RED₁, A und B die oben angegebene Bedeutung besitzen.

Insbesondere sind mit A-B-RED₁ die oben genannten Verbindungen der Formeln (IL), (L) und (LIII) gemeint. Mit OX₂ ist insbesondere eine der folgenden Verbindungen gemeint: 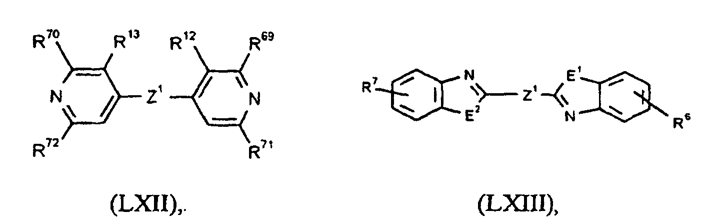 wonn
die Reste die oben angegebene Bedeutung besitzen.

Die Reaktionsbedingungen sind dann ganz analog zu den oben angegebenen.

In den obengenannten Substituentenbedeutungen sind Alkylreste, auch abgewandelte, wie z.B. Alkoxy- oder Aralkylreste, vorzugsweise solche mit 1 bis 12 C-Atomen, insbesondere mit 1 bis 8 C-Atomen, sofern nichts anderes angegeben ist. Sie können geradkettig oder verzweigt sein und gegebenenfalls weitere Substituenten tragen wie z.B. C₁- bis C₄-Alkoxy, Fluor, Chlor, Hydroxy, Cyano, C₁bis C₄-Alkoxycarbonyl oder COOH.

Unter Cycloalkylresten werden vorzugsweise solche mit 3 bis 7 C-Atomen, insbesondere 5 oder 6 C-Atomen verstanden.

Alkenylreste sind vorzugsweise solche mit 2 bis 8 C-Atomen, insbesondere 2 bis 4 C-Atomen.

Arylreste, auch solche in Aralkylresten, sind vorzugsweise Phenyl- oder Naphthylreste, insbesondere Phenylreste. Sie können durch 1 bis 3 der folgenden Reste substituiert sein: C₁- bis C₆-Alkyl, C,- bis C₆-Alkoxy, Fluor, Chlor, Brom, Cyano, Hydroxy, C₁- bis C₆-Alkoxycarbonyl oder Nitro. Zwei benachbarte Reste können auch einen Ring bilden.

Das erfindungsgemäße elektrochrome System enthält vorzugsweise mindestens ein Lösungsmittel, wodurch eine elektrochrome Flüssigkeit entsteht, die ebenfalls Gegenstand der vorliegenden Erfindung ist.

Geeignete Lösungsmittel sind alle unter den gewählten Spannungen redox-inerten Lösungsmittel, die keine Elektrophile oder Nukleophile abspalten können oder selber als ausreichend starke Elektrophile oder Nukleophile reagieren und so mit den farbigen Radikalionen reagieren könnten. Beispiele sind Propylencarbonat, γ-Butyrolacton, Acetonitril, Propionitril, Glutaronitril, Methylglutarnitril, 3,3'-Oxydipropionitril, Hydroxypropionitril, Dimethylformamid, N-Methylpyrrolidon, Sulfolan, 3-Methylsulfolan oder Mischungen davon. Bevorzugt sind Propylencarbonat und Mischungen davon mit Glutaronitril oder 3-Methylsulfolan.

Die erfindungsgemäße elektrochrome Flüssigkeit kann mindestens ein inertes Leitsalz enthalten.

Als inerte Leitsalze sind Lithium-, Natrium- und Tetraalkylammoniumsalze geeignet, insbesondere letztere. Die Alkylgruppen können zwischen 1 und 18 C-Atome aufweisen und gleich oder verschieden sein. Bevorzugt ist Tetrabutylammonium Als Anionen zu diesen Salzen, aber auch als Anionen X⁻ in den Formeln (I), (II), (IV), (VI), (VII) kommen alle redox-inerten, farblosen Anionen in Frage. Beispiele sind Tetrafluoroborat, Perchlorat, Methansulfonat, Trifluormethansulfonat, Perfluorbutansulfonat, Benzolsulfonat, Hexafluorophosphat, Hexafluoroarsenat und Hexafluorosilikat. Im letzteren Falle steht X⁻ für 1/2 SiF₆²⁻.

Die Leitsalze werden vorzugsweise im Bereich 0 bis 1 molar eingesetzt.

Als weitere Zusätze zu der elektrochromen Flüssigkeit können Verdicker eingesetzt werden, um die Viskosität der Flüssigkeit zu steuern. Das kann Bedeutung haben zur Vermeidung von Segregation, d.h. der Bildung von streifiger oder fleckiger Farbbildung bei längerem Betrieb einer die erfindungsgemäße elektrochrome Flüssigkeit enthaltenden elektrochromen Vorrichtung im eingeschalteten Zustand, und zur Steuerung der Ausbleichgeschwindigkeit nach Abschalten des Stroms.

Als Verdicker eignen sich alle für diese Zwecke üblichen Verbindungen wie z.B. Polyacrylat, Polymethacrylat (Luctite L®), Polycarbonat und Polyurethan.

Die elektrochrome Flüssigkeit kann auch gelförmig sein.

Als weitere Zusätze für die elektrochrome Flüssigkeit kommen UV-Absorber zur Verbesserung der Lichtechtheit in Frage. Beispiele sind Uvinul® 3000 (2,4-Dihydroxybenzophenon, BASF), SANDUVOR® 3035 (2-Hydroxy-4-n-octyloxybenzophenon, Clariant), Tinuvin® 571 (2-(2H-Benzotriazol-2-yl)-6-dodecyl-4-methylphenol, Ciba), Cyasorb 24™ (2,2'-Dihydroxy-4-methoxybenzophenon, American Cyanamid Company), UVINUL® 3035 (Ethyl-2-cyano-3,3-diphenylacrylat, BASF), Uvinul® 3039 (2-Ethylhexyl-2-cyano-3,3-diphenylacrylat, BASF), UVINUL® 3088 (2-Ethylhexyl-p-methoxycinnamat, BASF).

Die UV-Absorber werden im Bereich 0,01 bis 2 mol/l, vorzugsweise 0,04 bis 1 mol/l eingesetzt.

Die erfindungsgemäße elektrochrome Flüssigkeit enthält die Substanzen der Formel (I), insbesondere der Formeln (Ia) bis (Id), jeweils in einer Konzentration von mindestens 10⁻⁴ mol/l, vorzugsweise 0,001 bis 1 mol/l Es können auch Mischungen mehrerer elektrochromer Substanzen der Formel (I) eingesetzt werden.

Die erfindungsgemäßen elektrochromen Flüssigkeiten sind bestens als Bestandteil einer elektrochromen Vorrichtung geeignet. Weiterer Gegenstand der vorliegenden Erfindung sind demnach elektrochrome Vorrichtungen enthaltend eine erfindungsgemäße elektrochrome Flüssigkeit. Der Aufbau einer elektrochromen Vorrichtung, die z.B. als Fensterscheibe, Autosonnendach, Automobil-Rückspiegel oder Display ausgebildet sein kann, ist im Prinzip bekannt. Die erfindungsgemäße elektrochrome Vorrichtung besteht aus zwei einander zugewandten, lichtdurchlässigen Glas- oder Kunststoffscheiben, von denen gegebenenfalls eine verspiegelt ist und deren einander zugewandten Seiten elektrisch leitfähig beschichtet sind, z.B. mit Indium-Zinn-Oxid (ITO), zwischen denen sich die erfindungsgemäße elektrochrome Flüssigkeit befindet. Als leitfähige Materialien sind auch geeignet: Antimon-dotiertes Zinnoxid, Fluor-dotiertes Zinnoxid, Antimon-dotiertes Zinkoxid, Aluminium-dotiertes Zinkoxid, Zinnoxid; auch leitfähige organische Polymere wie gegebenenfalls substituierte Polythienyle, Polypyrrole, Polyaniline, Polyacetylen. Im Falle, daß eine der Scheiben verspiegelt ist, kann auch diese als leitfähige Schicht genutzt werden. Der Abstand der beiden Scheiben beträgt im allgemeinen 0,005-2 mm, vorzugsweise 0,02-0,5 mm. Der gewünschte Abstand zwischen den Scheiben wird im allgemeinen durch einen Dichtungsring hergestellt.

Die erfindungsgemäße selbstlöschende einzellige elektrochrome Vorrichtung kann zusätzlich zu den oben beschriebenen elektrochromen Substanzen der Formeln (I), insbesondere den Formeln (Ia) bis (Id), auch andere enthalten, wie sie beispielsweise in US-P 4,902,108, Topics in Current Chemistry, Vol. 92, S. 1-44 (1980) und Angew. Chem. 90, 927 (1978) beschrieben sind. Solche elektrochromen Substanzen entstammen beispielsweise den oben unter den Formeln (II) bis (XX) angegebenen Gruppen, wobei dann keiner der angeführten Reste die Bedeutung "direkte Bindung zur Brücke B" besitzen kann. Eine Zumischung solcher Redoxsysteme kann beispielsweise vorteilhaft sein, um bei der erfindungsgemäßen elektrochromen Vorrichtung den Farbton, z.B. des Displays, im eingeschalteten Zustand zu korrigieren oder zu intensivieren.

### Beispiel:

### Beispiel 1

### Herstellung einer elektrochromen Substanz der Formel (I)

a) 5.0 g 4,4'-Dipyridyl wurden in 30 ml wasserfreiem Acetonitril bei 50°C gelöst. Während 50 min tropften bei dieser Temperatur 2.7 g Benzylbromid dazu. Nach 3 h bei 50 °C wurde abgekühlt und der hellgelbe Niederschlag abgesaugt. Dieser wurde mit 60 ml Toluol gewaschen und im Vakuum getrocknet. Man erhielt 3.9 g (75 % d. Th.) des Produkts der Formel 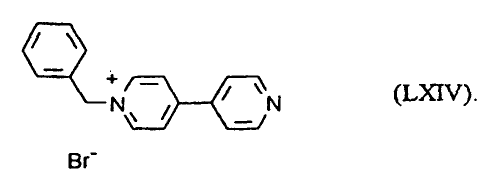
b) 10.1 g Phenothiazin wurden in 60 ml wasserfreiem N-Methylpyrrolidon unter N₂-Atmosphäre bei Raumtemperatur gelöst. 5.9 g Kalium-tert.-butylat wurden zugesetzt. Es entstand unter Erwärmung auf 30 °C eine orange Suspension, die 30 min bei 30 °C gerührt wurde. Dann wurden 54 g 1,4-Dibrombutan auf einmal zugesetzt. Die Temperatur stieg dabei bis auf 53°C an. Während 45 min wurde auf 70 °C geheizt, 15 min bei dieser Temperatur gehalten und dann abgekühlt. Die hellbraune Suspension wurde in 1 l Wasser eingetragen. Mit 3x200 ml Toluol wurde extrahiert, der Extrakt wurde mit 5x200 ml Wasser gewaschen, über Natriumsulfat getrocknet und am Rotationsverdampfer eingeengt. Der ölige Rückstand wurde in 400 ml Hexan gelöst, vom Unlöslichen filtriert und erneut eingeengt. Anschließend wurde bei 0.1 bis 0.5 mbar der Überschuß an 1,4-Dibrombutan abdestilliert. Man erhielt 9.6 g (57 % d. Th.) eines rötlichgelben, zähen Öls der Formel 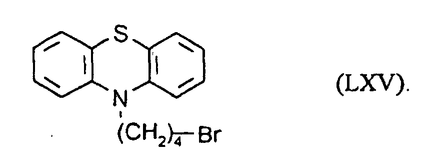
c) 3.7 g des Phenothiazins der Formel (LXV) wurden in 10 ml wasserfreiem N-Methylpyrrolidon bei Raumtemperatur unter N₂-Atmosphäre gelöst. 1.8 g des Dipyridiniumsalzes der Formel (LXIV) wurden zugesetzt. Die Suspension wurde während 1 h auf 80 °C geheizt und insgesamt 13 h bei dieser Temperatur gehalten. Dabei wurde die Suspension immer dicker. Nach Abkühlen auf Raumtemperatur wurde abgesaugt und mit 5 ml N-Methylpyrrolidon gewaschen. Das hygroskopische Rohprodukt der Formel 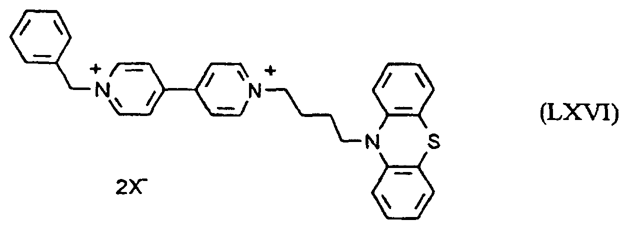 mit X⁻ = Br⁻ wurde in 7 ml Methanol aufgelöst und filtriert. In das Filtrat wurden während 2 h 3.0 g Tetrabutylammoniumtetrafluoroborat eingestreut. Langsam entstand eine Fällung, die während 18 h Rühren bei Raumtemperatur vervollständigt wurde. Schließlich wurde abgesaugt, mit Methanol bis zum farblosen Ablauf gewaschen und im. Vakuum getrocknet. Man erhielt 0.5 g (13 % d. Th.) blaß bläuliches Pulver der Formel (LXVI) mit X⁻ = BF₄⁻.
   In einer elektrochromeren Vorrichtung gemäß Beispiel 29-30 wurde eine violettblaue Färbung mit λₘₐₓ = 517 und 606 nm erzielt.

### Beispiel 2

a) Unter Stickstoffatmosphäre wurden 9,2 g Phenazin in 60 ml wasserfreiem Tetrahydrofuran suspendiert. Während 15 min tropften 30,8 ml 20 gew.-%ige Phenyllithiumlösung in Cyclohexan/Diethylether 7 : 3 dazu, wobei die Temperatur bei max. 35°C gehalten wurde. Die Lösung wurde 30 min bei Raumtemperatur nachgerührt.
   Bei 15°C wurden in einer Portion 30,2 ml 1,4-Dibrombutan dazugegeben. Die Temperatur stieg dabei bis 38°C an. Nach 6 h bei Raumtemperatur wurde mit 200 ml Wasser versetzt und der pH auf 7,0 gestellt. Die organische Phase wurde abgetrennt, dreimal mit je 100 ml Wasser gewaschen und im Vakuum eingeengt. Schließlich wurde überschüssiges 1,4-Dibrombutan bei einem Druck von 0,2 mbar abdestilliert. Der ölige Rückstand wurde in 400 ml Ethanol heiß gelöst. Das nach dem Erkalten ausgefallene Produkt wurde abgesaugt, mit Ethanol und Hexan gewaschen und getrocknet. Man erhielt 8,0 g (41 % d. Th.) blaßgelbes Pulver des 9,10-Dihydrophenazins der Formel 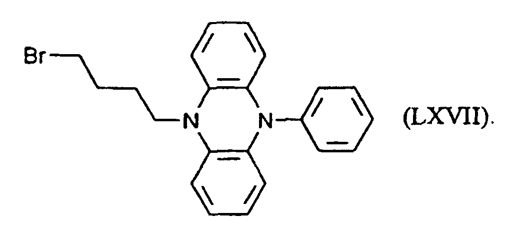
b) 7,5 g des 9,10-Dihydrophenazins der Formel (LXVII) aus a) und 6,1 g 4,4'-Dipyridyl wurden in 100 ml Acetonitril 24 h bei 70°C unter Stickstoffatmosphäre gerührt. Nach dem Abkühlen wurde abgesaugt und mit 50 ml Aceton gewaschen. Nach dem Trocknen erhielt man 6,3 g (60 % d. Th.) des Salzes der Formel 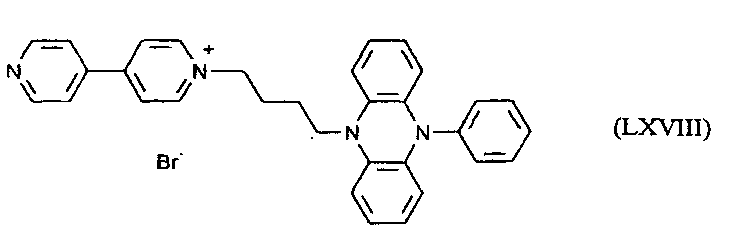
c) 6,1 g des unter b) erhaltenen Salzes wurden in 70 ml N-Methyl-2-pyrrolidon zusammen mit 2,7 ml Benzylbromid 7 h bei 70°C unter Stickstoffatmosphäre gerührt. Nach dem Abkühlen wurde mit 150 ml Toluol verdünnt und das ausgefallene Produkt abgesaugt. Es wurde gründlich mit 150 ml Toluol und 500 ml Hexan gewaschen und getrocknet. Man erhielt 5,5 g (69 % d. Th.) des Dipyridiniumsalzes der Formel 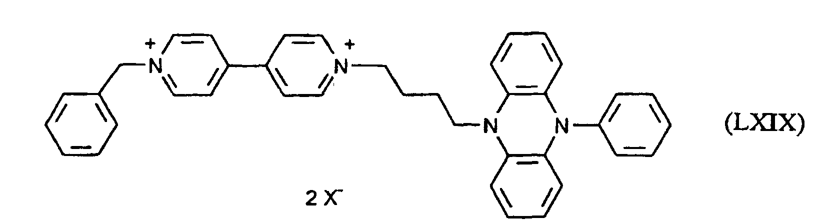 mit X⁻ = Br⁻ .
d) 4,0 g dieses Produkts aus c) wurden unter Stickstoffatmosphäre in 100 ml Methanol bei 65°C gelöst. Während 5 min wurden 7,4 g Tetrabutylammoniumtetrafluoroborat eingestreut. Es trat eine Fällung auf. Nach 5 min bei 65°C wurde abgekühlt; abgesaugt, mit 200 ml Methanol und 50 ml Hexan gewaschen und im Vakuum getrocknet. Man erhielt 3,4 g (83 % d. Th.) blaß beiges Pulver der Formel (LXIX) mit X⁻ = BF₄⁻.
   In einer elektrochromen Vorrichtung gemäß Beispiel 29-30 wurde eine grünlich-blaue Färbung mit λₘₐₓ = 466 und 407 nm erzielt.

### Beispiel 3

a) 45,3 g 2-Methylthiobenzthiazol wurden in 75 ml Toluol gelöst. 151 ml 1,4-Dibrombutan und eine Spatelspitze Kaliumiodid wurden zugesetzt. 4 h wurde gekocht und dann abgekühlt. Es wurde filtriert und mit 50 ml Toluol gewaschen. Das Filtrat wurde auf 50°C geheizt und mit 35,9 ml Dimethylsulfat versetzt. 8 h wurde bei 50°C gerührt, abgekühlt, abgesaugt und mit 250 ml Toluol gewaschen. Das Produkt wurde in 100 ml Aceton aufgerührt, erneut abgesaugt und mit 300 ml Aceton gewaschen. Man erhielt nach dem Trocknen im Vakuum 53,1 g (50 % d. Th.) des Salzes der Formel 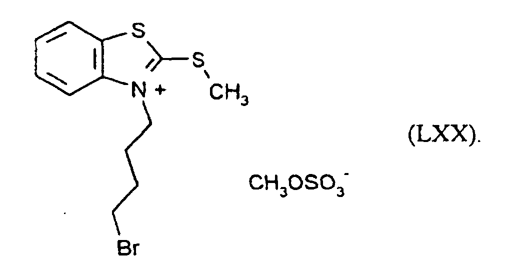
b) In 60 ml Acetonitril wurden unter Stickstoffatmosphäre 6,95 g des Benzthiazoliumsalzes der Formel (LXX) aus a) und 2,9 g des Hydrazons der Formel 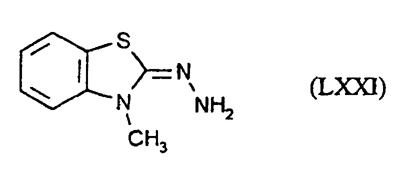 (von der Fa. Aldrich Chemical Company Ltd., England) suspendiert. Bei Raumtemperatur wurden 2,3 ml Triethylamin zugegeben. Es entstand kurzzeitig eine Lösung, dann bildete sich ein Niederschlag. Nach 5 h bei Raumtemperatur wurde schließlich abgesaugt, mit 50 ml Methanol, 100 ml Wasser und weiteren 50 ml Methanol bis zum farblosen Ablauf gewaschen und im Vakuum getrocknet. Man erhielt 6,0 g (83 % d. Th.) des Azins der Formel 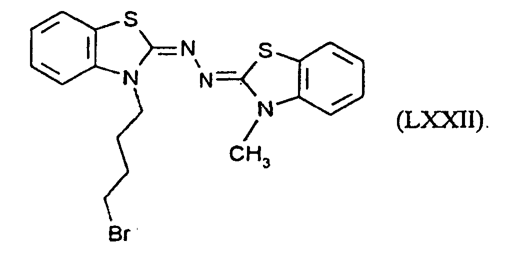
c) Wurde analog zu Beispiel 1 a) gearbeitet, aber statt Benzylbromid 6,8 ml Butylbromid eingesetzt, so erhielt man 5,2 g (57 % d. Th.) des Pyridiniumsalzes der Formel 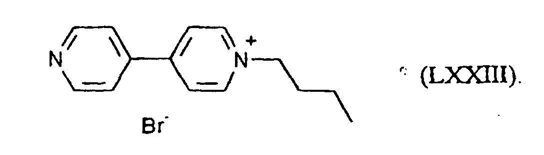
d) 2,0 g des Azins der Formel (LXXII) aus b) und 1,3 g des Pyridiniumsalzes der Formel (LXXIII) aus c) wurden in 20 ml N-Methyl-2-pyrrolidon unter Stickstoffatmosphäre 102 h bei 80°C gerührt. Nach dem Abkühlen wurde ein grünliches kristallines Produkt abgesaugt und mit 50 ml Aceton gewaschen. Nach dem Trocknen erhielt man 0,25 g (7,6 % d. Th.) des Dipyridiniumsalzes der Formel 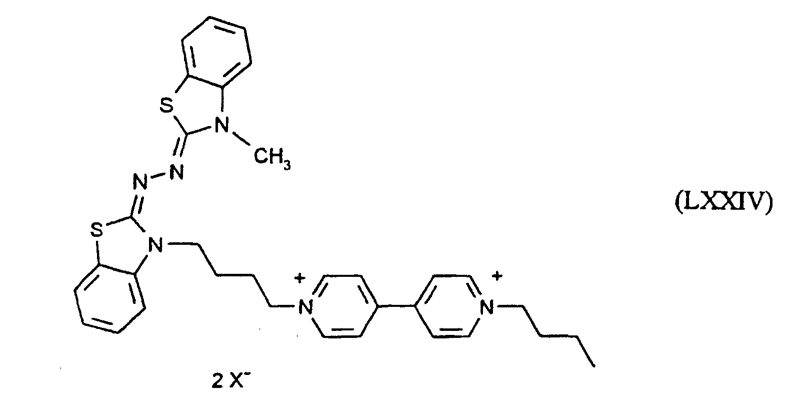 mit X⁻ = Br⁻.
e) 0,25 g des Produkts aus c) wurden in 5 ml Methanol fast vollständig gelöst. 0,45 g Tetrabutylammoniumtetrafluoroborat wurden zugesetzt. Es wurde 17 h bei Raumtemperatur gerührt, wobei das Produkt allmählich kristallin wurde. Es wurde abgesaugt, mit 25 ml Methanol, 25 ml Wasser und erneut mit 25 ml Methanol gewaschen. Nach dem Trocknen erhielt man 0,15 g (59 % d. Th.) hellgraues Pulver der Formel (LXXIV) mit X⁻ = BF₄⁻.
   In einer elektrochromen Vorrichtung gemäß Beispiel 29-30 wurde eine grüne Färbung mit λₘₐₓ = 402; 606; 734 nm erzielt.

### Beispiel 4

a) 4,0 g des Phenothiazins der Formel (LXV) aus Beispiel 1b) und 0,95 g 4,4'-Dipyridyl wurden in 10 ml Acetonitril unter Stickstoffatmosphäre 9 h bei 70°C gerührt. Die Suspension wurde dann mit 10 ml N-Methyl-2-pyrrolidon verdünnt und 25 h bei 70°C und 7 h bei 80°C gerührt. Nach dem Abkühlen wurde abgesaugt, mit 50 ml Methanol gewaschen und im Vakuum getrocknet. Man erhielt 1,6 g (32 % d. Th.) des Dipyridiniumsalzes der Formel 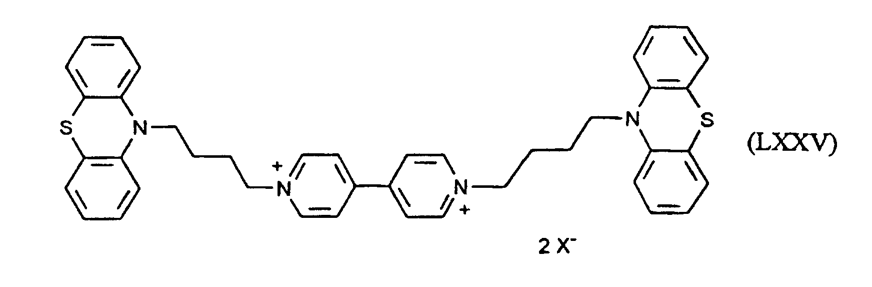 mit X⁻ = Br⁻.
b) 1,4 g des Salzes der Formel (LXXV) aus a) wurden in 20 ml Methanol bei Rückfluß teilweise gelöst. 2,3 g Tetrabutylammoniumtetrafluoroborat wurden zugesetzt. Es wurde noch 5 min gekocht und dann kaltgerührt. Das ausgefallene Produkt wurde abgesaugt, mit 50 ml Methanol, 50 ml Wasser und erneut mit 50 ml Methanol gewaschen und im Vakuum getrocknet. Man erhielt 1,1 g (77 % d. Th.) Dipyridiniumsalz der Formel (LXXV) mit X⁻ = BF₄⁻ .
   In einer elektrochromen Vorrichtung gemäß Beispiel 29-30 wurde eine violettblaue Färbung mit λₘₐₓ = 517 und 606 nm erzielt.
   Ganz analog wurden die folgenden Beispiele hergestellt.

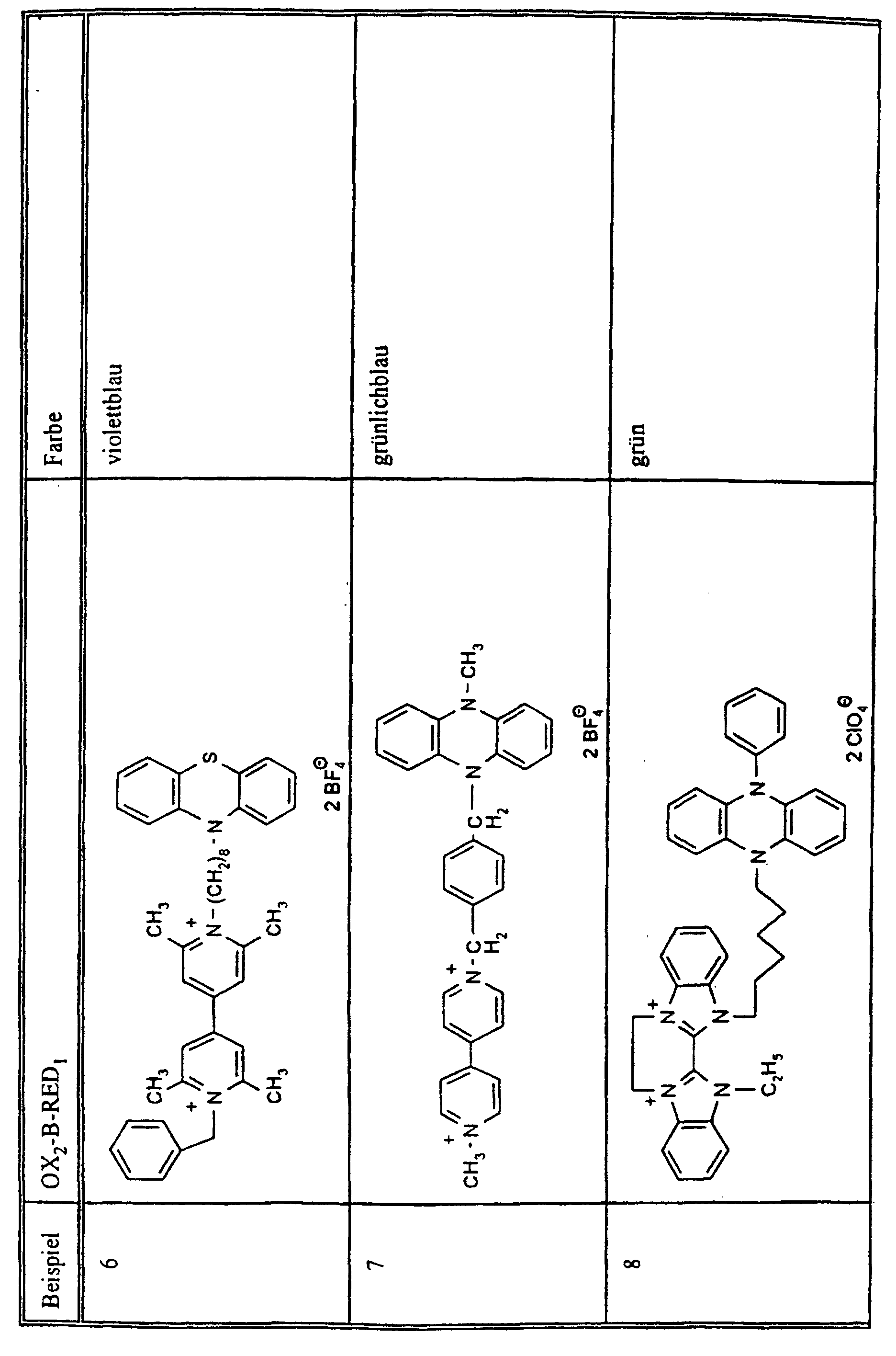

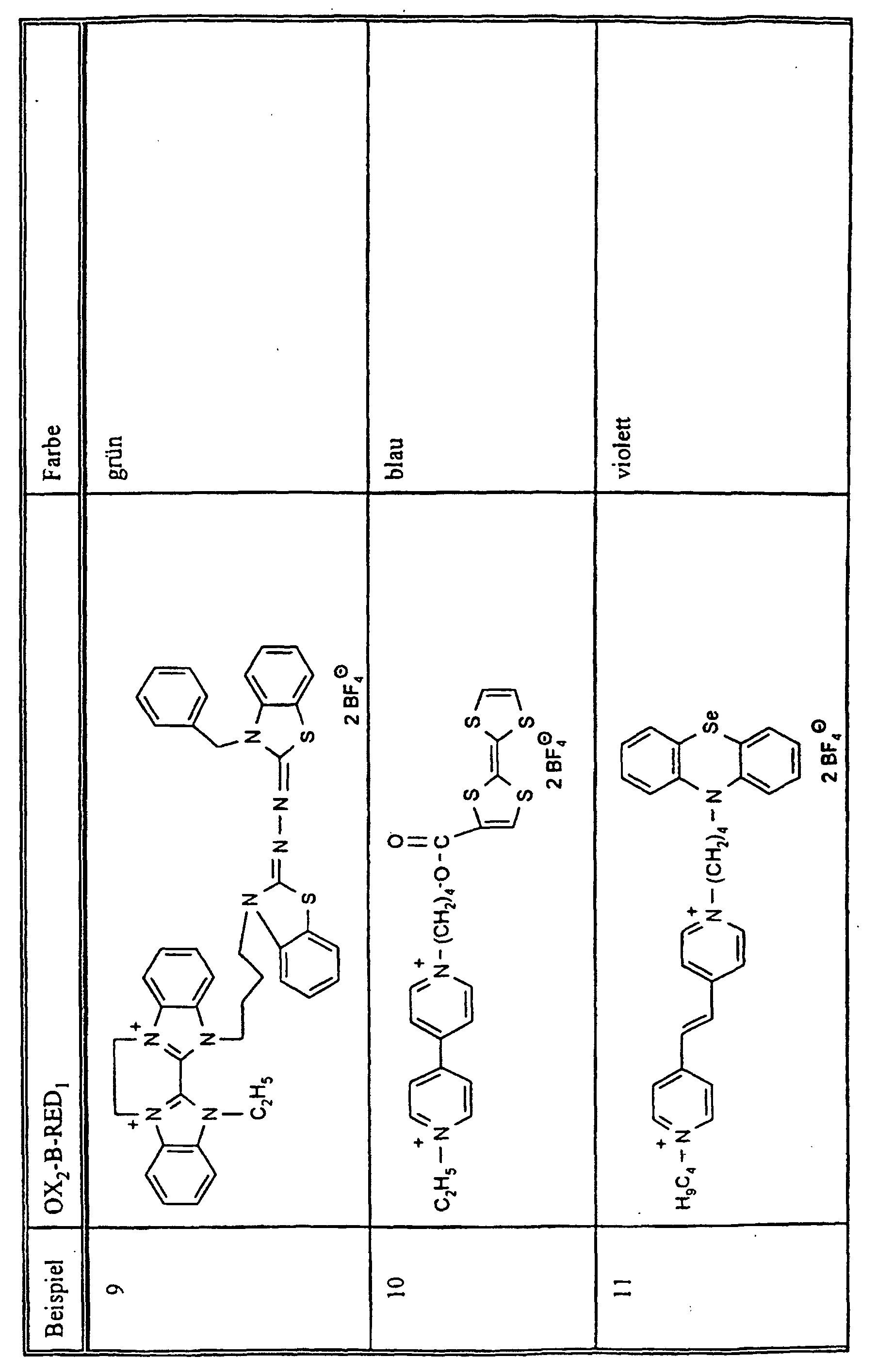

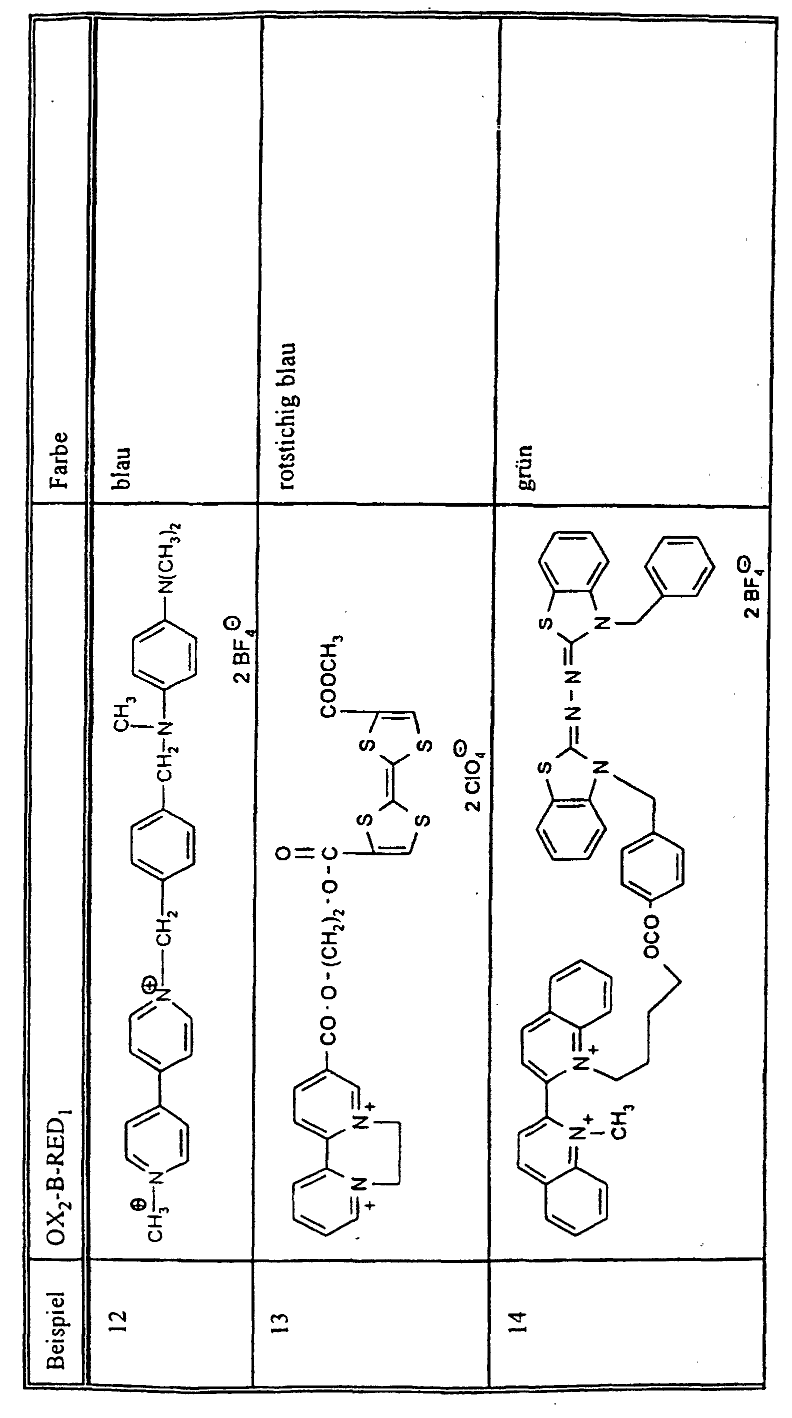

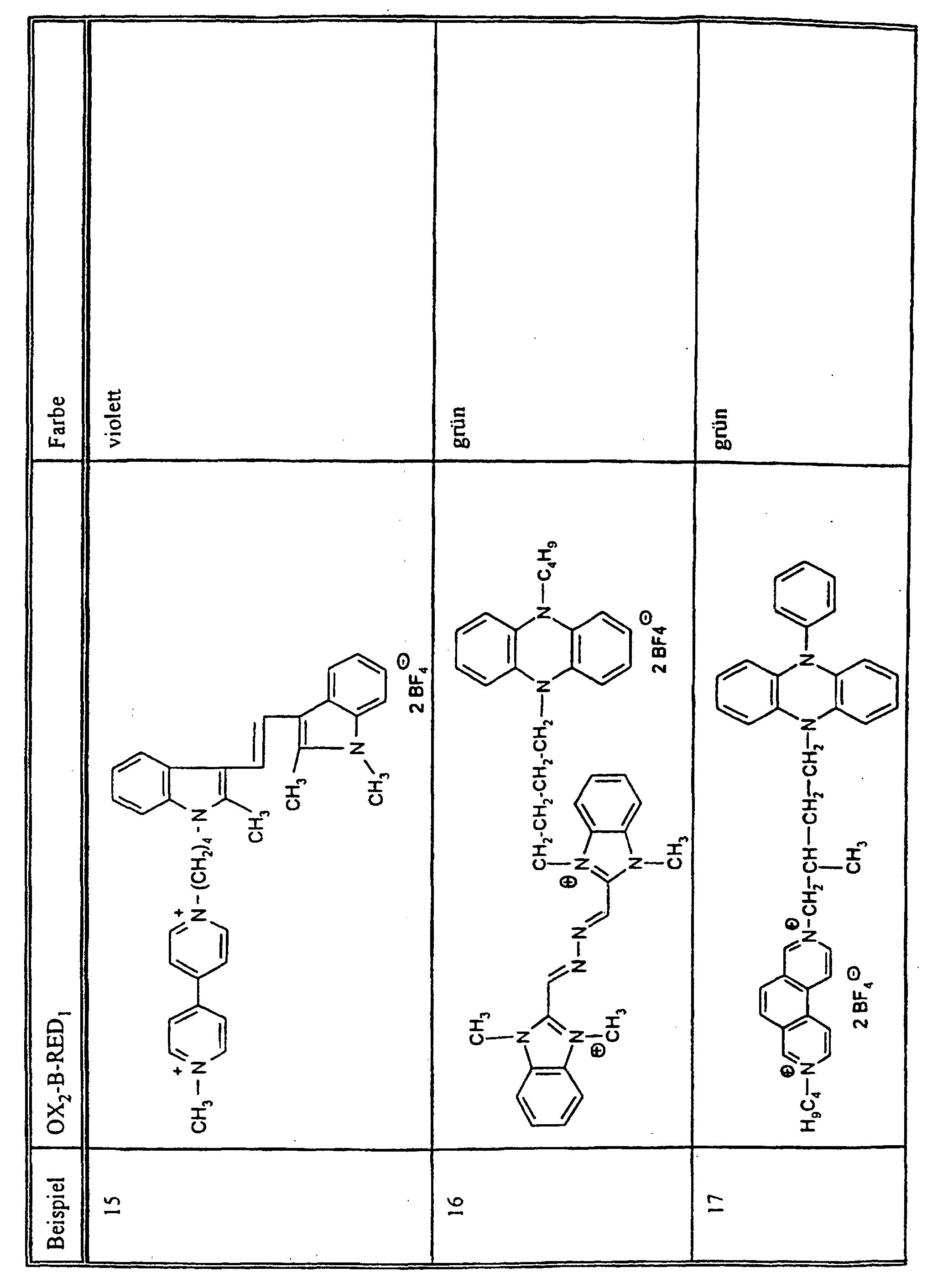

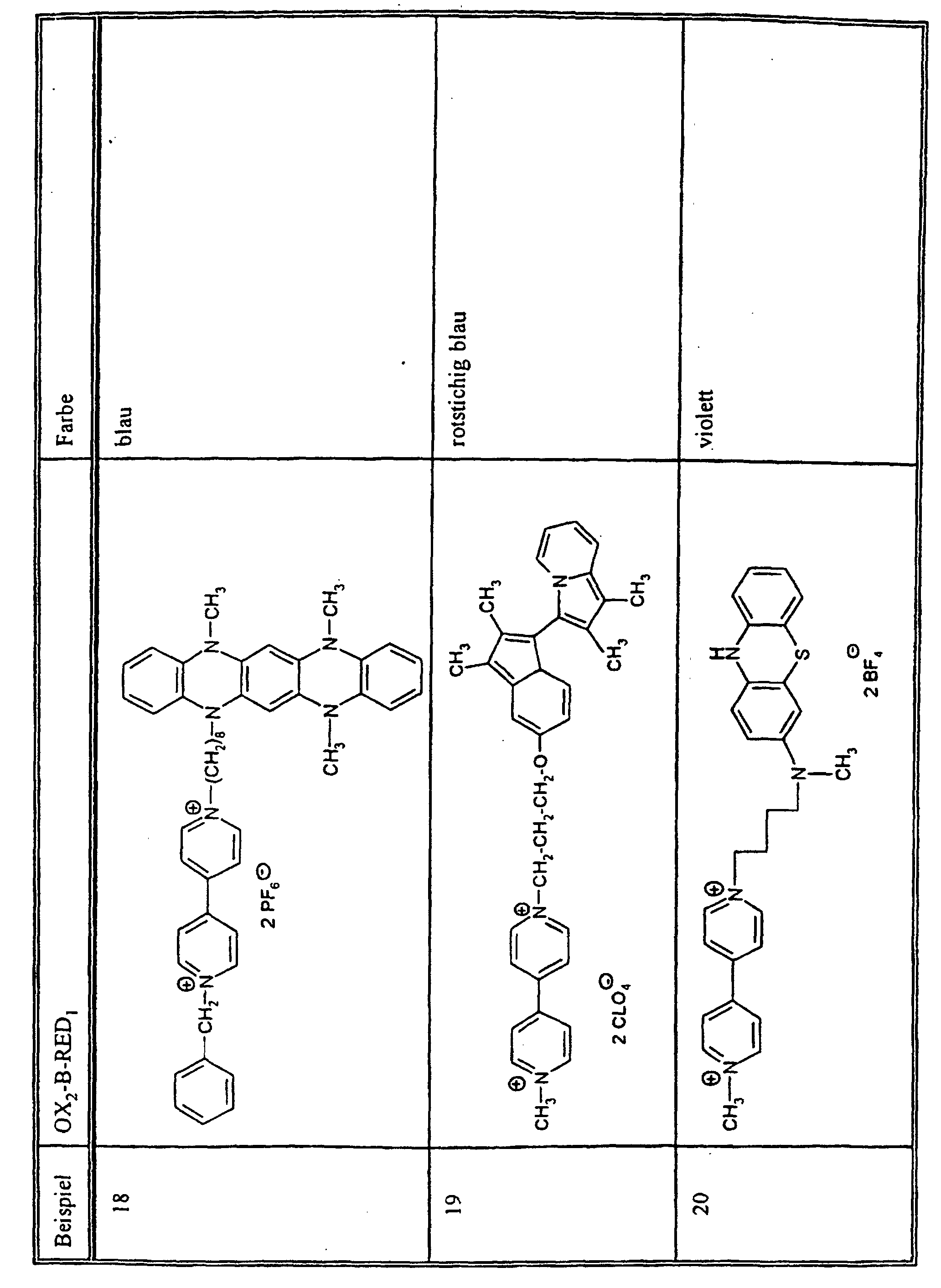

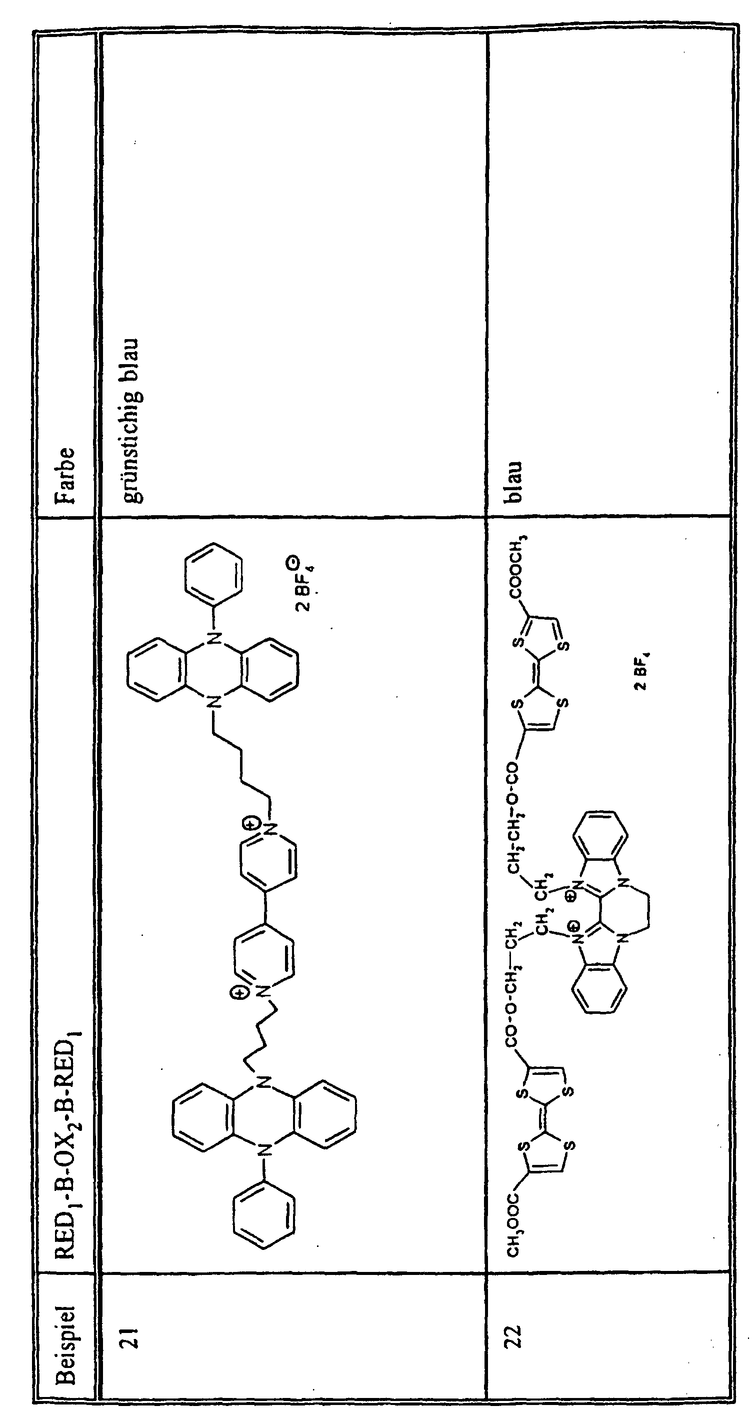

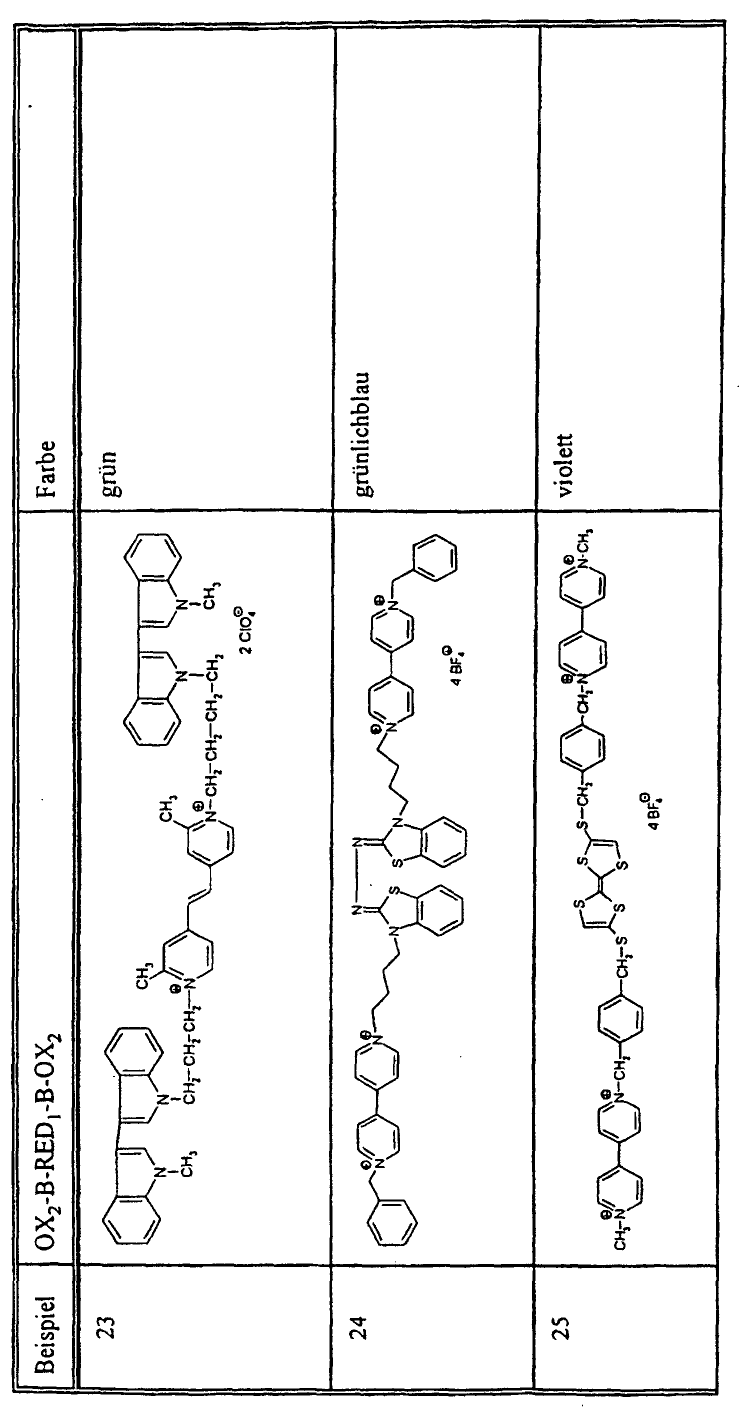

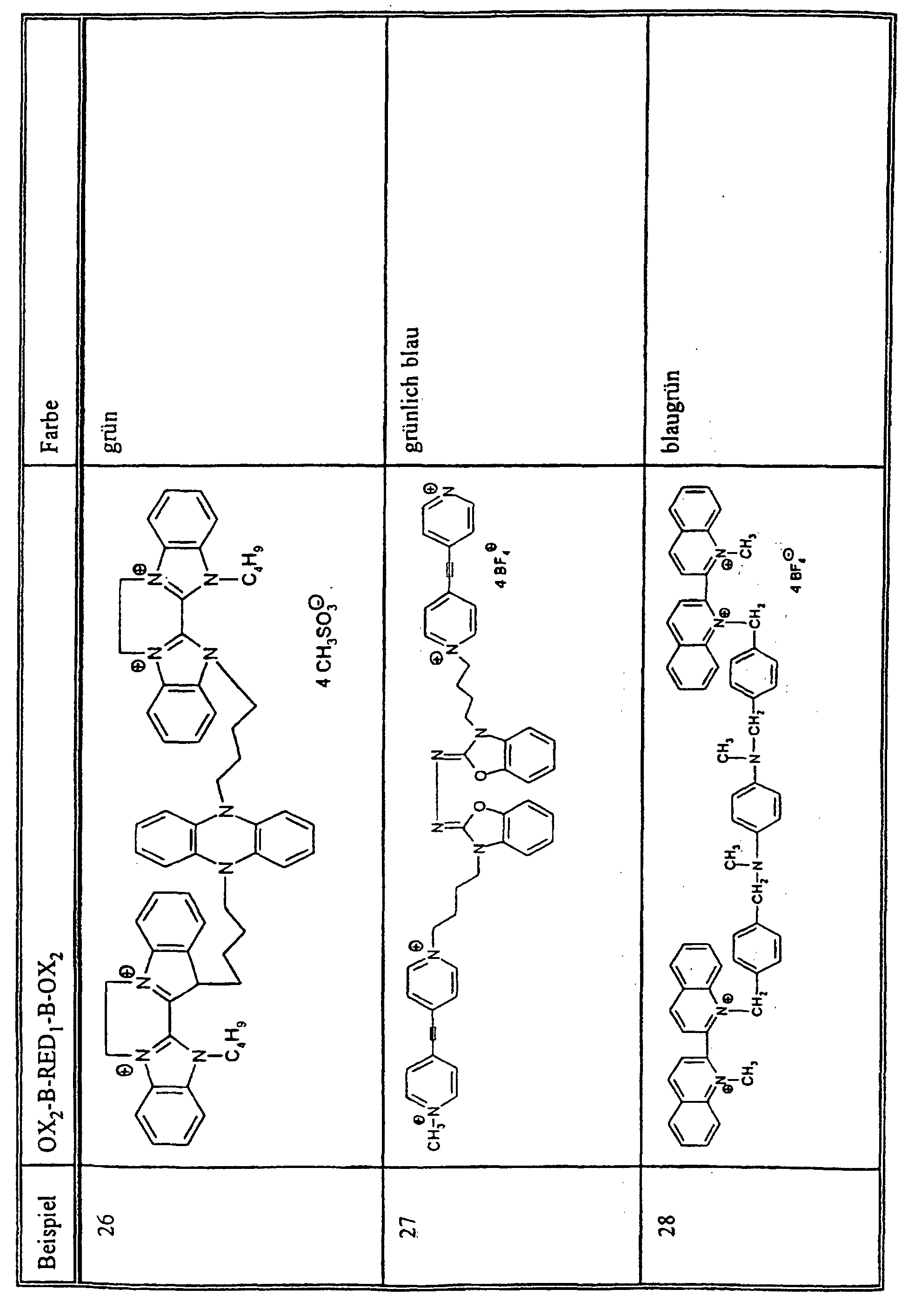

### Beispiel 29

Es wurde eine Zelle aus zwei Indium-Zinn-Oxid (ITO)-beschichteten Glasplatten und einem Dichtungsring gebaut, wie sie in US-P 4,902,108 beschrieben ist. Sie wurde unter Stickstoffatmosphäre über eine Öffnung in dem Dichtungsring befüllt mit einer Lösung, die 0,03 molar an der elektrochromen Substanz der Formel (LXXIV) mit X^{e} = BF₄^{e} gemäß Beispiel 3, in wasserfreiem Glutarsäuredinitril war. Die Zelle wurde luftdicht verschlossen. Die Lösung in der Zelle war blaßgelb. Nach Anlegen einer Spannung von 1.5 V färbte sich die Lösung rasch intensiv grün, nach Abschalten der Spannung entfärbte sich der Zellinhalt innerhalb von 1 min wieder vollständig. Durch Kurzschließen der Zelle erfolgte die Entfärbung rascher.

Nach 9 000 Färbe-/Entfärbe-Zyklen arbeitete die Zelle noch einwandfrei.

### Beispiel 30

Es wurde wie in Beispiel 4 eine Zelle gebaut. Eine der Glasplatten war jedoch auf der der ITO-Schicht abgewandten Seite verspiegelt.

Sie wurde unter N₂-Atmosphäre befüllt mit einer Lösung, die 0,03 molar an der elektrochromen Substanz der Formel (LXIX) mit X⊖ = BF₄⊖ gemäß Beispiel 2 in wasserfreiem Propylencarbonat war. Die Farbe der Lösung in der Zelle war blaßgelb. Nach Anlegen einer Spannung von 0,9 V färbte sie sich die Lösung rasch tief grünlich blau, nach Abschalten der Stromzufuhr und Kurzschließen entfärbte sich der Zellinhalt innerhalb von ca. 10 s wieder und resultierte in dem ursprünglichen Blaßgelb. Mehr als 30 000 solcher Schaltcyclen wurden ohne irgendwelche Veränderungen überstanden.

Ganz analog zu den Beispielen 29-30 wurden unter Verwendung der in den Beispielen 1-28 aufgeführten elektrochromen Substanzen elektrochrome Zellen aufgebaut, wobei ähnlich gute Ergebnisse erzielt wurden.

## Patentansprüche

1. Elektrochromes System, enthaltend mindestens eine oxidierbare Substanz RED₁, die durch Elektronenabgabe an einer Anode und mindestens eine reduzierbare Substanz OX₂, die durch Elektronenaufnahme an einer Kathode, jeweils unter Zunahme der Extinktion im sichtbaren Bereich des Spektrums von einer schwach gefärbten oder farblosen Form in eine gefärbte Form OX₁ bzw. RED₂ übergeht, wobei nach Ladungsausgleich jeweils die schwach gefärbte bzw. farblose Form zurückgebildet wird, **dadurch gekennzeichnet, daß** mindestens eine der enthaltenen Substanzen RED₁ und OX₂ über eine Brücke kovalent miteinander verknüpft sind.

2. Elektochromes System gemäß Anspruch 1, **dadurch gekennzeichnet, daß** es mindestens eine elektrochrome Substanz der Formel (I) 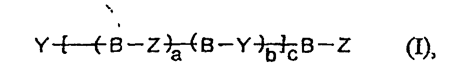 worin
Y und Z unabhängig voneinander für einen Rest OX₂ oder RED₁ stehen, wobei aber mindestens ein Y für OX₂ und mindestens ein Z für RED₁ steht,
wobei
OX₂ für den Rest eines reversibel elektrochemisch reduzierbaren Redoxsystems steht, und
RED₁ für den Rest eines reversibel elektrochemisch oxidierbaren Redoxsystems steht,
B für ein Brückenglied steht,
c für eine ganze Zahl von 0 bis 1 steht, und
a und b unabhängig voneinander für eine ganze Zahl von 0 bis 5 stehen, enthält.

3. Elektrochromes System gemäß Anspruch 1 und 2, **dadurch gekennzeichnet, daß** es mindestens eine elektrochrome Substanz der Formel (I), worin Y für OX₂ und Z für RED₁ steht und Y und Z in ihrer Reihenfolge alternieren enthält.

4. Elektrochromes System gemäß Ansprüchen 1 bis 3, **dadurch gekennzeichnet, daß** es mindestens eine elektrochrome Substanz der Formeln
OX₂-B-RED₁ (Ia),
OX₂-B-RED₁-B-OX₂ (Ib),
RED₁-B-OX₂-B-RED₁ (Ic),
oder
OX₂-(B-RED₁-B-OX₂)_{d}-B-RED₁ (Id),
worin
OX₂, RED₁ und B die oben angegebene Bedeutung haben, und
d für eine ganze Zahl von 1 bis 5 steht,
enthält.

5. Elektrochromes System gemäß Ansprüchen 1 bis 4, **dadurch gekennzeichnet, daß** es mindestens eine elektrochrome Substanz der Formeln (Ia)-(Id),
worin
OX₂ für den Rest einer kathodisch reduzierbaren Substanz steht, die im cyclischen Voltammogramm, aufgenommen in einem inerten Lösungsmittel bei Raumtemperatur, wenigstens zwei chemisch reversible Reduktionswellen zeigt, wobei die erste dieser Reduktionswellen zu einer Zunahme der Extinktion bei wenigstens einer Wellenlänge im sichtbaren Bereich des elektromagnetischen Spektrums führt,
RED₁ für den Rest der anodisch reversibel oxidierbaren Substanz steht, die im cyclischen Voltammogramm, aufgenommen in einem inerten Lösungsmittel bei Raumtemperatur, wenigstens zwei chemisch reversible Oxidationswellen zeigt, wobei die erste dieser OxidationsweUen zu einer Zunahme der Extinktion bei wenigstens einer Wellenlänge im sichtbaren Bereich des elektromagnetischen Spektrums führt, und
B für ein Brückenglied steht,
enthält.

6. Elektrochromes System gemäß Ansprüchen 1 bis 5, **dadurch gekennzeichnet, daß** es mindestens eine Substanz der Formel (Ia)-(Id) enthält, worin
OX₂ für einen Rest der Formeln 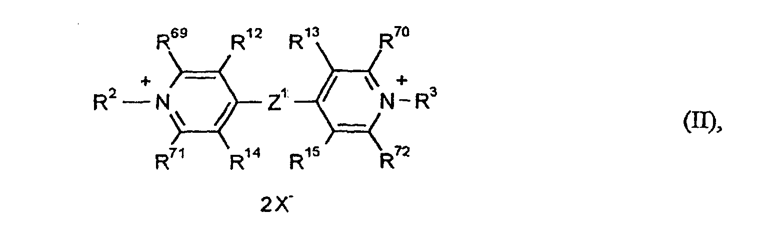 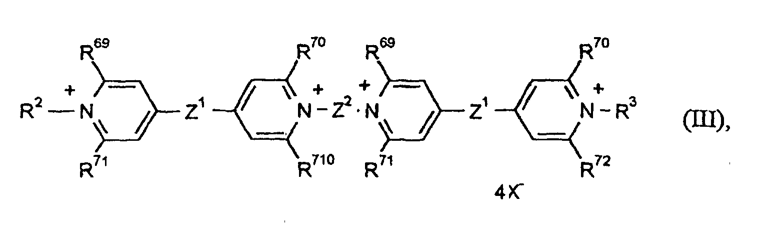 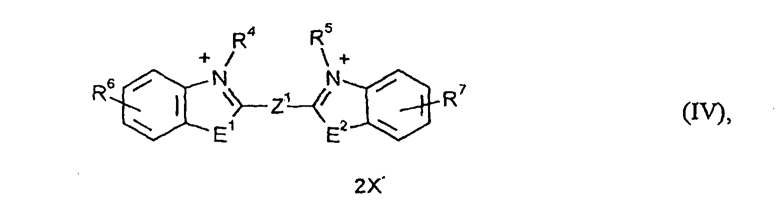 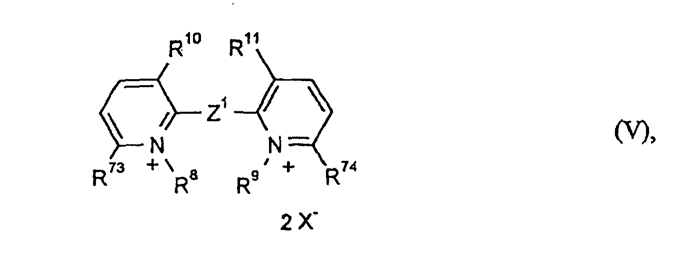 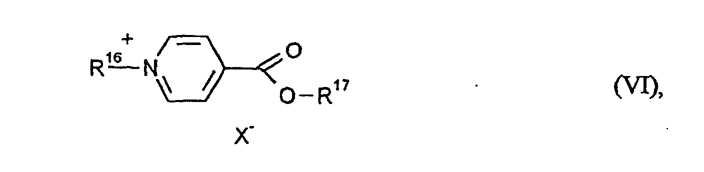 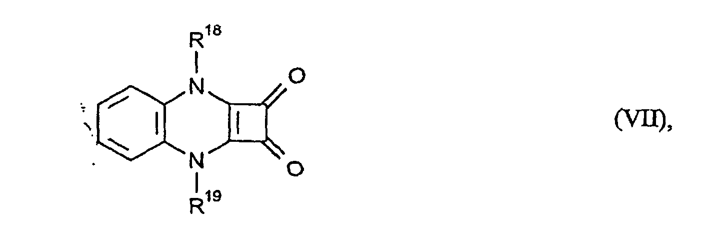 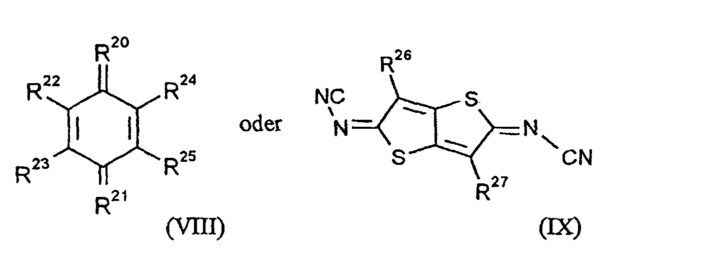
steht, wobei
R² bis R⁵, R⁸ , R⁹, R¹⁶ bis R¹⁹ unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten oder
R⁴ und R⁵ oder R⁸ und R⁹ gemeinsam eine -(CH₂)₂- oder -(CH)₃-Brücke bilden,
R⁶, R⁷ und R²² bis R²⁵ unabhängig voneinander Wasserstoff, C₁- bis C₄-Alkyl, C₁- bis C₄-Alkoxy, Halogen, Cyano, Nitro oder C₁- bis C₄-Alkoxycarbonyl bedeuten oder
R²² und R²³ und/oder R²⁴ und R²⁵ eine -CH=CH-CH=CH-Brücke bilden,
R¹⁰ und R¹¹; R¹² und R¹³; R¹⁴ und R¹⁵ unabhängig voneinander Wasserstoff oder paarweise eine -(CH₂)₂-, -(CH₂)₃- oder -CH=CH- Brücke bedeuten,
R⁶⁹ bis R⁷⁴ unabhängig voneinander Wasserstoff oder C₁-C₆-Alkyl bedeuten, oder
R⁶⁹; R¹² und/oder R⁷⁰; R¹³ eine -CH=CH-CH=CH-Brücke bilden,
R²⁰ und R²¹ unabhängig voneinander O, N-CN, C(CN)₂ oder N-C₆- bis C₁₀-Aryl bedeuten,
R²⁶ Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeutet, E¹ und E² unabhängig voneinander O, S, NR¹ oder C(CH₃)₂ bedeuten oder E¹ und E² gemeinsam eine -N-(CH₂)₂-N-Brücke bilden,
R¹ C₁- bis C₁₈-Alkyl, C₂- bis C₁₂-Alkenyl, C₄- bis C₇-Cycloalkyl, C₇- bis C₁₅-Aralkyl, C₆- bis C₁₀-Aryl bedeutet,
Z¹ eine direkte Bindung, -CH=CH-, -C(CH₃)=CH-, -C(CN)=CH-, -CCl=CCl-, -C(OH)=CH-, -CCl=CH-, -C≡C-, -CH=N-N=CH-, -C(CH₃)=N-N=C(CH₃)- oder -CCl=N-N=CCl- bedeutet,
Z² -(CH₂)ᵣ- oder -CH₂-C₆H₄-CH₂- bedeutet,
r eine ganze Zahl von 1 bis 10 bedeutet und
X⁻ ein unter den Bedingungen redox-inertes Anion bedeutet,
wobei die Bindung zum Brückenglied B über einen der Reste R²-R¹⁹, R²²-R²⁷ oder im Falle, daß E¹ oder E² für NR¹ steht über R¹ erfolgt und die genannten Reste dann für eine direkte Bindung stehen,
RED₁ für einen der folgenden Reste 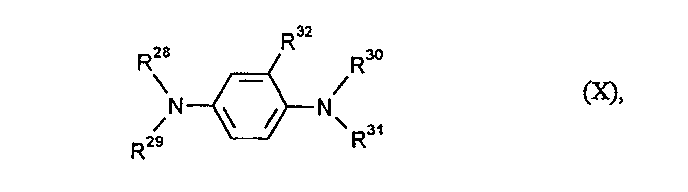 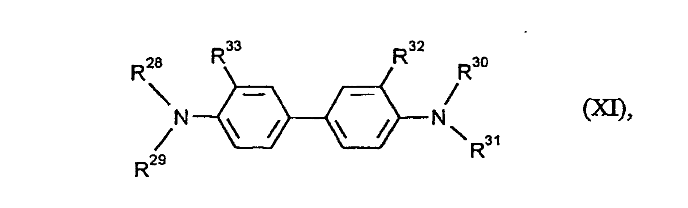 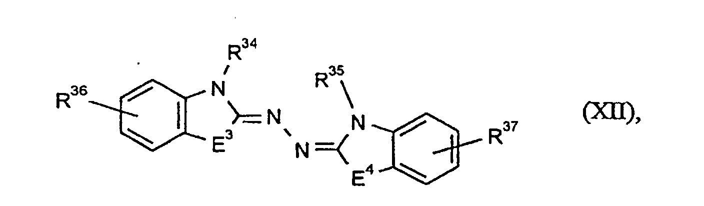 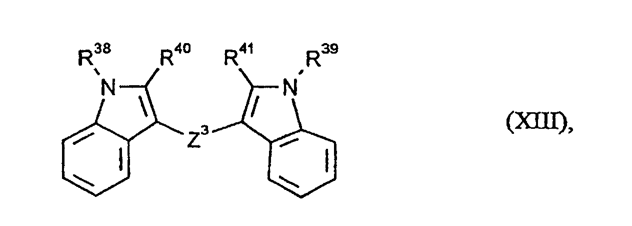 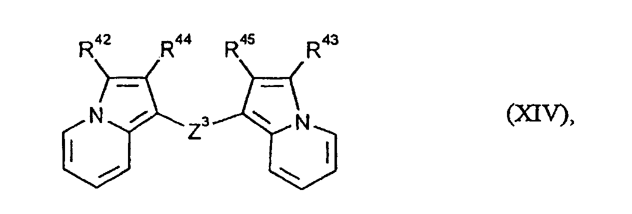 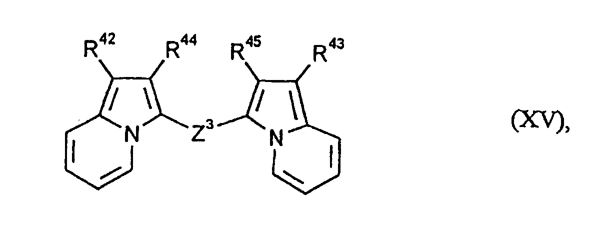 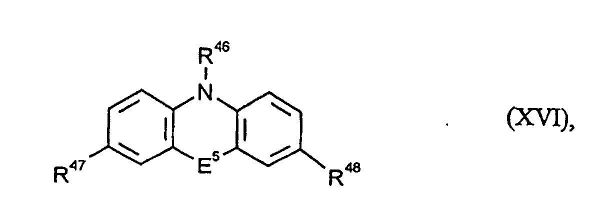 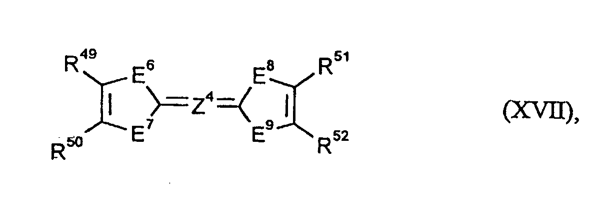 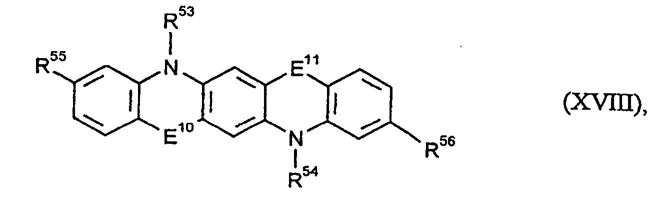 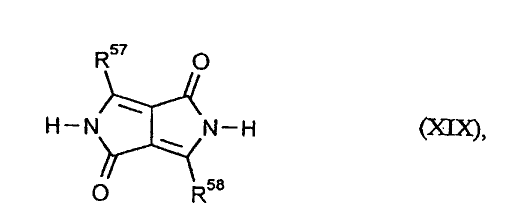 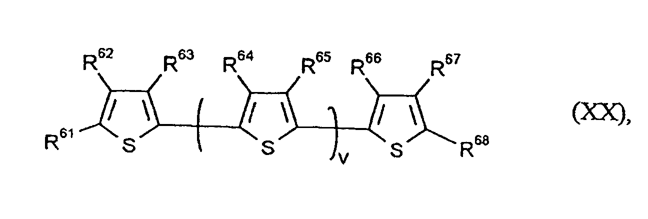
steht, worin
R²⁸ bis R³¹, R³⁴, R³⁵, R³⁸, R³⁹, R⁴⁶, R⁵³ und R⁵⁴ unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten, und R⁴⁶, R⁵³ und R⁵⁴ zusätzlich Wasserstoff bedeuten,
R³², R³³, R³⁶, R³⁷, R⁴⁰, R⁴¹, R⁴² bis R⁴⁵, R⁴⁷, R⁴⁸, R⁴⁹ bis R⁵² und R⁵⁵ bis R⁵⁷ unabhängig voneinander Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeuten und R⁵⁷ und R⁵⁸ zusätzlich einen gegebenenfalls benzanellierten aromatischen oder quasiaromatischen fünf- oder sechsgliedrigen heterocyclischen Ring bedeuten und R⁴⁸ zusätzlich NR⁷⁵R⁷⁶ bedeutet,
R⁴⁹ und R⁵⁰ und/oder R⁵¹ und R⁵² eine -(CH₂)₃-, -(CH₂)₄-, -(CH₂)₅- oder -CH=CH-CH=CH-Brücke bilden,
Z³ eine direkte Bindung, eine -CH=CH- oder -N=N-Brücke bedeutet,
=Z⁴= eine direkte Doppelbindung, eine =CH-CH= oder =N-N=-Brücke bedeutet,
E³ bis E⁵, E¹⁰ und E¹¹ unabhängig voneinander O, S, NR⁵⁹ oder C(CH₃)₂ bedeuten und E⁵ zusätzlich C = O oder SO₂ bedeutet, oder
E³ und E⁴ unabhängig voneinander -CH=CH- bedeuten,
E⁶ bis E⁹ unabhängig voneinander S, Se oder NR⁵⁹ bedeuten,
R⁵⁹, R⁷⁵ und R⁷⁶ unabhängig voneinander C₁- bis C₁₂-Alkyl, C₂- bis C₈-Alkenyl, C₃- bis C₇-Cycloalkyl, C₇- bis C₁₅-Aralkyl oder C₆- bis C₁₀-Aryl bedeuten und R⁷³ zusätzlich Wasserstoff bedeutet oder
R⁷³ und R⁷⁴ in der Bedeutung von NR⁷³R⁷⁴ gemeinsam mit dem N-Atom, an das sie gebunden sind, einen fünf- oder sechsgliedrigen, gesättigten Ring bedeuten, der weitere Heteroatome enthalten kann,
R⁶¹ bis R⁶⁸ unabhängig voneinander Wasserstoff, C₁- bis C₆-Alkyl, C₁- bis C₄-Alkoxy, Cyano, C₁- bis C₄-Alkoxycarbonyl oder C₆- bis C₁₀-Aryl bedeuten, oder
R⁶¹; R⁶² und R⁶⁷; R⁶⁸ unabhängig voneinander gemeinsam eine -(CH₂)₃-, -(CH₂)₄- oder -CH=CH-CH=CH-Brücke bilden,
v eine ganze Zahl zwischen 0 und 10 bedeutet,
wobei die Bindung zum Brückenglied B einen der Reste R²⁸-R⁵⁸, R⁶¹, R⁶², R⁶⁷, R⁶⁸ oder im Falle, daß einer der Reste E³-E¹¹ für NR⁵⁹ steht über R⁵⁹ erfolgt und die genannten Reste dann für eine direkte Bindung stehen, und
B für ein Brückenglied der Formeln -(CH₂)ₙ- oder -[Y¹ₛ(CH₂)ₘ-Y²]ₒ-(CH₂)ₚ-Y³_{q}- steht, welche jeweils gegebenenfalls durch C₁- bis C₄-Alkoxy, Halogen oder Phenyl substituiert sind,
Y¹ bis Y³ unabhängig voneinander für O, S, NR⁶⁰, COO, CONH, NHCONH, Cyclopentandiyl, Cyclohexandiyl, Phenylen oder Naphthylen stehen,
R⁶⁰ C₁- bis C₆-Alkyl, C₂- bis C₆-Alkenyl, C₄- bis C₇-Cycloalkyl, C₇- bis C₁₅-Aralkyl oder C₆- bis C₁₀-Aryl bedeutet,
n eine ganze Zahl von 1 bis 12 bedeutet,
m und p unabhängig voneinander eine ganze Zahl von 0 bis 8 bedeuten,
o eine ganze Zahl von 0 bis 6 bedeutet und
q und s unabhängig voneinander 0 oder 1 bedeuten.

7. Elektrochromes System gemäß Ansprüchen 1 bis 6, **dadurch gekennzeichnet, daß** es mindestens eine Substanz der Formel (Ia)-(Id) enthält,
worin
OX₂ für einen Rest der Formeln (II), (III), (IV) oder (V) steht,
wobei
R², R³, R⁴, R⁵, R⁸ und R⁹ unabhängig voneinander für C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl stehen,
R⁶ und R⁷ unabhängig voneinander für Wasserstoff, Methyl, Ethyl, Methoxy, Ethoxy, Fluor, Chlor, Brom, Cyano, Nitro, Methoxycarbonyl oder Ethoxycarbonyl stehen,
R¹⁰, R¹¹; R¹², R¹³ und R¹⁴, R¹⁵ unabhängig voneinander für Wasserstoff oder, falls Z¹ eine direkte Bindung bedeutet, jeweils paarweise gemeinsam für eine -(CH₂)₂-, -(CH₂)₃- oder -CH=CH-Brücke stehen,
oder
R⁴, R⁵ und R⁸, R⁹ unabhängig voneinander jeweils paarweise gemeinsam für -(CH₂)₂- oder -(CH₃)₃-Brücke stehen, falls Z¹ eine direkte Bindung bedeutet,
R⁶⁹ bis R⁷⁴ unabhängig voneinander Wasserstoff oder C₁-C₄-Alkyl bedeuten,
E¹ und E² gleich sind und für O, S, NR¹ oder C(CH₃)₂ stehen oder gemeinsam eine -N-(CH₂)₂-N-Brücke bilden,
R¹ für C₁- bis C₁₂-Alkyl, C₂- bis C₄-Alkenyl, C₅- bis C₇-Cycloalkyl, C₇- bis C₁₅-Aralkyl oder C₆- bis C₁₀-Aryl steht,
Z¹ für eine direkte Bindung, -CH=CH-, -C(CH₃)=CH-, -C(CN)=CH-, -C≡C- oder -CH=N-N=CH- steht,
Z² für -(CH)ᵣ- oder -CH₂-C₆H₄-CH₂- steht,
r für eine ganze Zahl zwischen 1 und 6 steht,
X⁻ für ein unter den Bedingungen redox-inertes, farbloses Anion steht,
wobei die Bindung zum Brückenglied B über einen der Reste R²-R¹¹ oder im Falle, daß E¹ oder E² für NR¹ steht über R¹ erfolgt und die genannten Reste dann für eine direkte Bindung stehen,
RED₁ für einen Rest der Formeln (X), (XI), (XII), (XIII), (XVI), (XVII), (XVIII) oder (XX) steht,
wobei
R²⁸ bis R³¹, R³⁴, R³⁵, R³⁸, R³⁹, R⁴⁶, R⁵³ und R⁵⁴ unabhängig voneinander C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl bedeuten und
R⁴⁶, R⁵³ und R⁵⁴ zusätzlich Wasserstoff bedeuten,
R³², R³³, R³⁶, R³⁷, R⁴⁰, R⁴¹, R⁴⁷ bis R⁵², R⁵⁵ und R⁵⁶ unabhängig voneinander Wasserstoff, Methyl, Ethyl, Methoxy, Ethoxy, Fluor, Chlor, Brom, Cyan, Nitro, Methoxycarbonyl, Ethoxycarbonyl oder Phenyl bedeuten und
R⁵⁷ und R⁵⁸ zusätzlich 2- oder 4-Pyridyl bedeuten und
R⁴⁸ zusätzlich NR⁷⁵R⁷⁶ bedeutet,
Z³ eine direkte Bindung, eine -CH=CH- oder -N=N-Brücke bedeutet,
=Z⁴= eine direkte Doppelbindung, eine =CH-CH= oder =N-N=-Brücke bedeutet,
E³ bis E⁵, E¹⁰ und E¹¹ unabhängig voneinander O, S, NR⁵⁹ oder C(CH₃)₂ bedeuten, E³ und E⁴ aber die gleiche Bedeutung haben,
E⁶ bis E⁹ untereinander gleich sind und S, Se oder NR⁵⁹ bedeuten und
E⁵ zusätzlich C = O bedeutet,
E⁶ für NR⁵⁹ steht, wobei R⁵⁹ eine direkte Bindung zur Brücke B bedeutet und
E⁷ bis E⁹ die oben angegebene Bedeutung besitzen, aber untereinander nicht gleich sein müssen,
R⁵⁹, R⁷⁵ und R⁷⁶ unabhängig voneinander C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl bedeuten, und R⁷³ zusätzlich Wasserstoff bedeutet oder
R⁷³ und R⁷⁴ in der Bedeutung von NR⁷³R⁷⁴ gemeinsam mit dem N-Atorn, an das sie gebunden sind, Pyrrolidino, Piperidino oder Morpholino bedeuten,
R⁶¹, R⁶² und R⁶⁷, R⁶⁸ unabhängig voneinander für Wasserstoff, C₁- bis C₄-Alkyl, Methoxycarbonyl, Ethoxycarbonyl oder Phenyl oder paarweise gemeinsam für eine -(CH₂)₃- oder -(CH₂)₄-Brücke stehen,
R⁶³ bis R⁶⁶ für Wasserstoff stehen und
v für eine ganze Zahl von 1 bis 6 steht,
wobei die Bindung zum Brückenglied B über einen der Reste R²⁸-R⁴¹, R⁴⁶-R⁵⁶, R⁶¹, R⁶², R⁶⁷, R⁶⁸ oder im Falle, daß einer der Reste E³-E¹¹ für NR⁵⁹ steht, über R⁵⁹ erfolgt und die genannten Reste dann für eine direkte Bindung stehen,
B für ein Brückenglied der Formeln -(CH₂)ₙ-, -(CH₂)ₘ-O-(CH₂)ₚ-, -(CH)ₘ-NR⁶⁰-(CH₂)ₚ-, -(CH₂)ₘ-C₆H₄-(CH₂)ₚ-, -[O-(CH₂)ₚ]ₒ-O-, -[NR⁶⁰-(CH₂)ₚ]ₒ-NR⁶⁰-, -[C₆H₄-(CH₂)ₚ]ₒ-C₆H₄-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)-, -(CH₂)ₘ-NHCO-(CH₂)ₜ-CONH-(CH)ₚ-, -(CH₂)ₘ-NHCONH-(CH₂)ₜ-NHCONH-(CH₂)ₚ- steht,
R⁶⁰ für Methyl, Ethyl, Benzyl oder Phenyl steht,
n für eine ganze Zahl von 1 bis 10 steht,
m und p unabhängig voneinander für eine ganze Zahl von 0 bis 4 stehen,
o für eine ganze Zahl von 0 bis 2 steht und
t für eine ganze Zahl von 1 bis 6 steht.

8. Elektrochromes System gemäß Ansprüchen 1 bis 7, **dadurch gekennzeichnet, daß** es mindestens eine Substanz der Formel (Ia)-(Id) enthält,
worin
OX₂ für einen Rest der Formeln (II), (IV) oder (V) steht,
wobei
R², R⁴ und R⁸ für eine direkte Bindung zum Brückenglied B stehen,
R³, R⁵ und R⁹ unabhängig voneinander für Methyl, Ethyl, Propyl, Butyl, Pentyl, Hexyl, Heptyl, Octyl, Benzyl oder Phenyl stehen, oder R³, R⁵ und R⁹ im Falle der Formeln Ic und Id auch für eine direkte Bindung zum Brückenglied B stehen,
R⁶ und R⁷ gleich sind und für Wasserstoff, Methyl, Methoxy, Chlor, Cyano oder Methoxycarbonyl stehen,
R¹⁰, R¹¹; R¹², R¹³ und R¹⁴, R¹⁵ unabhängig voneinander für Wasserstoff oder, falls Z¹ eine direkte Bindung bedeutet, jeweils paarweise zusammen für eine -CH=CH-Brücke stehen,
R⁶⁹ bis R⁷² gleich sind und Wasserstoff, Methyl oder Ethyl bedeuten,
R⁷³ und R⁷⁴ Wasserstoff bedeuten,
E¹ und E² gleich sind und für O oder S stehen,
Z¹ für eine direkte Bindung oder -CH=CH- steht,
X⁻ für ein unter den Bedingungen redox-inertes, farbloses Anion steht;
RED₁ für einen Rest der Formeln (X), (XII), (XIII), (XVI) oder (XVII) steht,
R²⁸, R³⁴, R³⁸, R⁴⁶ und R⁴⁹ für eine direkte Bindung zum Brückenglied B stehen,
R²⁹ bis R³¹, R³⁵ und R³⁹ unabhängig voneinander für Methyl, Ethyl, Propyl, Butyl, Pentyl, Hexyl, Heptyl, Octyl, Benzyl oder Phenyl stehen, oder
R³⁰, R³⁵ und R³⁹ im Falle der Formeln Ib und Ic auch für eine direkte Bindung zum Brückenglied B stehen,
R³², R⁴⁷ und R⁴⁸ für Wasserstoff stehen,
R³⁶, R³⁷, R⁴⁰, R⁴¹ und R⁵⁰ bis R⁵² unabhängig voneinander für Wasserstoff, Methyl, Methoxy, Chlor, Cyano, Methoxycarbonyl oder Phenyl stehen, oder
R⁵¹ im Falle der Formeln Ib und Id auch für eine direkte Bindung zum Brückenglied B steht,
Z³ für eine direkte Bindung, eine -CH=CH- oder -N=N-Brücke steht,
=Z⁴= für eine direkte Doppelbindung, eine =CH-CH= oder =N-N=-Brücke steht,
E³ bis E⁵ unabhängig voneinander für O, S oder NR⁵⁹ stehen, E³ und E⁴ aberdie gleiche Bedeutung haben,
E⁶ bis E⁹ untereinander gleich sind und für S, Se oder NR⁵⁹ stehen,
R⁵⁹ für Methyl, Ethyl, Propyl, Butyl, Pentyl, Hexyl, Heptyl, Octyl, Benzyl oder Phenyl steht, oder
R⁵⁹ im Falle der Formel XVI in Formel Ib und Id auch für eine direkte Bindung zum Brückenglied B steht,
B für ein Brückenglied der Formeln -(CH₂)ₙ-, -(CH₂)ₘ-O-(CH₂)ₚ-, -(CH₂)ₘ-NR⁶⁰-(CH₂)ₚ-, -(CH₂)ₘ-C₆H₄-(CH₂)ₚ-, -O-(CH₂)ₚ-O-, -NR⁶⁰-(CH₂)ₚ-NR⁶⁰-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)ₚ-, -(CH₂)ₘ-NHCO-(CH₂)ₜ-CONH-(CH₂)ₚ-, -(CH₂)ₘ-NHCONH-(CH₂)ₜ-NHCONH-(CH₂)ₚ- steht,
R⁶⁰ für Methyl steht,
n für eine ganze Zahl von 1 bis 10 steht,
m und p gleich sind und für eine ganze Zahl von 0 bis 2 stehen und
t für eine ganze Zahl von 1 bis 6 steht.

9. Elektrochromes System gemäß Ansprüchen 1 bis 8, **dadurch gekennzeichnet, daß** es mindestens eine elektrochrome Substanz der Formel (Ia)
OX₂-B-RED₁ (Ia)
entsprechend einer der Formeln 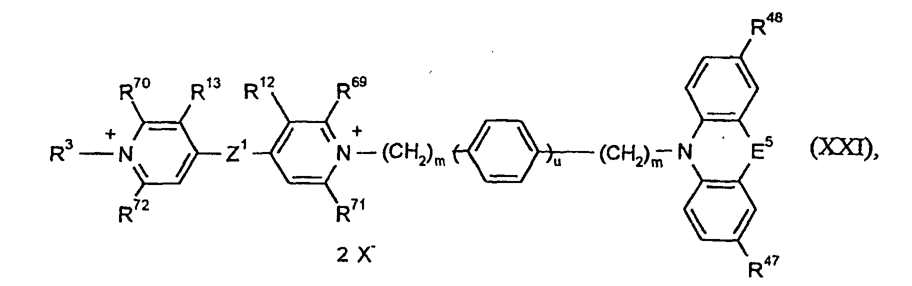 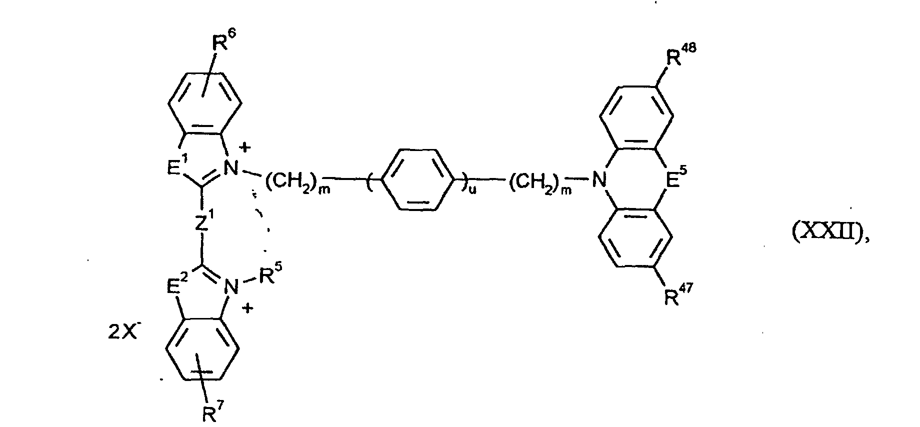 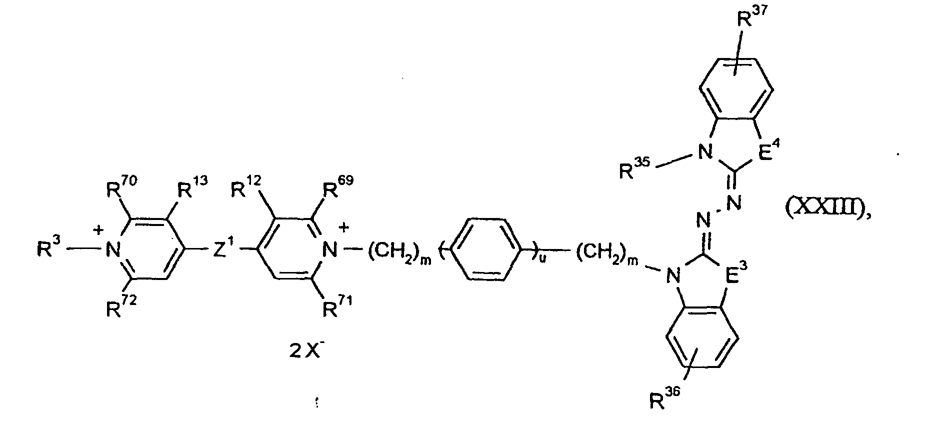 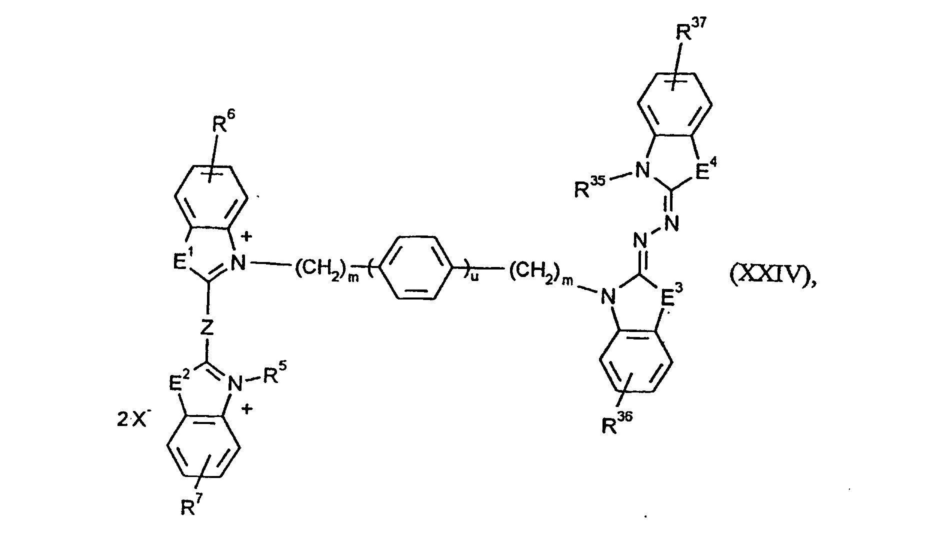 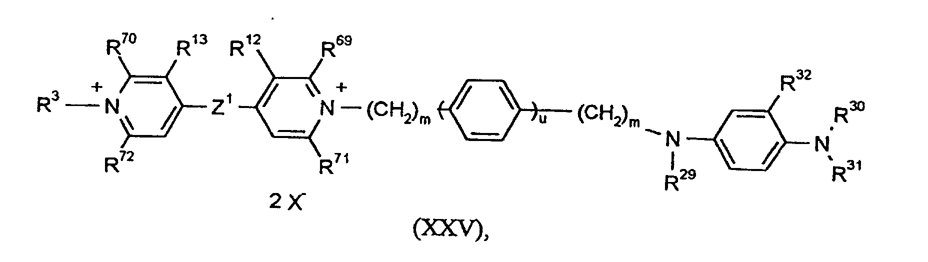 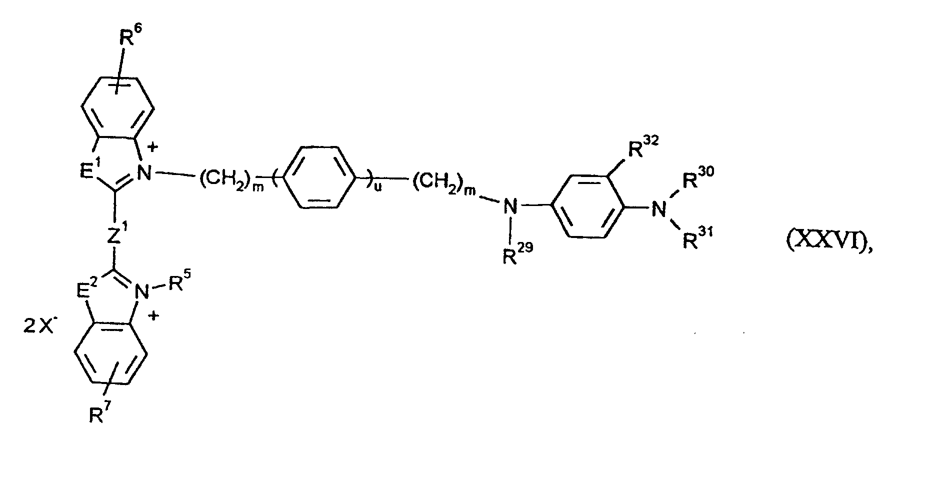 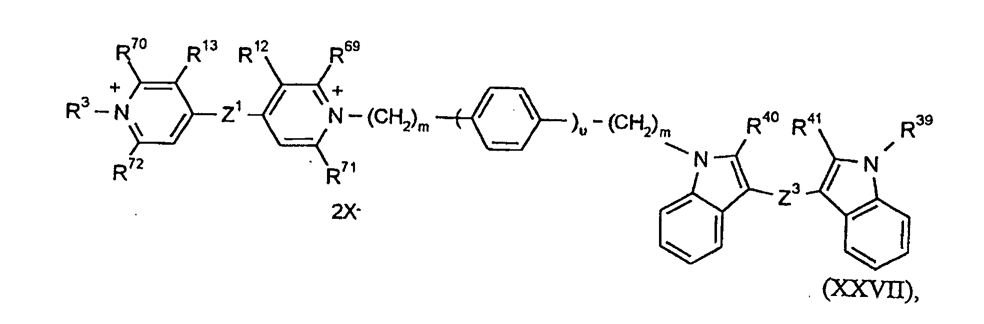 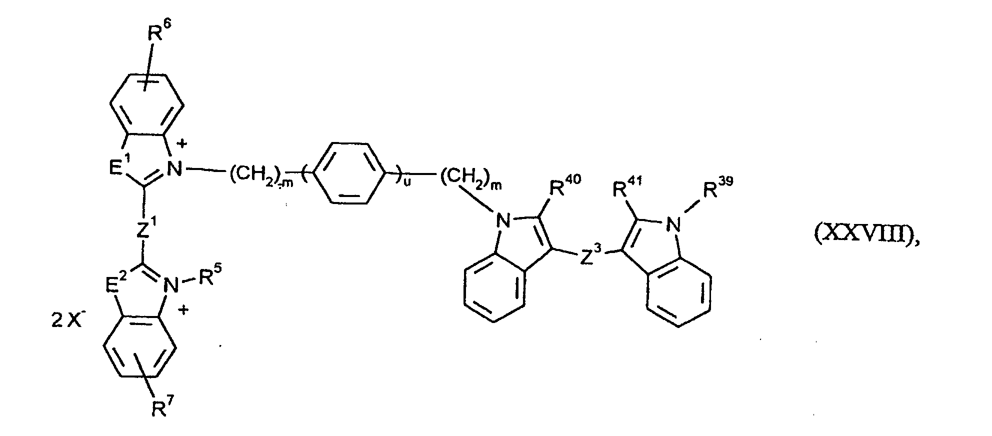 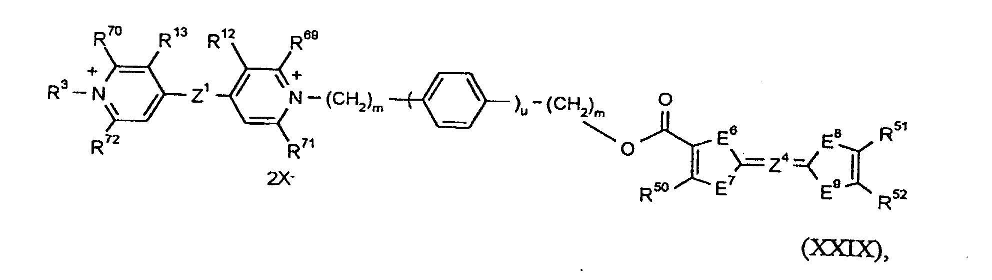 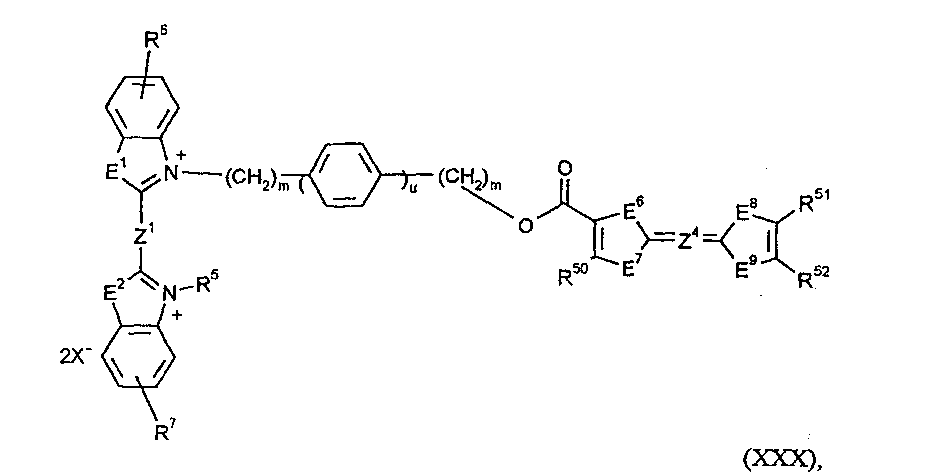 oder 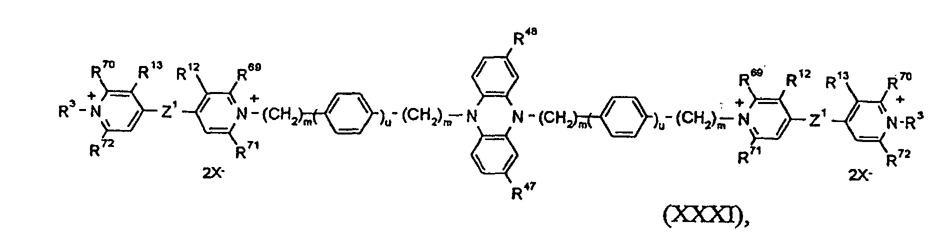 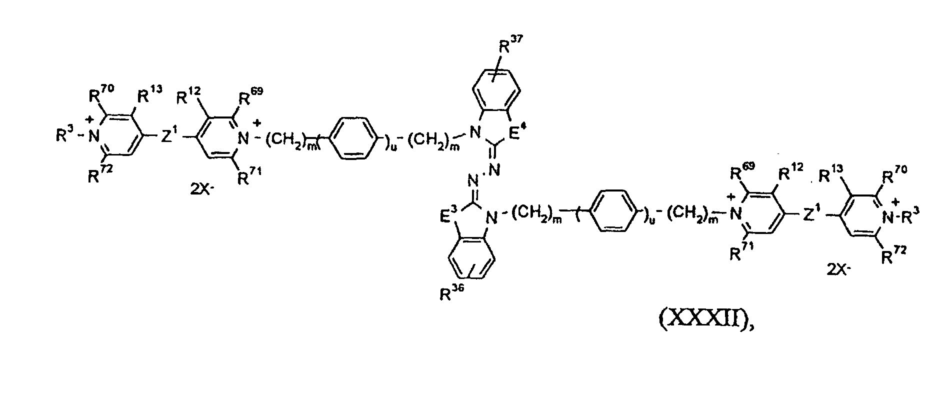 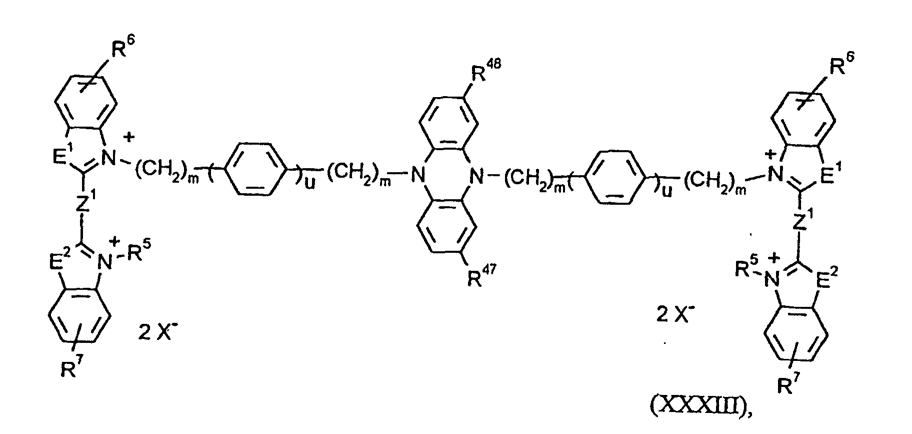 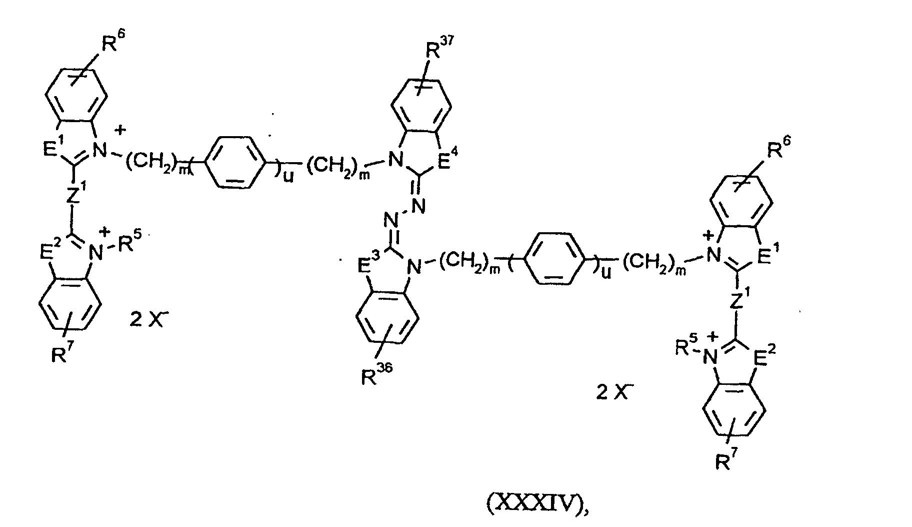 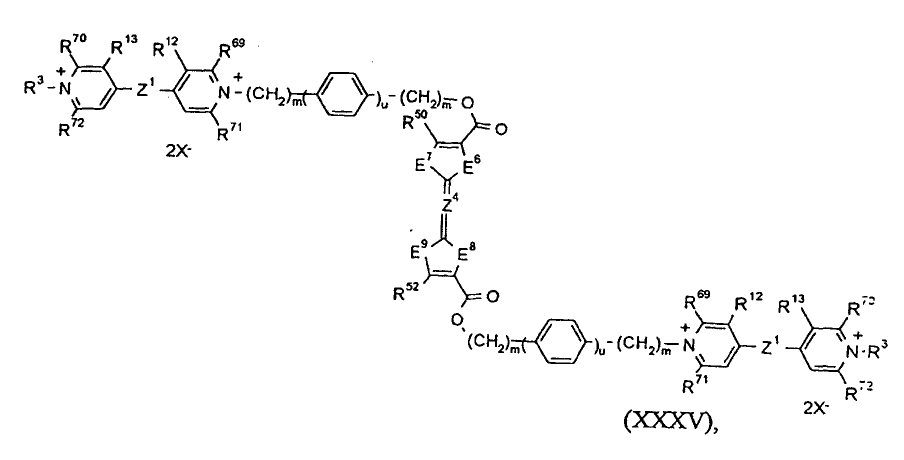 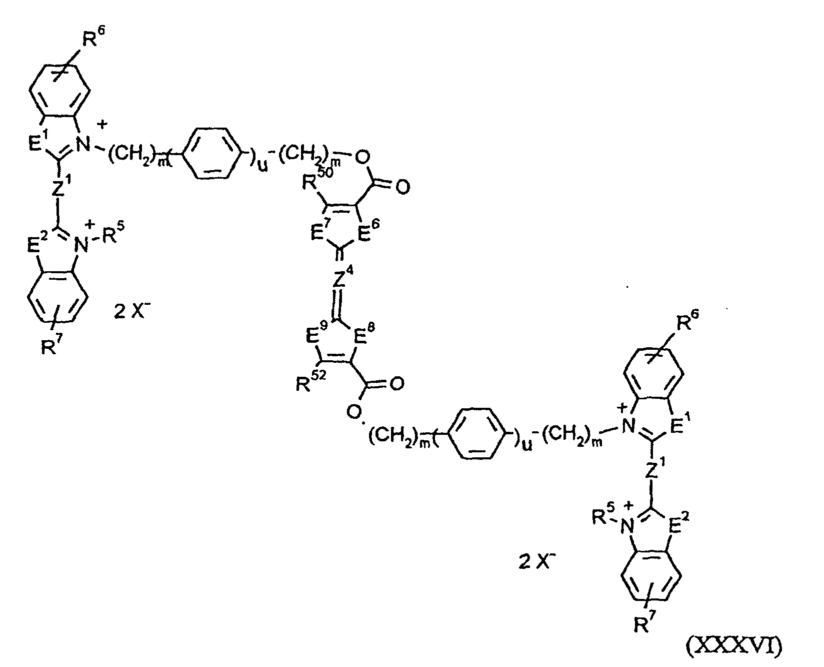 oder mindestens eine Substanz der Formel (Ic) entsprechend einer der Formeln 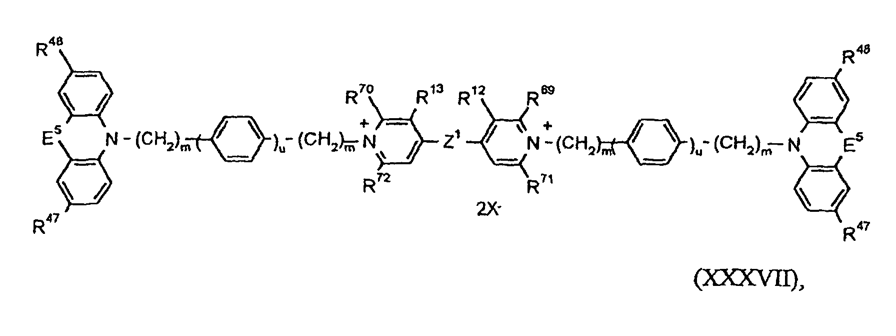 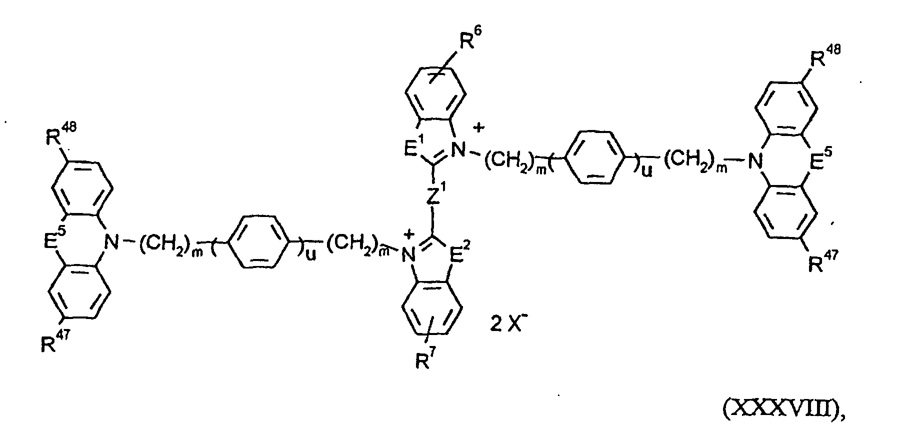 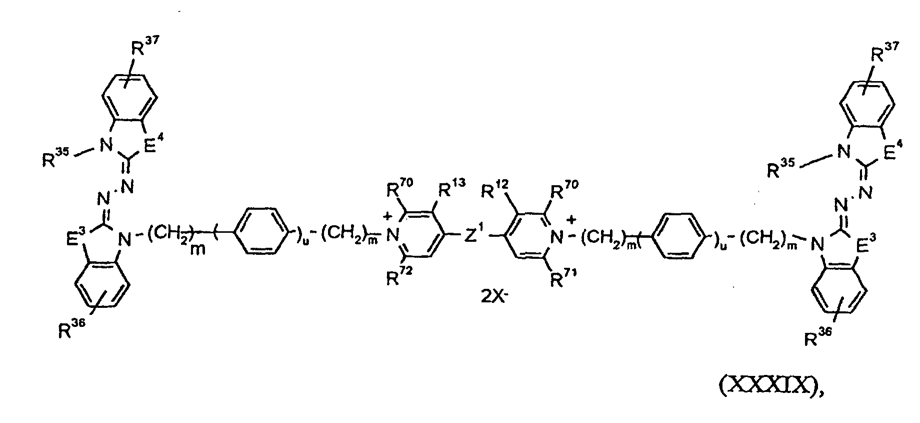 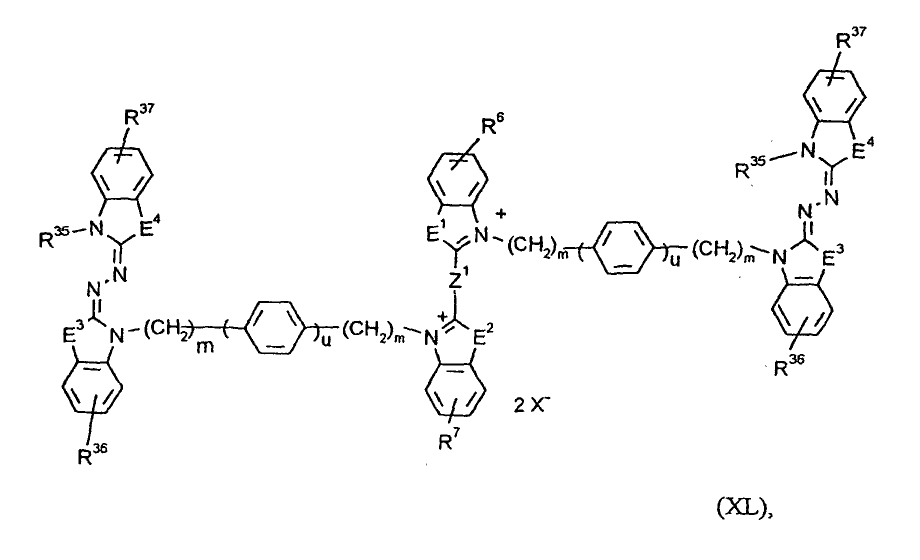 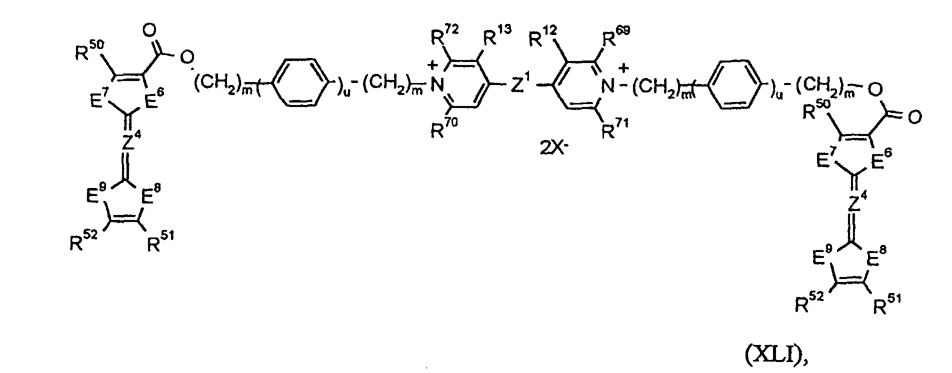 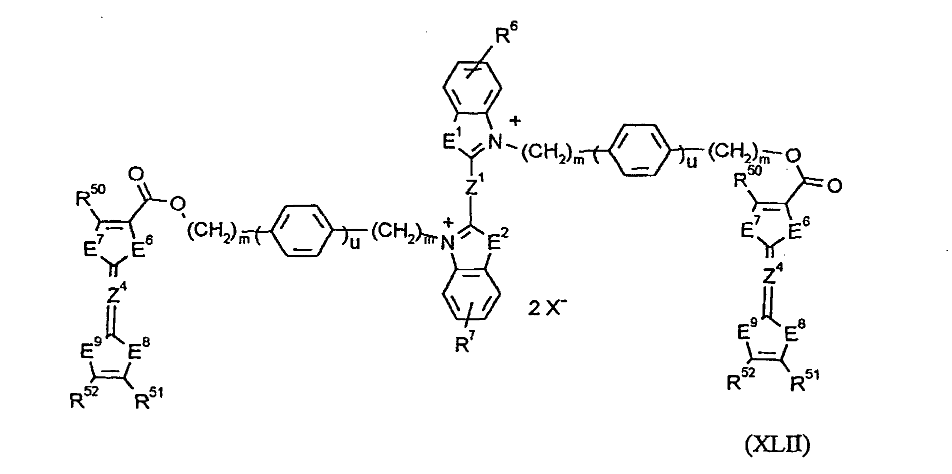 enthält, worin
R³, R⁵, R³⁵ und R³⁹ unabhängig voneinander für Methyl, Ethyl, Propyl, Butyl, Pentyl, Hexyl oder Benzyl stehen,
R⁶, R⁷ und R³⁶, R³⁷ paarweise gleich sind und für Wasserstoff, Methyl, Methoxy, Chlor, Cyano oder Methoxycarbonyl stehen,
R¹² und R¹³ für Wasserstoff oder, wenn Z¹ eine direkte Bindung bedeutet, gemeinsam für eine CH=CH-Brücke stehen,
R⁶⁹ bis R⁷² gleich sind und für Wasserstoff oder Methyl stehen,
E¹ und R² gleich sind und für O oder S stehen,
Z¹ für eine direkte Bindung oder -CH=CH steht,
R³², R⁴⁷ und R⁴⁸ für Wasserstoff stehen,
E³ bis E⁵ unabhängig voneinander für O, S oder NR⁵⁹ stehen, wobei E³ und E⁴ aber gleich sind,
R²⁹ bis R³¹ und R⁵⁹ unabhängig voneinander für Methyl, Ethyl, Propyl, Butyl, Pentyl, Hexyl oder Benzyl stehen, wobei R²⁹ bis R³¹ vorzugsweise gleich sind,
R⁴⁰ und R⁴¹ gleich sind und für Wasserstoff, Methyl, Ethyl, Propyl, Butyl oder Phenyl stehen,
Z³ für eine direkte Bindung, -CH=CH- oder -N=N- steht,
R⁵⁰ bis R⁵² unabhängig voneinander für Wasserstoff, Methyl, Methoxy, Chlor, Cyano, Methoxycarbonyl, Ethoxycarbonyl oder Phenyl stehen, vorzugsweise jedoch gleich sind,
E⁶ bis E⁹ untereinander gleich sind und für S, Se oder NR⁵⁹ stehen,
Z⁴ für eine direkte Doppelbindung, eine =CH-CH= oder =N-N=-Brücke steht,
m für eine ganze Zahl von 1 bis 5 steht,
u für 0 oder 1 steht und
X⁻ für ein unter den Bedingungen redox-inertes, farbloses Anion steht.

10. Elektrochrome Substanz entsprechend der Formel 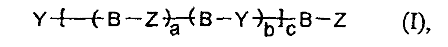 worin
Y und Z unabhängig voneinander für einen Rest OX₂ oder RED₁ stehen, wobei aber mindestens ein Y für OX₂ und mindestens ein Z für RED₁ steht,
wobei
OX₂ für den Rest eines reversibel elektrochemisch reduzierbaren Redoxsystems steht, und
RED₁ für den Rest eines reversibel elektrochemisch oxidierbaren Redoxsystems steht, wobei der Rest der Formel (XX) 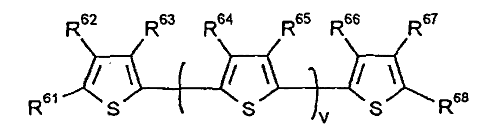 wobei R⁶¹-R⁶⁸ und v die in Anspruch 6 angegebene Bedeutung haben, ausgenommen ist,
B für ein Brückenglied steht,
c für eine ganze Zahl von 0 bis 1 steht, und
a und b unabhängig voneinander für eine ganze Zahl von 0 bis 5 stehen, vorzugsweise für eine ganze Zahl von 0 bis 3 stehen,
mit Ausnahme von Verbindungen der Formel 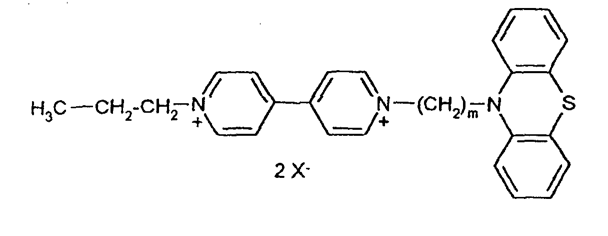 worin
m für eine ganze Zahl von 2 bis 16 steht, und
X⁻ die oben angegebene Bedeutung hat,

11. Elektrochrome Substanzen gemäß Anspruch 10 entsprechend einer der Formeln
OX₂-B-RED₁ (Ia),
OX₂-B-RED₁-B-OX₂ (Ib),
RED₁-B-OX₂-B-RED₁ (Ic),
OX₂-(B-RED₁-B-OX₂)_{d}-B-RED₁ (Id),
worin OX₂, RED₁ und B die in Anspruch 10 angegebenen Bedeutungen haben und
d für eine ganze Zahl von 1 bis 5 steht.

12. Elektrochrome Substanzen gemäß Ansprüchen 10 und 11 der Formeln 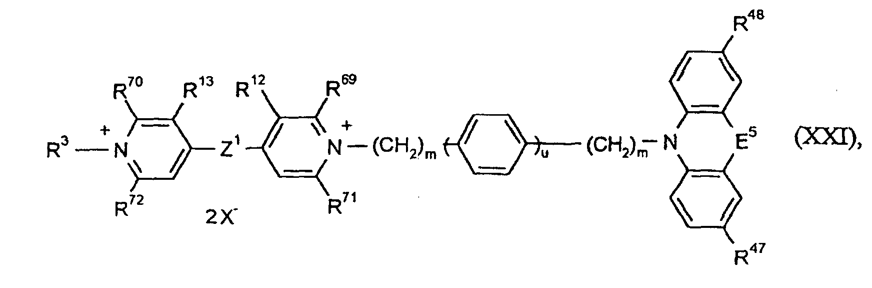 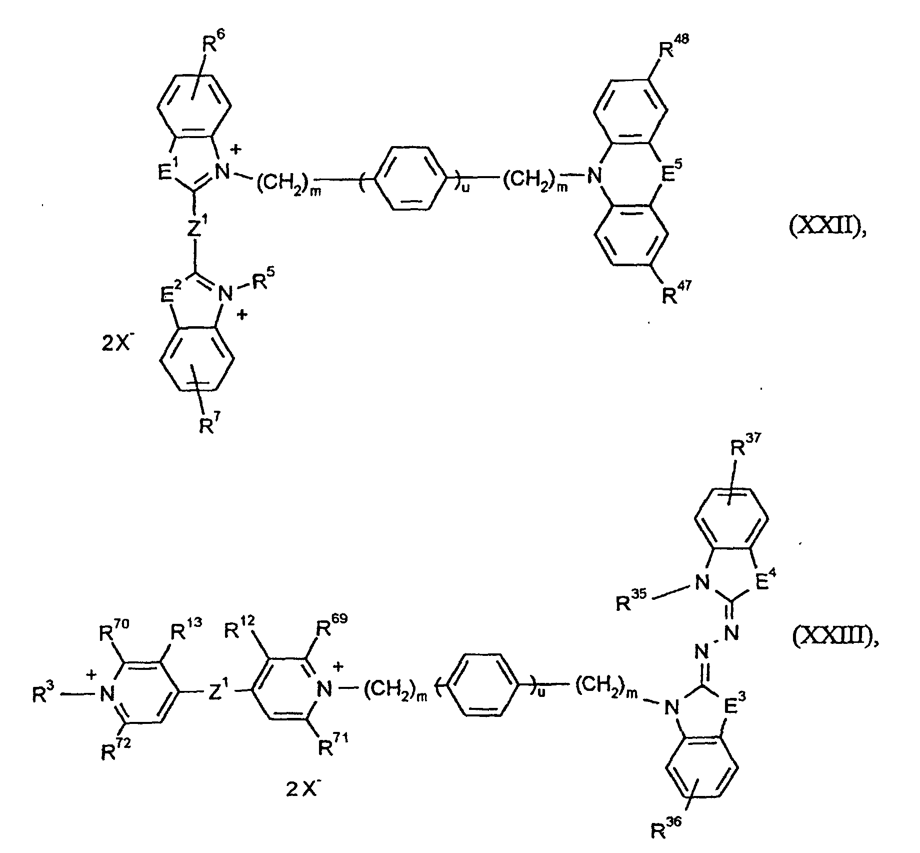 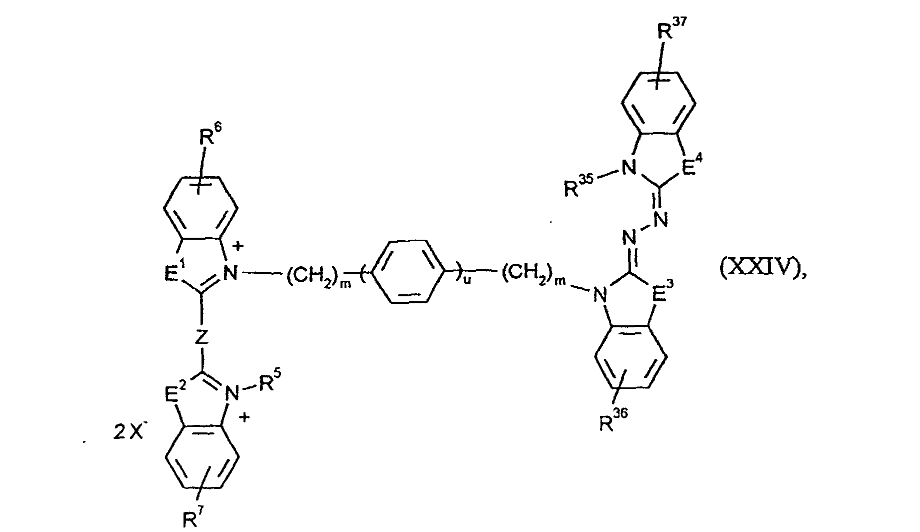 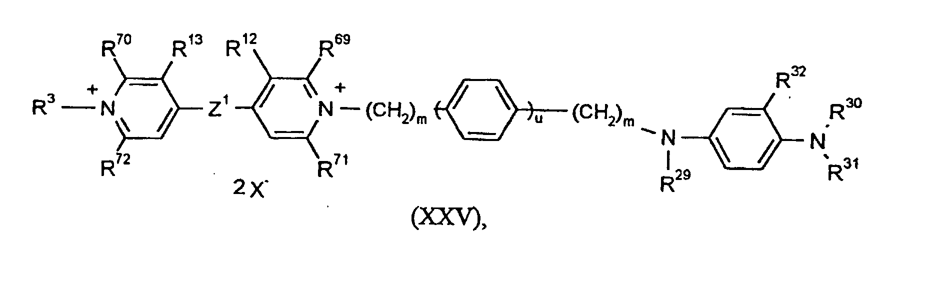 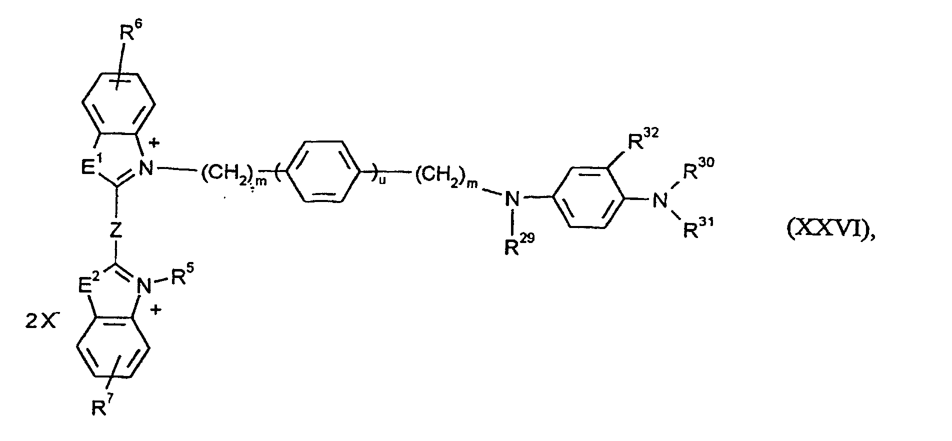 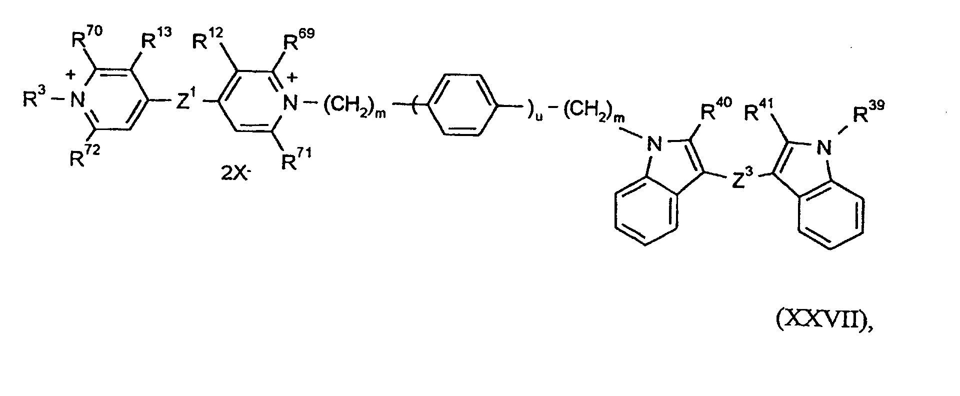 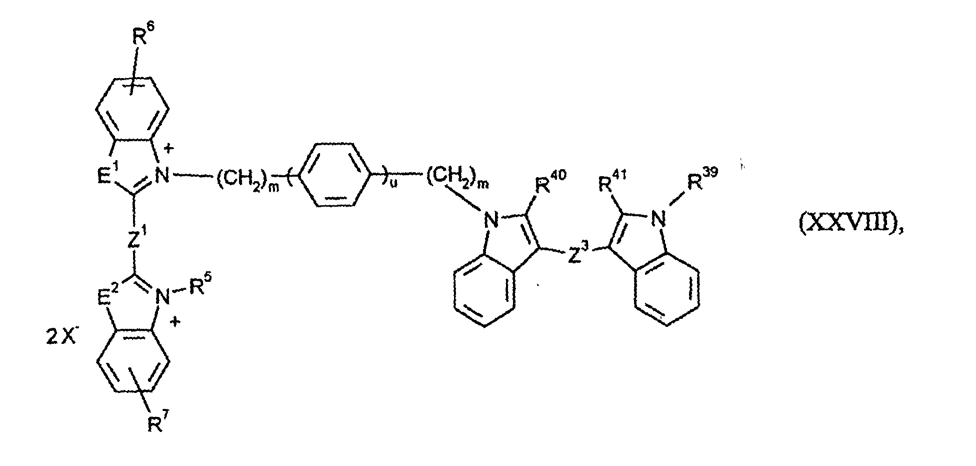 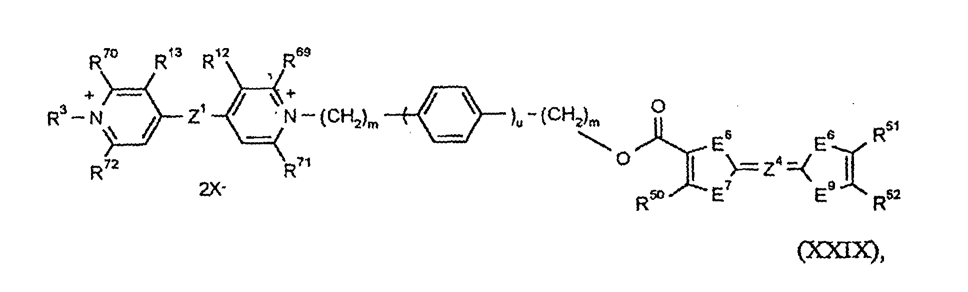 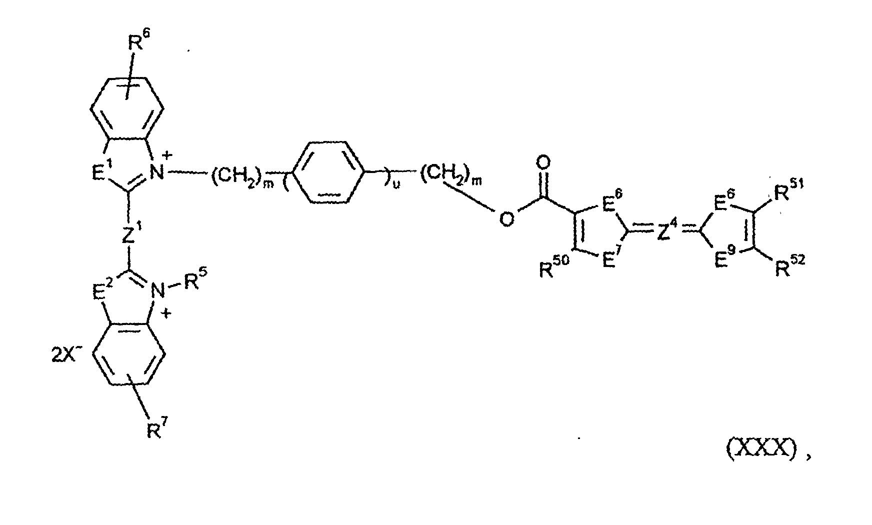 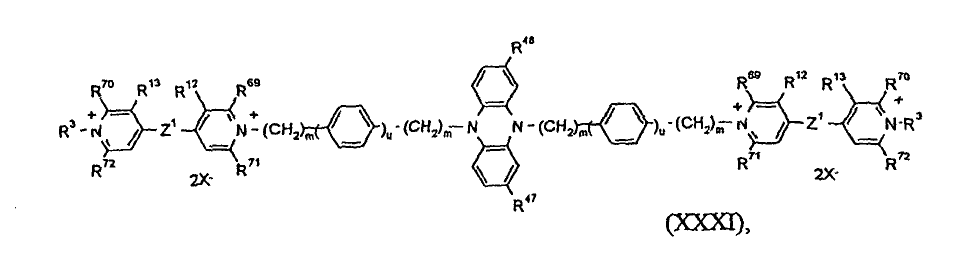 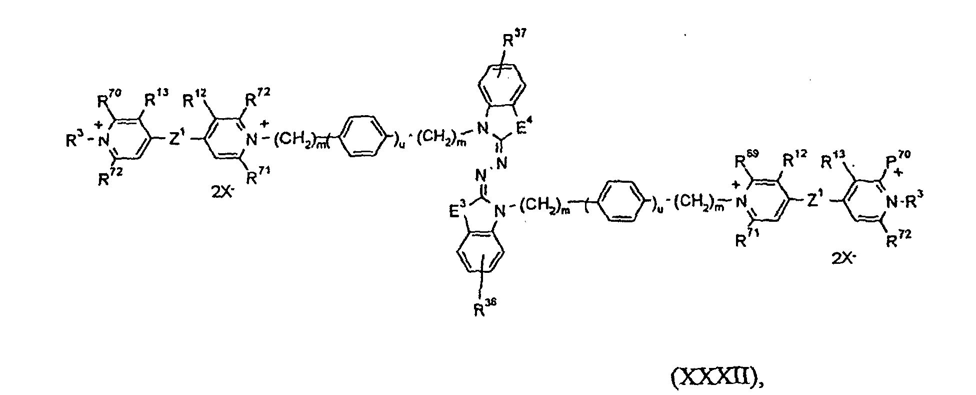 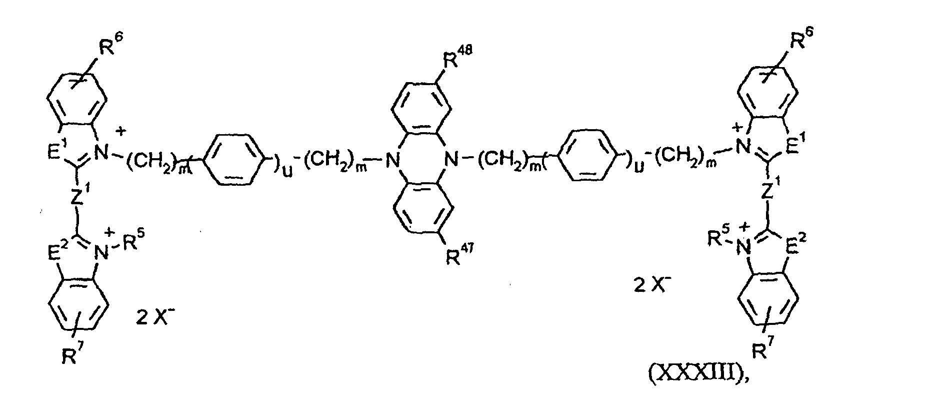 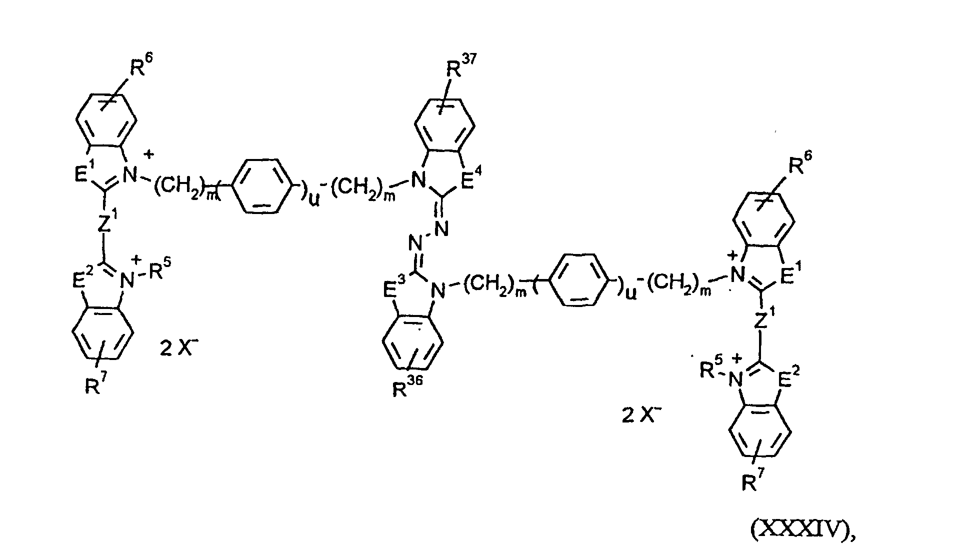 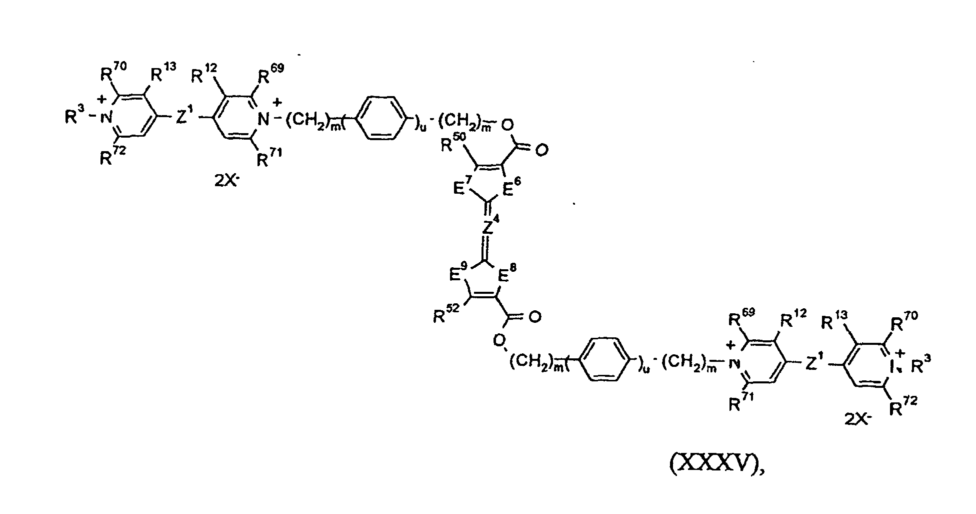 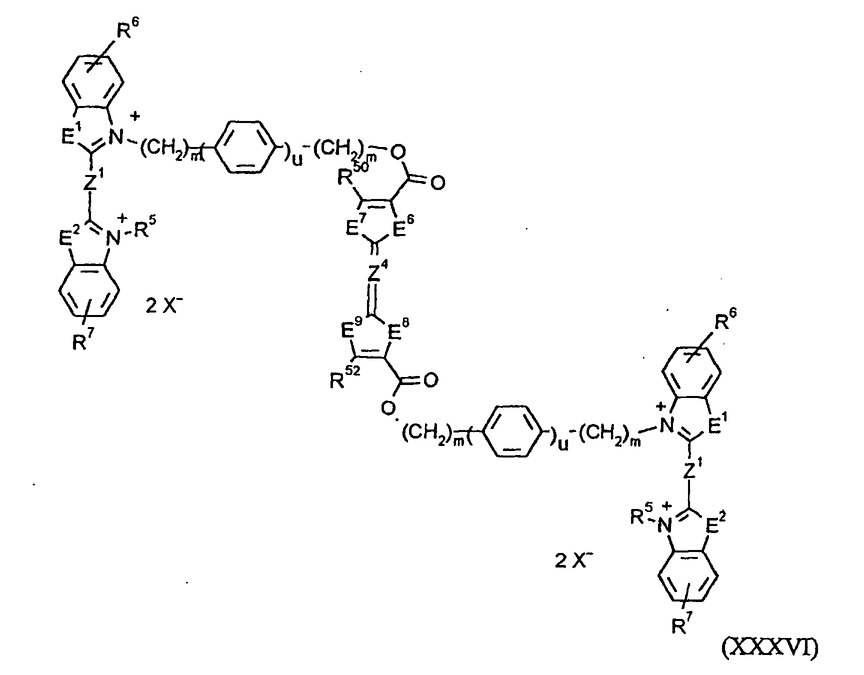 oder mindestens eine Substanz der Formel (Ic) entsprechend einer der Formeln 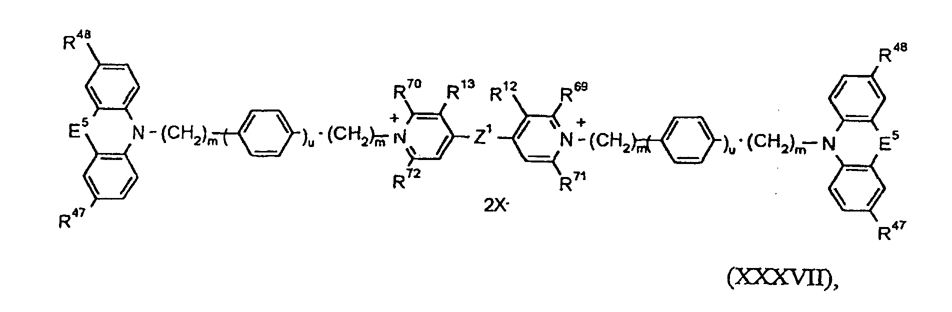 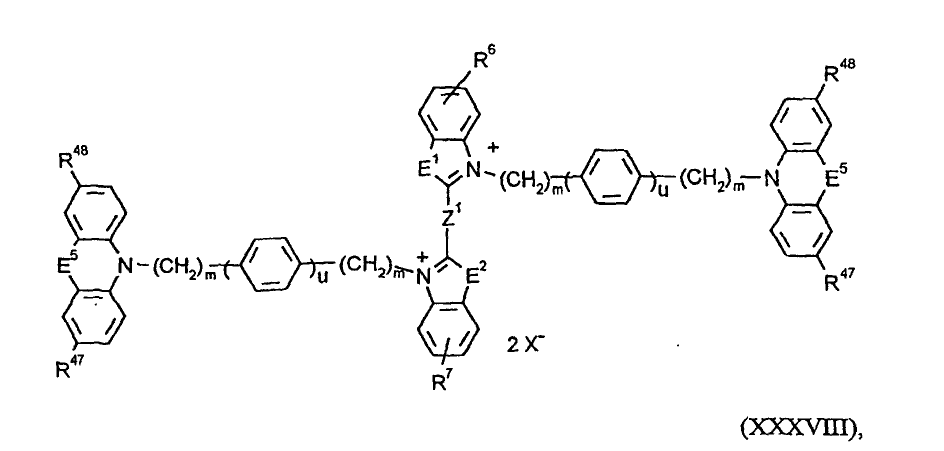 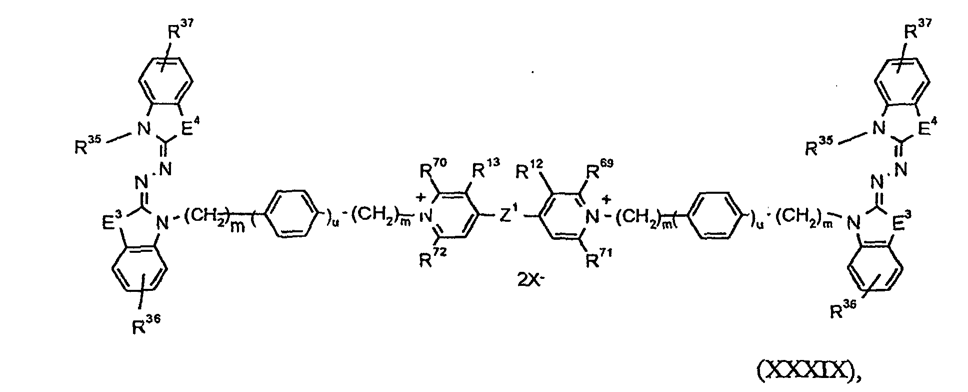 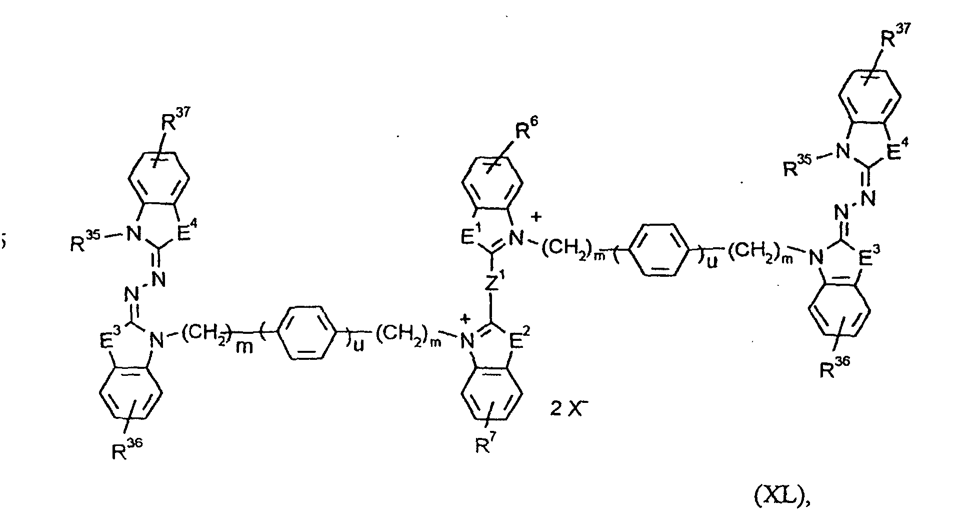 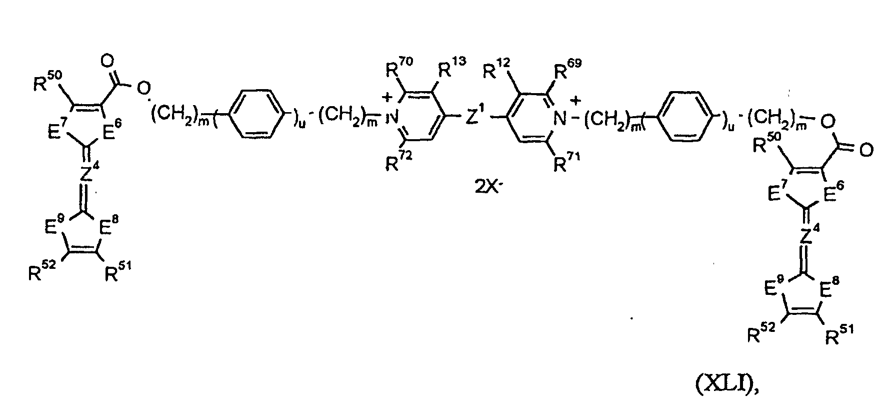 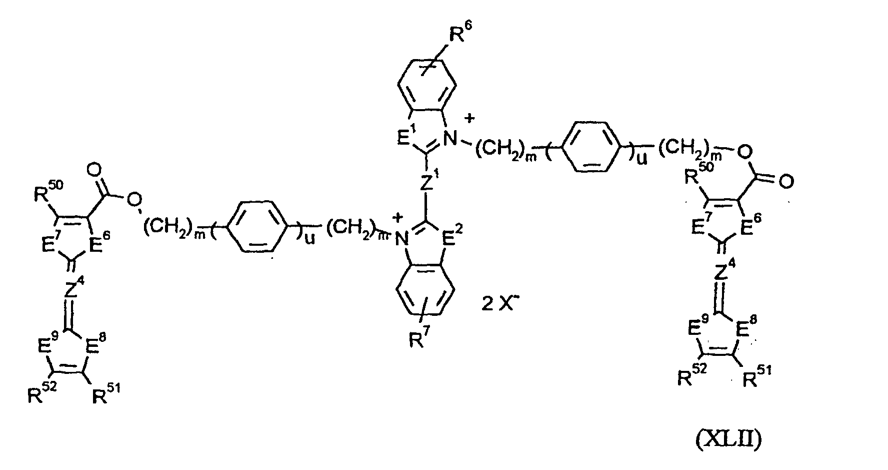 enthält, worin
R³, R⁵, R³⁵ und R³⁹ unabhängig voneinander für Methyl, Ethyl, Propyl, Butyl, Pentyl, Hexyl oder Benzyl stehen,
R⁶, R⁷ und R³⁶, R³⁷ paarweise gleich sind und für Wasserstoff, Methyl, Methoxy, Chlor, Cyano oder Methoxycarbonyl stehen,
R¹² und R¹³ für Wasserstoff oder, wenn Z¹ eine direkte Bindung bedeutet, gemeinsam für eine CH=CH-Brücke stehen,
R⁶⁹ bis R⁷² gleich sind und für Wasserstoff oder Methyl stehen
E¹ und R² gleich sind und für O oder S stehen,
Z¹ für eine direkte Bindung oder -CH=CH- steht,
R³², R⁴⁷ und R⁴⁸ für Wasserstoff stehen,
E³ bis E⁵ unabhängig voneinander für O, S oder NR⁵⁹ stehen, wobei E³ und E⁴ aber gleich sind,
R²⁹ bis R³¹ und R⁵⁹ unabhängig voneinander für Methyl, Ethyl, Propyl, Butyl, Pentyl, Hexyl oder Benzyl stehen, wobei R²⁹ bis R³¹ vorzugsweise gleich sind,
R⁴⁰ und R⁴¹ gleich sind und für Wasserstoff, Methyl, Ethyl, Propyl, Butyl oder Phenyl stehen,
Z³ für eine direkte Bindung, -CH=CH- oder -N=N- steht,
R⁵⁰ bis R⁵² unabhängig voneinander für Wasserstoff, Methyl, Methoxy, Chlor, Cyano, Methoxycarbonyl, Ethoxycarbonyl oder Phenyl stehen, vorzugsweise jedoch gleich sind,
E⁶ bis E⁹ untereinander gleich sind und für S, Se oder NR⁵⁹ stehen,
Z⁴ für eine direkte Doppelbindung, eine =CH-CH= oder =N-N=-Brücke steht,
m für eine ganze Zahl von 1 bis 5 steht,
u für 0 oder 1 steht und
X⁻ für ein unter den Bedingungen redox-inertes, farbloses Anion steht.

13. Verfahren zur Herstellung elektrochromer Substanzen gemäß Ansprüche 10 bis 12, **dadurch gekennzeichnet, daß** Verbindungen der Formel
OX₂-B-A (XLIII)
mit Verbindungen der Formel
RED₁ (XLIV)
oder daß Verbindungen der Formel
OX₂ (XLV)
mit Verbindungen der Formel
A-B-RED₁ (XLVI)
wobei
A eine Abgangsgruppe wie Chlor, Brom, Iod, OSO₂-Alkyl, OSO₂-Perfluoralkyl oder OSO₂-Aryl bedeutet
und OX₂, RED₁ und B die in Anspruch 10 angegebene Bedeutung besitzen,
umgesetzt werden.

14. Elektrochrome Flüssigkeit enthaltend ein elektrochromes System gemäß wenigstens einem der Ansprüche 1 bis 9 und mindestens ein inertes Lösungsmittel.

15. Elektrochrome Vorrichtung enthaltend eine elektrochrome Flüssigkeit gemäß Anspruch 14.

16. Elektrochrome Vorrichtung gemäß Anspruch 15, **dadurch gekennzeichnet, daß** sie als Zelle wie z.B. eine Solarzelle, als Fensterscheibe, Spiegel, Sonnendach oder Display ausgebildet ist.

17. Elektrochrome Vorrichtung gemäß Ansprüchen 15 und 16, **dadurch gekennzeichnet, daß** sie aus zwei einander zugewandten lichtdurchlässigen Glasoder Kunststoffscheiben besteht, von denen gegebenenfalls eine verspiegelt ist und deren einander zugewandten Seiten elektrisch leitfähig beschichtet sind zwischen denen die elektrochrome Flüssigkeit enthalten ist.

## Claims

1. Electrochromic system comprising at least one oxidizable substance RED₁ and at least one reducible substance OX₂ which, by electron donation of the former at an anode and by electron acceptance of the latter at a cathode, in each case with an increase in the extinction in the visible range of the spectrum, are converted from a slightly coloured or colourless form into a coloured form OX₁ and RED₂ respectively, in each case the slightly coloured or colourless form being reformed after charge compensation, **characterized in that** at least one of the substances RED₁ and OX₂ contained therein are linked together covalently via a bridge.

2. Electrochromic system according to Claim 1, **characterized in that** it comprises at least one electrochromic substance of the formula (I) 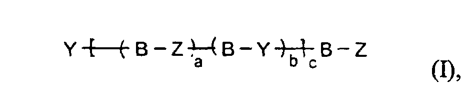 in which
Y and Z independently of one another represent a radical OX₂ or RED₁, but in which at least one Y represents OX₂ and at least one Z represents RED₁,
in which
OX₂ represents the radical of a reversibly electrochemically reducible redox system and
RED₁ represents the radical of a reversibly electrochemically oxidizable redox system,
B represents a bridge member,
c represents an integer from 0 to 1 and
a and b independently of one another represent an integer from 0 to 5.

3. Electrochromic system according to Claims 1 and 2, **characterized in that** it comprises at least one electrochromic substance of the formula (I) in which Y represents OX₂ and Z represents RED₁ and Y and Z alternate in their sequence.

4. Electrochromic system according to Claims 1 to 3, **characterized in that** it comprises at least one electrochromic substance of the formulae
OX₂-B-RED₁ (Ia),
OX₂-B-RED₁-B-OX₂ (Ib),
RED₁-B-OX₂-B-RED₁ (Ic),
or
OX₂-(B-RED₁-B-OX₂)d-B-RED₁ (Id),
in which
OX₂, RED₁ and B have the abovementioned meaning and
d represents an integer from 1 to 5.

5. Electrochromic system according to Claims 1 to 4, **characterized in that** it comprises at least one electrochromic substance of the formulae (Ia)-(Id),
in which
OX₂ represents the radical of a cathodically reducible substance which, in the cyclic voltammogram recorded in an inert solvent at room temperature, shows at least two chemically reversible reduction waves, the first of these reduction waves leading to an increase in the extinction at not less than one wavelength in the visible range of the electromagnetic spectrum,
RED₁ represents the radical of the anodically reversibly oxidizable substance which, in the cyclic voltammogram recorded in an inert solvent at room temperature, shows at least two chemically reversible oxidation waves, the first of these oxidation waves leading to an increase in the extinction at not less than one wavelength in the visible range of the electromagnetic spectrum and
B represents a bridge member.

6. Electrochromic system according to Claims 1 to 5, **characterized in that** it comprises at least one substance of the formula (Ia)-(Id), in which
OX₂ represents a radical of the formulae 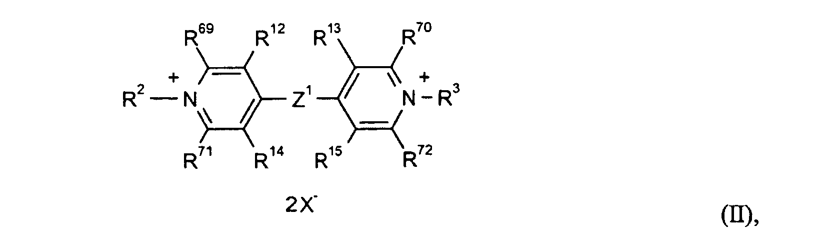 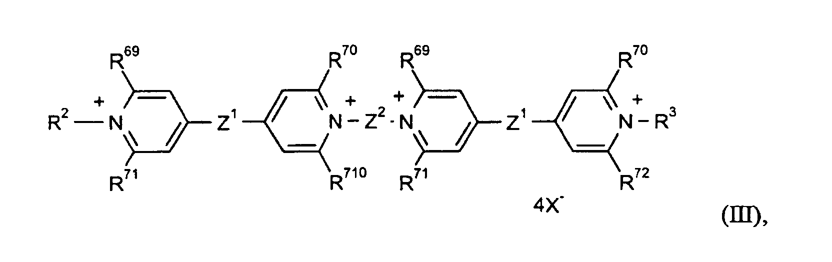 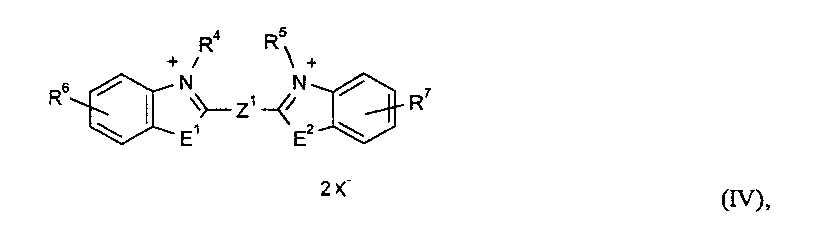 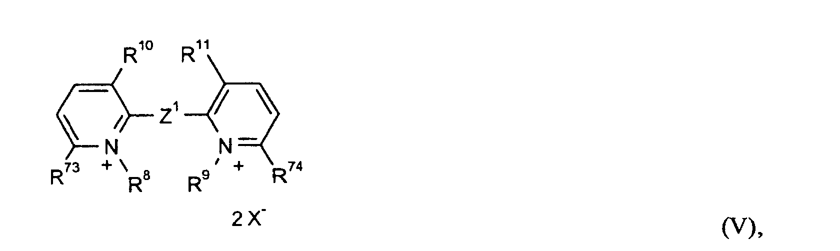 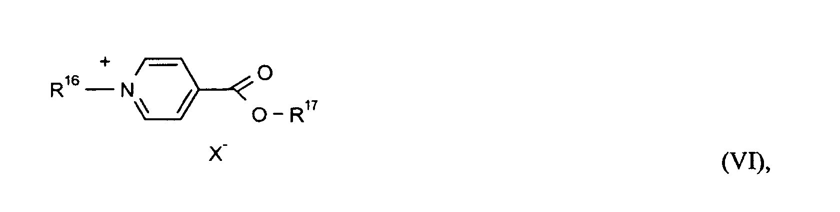 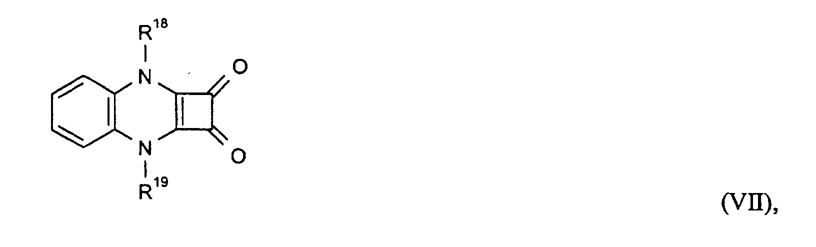 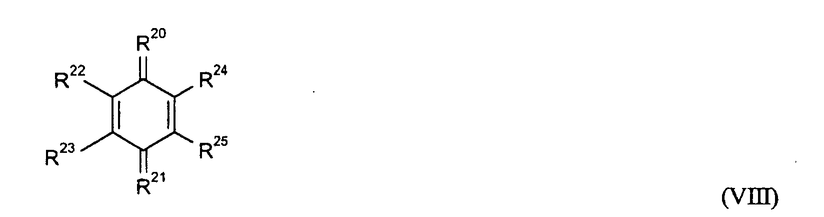 or 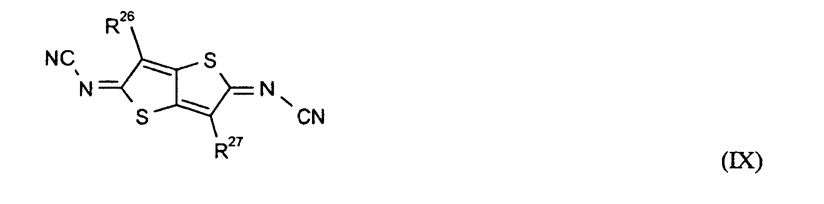
in which
R² to R⁵, R⁸, R⁹ and R¹⁶ to R¹⁹ independently of one another denote C₁- to C₁₈-alkyl, C₂- to C₁₂-alkenyl, C₃- to C₇-cycloalkyl, C₇- to C₁₅-aralkyl or C₆- to C₁₀-aryl or
R⁴ and R⁵ or R⁸ and R⁹ together form a -(CH₂)₂- or -(CH₃)- bridge,
R⁶, R⁷ and R²² to R²⁵ independently of one another denote hydrogen, C₁- to C₄-alkyl, C₁- to C₄-alkoxy, halogen, cyano, nitro or C₁- to C₄-alkoxycarbonyl or
R²² and R²³ and/or R²⁴ and R²⁵ form a -CH=CH-CH=CH- bridge,
R¹⁰ and R¹¹; R¹² and R¹³; and R¹⁴ and R¹⁵ independently of one another denote hydrogen or, in pairs, a -(CH₂)₂-, -(CH₂)₃- or -CH=CH- bridge,
R⁶⁹ to R⁷⁴ independently of one another denote hydrogen or C₁-C₆-alkyl, or
R⁶⁹; R¹² and/or R⁷⁰; and R¹³ form a -CH=CH-CH=CH- bridge,
R²⁰ and R²¹ independently of one another denote O, N-CN, C(CN)₂ or N-C₆- to C₁₀-aryl,
R²⁶ denotes hydrogen, C₁- to C₄-alkyl, C₁- to C₄-alkoxy, halogen, cyano, nitro, C₁- to C₄-alkoxycarbonyl or C₆- to C₁₀-aryl,
E¹ and E² independently of one another denote O, S, NR¹ or C(CH₃)₂ or
E¹ and E² together form a -N-(CH₂)₂-N- bridge,
R¹ denotes C₁- to C₁₈-alkyl, C₂- to C₁₂-alkenyl, C₄- to C₇-cycloalkyl, C₇- to C₁₅-aralkyl or C₆- to C₁₀-aryl,
Z¹ denotes a direct bond -CH=CH-, -C(CH3)=CH-, -C(CN)=CH-, -CCl=CCl-, -C(OH)=CH-, -CCl=CH-, -C=C-, -CH=N-N=CH-, -C(CH3)=N-N=C(CH3)- or -CCl=N-N=CCl-,
Z² denotes -(CH₂)ᵣ- or -CH₂-C₆H₄-CH₂-,
r denotes an integer from 1 to 10,
X⁻ denotes an anion which is redox-inert under the conditions,
wherein bonding to the bridge member B is effected via one of the radicals R²-R¹⁹, R²²-R²⁷ or, in the case where E¹ or E² represents NR¹, via R¹ and the radicals mentioned then represent a direct bond,
RED 1 represents one of the following radicals 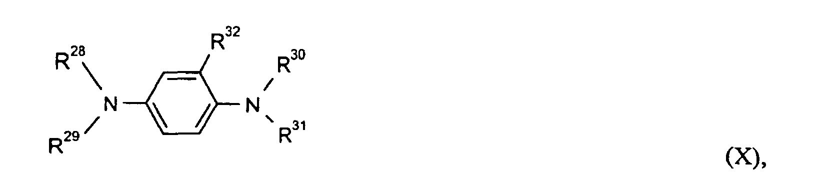 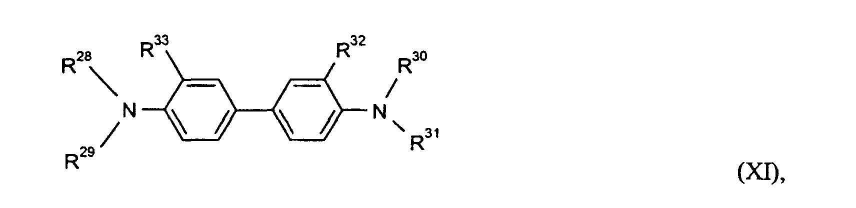 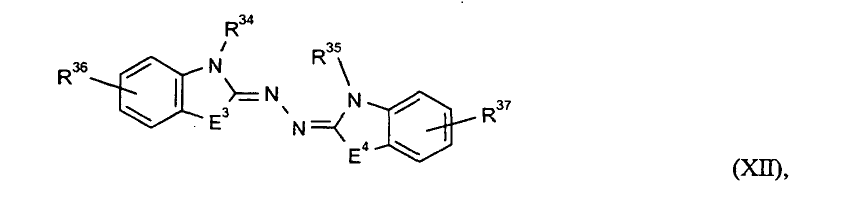 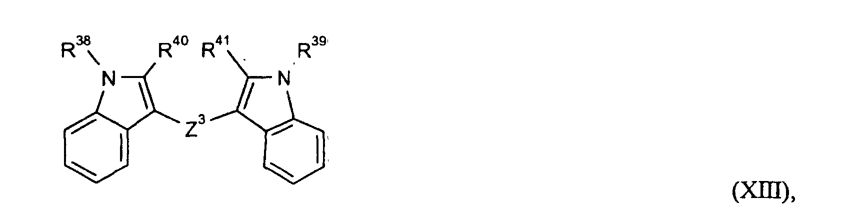 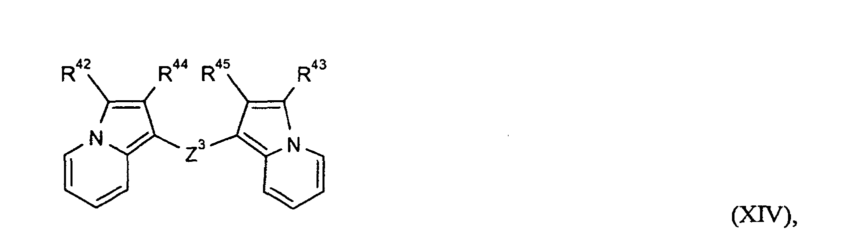 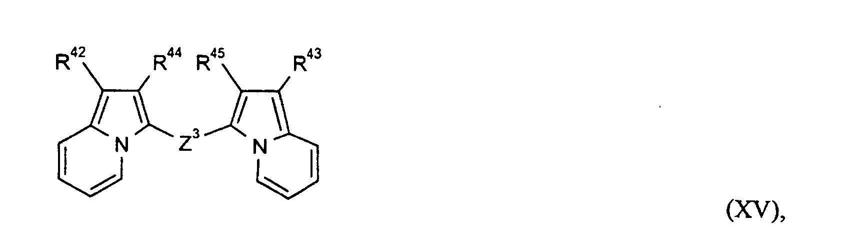 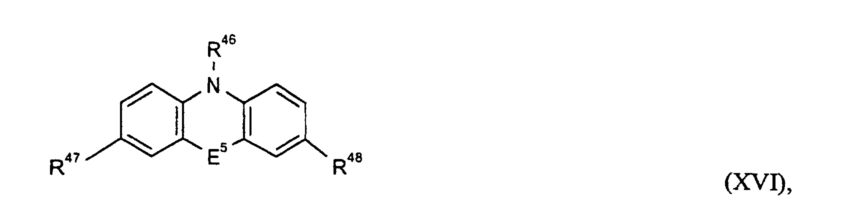 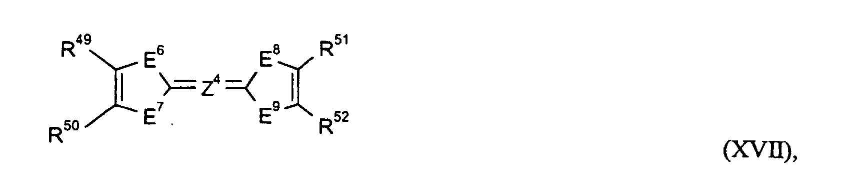 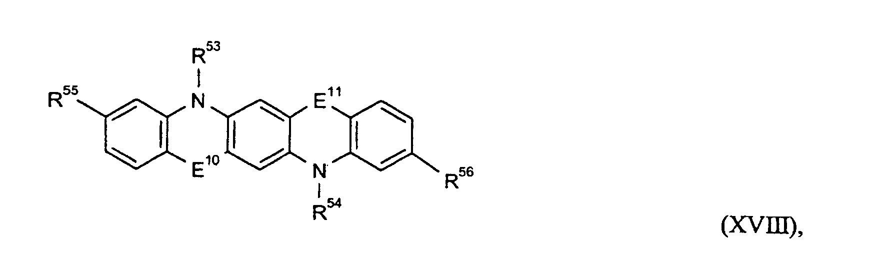 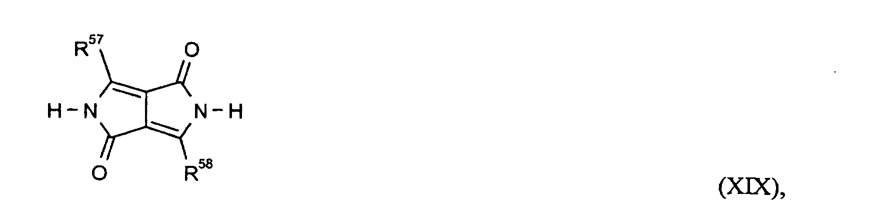 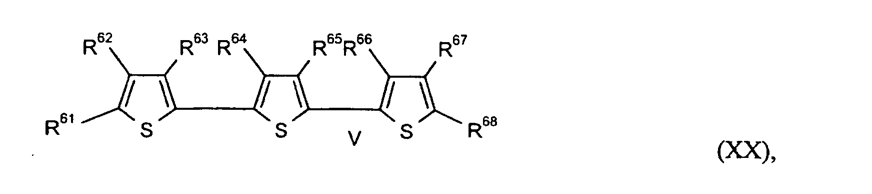
wherein
R²⁸ to R³¹, R³⁴, R³⁵, R³⁸, R³⁹, R⁴⁶, R⁵³ and R⁵⁴ independently of one another denote C₁- to C₁₈-alkyl, C₂- to C₁₂-alkenyl, C₃- to C₇-cycloalkyl, C₇- to C₁₅-aralkyl or C₆- to C₁₀-aryl, and R⁴⁶, R⁵³ and R⁵⁴ additionally denote hydrogen,
R³², R³³, R³⁶, R³⁷, R⁴⁰, R⁴¹, R⁴² to R⁴⁵, R⁴⁷, R⁴⁸, R⁴⁹ to R⁵² and R⁵⁵ to R⁵⁷ independently of one another denote hydrogen, C₁- to C₄-alkyl, C₁- to C₄-alkoxy, halogen, cyano, nitro, C₁- to C₄-alkoxycarbonyl or C₆- to C₁₀-aryl and R⁵⁷ and R⁵⁸ additionally denote an optionally benzofused aromatic or quasiaromatic five- or six-membered heterocyclic ring and R⁴⁸ additionally denotes NR⁷⁵R⁷⁶,
R⁴⁹ and R⁵⁰ and/or R⁵¹ and R⁵² form a -(CH₂)₃-, -(CH₂)₄-, -(CH₂)₅- or -CH=CH-CH=CH- bridge,
Z³ denotes a direct bond or a -CH=CH- or -N=N- bridge,
=Z⁴= denotes a direct double bond or a =CH-CH= or =N-N= bridge
E³ to E⁵, E¹⁰ and E¹¹ independently of one another denote O, S, NR⁵⁹ or C(CH₃)₂ and E⁵ additionally denotes C = O or SO₂, or
E³ and E⁴ independently of one another denote -CH=CH-,
E⁶ to E⁹ independently of one another denote S, Se or NR⁵⁹,
R⁵⁹, R⁷⁵ and R⁷⁶ independently of one another denote C₁- to C₁₂-alkyl, C₂- to C₈-alkenyl, C₃- to C₇-cycloalkyl, C₇- to C₁₅-aralkyl or C₆- to C₁₀- aryl and R⁷³ additionally denotes hydrogen, or
R⁷³ and R⁷⁵ in the meaning of NR⁷³R⁷⁴, together with the N atom to which they are bonded, form a five- or six-membered saturated ring which can contain further heteroatoms,
R⁶¹ to R⁶⁸ independently of one another denote hydrogen, C₁- to C₆-alkyl, C₁- to C₄-alkoxy, cyano, C₁- to C₄-alkoxycarbonyl or C₆- to C₁₀-aryl, or
R⁶¹; R⁶² and R⁶⁷; and R⁶⁸ independently of one another together form a -(CH₂)₃-, -(CH₂)₄- or -CH=CH-CH=CH- bridge,
v denotes an integer between 0 and 10,
wherein bonding to the bridge member B is effected by one of the radicals R²⁸-R⁵⁸, R⁶¹, R⁶², R⁶⁷, R⁶⁸ or, in the case where one of the radicals E³-E¹¹ represents NR⁵⁹, via R⁵⁹ and the radicals mentioned then represent a direct bond, and
B represents a bridge member of the formula -(CH₂)ₙ- or -[Y¹ₛ(CH₂)ₘ-Y²]ₒ-(CH₂)ₚ-Y³_{q}-, each of which is optionally substituted by C₁- to C₄-alkoxy, halogen or phenyl,
Y¹ to Y³ independently of one another represent O, S, NR⁶⁰, COO, CONH, NHCONH, cyclopentanediyl, cyclohexanediyl, phenylene or naphthylene,
R⁶⁰ denotes C₁- to C₆-alkyl, C₂- to C₆-alkenyl, C₄- to C₇-cycloalkyl, C₇- to C₁₅-aralkyl or C₆- to C₁₀-aryl,
n denotes an integer from 1 to 12,
m and p independently of one another denote an integer from 0 to 8;
o denotes an integer from 0 to 6 and
q and s independently of one another denote 0 or 1.

7. Electrochromic system according to Claims 1 to 6, **characterized in that** it comprises at least one substance of the formula (Ia)-(Id)
in which
OX₂ represents a radical of the formulae (II), (III), (IV) or (V),
in which
R², R³, R⁴, R⁵, R⁸ and R⁹ independently of one another represent C₁- to C₁₂alkyl, C₂- to C₈-alkenyl, C₅- to C₇-cycloalkyl, C₇- to C₁₅-aralkyl or C₆- to C₁₀-aryl,
R⁶ and R⁷ independently of one another represent hydrogen, methyl, ethyl, methoxy, ethoxy, fluorine, chlorine, bromine, cyano, nitro, methoxycarbonyl or ethoxycarbonyl,
R¹⁰, R¹¹; R¹², R¹³ and R¹⁴, R¹⁵ independently of one another represent hydrogen or, if Z¹ denotes a direct bond, in each case together in pairs represent a -(CH₂)₂-, -(CH₂)₃- or -CH=CH- bridge,
or
R⁴, R⁵ and R⁸, R⁹ independently of one another in pairs together represent a -(CH₂)₂- or -(CH₃)₃- bridge, if Z¹ denotes a direct bond,
R⁶⁹ to R⁷⁴ independently of one another denote hydrogen or C₁-C₄-alkyl,
E¹ and E² are identical and represent O, S, NR¹ or C(CH₃)₂ or together form a -N-(CH₂)₂-N- bridge,
R¹ represents C₁- to C₁₂-alkyl, C₂- to C₄-alkenyl, C₅- to C₇-cycloalkyl, C₇- to C₁₅-aralkyl or C₆- to C₁₀-aryl,
Z¹ represents a direct bond, -CH=CH-, -C(CH3)=CH-, -C(CN)≡CH-, -C=C- or -CH=N-N=CH-,
Z² represents -(CH)ᵣ- or -CH₂-C₆H₄-CH₂-,
r represents an integer between 1 and 6,
X⁻ represents a colourless anion which is redox-inert under the conditions,
wherein bonding to the bridge member B is effected by one of the radicals R²-R¹¹ or, in the case where E¹ or E² represents NR¹, via R¹ and the radicals mentioned then represent a direct bond,
RED₁ represents a radical of the formulae (X), (XI), (XII), (XIII), (XVI), (XVII), (XVIII) or (XX),
in which
R²⁸ to R³¹, R³⁴, R³⁵, R³⁸, R³⁹, R⁴⁶, R⁵³ and R⁵⁴ independently of one another denote C₁- to C₁₂-alkyl, C₂- to C₈-alkenyl, C₅- to C₇-cycloalkyl, C₇- to C₁₅-aralkyl or C₆- to C₁₀-aryl and
R⁴⁶, R⁵³ and R⁵⁴ additionally denote hydrogen,
R³², R³³, R³⁶, R³⁷, R⁴⁰, R⁴¹, R⁴⁷ to R⁵², R⁵⁵ and R⁵⁶ independently of one another denote hydrogen, methyl, ethyl, methoxy, ethoxy, fluorine, chlorine, bromine, cyano, nitro, methoxycarbonyl, ethoxycarbonyl or phenyl and
R⁵⁷ and R⁵⁸ additionally denote 2- or 4-pyridyl and
R⁴⁸ additionally denotes NR⁷⁵R⁷⁶,
Z³ denotes a direct bond or a -CH=CH- or -N=N- bridge,
=Z⁴= denotes a direct double bond or a =CH-CH= or =N-N= bridge,
E³ to E⁵, E¹⁰ and E¹¹ independently of one another denote O, S, NR⁵⁹ or C(CH₃)₂, but E³ and E⁴ have the same meaning,
E⁶ to E⁹ are identical to one another and denote S, Se or NR⁵⁹ and
E⁵ additionally denotes C = O,
E⁶ represents NR⁵⁹, in which R⁵⁹ denotes a direct bond to the bridge B and
E⁷ to E⁹ have the abovementioned meaning but must not be identical to one another,
R⁵⁹, R⁷⁵ and R⁷⁶ independently of one another denote C₁- to C₁₂-alkyl, C₂- to C₈-alkenyl, C₅- to C₇-cycloalkyl, C₇- to C₁₅-aralkyl or C₆- to C₁₀-aryl, and R⁷³ additionally denotes hydrogen or
R⁷³ and R⁷⁴ in the meaning of NR⁷³R⁷⁴, together with the N atom to which they are bonded, denote pyrrolidino, piperidino or morpholino,
R⁶¹, R⁶² and R⁶⁷, R⁶⁸ independently of one another represent hydrogen, C₁- to C₄-alkyl, methoxycarbonyl, ethoxycarbonyl or phenyl or in pairs together represent a -(CH₂)₃- or -(CH₂)₄- bridge,
R⁶³ to R⁶⁶ represents hydrogen and
v represents an integer from 1 to 6,
wherein bonding to the bridge member B is effected by one of the radicals R²⁸-R⁴¹, R⁴⁶-R⁵⁶, R⁶¹, R⁶², R⁶⁷, R⁶⁸ or, in the case where one of the radicals E³-E¹¹ represents NR⁵⁹, via R⁵⁹ and the radicals mentioned then represent a direct bond,
B represents a bridge member of the formulae -(CH₂)ₙ-, -(CH₂)ₘ-O-(CH₂)ₚ-, -(CH)ₘ-NR⁶⁰-(CH₂)ₚ-, -(CH₂)ₘ-C₆H₄-(CH₂)ₚ-, -[O-(CH₂)ₚ]ₒ-O-, -[NR⁶⁰-(CH₂)ₚ]ₒ-NR⁶⁰-, -[C₆H₄-(CH₂)ₚ]ₒ-C₆H₄-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)-, -(CH₂)ₘ-NHCO-(CH₂)ₜ-CONH-(CH)ₚ-, -(CH₂)ₘ-NHCONH-(CH₂)ₜ-NHCONH-(CH₂)ₚ-,
R⁶⁰ represents methyl, ethyl, benzyl or phenyl,
n represents an integer from 1 to 10,
m and p independently of one another represent an integer from 0 to 4,
o represents an integer from 0 to 2 and
t represents an integer from 1 to 6.

8. Electrochromic system according to Claims 1 to 7, **characterized in that** it comprises at least one substance of the formula (Ia)-(Id)
in which
OX₂ represents a radical of the formulae (II), (IV) or (V),
in which
R², R⁴ and R⁸ represent a direct bond to the bridge member B,
R³, R⁵ and R⁹ independently of one another represent methyl, ethyl, propyl, butyl, pentyl, hexyl, heptyl, octyl, benzyl or phenyl, or
R³, R⁵ and R⁹ in the case of the formulae Ic or Id also represent a direct bond to the bridge member B,
R⁶ and R⁷ are identical and represent hydrogen, methyl, methoxy, chlorine, cyano or methoxycarbonyl,
R¹⁰, R¹¹; R¹², R¹³ and R¹⁴, R¹⁵ independently of one another represent hydrogen or, if Z¹ denotes a direct bond, in each case in pairs together represent a -CH=CH- bridge,
R⁶⁹ to R⁷² are identical and denote hydrogen, methyl or ethyl,
R⁷³ and R⁷⁴ denote hydrogen,
E¹ and E² are identical and represent O or S,
Z¹ represents a direct bond or -CH=CH-,
X⁻ represents a colourless anion which is redox-inert under the conditions,
RED₁ represents a radical of the formulae (X), (XII), (XIII), (XVI) or (XVII),
R²⁸, R³⁴, R³⁸, R⁴⁶ and R⁴⁹ represent a direct bond to the bridge member B,
R²⁹ to R³¹, R³⁵ and R³⁹ independently of one another represent methyl, ethyl, propyl, butyl, pentyl, hexyl, heptyl, octyl, benzyl or phenyl, or
R³⁰, R³⁵ and R³⁹ in the case of the formulae Ib and Ic also represent a direct bond to the bridge member B,
R³², R⁴⁷ and R⁴⁸ represent hydrogen,
R³⁶, R³⁷, R⁴⁰, R⁴¹ and R⁵⁰ to R⁵² independently of one another represent hydrogen, methyl, methoxy, chlorine, cyano, methoxycarbonyl or phenyl, or,
R⁵¹ in the case of the formulae Ib and Id also represent a direct bond to the bridge member B,
Z³ represents a direct bond or a -CH=CH- or -N=N- bridge,
=Z⁴= represents a direct double bond or a =CH-CH= or =N-N= bridge,
E³ to E⁵ independently of one another represent O, S or NR⁵⁹, but E³ and E⁴ have the same meaning,
E⁶ to E⁹ are identical to one another and represent S, Se or NR⁵⁹,
R⁵⁹ represents methyl, ethyl, propyl, butyl, pentyl, hexyl, heptyl, octyl, benzyl or phenyl, or
R⁵⁹ in the case of the formula XVI in formula Ib or Id also represents a direct bond to the bridge member B,
B represents a bridge member of the formulae -(CH₂)ₙ-, -(CH₂)ₘ-O-(CH₂)ₚ-, -(CH₂)ₘ-NR⁶⁰-(CH₂)ₚ-, -(CH₂)ₘ-C₆H₄-(CH₂)ₚ-, -[O-(CH₂)ₚ]-O-, -NR⁶⁰-(CH₂)ₚ-NR⁶⁰-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)ₚ-, -(CH₂)ₘ-NHCO-(CH₂)ₜ-CONH-(CH₂)ₚ-, -(CH₂)ₘ-NHCONH-(CH₂)ₜ-NHCONH-(CH₂)ₚ-,
R⁶⁰ represents methyl,
n represents an integer from 1 to 10,
m and p are identical and represent an integer from 0 to 2 and
t represents an integer from 1 to 6.

9. Electrochromic system according to Claims 1 to 8, **characterized in that** it comprises at least one electrochromic substance of the formula (Ia)
OX₂-B-RED₁ (Ia)
corresponding to one of the formulae 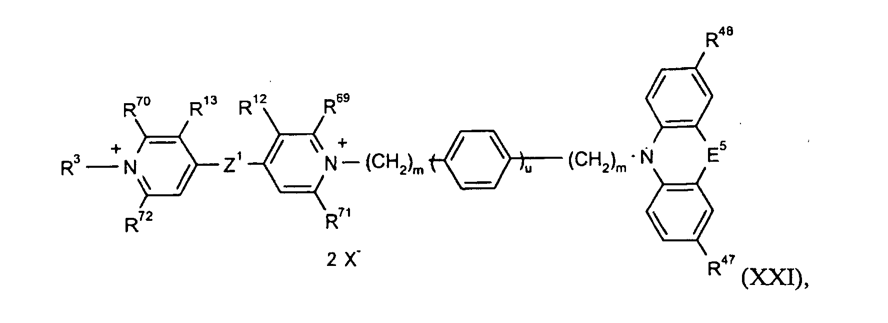 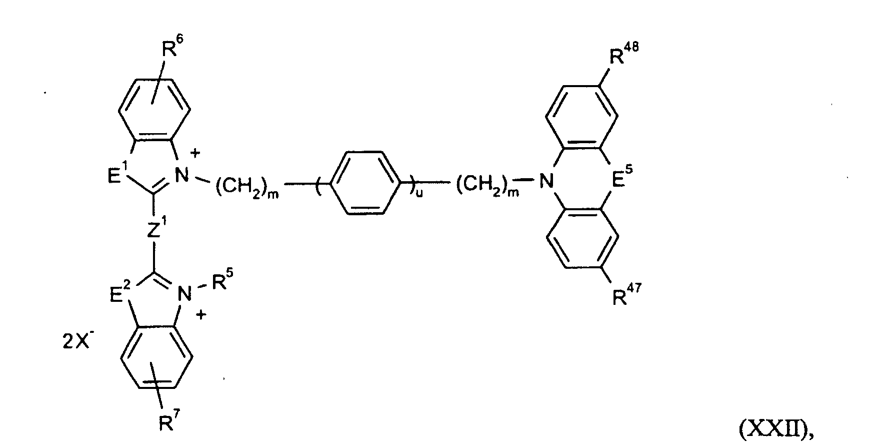 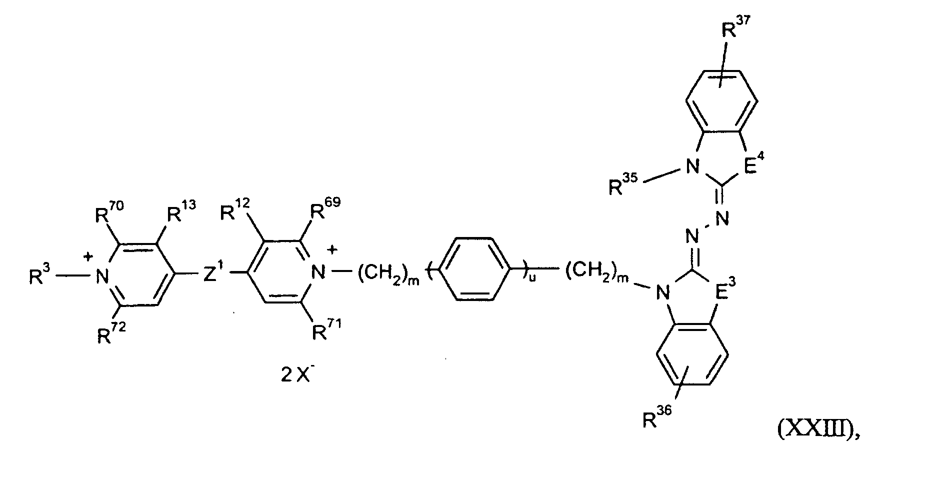 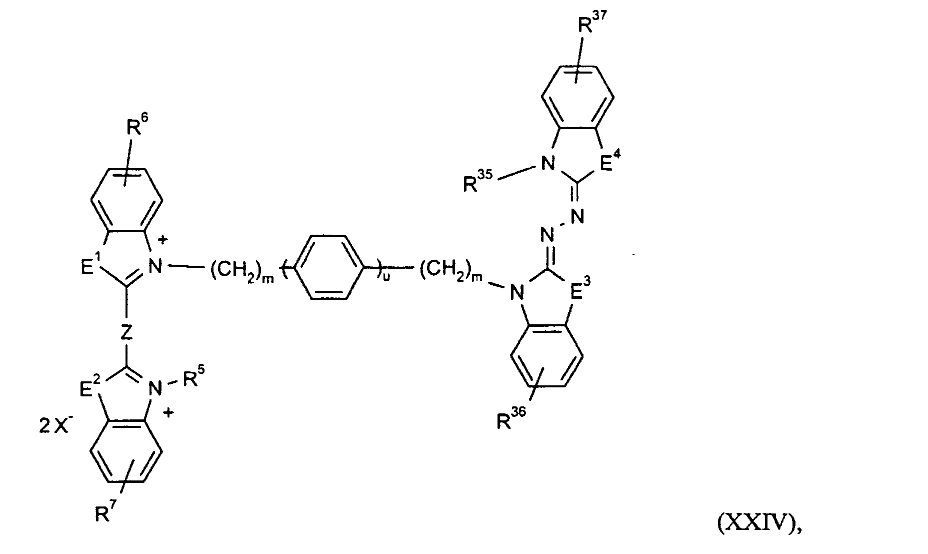 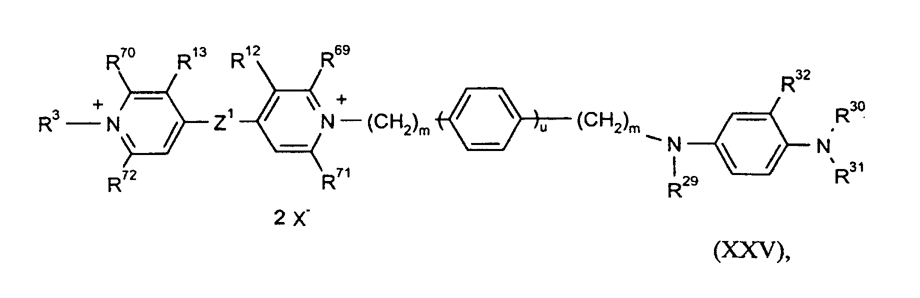 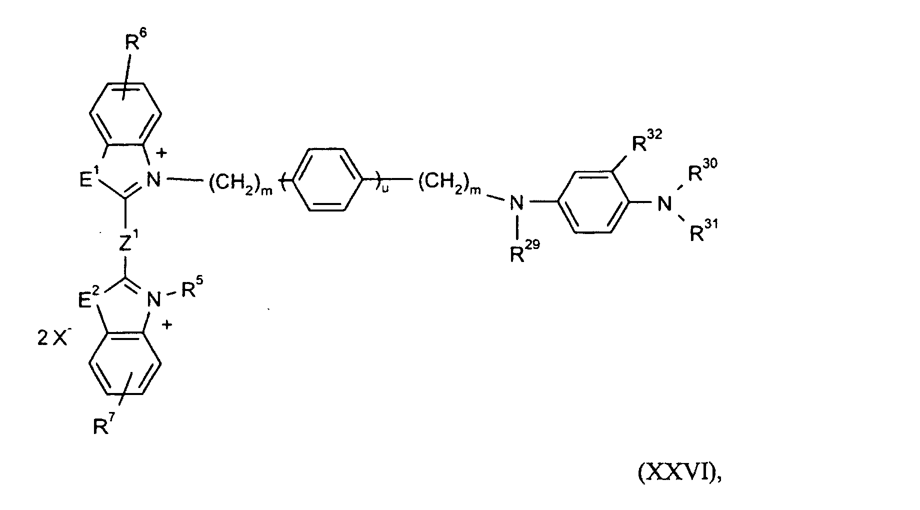 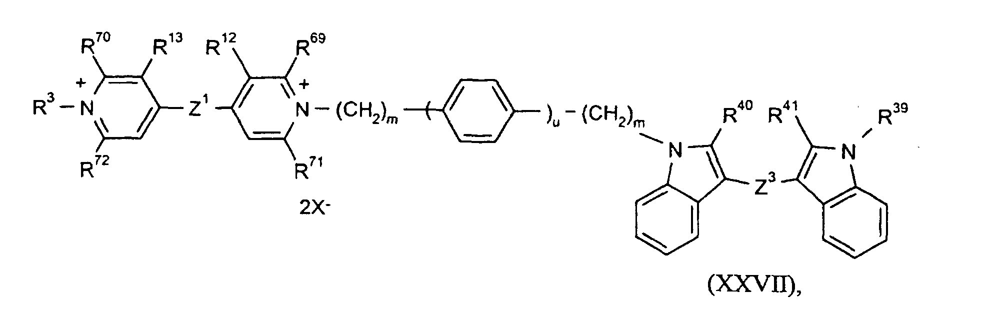 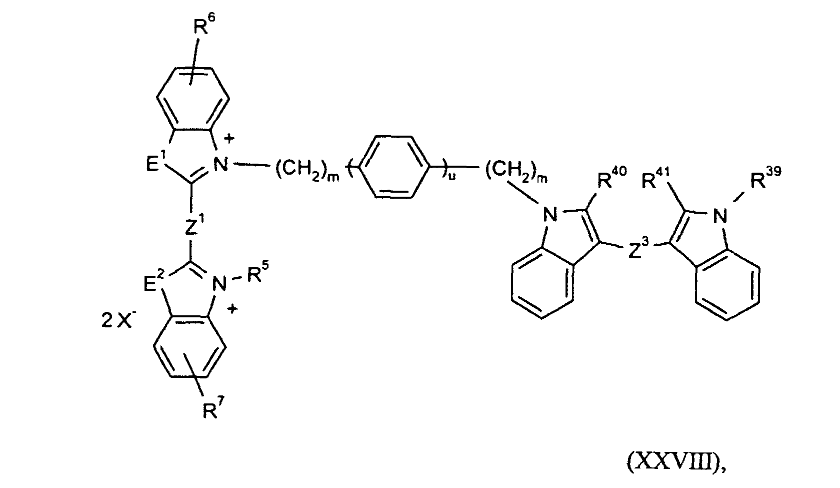 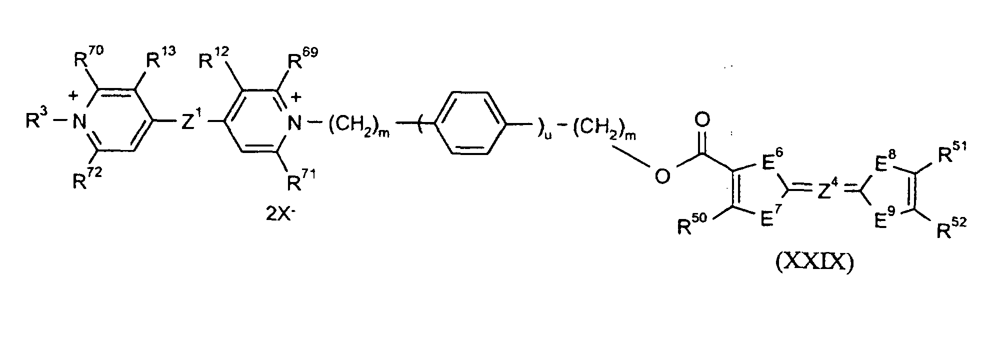 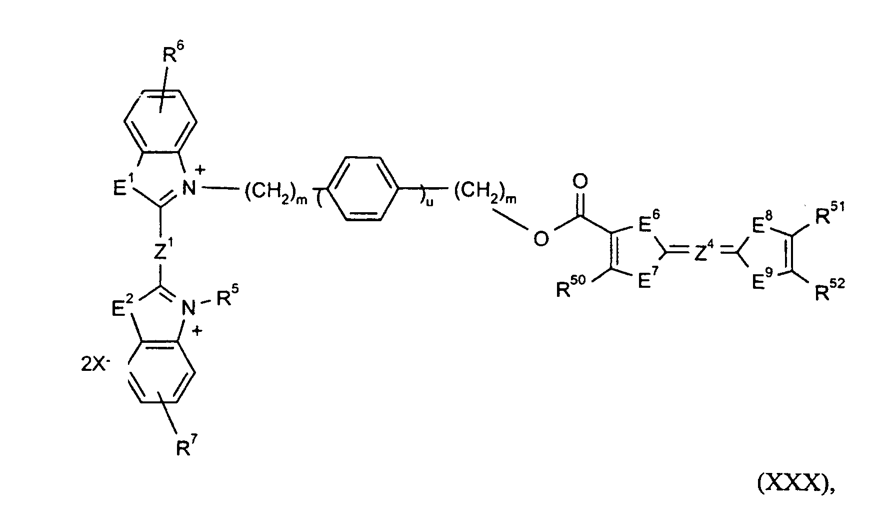 or 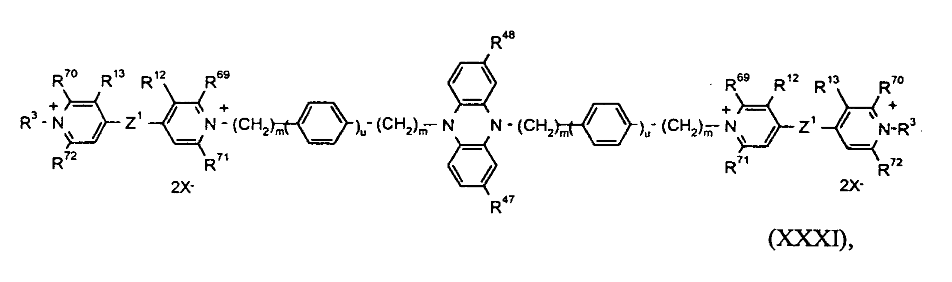 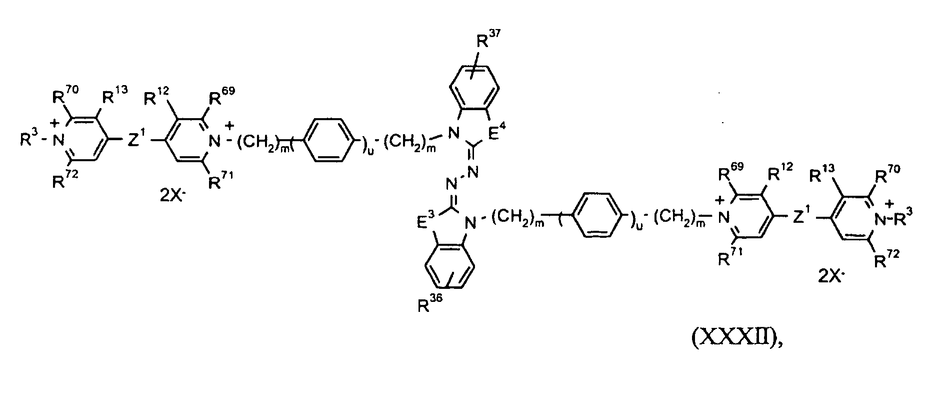 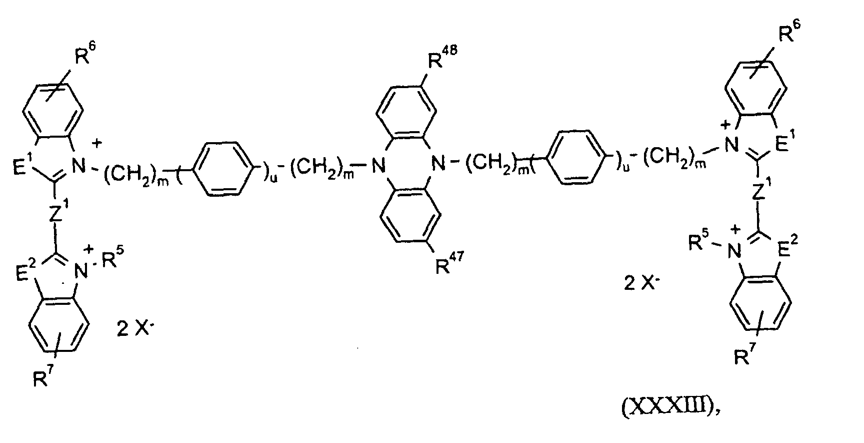 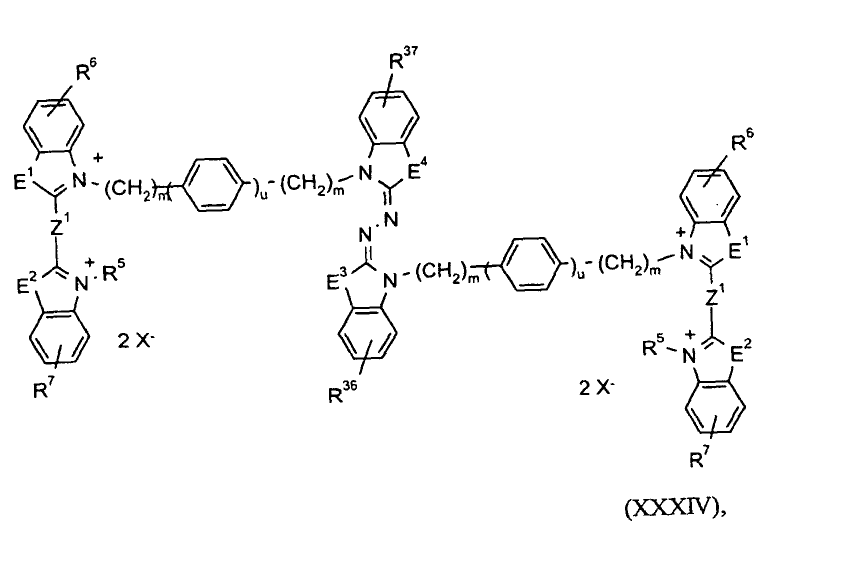 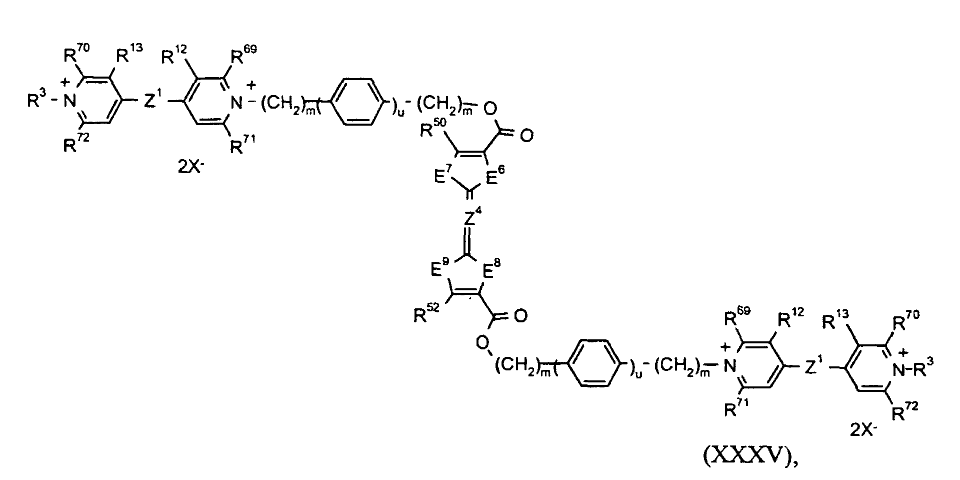 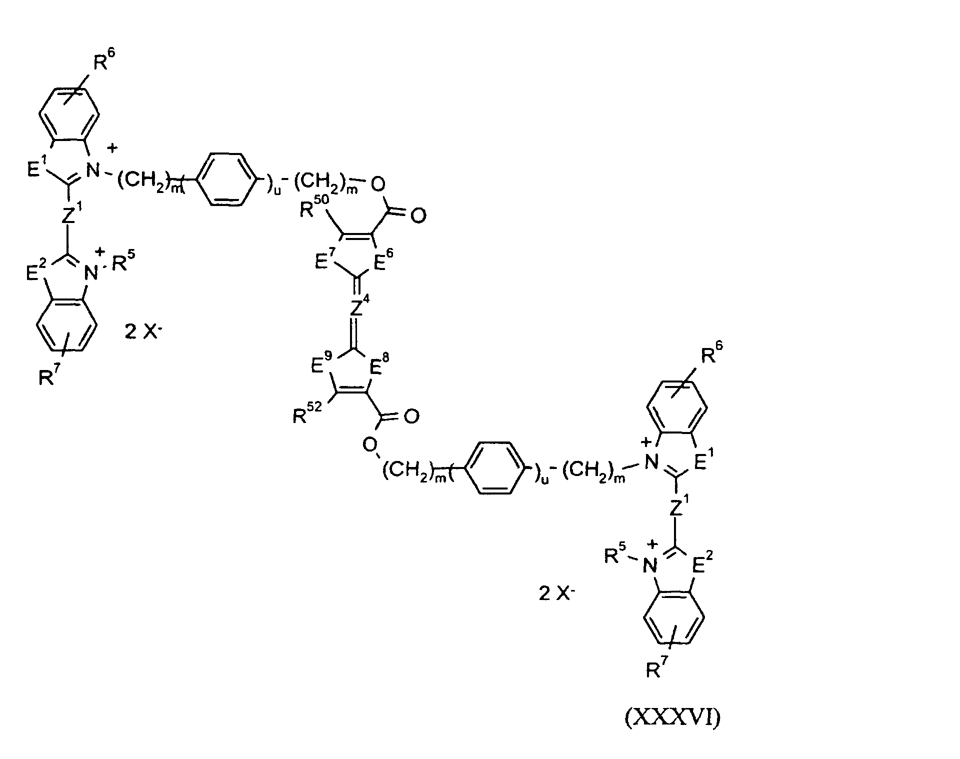 or at least one substance of the formula (Ic) corresponding to one of the formulae 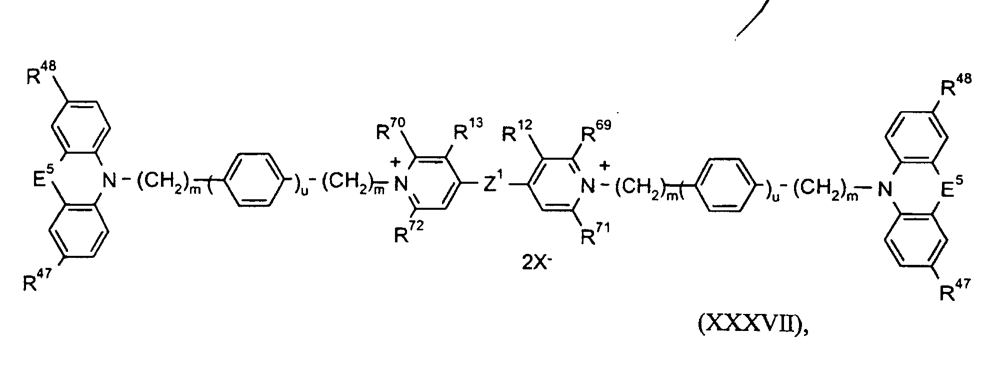 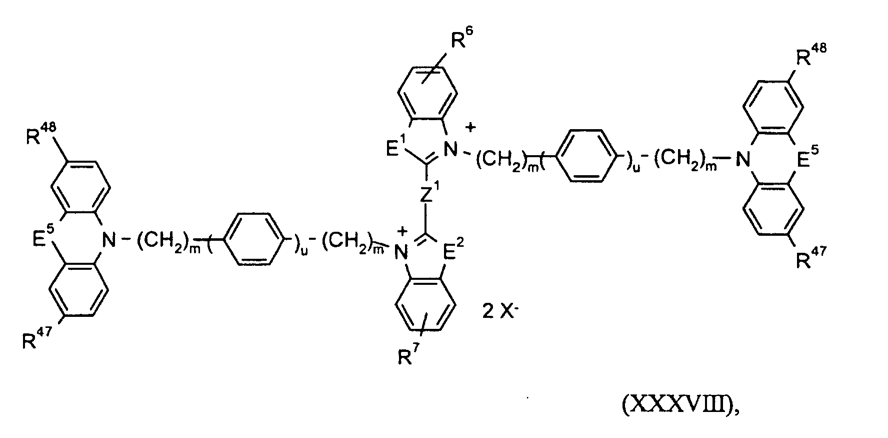 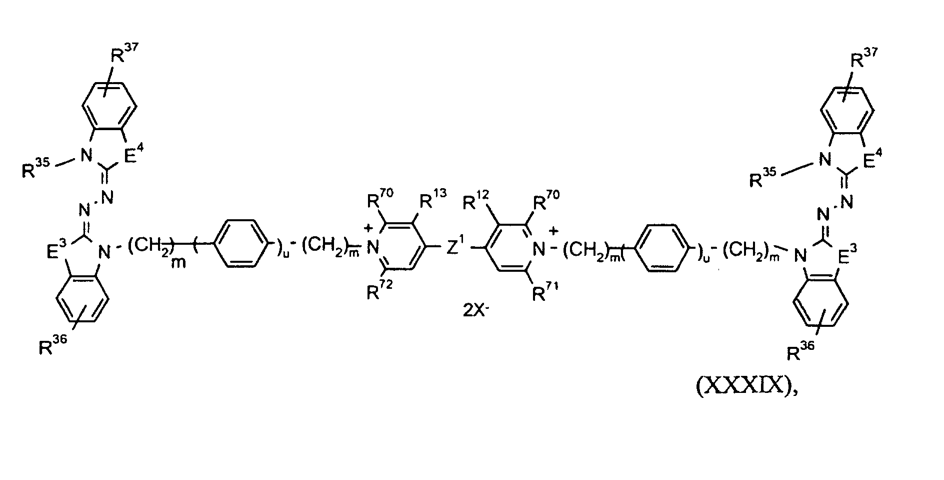 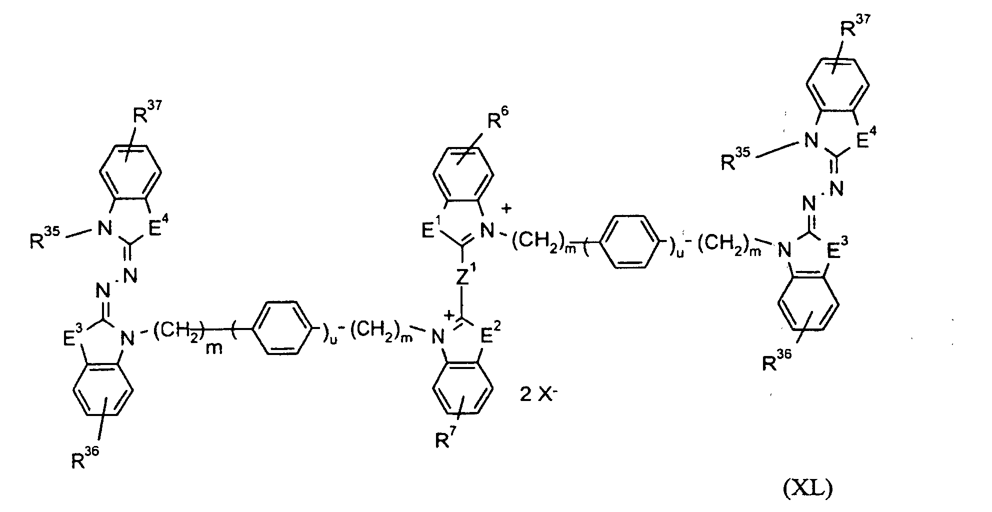 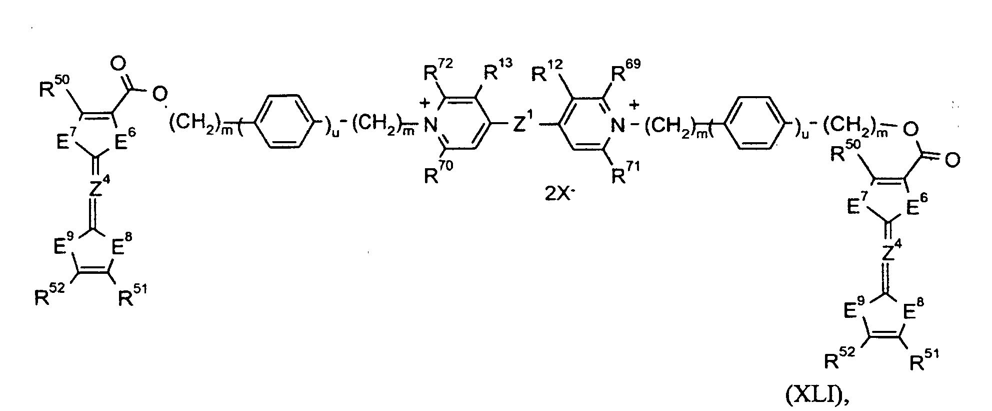 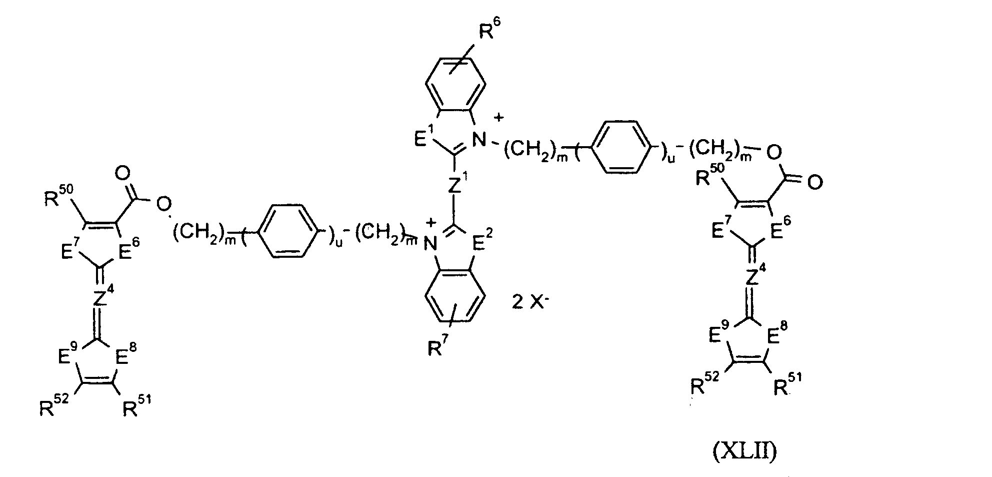 in which
R³, R⁵, R³⁵ and R³⁹ independently of one another represent methyl, ethyl, propyl, butyl, pentyl, hexyl or benzyl,
R⁶, R⁷ and R³⁶, R³⁷ in pairs are identical and represent hydrogen, methyl, methoxy, chlorine, cyano or methoxycarbonyl,
R¹² and R¹³ represent hydrogen or, if Z¹ denotes a direct bond, together represent a CH=CH bridge,
R⁶⁹ to R⁷² are identical and represent hydrogen or methyl,
E¹ and R² are identical and represent O or S,
Z¹ represents a direct bond or -CH=CH-,
R³², R⁴⁷ and R⁴⁸ represent hydrogen,
E³ to E⁵ independently of one another represent O, S or NR⁵⁹, but where E³ and E⁴ are identical,
R²⁹ to R³¹ and R⁵⁹ independently of one another represent methyl, ethyl, propyl, butyl, pentyl, hexyl or benzyl, where R²⁹ to R³¹ are preferably identical,
R⁴⁰ and R⁴¹ are identical and represent hydrogen, methyl, ethyl, propyl, butyl or phenyl,
Z³ represents a direct bond, -CH=CH- or -N=N-,
R⁵⁰ to R⁵² independently of one another represent hydrogen, methyl, methoxy, chlorine, cyano, methoxycarbonyl, ethoxycarbonyl or phenyl, but are preferably identical,
E⁶ to E⁹ are identical to one another and represent S, Se or NR⁵⁹,
Z⁴ represents a direct double bond or a =CH-CH= or =N-N= bridge,
m represents an integer from 1 to 5,
u represents 0 or 1 and
X⁻ represents a colourless anion which is redox-inert under the conditions.

10. Electrochromic substance corresponding to the formula 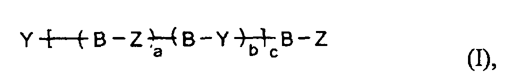 in which
Y and Z independently of one another represent a radical OX₂ or RED₁, but in which at least one Y represents OX₂ and at least one Z represents RED₁,
in which
OX₂ represents the radical of a reversibly electrochemically reducible redox system and
RED₁ represents the radical of a reversibly electrochemically oxidizable redox system, where the radical of the formula (XX) 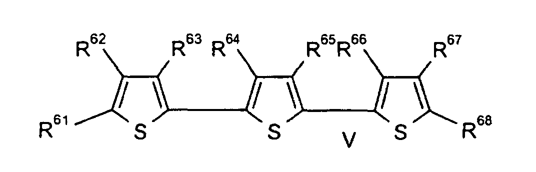 where R⁶¹ - R⁶⁸ and v have the meaning given in Claim 6 is excluded,
B represents a bridge member,
c represents an integer from 0 to 1 and
a and b independently of one another represent an integer from 0 to 5, and preferably represent an integer from 0 to 3,
with the exception of compounds of the formula 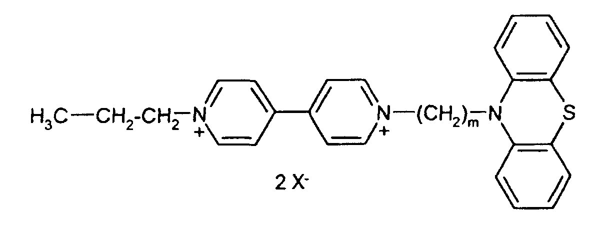 in which
m represents an integer from 2 to 16 and
X⁻ has the abovementioned meaning.

11. Electrochromic substances according to Claim 10 corresponding to one of the formulae
OX₂-B-RED₁ (Ia),
OX₂-B-RED₁-B-OX₂ (Ib),
RED₁-B-OX₂-B-RED₁ (Ic),
or
OX₂-(B-RED₁-B-OX₂)d-B-RED₁ (Id),
in which OX₂, RED₁ and B have the meanings given in Claim 10 and
d is an integer from 1 to 5.

12. Electrochromic substance according to Claims 10 and 11 of the formulae 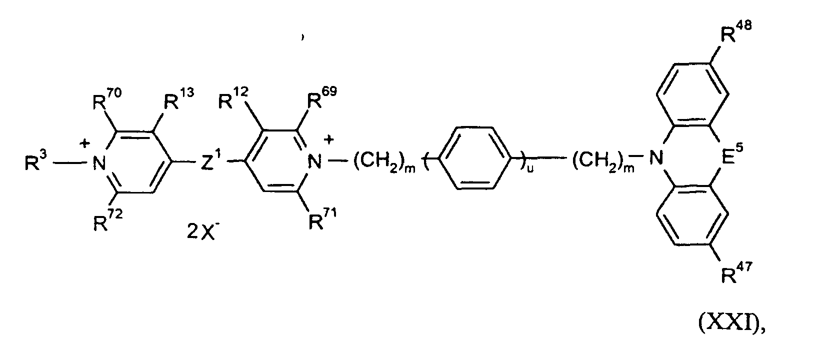 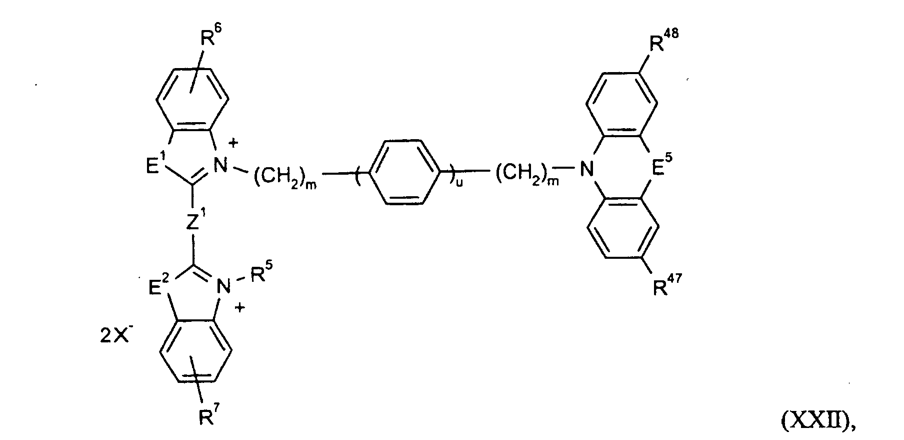 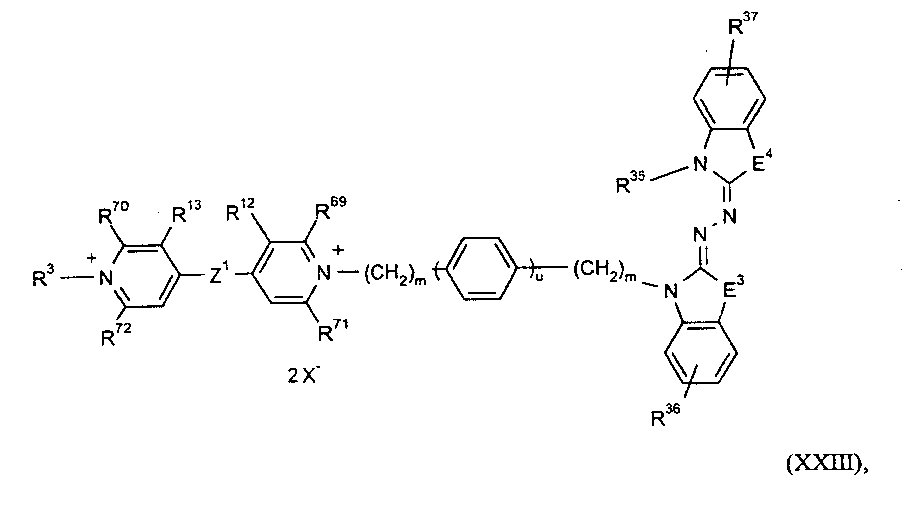 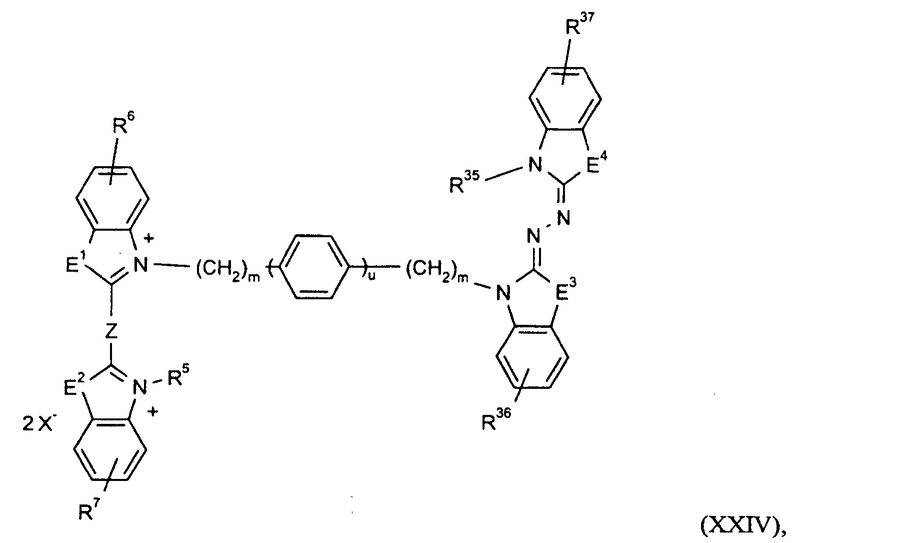 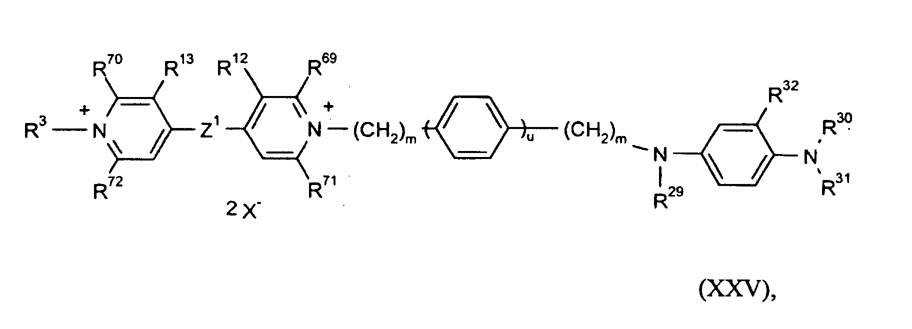 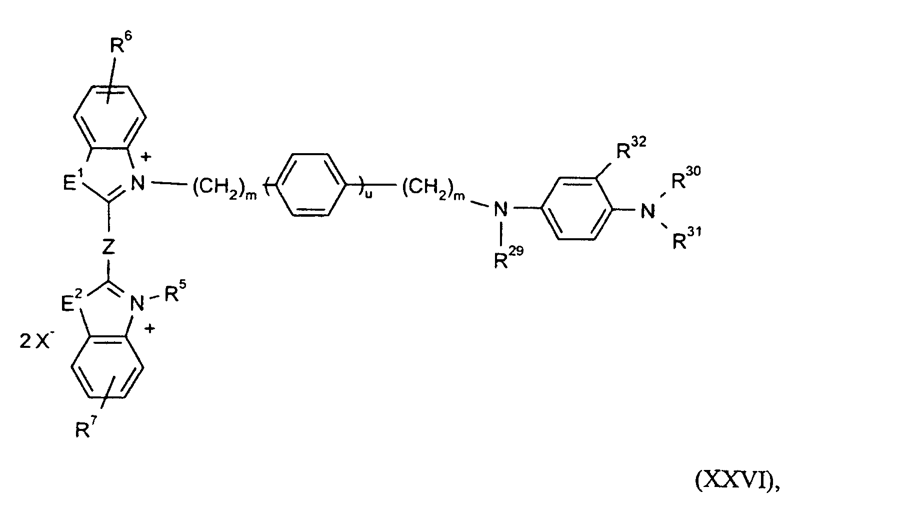 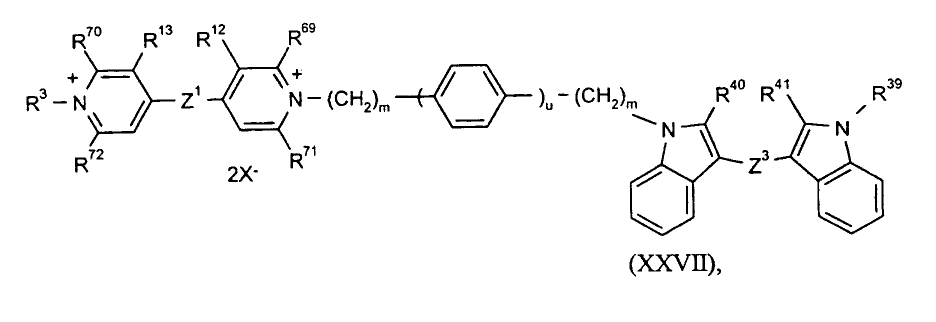 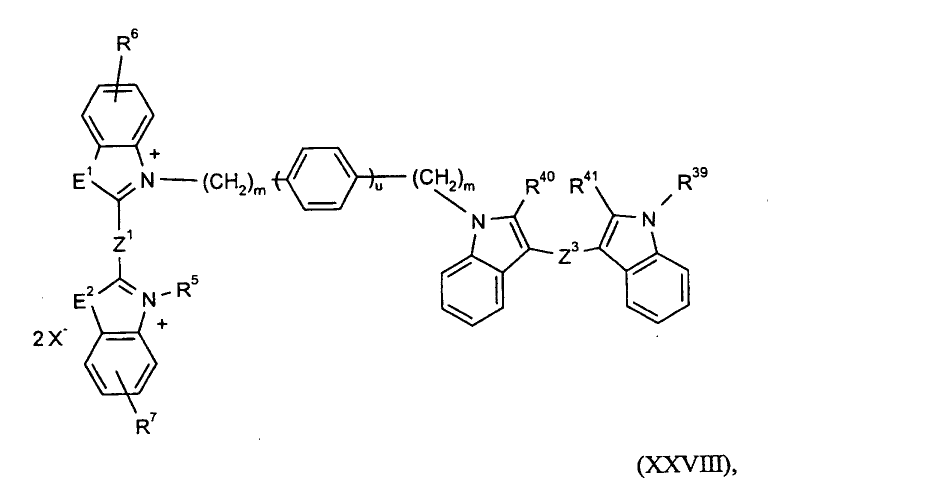 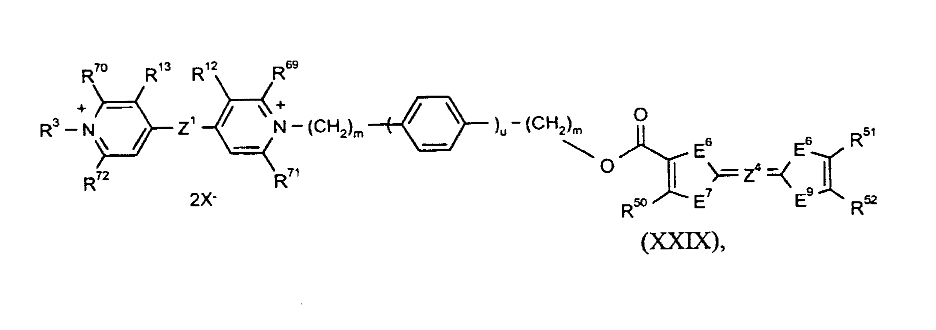 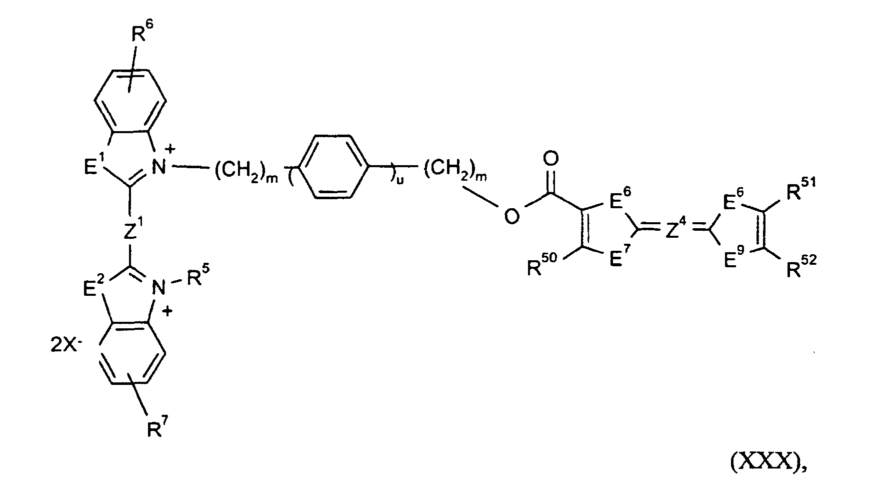 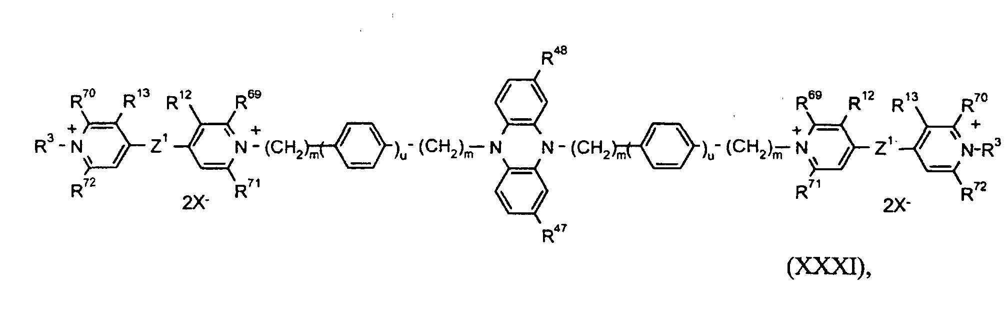 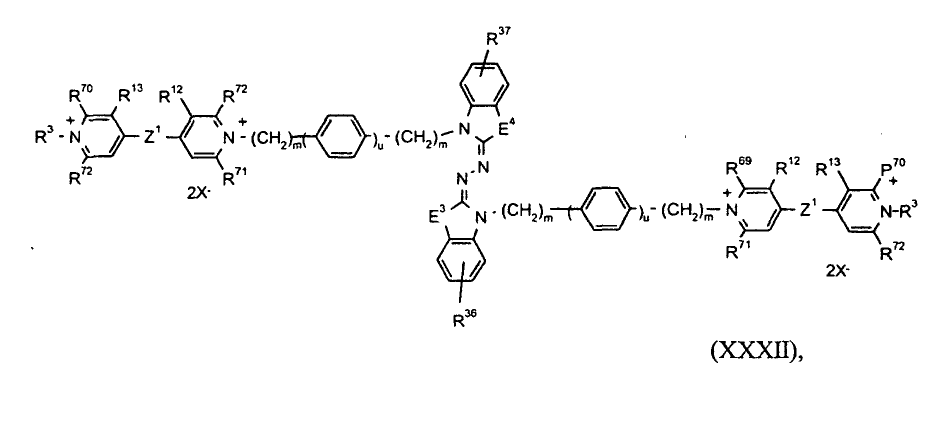 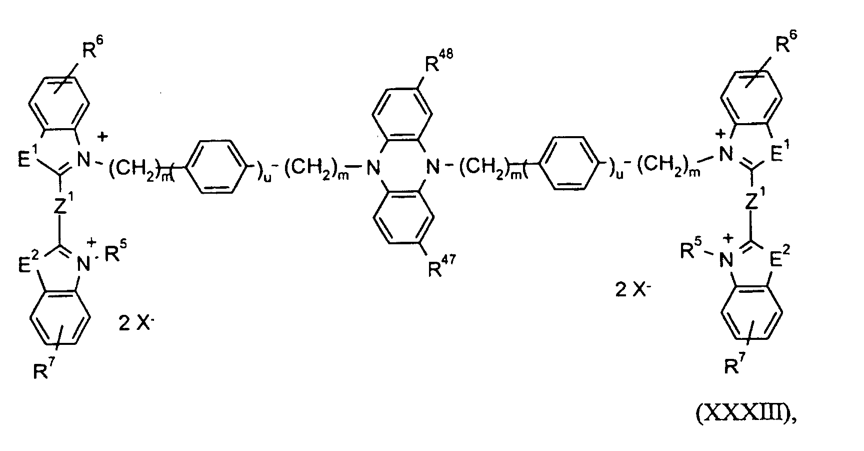 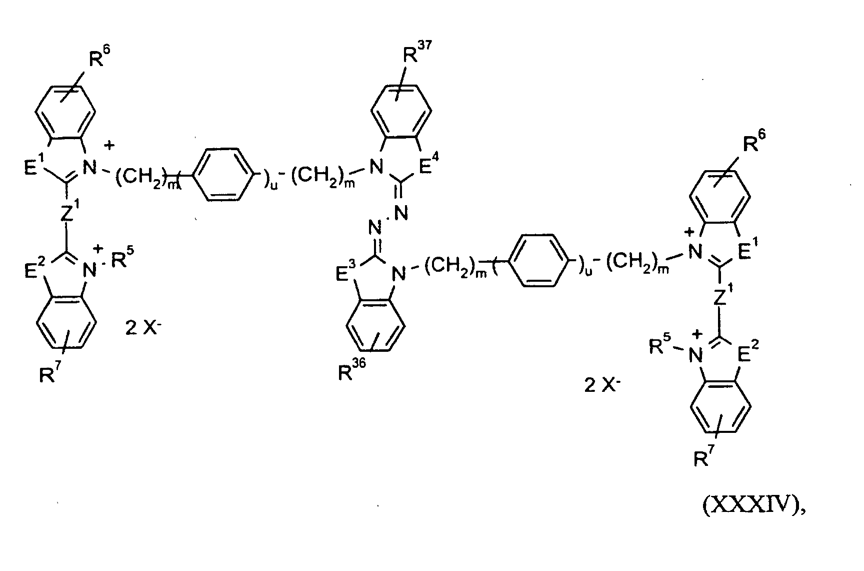 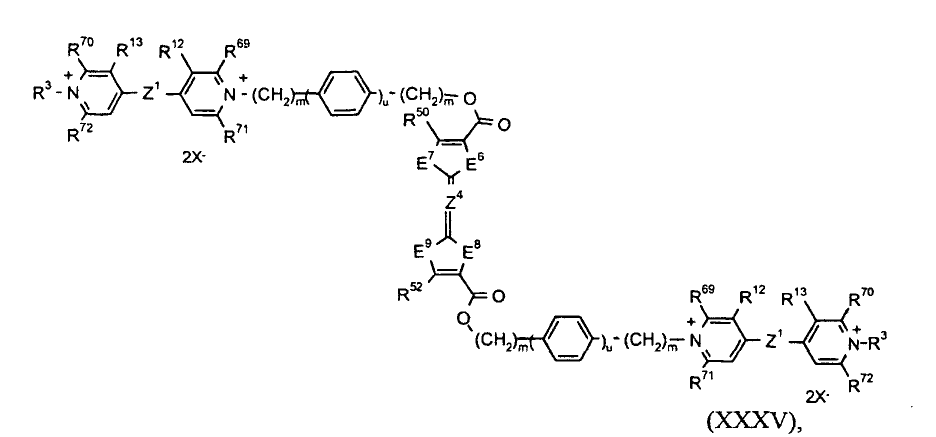 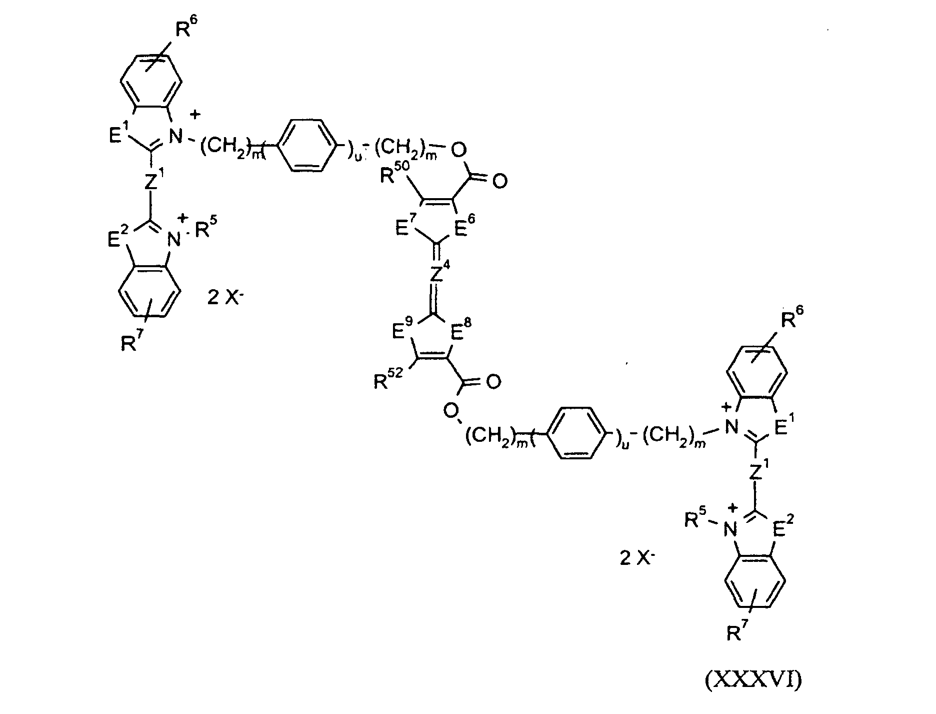 or at least one substance of the formula (Ic) corresponding to one of the formulae 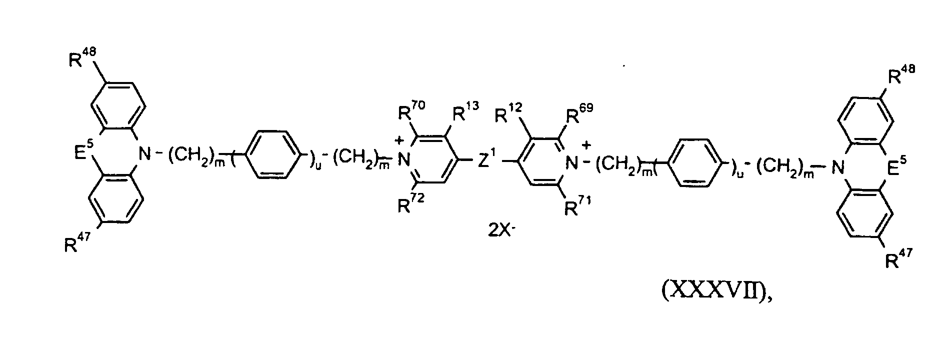 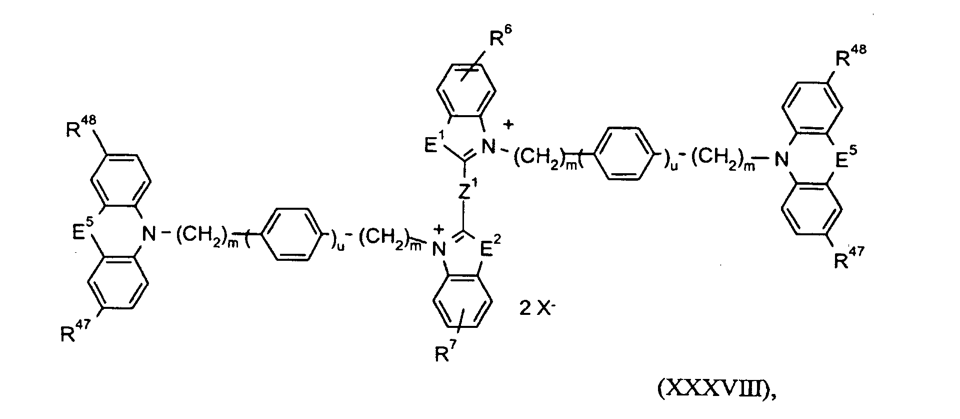 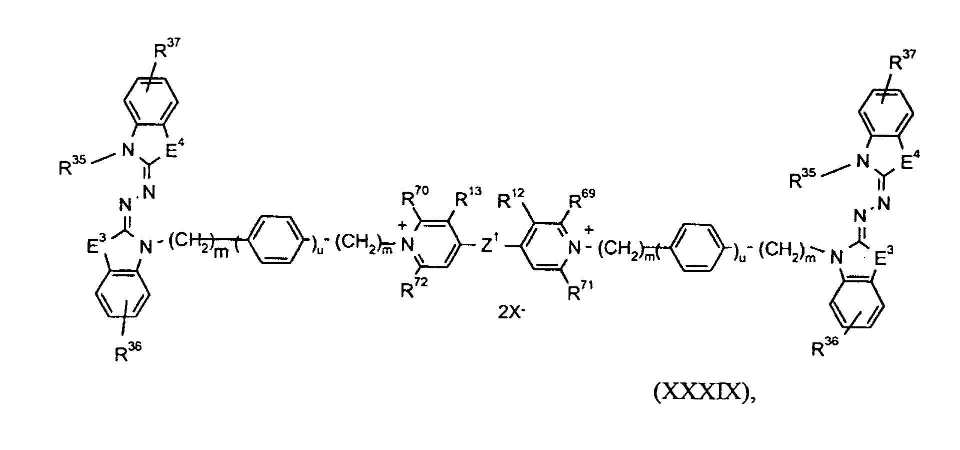 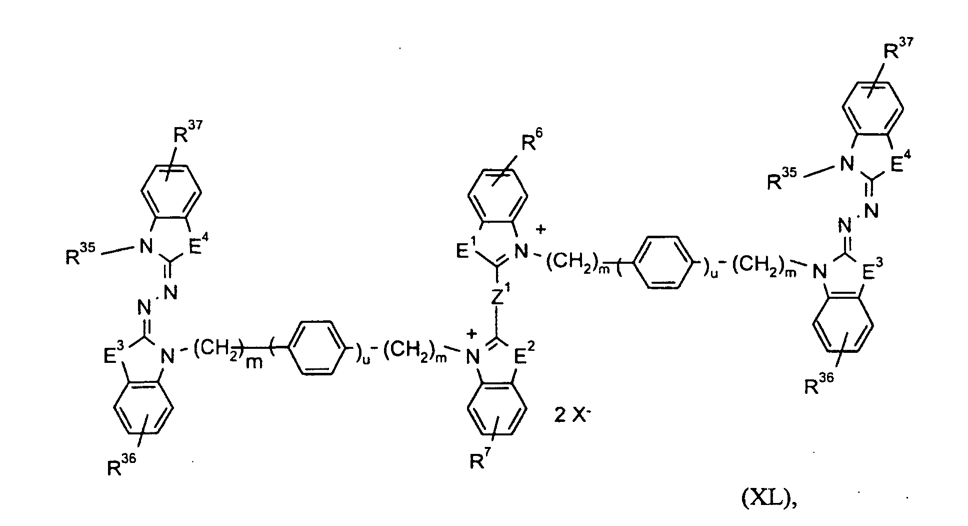 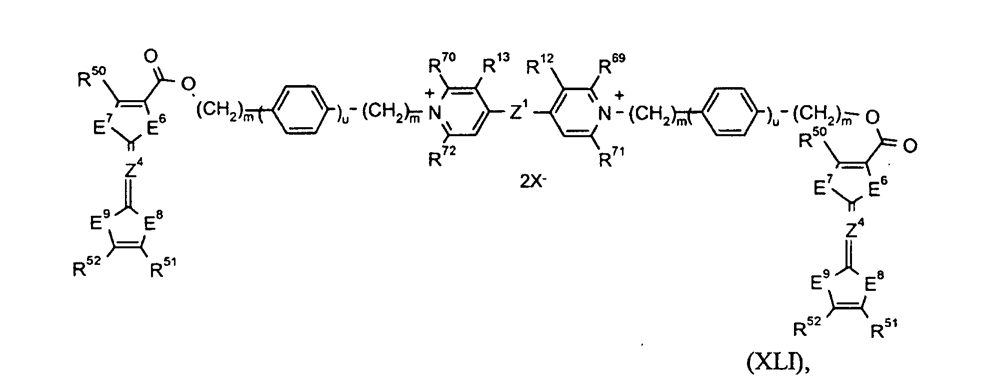 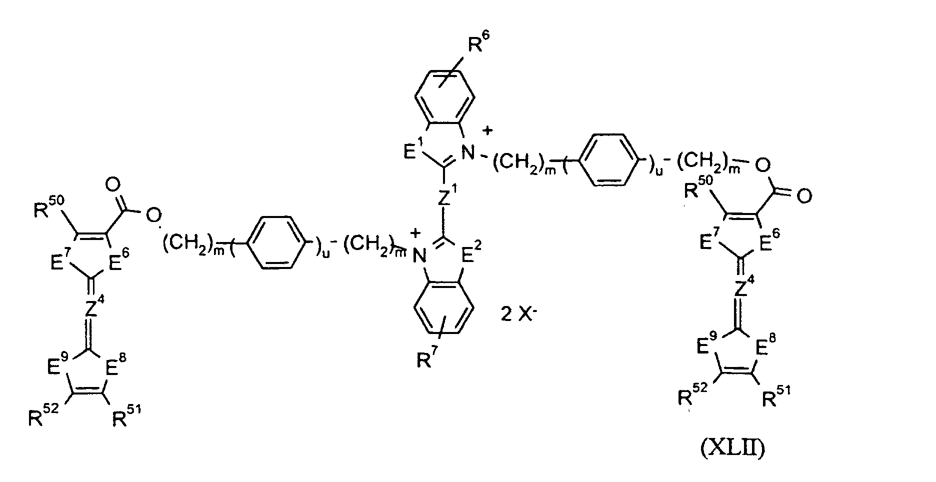 in which
R³, R⁵, R³⁵ and R³⁹ independently of one another represent methyl, ethyl, propyl, butyl, pentyl, hexyl or benzyl,
R⁶, R⁷ and R³⁶, R³⁷ in pairs are identical and represent hydrogen, methyl, methoxy, chlorine, cyano or methoxycarbonyl,
R¹² and R¹³ represent hydrogen or, if Z¹ denotes a direct bond, together represent a CH=CH bridge,
R⁶⁹ to R⁷² are identical and represent hydrogen or methyl,
E¹ and R² are identical and represent O or S,
Z¹ represents a direct bond or -CH=CH-,
R³², R⁴⁷ and R⁴⁸ represent hydrogen,
E³ to E⁵ independently of one another represent O, S or NR⁵⁹, but where E³ and E⁴ are identical,
R²⁹ to R³¹ and R⁵⁹ independently of one another represent methyl, ethyl, propyl, butyl, pentyl, hexyl or benzyl, where R²⁹ to R³¹ are preferably identical,
R⁴⁰ and R⁴¹ are identical and represent hydrogen, methyl, ethyl, propyl, butyl or phenyl,
Z³ represents a direct bond, -CH=CH- or -N=N-,
R⁵⁰ to R⁵² independently of one another represent hydrogen, methyl, methoxy, chlorine, cyano, methoxycarbonyl, ethoxycarbonyl or phenyl, but are preferably identical,
E⁶ to E⁹ are identical to one another and represent S, Se or NR⁵⁹,
Z⁴ represents a direct double bond or a =CH-CH= or =N-N= bridge,
m represents an integer from 1 to 5,
u represents 0 or 1 and
X⁻ represents a colourless anion which is redox-inert under the conditions.

13. Process for the preparation of electrochromic substances according to Claims 10 to 12, **characterized in that** it comprises reacting compounds of the formula
OX₂-B-A (XLIII)
with compounds of the formula
RED₁ (XLIV)
or compounds of the formula
OX₂ (XLV)
with compounds of the formula
A-B-RED₁ (XLVI)
in which
A denotes a leaving group, such as chlorine, bromine, iodine, OSO₂-alkyl, OSO₂-perfluoroalkyl or OSO₂-aryl
and OX₂, RED₁ and B have the meaning given in Claim 10.

14. Electrochromic liquid comprising an electrochromic system according to at least one of Claims 1 to 9 and at least one inert solvent.

15. Electrochromic device comprising an electrochromic liquid according to Claim 14.

16. Electrochromic device according to Claim 15, **characterized in that** it is constructed as a cell, such as, for example, a solar cell, or as a window pane, mirror, sunroof or display.

17. Electrochromic device according to Claims 15 and 16, **characterized in that** it comprises two transparent panes of glass or plastic facing one another, one of which is optionally metallized and the sides of which facing one another are coated with an electrically conductive coating, the electrochromic liquid being contained between the two panes.

## Revendications

1. Système électrochrome contenant au moins une substance oxydable RED₁ qui, en cédant des électrons à une anode, et au moins une substance réductible OX₂ qui, en prélevant des électrons sur une cathode, dans chaque cas avec augmentation de l'extinction dans le domaine visible du spectre, passe d'une forme faiblement colorée ou incolore à une forme colorée OX₁ ou RED₂, où, après égalisation des charges, la forme faiblement colorée ou incolore est reformée, **caractérisé en ce qu'**au moins l'une des substances RED₁ et OX₂ contenues sont liées l'une à l'autre de manière covalente par le biais d'un pont.

2. Système électrochrome selon la revendication 1, **caractérisé en ce qu'**il contient au moins une substance électrochrome de formule (I) 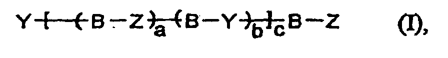 où
Y et Z représentent indépendamment l'un de l'autre un reste OX₂ ou RED₁, mais où au moins un Y représente OX₂ et au moins un Z représente RED₁,
où
OX₂ représente le reste d'un système redox électrochimiquement réductible de manière réversible, et
RED₁ représente le reste d'un système redox électrochimiquement oxydable de manière réversible,
B représente un élément de pont,
c représente un nombre entier de 0 à 1, et
a et b représentent indépendamment l'un de l'autre un nombre entier de 0 à 5.

3. Système électrochrome selon la revendication 1 et 2,: **caractérisé en ce qu'**il contient au moins une substance électrochrome de formule (I) où Y représente OX₂ et Z représente RED₁ et Y et Z alternent dans leur succession.

4. Système électrochrome selon les revendications 1 à 3 **caractérisé en ce qu'**il contient au moins une substance électrochrome des formules :
OX₂-B-RED₁ (Ia),
OX₂-B-RED₁-B-OX₂ (Ib),
RED₁-B-OX₂-B-RED₁ (Ic)
ou
OX₂-(B-RED₁-B-OX₂)_{d}-B-RED₁ (Id),
où
OX₂, RED₁ et B ont la signification indiquée ci-dessus, et
d représente un nombre entier de 1 à 5.

5. Système électrochrome selon les revendications 1 à 4, **caractérisé en ce qu'**il contient au moins une substance électrochrome des formules (Ia)-(Id),
où
OX₂ représente le reste d'une substance cathodiquement réductible qui présente, dans le voltammogramme cyclique enregistré dans un solvant inerte à la température ambiante, au moins deux ondes de réduction chimiquement réversibles, où la première de ces ondes de réduction conduit à une augmentation de l'extinction à au moins une longueur d'onde dans le domaine visible du spectre électromagnétique,
RED₁ représente le reste de la substance anodiquement oxydable de manière réversible qui, dans le voltammogramme cyclique enregistré dans un solvant inerte à la température ambiante, présente au moins deux ondes d'oxydation chimiquement réversibles, où la première de ces ondes d'oxydation conduit à une augmentation de l'extinction à au moins une longueur d'onde dans le domaine visible du spectre électromagnétique, et
B représente un élément de pont.

6. Système électrochrome selon les revendications 1 à 5, **caractérisé en ce qu'**il contient au moins une substance des formules (Ia)-(Id) où
OX₂ représente un reste des formules 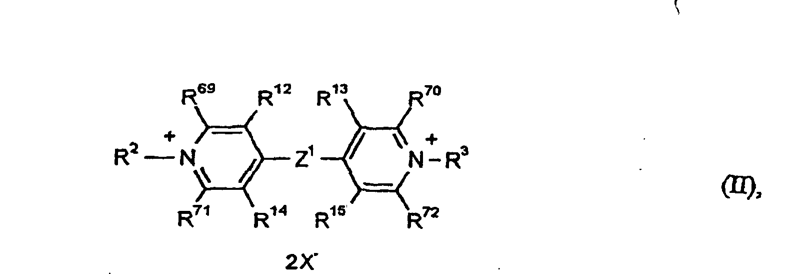 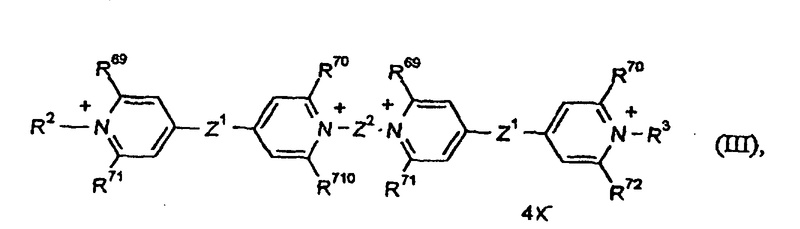 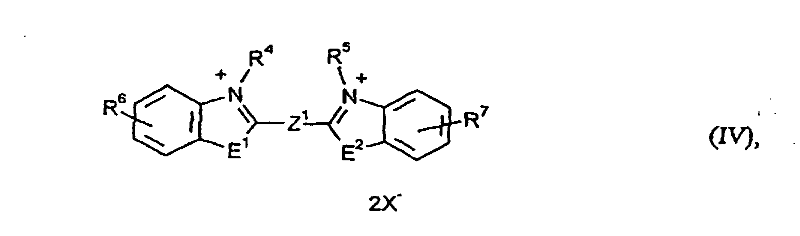 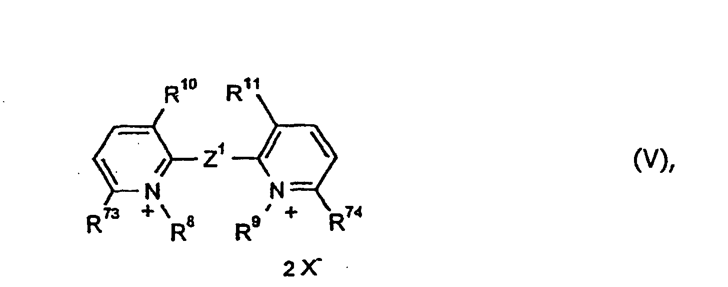 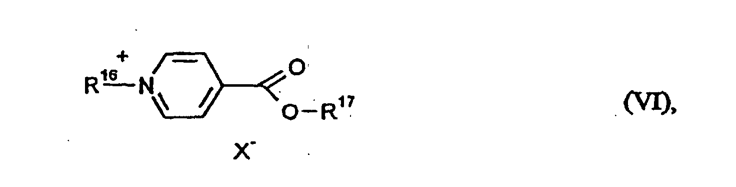 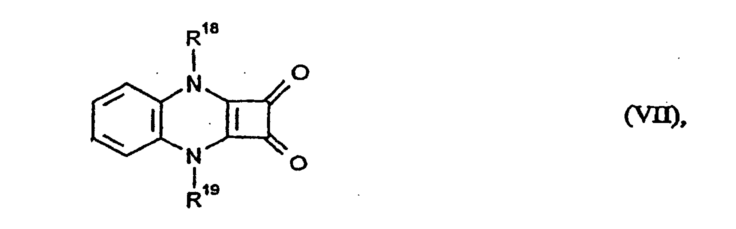 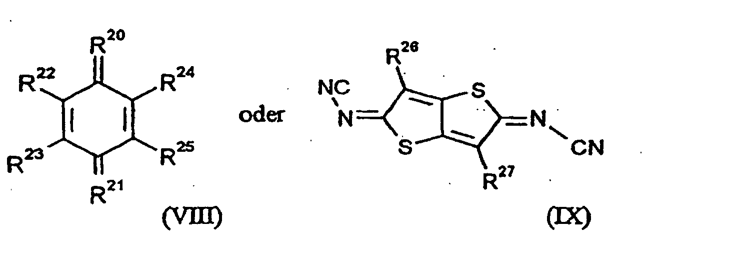
où
R² à R⁵, R⁸, R⁹, R¹⁶ à R¹⁹ représentent indépendamment les uns des autres alkyle en C₁ à C₁₈, alcényle en C₂ à C₁₂, cycloalkyle en C₃ à C₇, aralkyle en C₇ à C₁₅ ou aryle en C₆ à C₁₀ ou bien
R⁴ et R⁵ ou R⁸ et R⁹ forment ensemble un pont -(CH₂)₂- ou -(CH)₃-,
R⁶, R⁷ et R²² à R²⁵ représentent indépendamment les uns des autres l'hydrogène, alkyle en C₁ à C₄, alcoxy en C₁ à C₄, halogène, cyano, nitro ou alcoxy en C₁-C₄-carbonyle ou bien
R²² et R²³ et/ou R²⁴ et R²⁵ forment un pont -CH=CH-CH=CH-,
R¹⁰ et R¹¹ ; R¹² et R¹³ ; R¹⁴ et R¹⁵ représentent indépendamment les uns des autres l'hydrogène ou par paire un pont -(CH₂)₂-, -(CH₂)₃- ou -CH=CH-,
R⁶⁹ à R⁷⁴ représentent indépendamment les uns des autres l'hydrogène ou alkyle en C₁-C₆, ou bien
R⁶⁹ ; R¹² et/ou R⁷⁰ ; R¹³ forment un pont -CH=CH-CH=CH-,
R²⁰ et R²¹ représentent indépendamment l'un de l'autre O, N-CN, C(CN)₂
ou N-aryle en C₆-C₁₀,
R²⁶ représente l'hydrogène, alkyle en C₁ à C₄, alcoxy en C₁ à C₄, halogène, cyano, nitro, alcoxy en C₁-C₄-carbonyle ou aryle en C₆-C₁₀,
E¹ et E² représentent indépendamment l'un de l'autre O, S, NR¹ ou C(CH₃)₂ ou bien
E¹ et E² forment ensemble un pont -N-(CH₂)₂-N-,
R¹ représente alkyle en C₁ à C₁₈, alcényle en C₂ à C₁₂, cycloalkyle en C₄ à C₇, aralkyle en C₇ à C₁₅, aryle en C₆ à C₁₀,
Z¹ représente une liaison directe, -CH=CH-, -C(CH₃)=CH-, -C(CN)=CH-, -CCl=CCl-, -C(OH)=CH-, -CCl=CH-, -C≡C-, -CH=N-N=CH-, -C(CH₃)=N-N=C(CH₃)- ou -CCl=N-N=CCl-,
Z² représente -(CH₂)ᵣ- ou -CH₂-C₆H₄-CH₂-,
r représente un nombre entier de 1 à 10 et
X⁻ représente un anion inerte dans les conditions redox,
où la liaison avec l'élément de pont B a lieu par le biais de l'un des restes R²-R¹⁹, R²²-R²⁷ ou bien, dans le cas où E¹ ou E² représente NR¹, par le biais de R¹, et les restes cités représentent alors une liaison directe,
RED₁ représente l'un des restes suivants 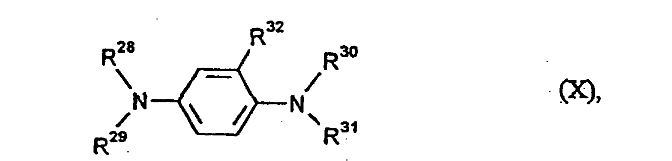 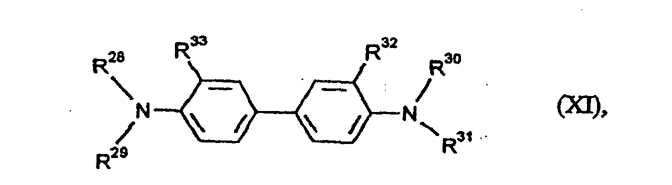 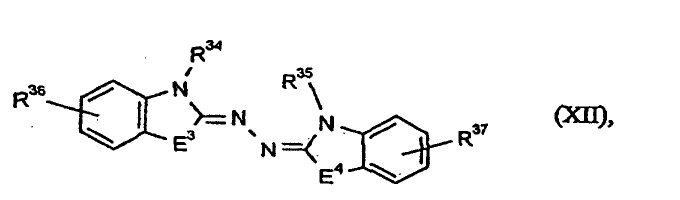 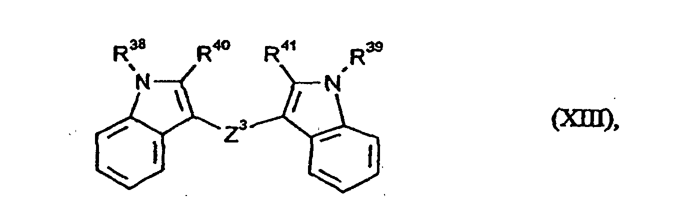 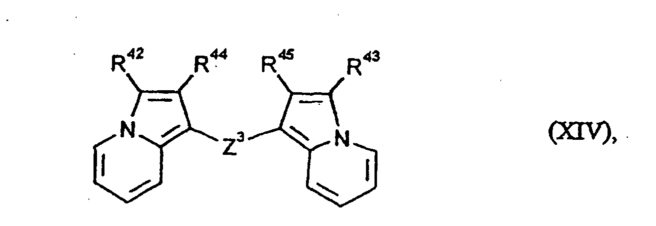 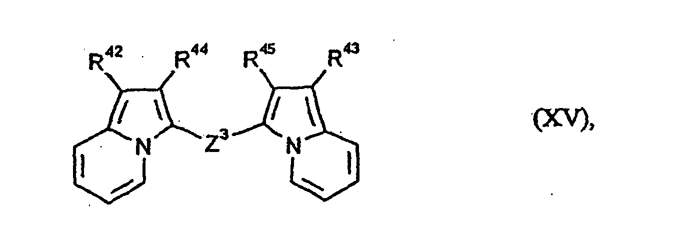 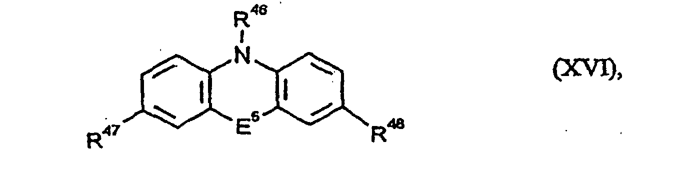 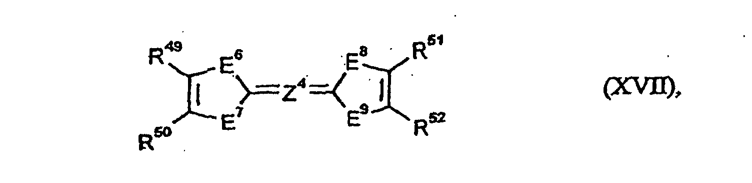 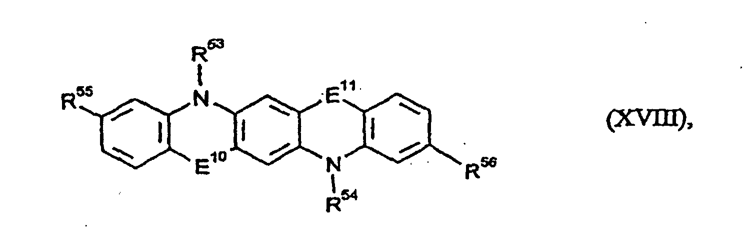 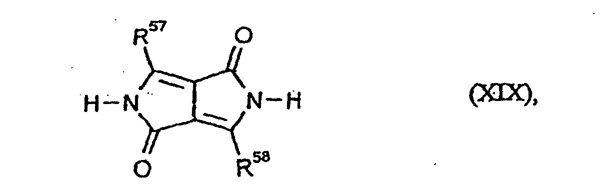 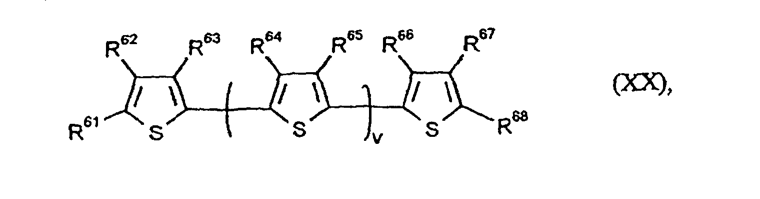
où
R²⁸ à R³¹, R³⁴, R³⁵, R³⁸, R³⁹, R⁴⁶, R⁵³ et R⁵⁴ représentent indépendamment les uns des autres alkyle en C₁ à C₁₈, alcényle en C₂ à C₁₂, cycloalkyle en C₃ à C₇, aralkyle en C₇ à C₁₅ ou aryle en C₆ à C₁₀, et R⁴⁶, R⁵³ et R⁵⁴ représentent en outre l'hydrogène,
R³², R³³, R³⁶, R³⁷, R⁴⁰, R⁴¹, R⁴² à R⁴⁵, R⁴⁷, R⁴⁸, R⁴⁹ à R⁵² et R⁵⁵ à R⁵⁷ représentent indépendamment les uns des autres l'hydrogène, alkyle en C₁ à C₄, alcoxy en C₁ à C₄, halogène, cyano, nitro, alcoxy en C₁ à C₄-carbonyle ou aryle en C₆ à C₁₀ et R⁵⁷ et R⁵⁸ représentent en outre un cycle hétérocyclique à 5 ou 6 chaînons aromatique ou quasi-aromatique éventuellement condensé au benzène et R⁴⁸ représente en outre NR⁷⁵R⁷⁶,
R⁴⁹ et R⁵⁰ et/ou R⁵¹ et R⁵² forment un pont -(CH₂)₃-, -(CH₂)₄-, -(CH₂)₅- ou -CH=CH-CH=CH-,
Z³ représente une liaison directe, un pont -CH=CH- ou -N=N-,
=Z⁴= représente une double liaison directe, un pont =CH-CH= ou =N-N=,
E³ à E⁵, E¹⁰ et E¹¹ représentent indépendamment les uns des autres O, S, NR⁵⁹ ou C(CH₃)₂ et E⁵ représente en outre C=O ou SO₂, ou bien
E³ et E⁴ représentent indépendamment l'un de l'autre -CH=CH-,
E⁶ à E⁹ représentent indépendamment les uns des autres S, Se ou NR⁵⁹,
R⁵⁹, R⁷⁵ et R⁷⁶ représentent indépendamment les uns des autres alkyle en C₁ à C₁₂, alcényle en C₂ à C₈, cycloalkyle en C₃ à C₇, aralkyle en C₇ à C₁₅ ou aryle en C₆ à C₁₀ et R⁷³ représente en outre l'hydrogène ou bien
R⁷³ et R⁷⁴ dans la signification de NR⁷³R⁷⁴ représentent avec l'atome N auquel ils sont liés un cycle saturé à 5 ou 6 chaînons qui peut contenir d'autres hétéroatomes,
R⁶¹ à R⁶⁸ représentent indépendamment les uns des autres l'hydrogène, alkyle en C₁ à C₆, alcoxy en C₁ à C₄, cyano, alcoxy en C₁ à C₄-carbonyle ou aryle en C₆ à C₁₀, ou bien
R⁶¹ ; R⁶² et R⁶⁷ ; R⁶⁸ forment ensemble indépendamment les uns des autres un pont -(CH₂)₃-, -(CH₂)₄- ou -CH=CH-CH=CH-,
v représente un nombre entier entre 0 et 10,
où la liaison avec l'élément de pont B a lieu par le biais de l'un des restes R²⁸-R⁵⁸, R⁶¹, R⁶², R⁶⁷, R⁶⁸ ou bien, dans le cas où l'un des restes E³-E¹¹ représente NR⁵⁹, par le biais de R⁵⁹, et les restes cités représentent alors une liaison directe, et
B représente un élément de pont de formule -(CH₂)ₙ- ou -[Y¹ₛ(CH₂)ₘ-Y²]ₒ-(CH₂)ₚ-Y³_{q}- qui sont chacun éventuellement substitués par alcoxy en C₁ à C₄, halogène ou phényle,
Y¹ à Y³ représentent indépendamment les uns des autres O, S, NR⁶⁰, COO, CONH, NHCONH, cyclopentanediyle, cyclohexanediyle, phénylène ou naphtylène,
R⁶⁰ représente alkyle en C₁ à C₆, alcényle en C₂ à C₆, cycloalkyle en C₄ à C₇, aralkyle en C₇ à C₁₅ ou aryle en C₆ à C₁₀,
n représente un nombre entier de 1 à 12,
m et p représentent indépendamment l'un de l'autre un nombre entier de 0 à 8,
o représente un nombre entier de 0 à 6 et
q et s représentent indépendamment l'un de l'autre 0 ou 1.

7. Système électrochrome selon les revendications 1 à 6, **caractérisé en ce qu'**il contient au moins une substance des formules (Ia)-(Id),
où
OX₂ représente un reste des formules (II), (III), (IV) ou (V),
où
R², R³, R⁴, R⁵, R⁸ et R⁹ représentent indépendamment les uns des autres alkyle en C₁ à C₁₂, alcényle en C₂ à C₈, cycloalkyle en C₅ à C₇, aralkyle en C₇ à C₁₅ ou aryle en C₆ à C₁₀,
R⁶ et R⁷ représentent indépendamment l'un de l'autre l'hydrogène, méthyle, éthyle, méthoxy, éthoxy, le fluor, le chlore, le brome, cyano, nitro, méthoxycarbonyle ou éthoxycarbonyle,
R¹⁰, R¹¹ ; R¹², R¹³ et R¹⁴, R¹⁵ représentent indépendamment les uns des autres l'hydrogène ou bien, dans le cas où Z¹ représente une liaison directe, dans chaque cas par paires conjointement un pont -(CH₂)₂-, -(CH₂)₃- ou -CH=CH-,
ou bien
R⁴, R⁵ et R⁸, R⁹ représentent indépendamment les uns des autres dans chaque cas par paires conjointement un pont -(CH₂)₂- ou -(CH₃)₃- lorsque Z¹ représente une liaison directe,
R⁶⁹ à R⁷⁴ représentent indépendamment les uns des autres l'hydrogène ou alkyle en C₁ à C₄,
E¹ et E² sont identiques et représentent O, S, NR¹ ou C(CH₃)₂ ou forment ensemble un pont -N-(CH₂)₂-N-,
R¹ représente alkyle en C₁ à C₁₂, alcényle en C₂ à C₄, cycloalkyle en C₅ à C₇, aralkyle en C₇ à C₁₅ ou aryle en C₆ à C₁₀,
Z¹ représente une liaison directe, -CH=CH-, -C(CH₃)=CH-, -C(CN)=CH-, -C≡C- ou -CH=N-N=CH-,
Z² représente -(CH)ᵣ- ou -CH₂-C₆H₄-CH₂-,
r représente un nombre entier entre 1 et 6,
X⁻ représente un anion incolore inerte dans les conditions redox,
où la liaison avec l'élément de pont B a lieu par le biais de l'un des restes R²-R¹¹ ou bien, dans le cas où E¹ ou E² représente NR¹, par le biais de R¹, et les restes cités représentent alors une liaison directe,
RED₁ représente un reste des formules (X), (XI), (XII), (XIII), (XVI), (XVII), (XVIII) ou (XX),
où
R²⁸ à R³¹, R³⁴, R³⁵, R³⁸, R³⁹, R⁴⁶, R⁵³ et R⁵⁴ représentent indépendamment les uns des autres alkyle en C₁ à C₁₂, alcényle en C₂ à C₈, cycloalkyle en C₅ à C₇, aralkyle en C₇ à C₁₅ ou aryle en C₆ à C₁₀ et
R⁴⁶, R⁵³ et R⁵⁴ représentent en outre l'hydrogène,
R³², R³³, R³⁶, R³⁷, R⁴⁰, R⁴¹, R⁴⁷ à R⁵², R⁵⁵ et R⁵⁶ représentent indépendamment les uns des autres l'hydrogène, méthyle, éthyle, méthoxy, éthoxy, le fluor, le chlore, le brome, cyano, nitro, méthoxycarbonyle, éthoxycarbonyle ou phényle et
R⁵⁷ et R⁵⁸ représentent en outre 2- ou 4-pyridyle et
R⁴⁸ représente en outre NR⁷⁵R⁷⁶,
Z³ représente une liaison directe, un pont -CH=CH- ou -N=N-,
=Z⁴= représente une double liaison directe, un pont =CH-CH= ou =N-N=,
E³ à E⁵, E¹⁰ et E¹¹ représentent indépendamment les uns des autres O, S, NR⁵⁹ ou C(CH₃)₂, E³ et E⁴ ont cependant la même signification,
E⁶ à E⁹ sont identiques et représentent S, Se ou NR⁵⁹ et
E⁵ représente en outre C=O,
E⁶ représente NR⁵⁹ où R⁵⁹ représente une liaison directe avec le pont B et
E⁷ à E⁹ possèdent la signification indiquée ci-dessus mais ne doivent pas être identiques entre eux,
R⁵⁹, R⁷⁵ et R⁷⁶ représentent indépendamment les uns des autres alkyle en C₁ à C₁₂, alcényle en C₂ à C₈, cycloalkyle en C₅ à C₇, aralkyle en C₇ à C₁₅ ou aryle en C₆ à C₁₀, et R⁷³ représente en outre l'hydrogène ou bien
R⁷³ et R⁷⁴ dans la signification de NR⁷³R⁷⁴ représentent avec l'atome N auquel ils sont liés pyrrolidino, pipéridino ou morpholino,
R⁶¹, R⁶² et R⁶⁷, R⁶⁸ représentent indépendamment les uns des autres l'hydrogène, alkyle en C₁ à C₄, méthoxycarbonyle, éthoxycarbonyle ou phényle ou représentent par paires conjointement un pont -(CH₂)₃- ou -(CH₂)₄-,
R⁶³ à R⁶⁶ représentent l'hydrogène et
v représente un nombre entier de 1 à 6,
où la liaison avec l'élément de pont B a lieu par le biais de l'un des restes R²⁸-R⁴¹, R⁴⁶-R⁵⁶, R⁶¹, R⁶², R⁶⁷, R⁶⁸ ou bien, dans le cas où l'un des restes E³-E¹¹ représente NR⁵⁹, par le biais de R⁵⁹, et les restes cités représentent alors une liaison directe,
B représente un élément de pont des formules -(CH₂)ₙ-, -(CH₂)ₘ-O-(CH₂)ₚ-, -(CH₂)ₘ-NR⁶⁰-(CH₂)ₚ-, -(CH₂)ₘ-C₆H₄-(CH₂)ₚ-, -[O-(CH₂)ₚ]ₒ-O-, -[NR⁶⁰-(CH₂)ₚ]ₒ-NR⁶⁰-, -[C₆H₄-(CH₂)ₚ]ₒ-C₆H₄-, -(CH₂)m-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)ₚ-, -(CH₂)ₘ-NHCO-(CH₂)ₜ-CONH-(CH₂)ₚ-, -(CH₂)ₘ-NHCONH-(CH₂)ₜ-NHCONH-(CH₂)ₚ-
R⁶⁰ représente méthyle, éthyle, benzyle ou phényle,
n représente un nombre entier de 1 à 10,
m et p représentent indépendamment l'un de l'autre un nombre entier de 0 à 4,
o représente un nombre entier de 0 à 2 et
t représente un nombre entier de 1 à 6.

8. Système électrochrome selon les revendications 1 à 7, **caractérisé en ce qu'**il contient au moins une substance des formules (Ia)-(Id) où
OX₂ représente un reste des formules (II), (IV) ou (V),
où
R², R⁴ et R⁸ représentent une liaison directe avec l'élément de pont B,
R³, R⁵ et R⁹ représentent indépendamment les uns des autres méthyle, éthyle, propyle, butyle, pentyle, hexyle, heptyle, octyle, benzyle ou phényle, ou bien
R³, R⁵ et R⁹, dans le cas des formules Ic et Id, représentent aussi une liaison directe avec l'élément de pont B,
R⁶ et R⁷ sont identiques et représentent l'hydrogène, méthyle, méthoxy, le chlore, cyano ou méthoxycarbonyle,
R¹⁰, R¹¹ ; ¹², R¹³ et R^{14'}, R¹⁵ Rreprésentent indépendamment les uns des autres l'hydrogène ou, dans le cas où Z¹ représente une liaison directe, dans chaque cas par paires conjointement un pont -CH=CH-,
R⁶⁹ à R⁷² sont identiques et représentent l'hydrogène, méthyle ou éthyle,
R⁷³ et R⁷⁴ représentent l'hydrogène,
E¹ et E² sont identiques et représentent O ou S,
Z¹ représente une liaison directe ou -CH=CH-,
X⁻ représente un anion incolore inerte dans les conditions redox;
RED₁ représente un reste des formules (X), (XII), (XIII), (XVI) ou (XVII),
R²⁸, R³⁴, R³⁸, R⁴⁶ et R⁴⁹ représentent une liaison directe avec l'élément de pont B,
R²⁹ à R³¹, R³⁵ et R³⁹ représentent indépendamment les uns des autres méthyle, éthyle, propyle, butyle, pentyle, hexyle, heptyle, octyle, benzyle ou phényle, ou bien
R³⁰, R³⁵ et R³⁹, dans le cas des formules Ib et Ic, représentent aussi une liaison directe avec l'élément de pont B,
R³², R⁴⁷ et R⁴⁸ représentent l'hydrogène,
R³⁶, R³⁷, R⁴⁰, R⁴¹ et R⁵⁰ à R⁵² représentent indépendamment les uns des autres l'hydrogène, méthyle, méthoxy, le chlore, cyano, méthoxycarbonyle ou phényle, ou bien
R⁵¹ dans le cas des formules Ib et Id représente aussi une liaison directe avec l'élément de pont B,
Z³ représente une liaison directe, un pont -CH=CH- ou -N=N-,
=Z⁴= représente une double liaison directe, un pont =CH-CH= ou =N-N=,
E³ à E⁵ représentent indépendamment les uns des autres O, S ou NR⁵⁹, E³ et E⁴ ont toutefois la même signification,
E⁶ à E⁹ sont identiques entre eux et représentent S, Se ou NR⁵⁹,
R⁵⁹ représente méthyle, éthyle, propyle, butyle, pentyle, hexyle, heptyle, octyle, benzyle ou phényle, ou bien
R⁵⁹ dans le cas de la formule XVI dans la formule Ib et Id représente aussi une liaison directe avec l'élément de pont B,
B représente un élément de pont des formules -(CH₂)ₙ-, -(CH₂)ₘ-O-(CH₂)ₚ-, -(CH₂)ₘ-NR⁶⁰-(CH₂)ₚ-, -(CH₂)ₘ-C₆H₄-(CH₂)ₚ-, -O-(CH₂)ₚ-O-, -NR⁶⁰-(CH₂)ₚ-NR⁶⁰-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)ₚ-, -(CH₂)ₘ-NHCO-(CH₂)ₜ-CONH-(CH₂)ₚ-, -(CH₂)ₘ-NHCONH-(CH₂)ₜ-NHCONH-(CH₂)ₚ-
R⁶⁰ représente méthyle,
n représente un nombre entier de 1 à 10,
m et p sont identiques et représentent un nombre entier de 0 à 2 et
t représente un nombre entier de 1 à 6

9. Système électrochrome selon les revendications 1 à 8, **caractérisé en ce qu'**il contient au moins une substance électrochrome de formule (Ia)
OX₂-B-RED₁ (Ia)
correspondant à l'une des formules 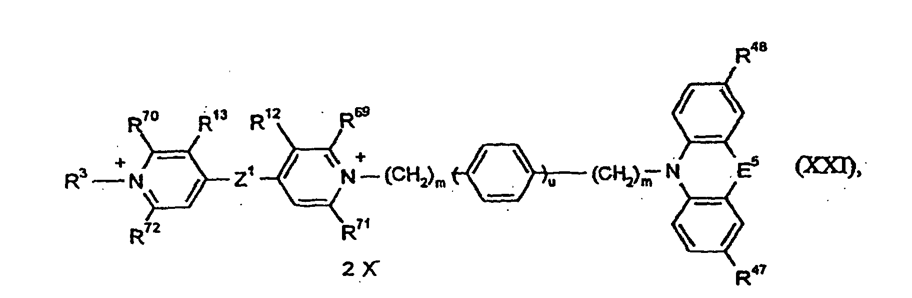 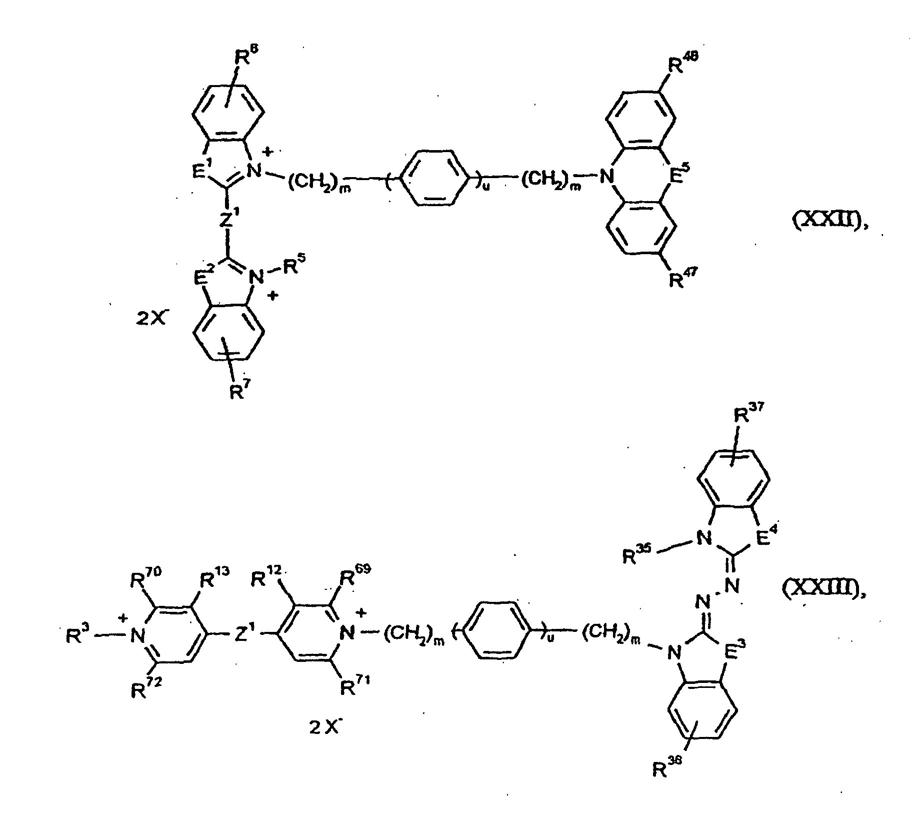 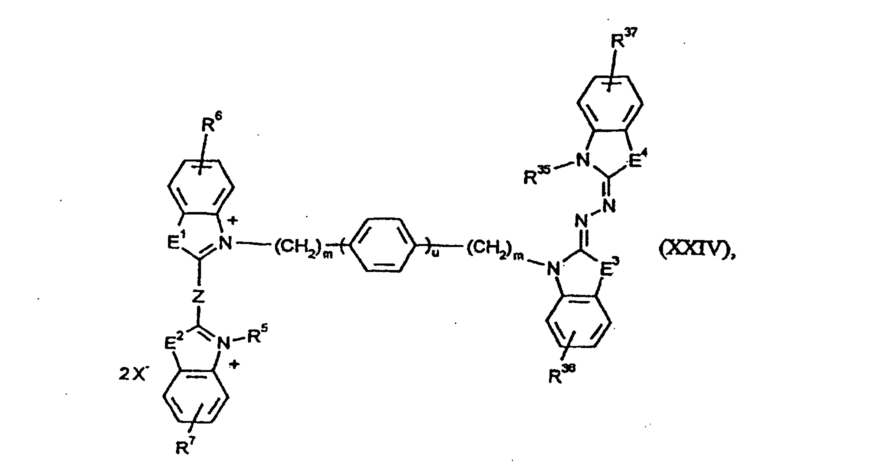 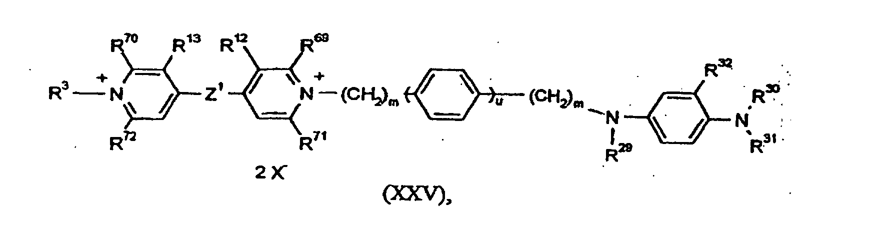 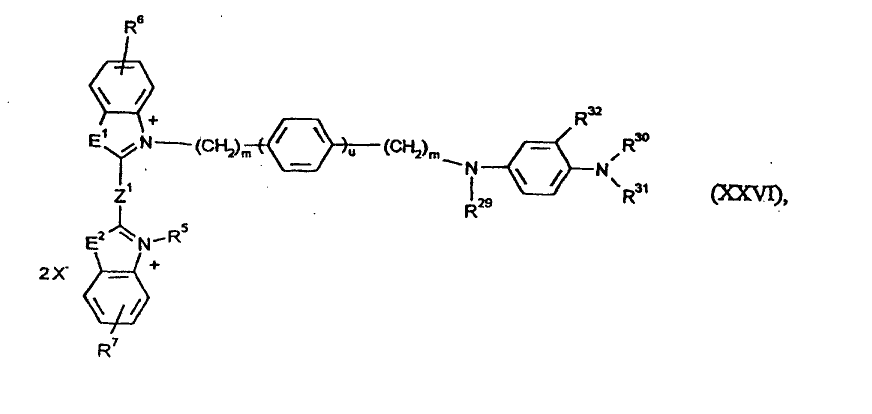 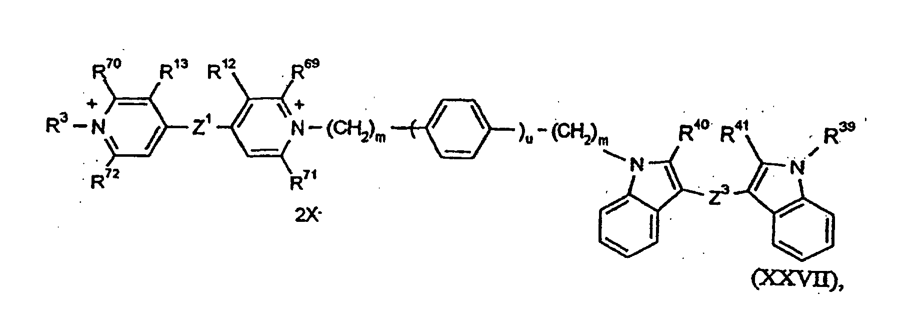 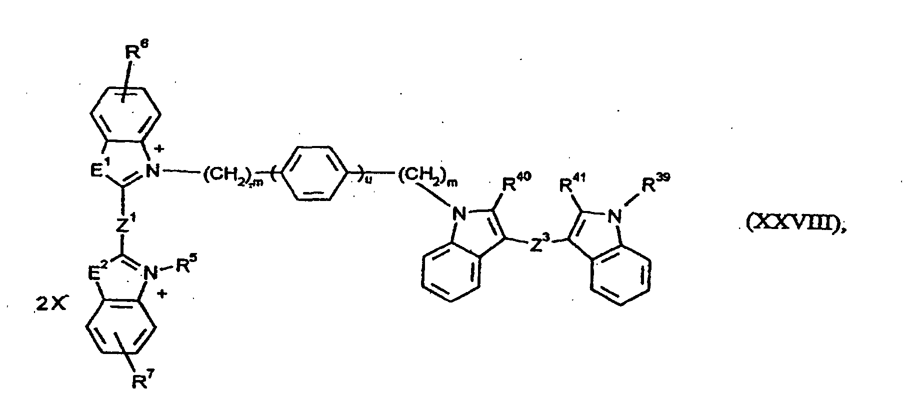 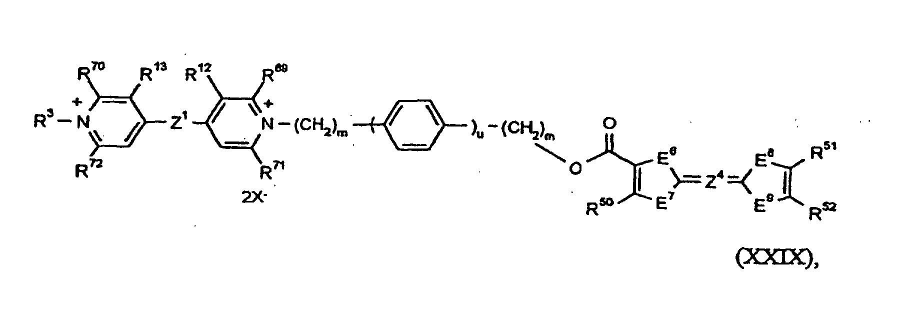 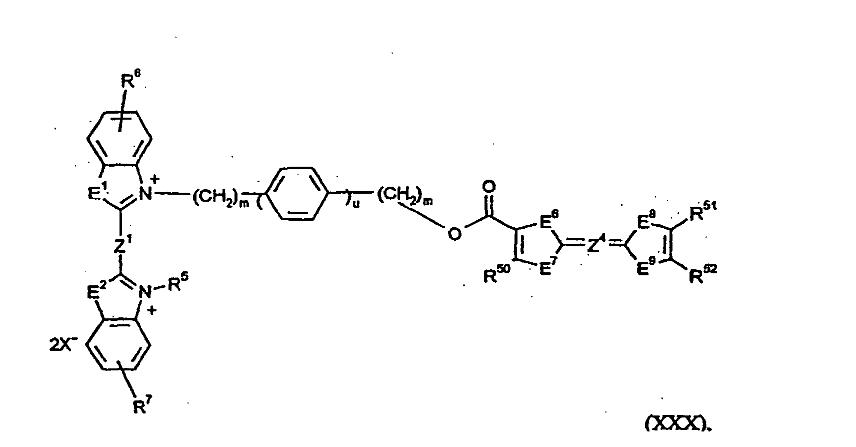 ou 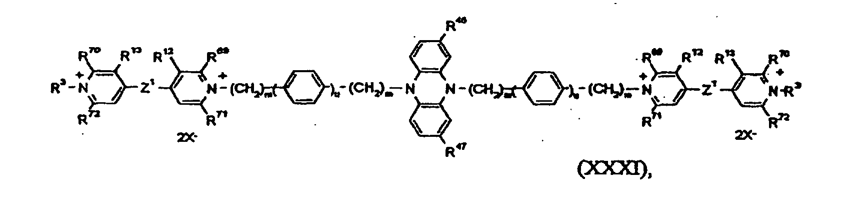 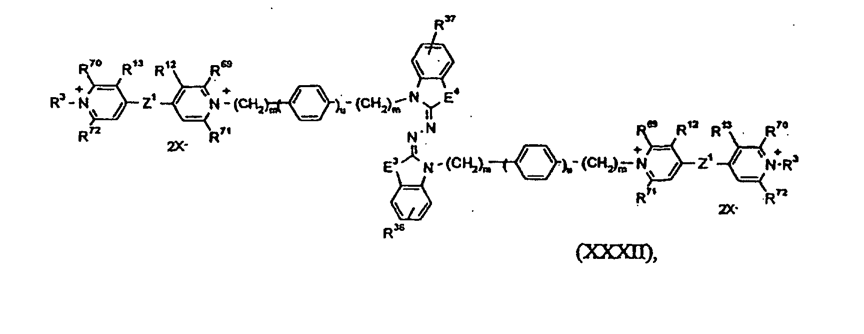 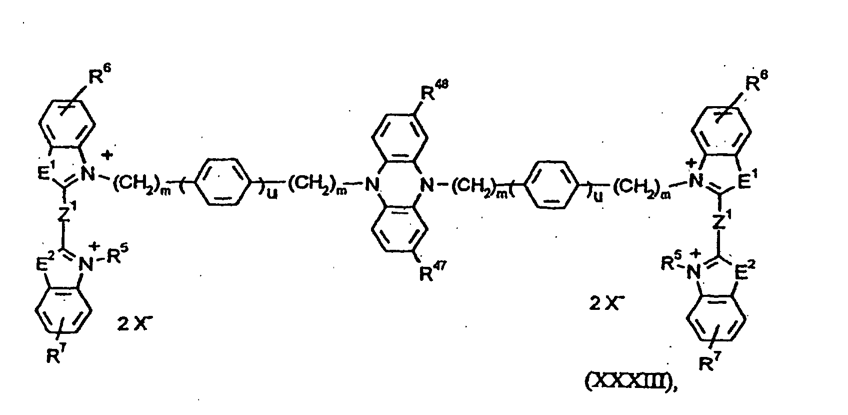 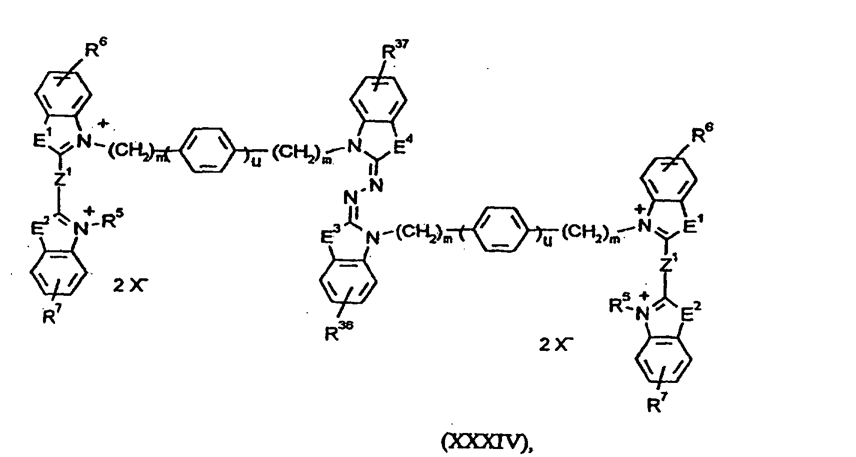 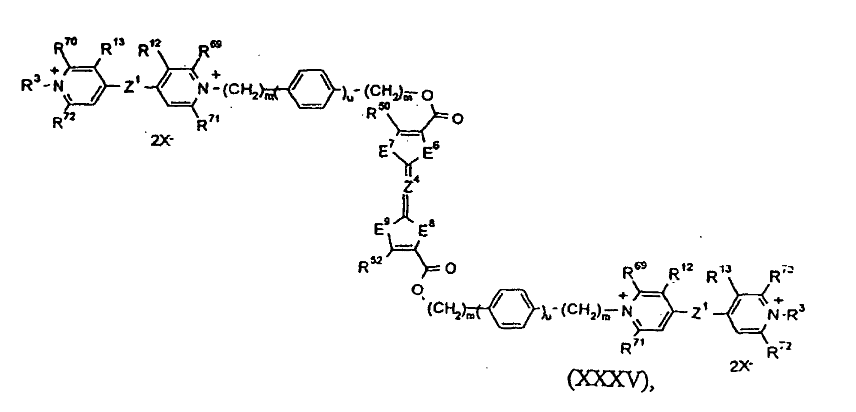 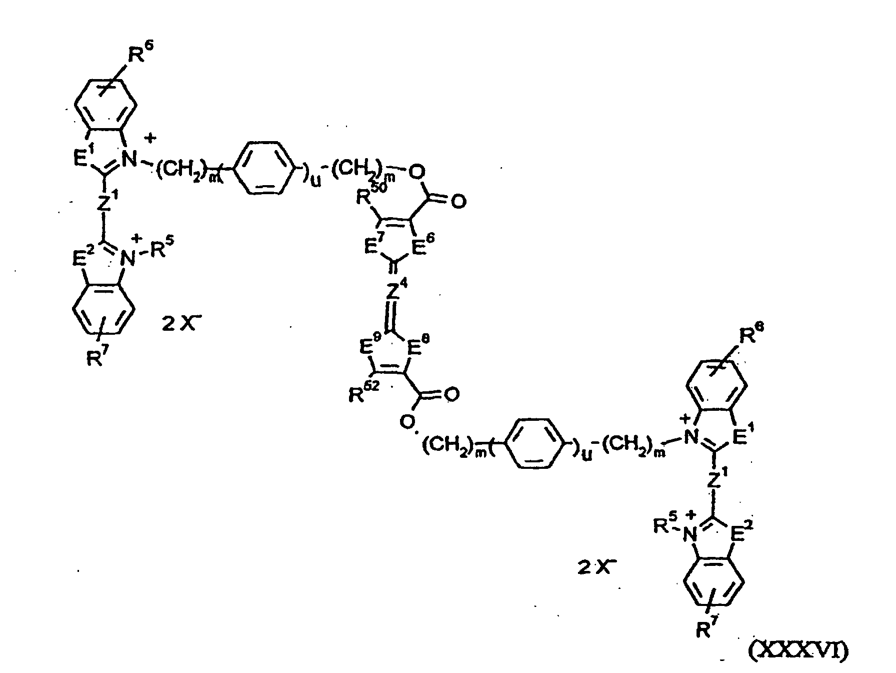 ou au moins une substance de formule (Ic) correspondant à l'une des formules 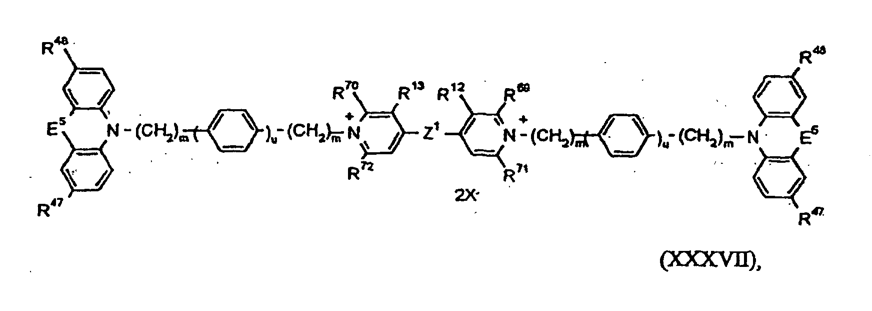 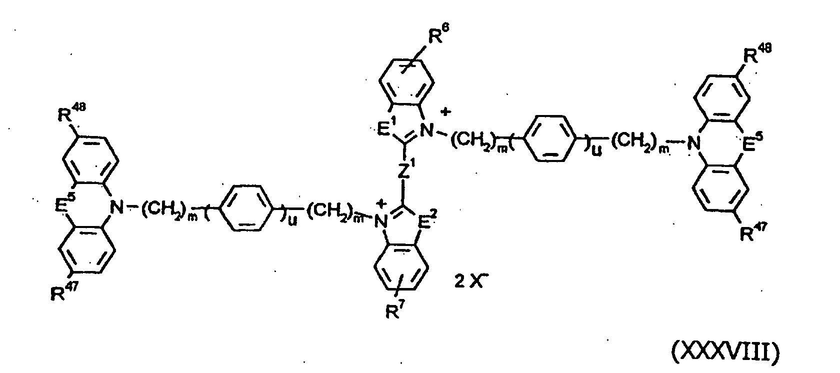 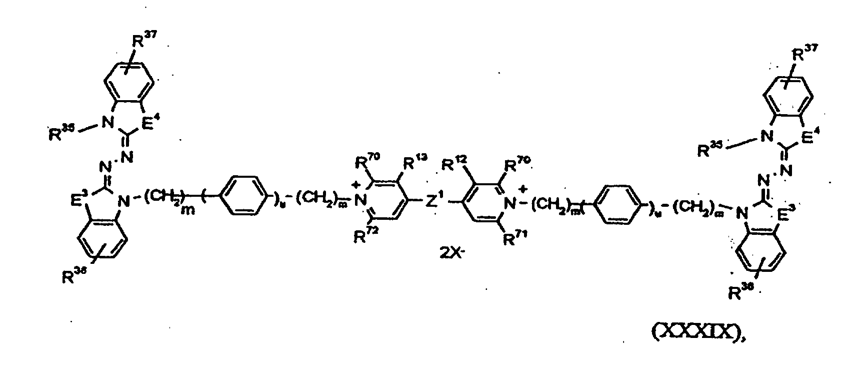 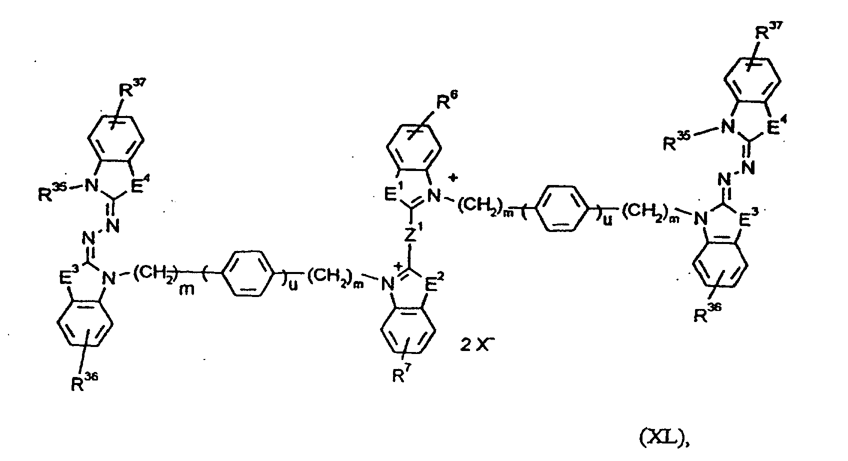 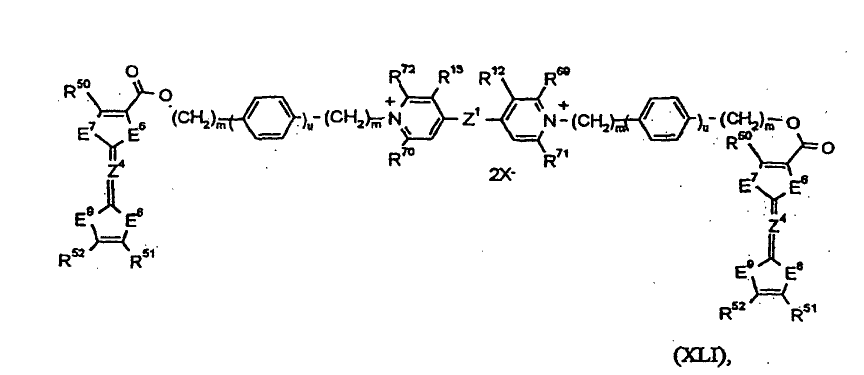 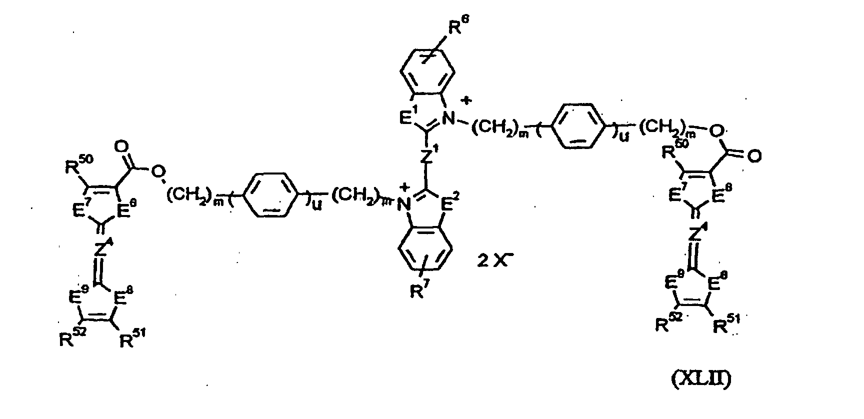 où
R³, R⁵, R³⁵ et R³⁹ représentent indépendamment les uns des autres méthyle, éthyle, propyle, butyle, pentyle, hexyle ou benzyle,
R⁶, R⁷ et R³⁶, R³⁷ sont identiques par paires et représentent l'hydrogène, méthyle, méthoxy, chloro, cyano ou méthoxycarbonyle,
R¹² et R¹³ représentent l'hydrogène ou bien, quand Z¹ représente une liaison directe, conjointement un pont -CH=CH-,
R⁶⁹ à R⁷² sont identiques et représentent l'hydrogène ou méthyle,
E¹ et E² sont identiques et représentent O ou S,
Z¹ représente une liaison directe ou -CH=CH-,
R³², R⁴⁷ et R⁴⁸ représentent l'hydrogène,
E³ à E⁵ représentent indépendamment les uns des autres O, S ou NR⁵⁹, où E³ et E⁴ sont toutefois identiques,
R²⁹ à R³¹ et R⁵⁹ représentent indépendamment les uns des autres méthyle, éthyle, propyle, butyle, pentyle, hexyle ou benzyle, où R²⁹ à R³¹ sont de préférence identiques,
R⁴⁰ et R⁴¹ sont identiques et représentent l'hydrogène, méthyle, éthyle, propyle, butyle ou phényle,
Z³ représente une liaison directe, -CH=CH- ou -N=N-,
R⁵⁰ à R⁵² représentent indépendamment les uns des autres l'hydrogène, méthyle, méthoxy, chloro, cyano, méthoxycarbonyle, éthoxycarbonyle ou phényle, mais sont toutefois de préférence identiques,
E⁶ à E⁹ sont identiques entre eux et représentent S, Se ou NR⁵⁹,
Z⁴ représente une double liaison directe, un pont =CH-CH= ou =N-N=,
m représente un nombre entier de 1 à 5,
u représente 0 ou 1 et
X⁻ représente un anion incolore inerte dans les conditions redox.

10. Substance électrochrome correspondant à la formule 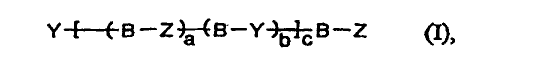 où
Y et Z où représentent indépendamment l'un de l'autre un reste OX₂ ou RED₁, où toutefois au moins un Y représente OX₂ et au moins un Z représente RED₁,
où
OX₂ représente le reste d'un système redox électrochimiquement réductible de manière réversible et RED₁ représente le reste d'un système redox électrochimiquement oxydable de manière réversible, où le reste de formule (XX) 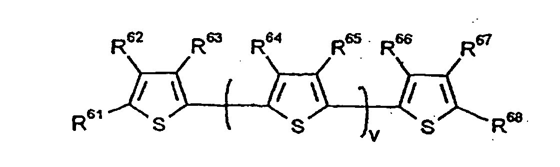 où R⁶¹-R⁶⁸ et v ont la signification indiquée dans la revendication 6, est exclus,
B représente un élément de pont,
c représente un nombre entier de 0 à 1, et
a et b représentent indépendamment l'un de l'autre un nombre entier de 0 à 5, de préférence un nombre entier de 0 à 3,
à l'exception de composés des formules 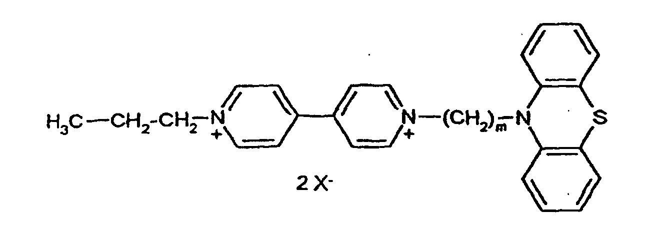 où
m représente un nombre entier de 2 à 16 et
X⁻ a la signification indiquée ci-dessus.

11. Substances electrochromes selon la revendication 10 correspondant à l'une des formules
OX₂-B-RED₁ (Ia),
OX₂-B-RED₁-B-OX₂ (Ib),
RED₁-B-OX₂-B-RED₁ (Ic)
ou
OX₂-(B-RED₁-B-OX₂)_{d}-B-RED₁ (Id),
où OX₂, RED₁ et B ont les significations indiquées dans la revendication 10 et d représente un nombre entier de 1 à 5.

12. Substances électrochromes selon les revendications 10 et 11 des formules 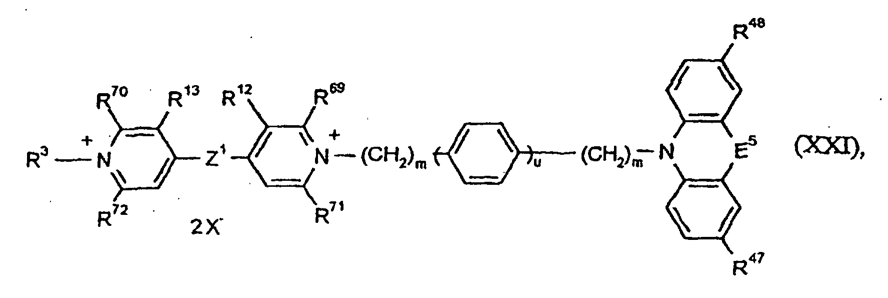 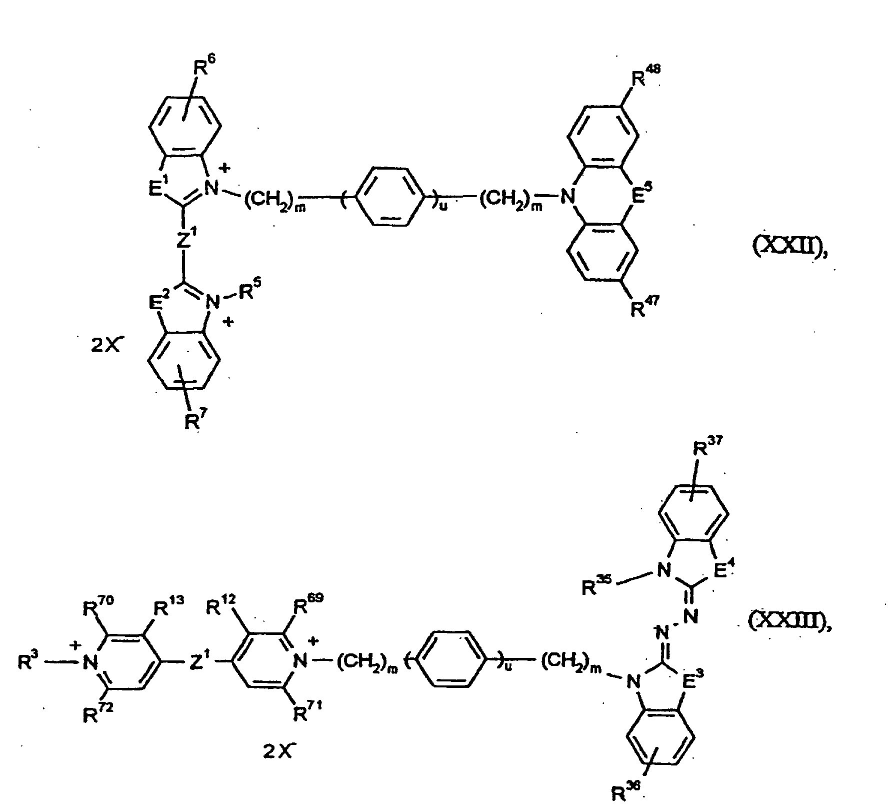 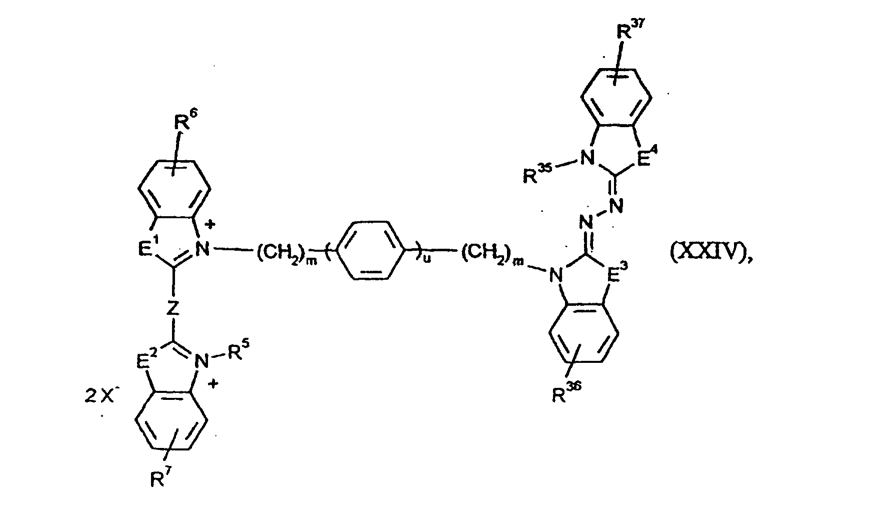 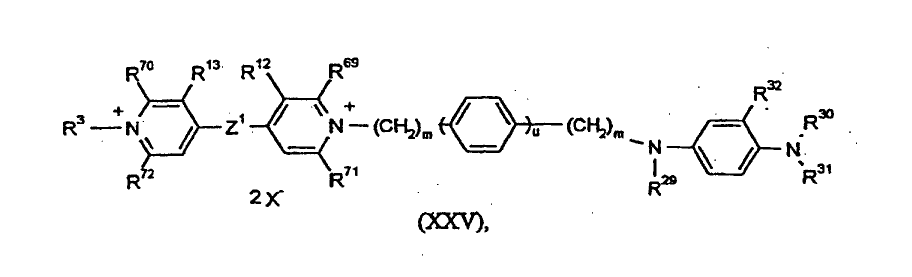 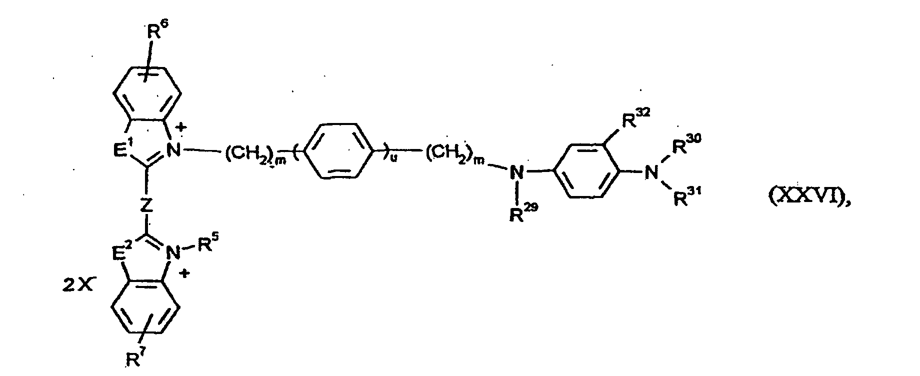 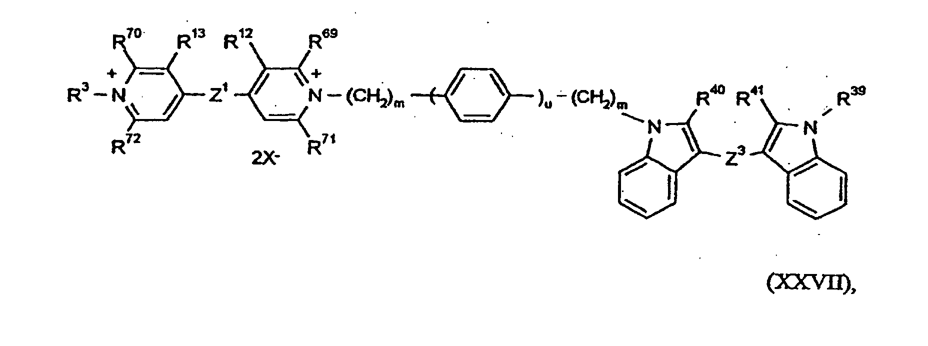 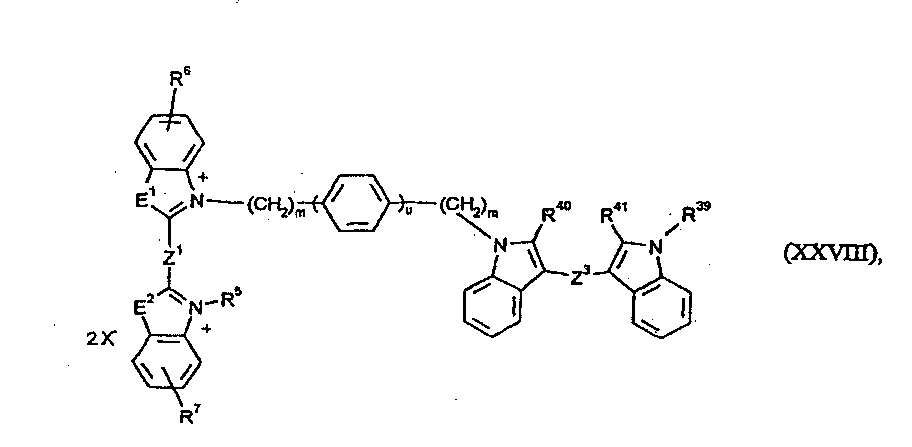 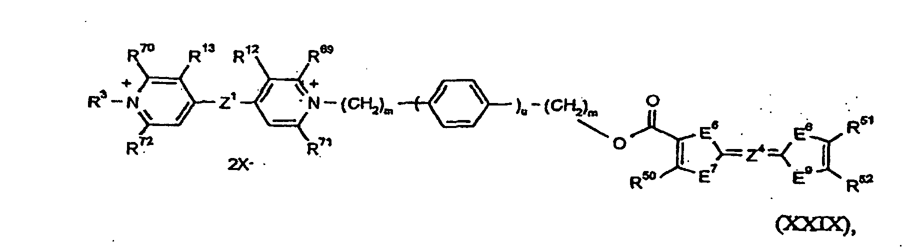 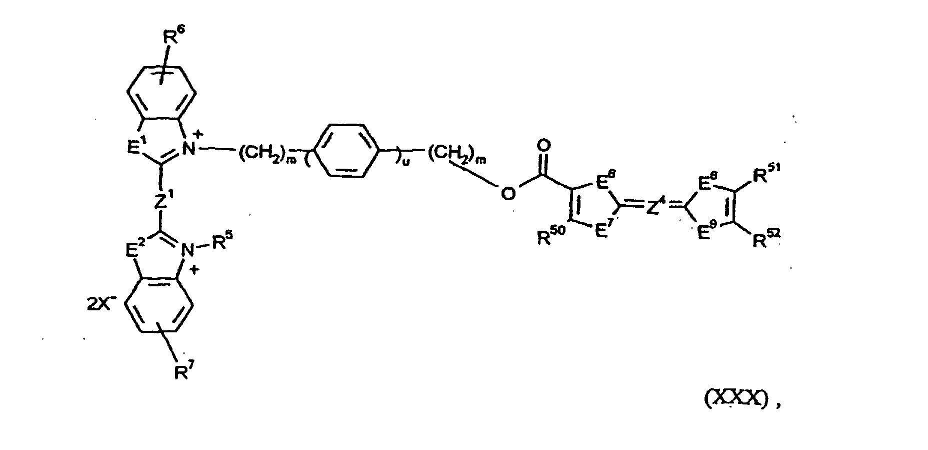 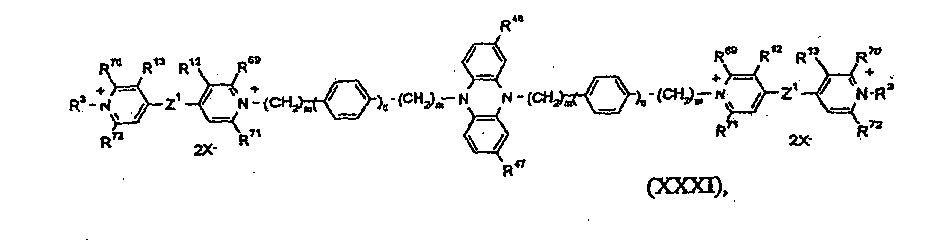 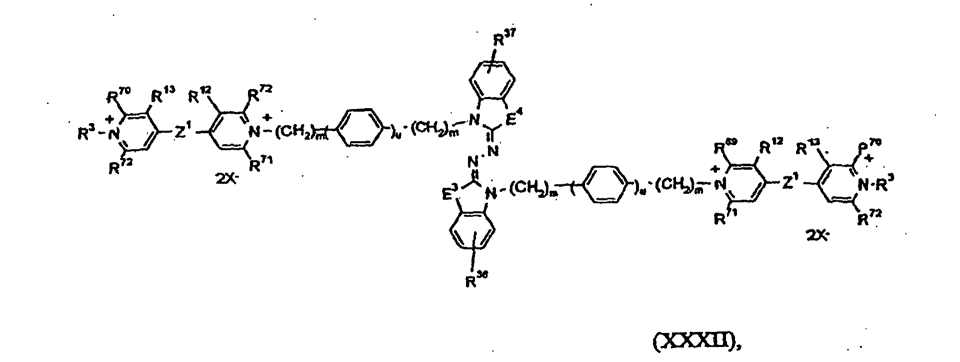 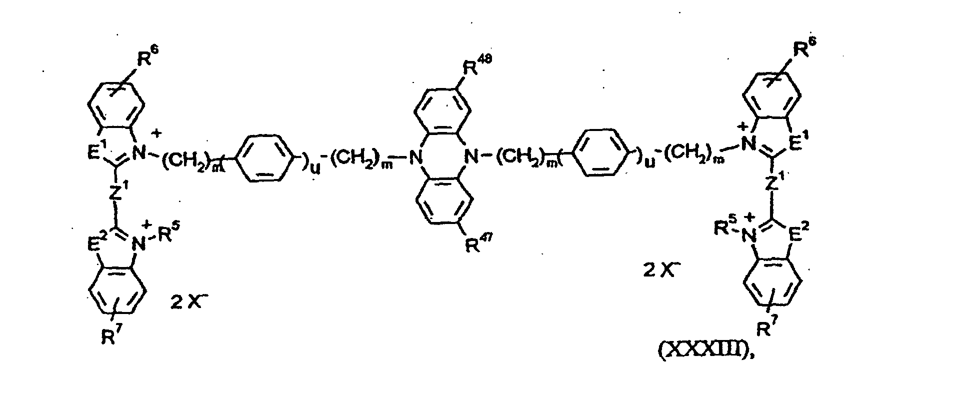 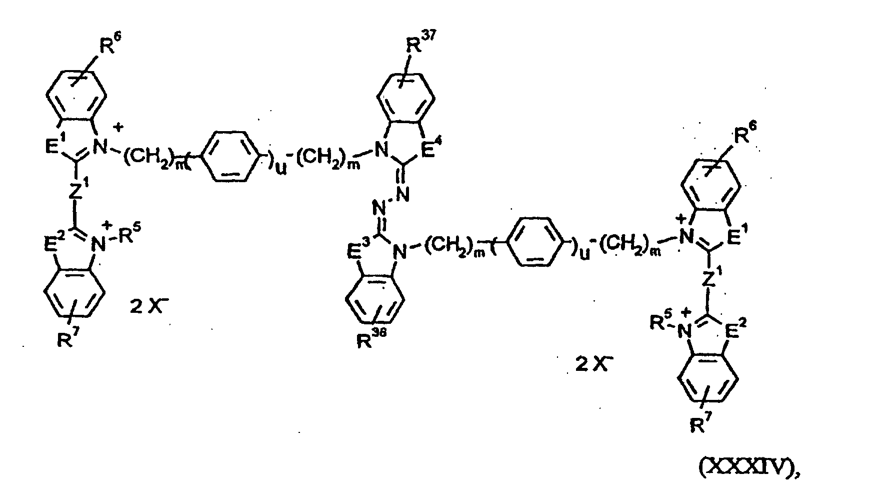 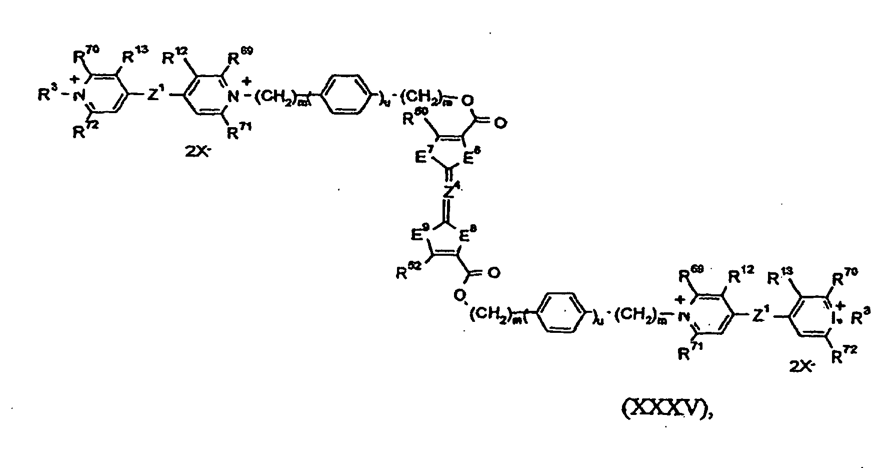 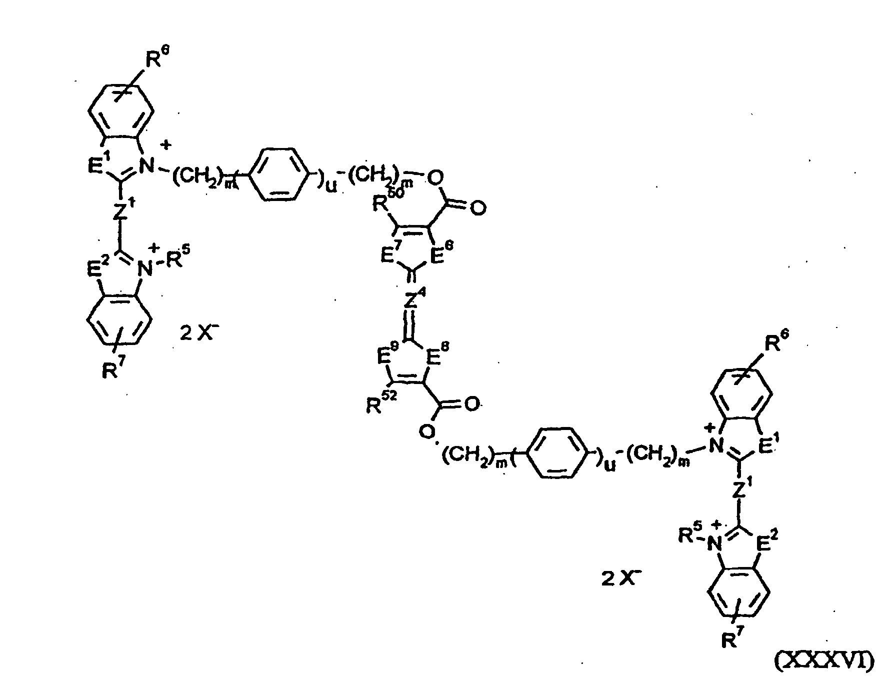 ou contient au moins une substance de formule (Ic) selon l'une des formules 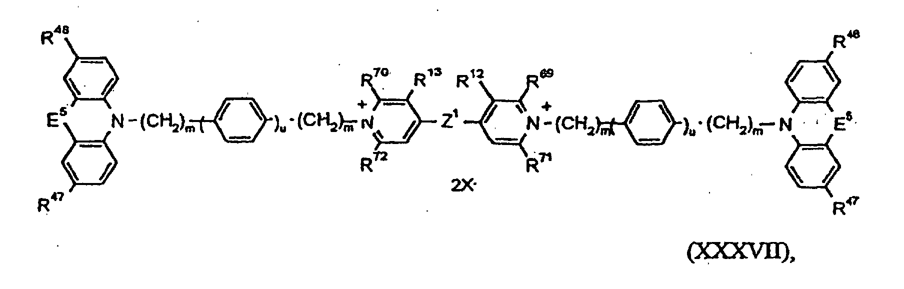 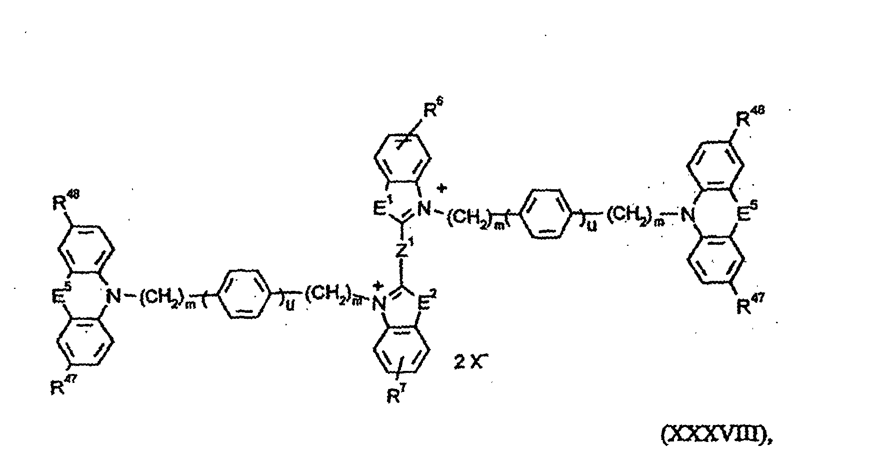 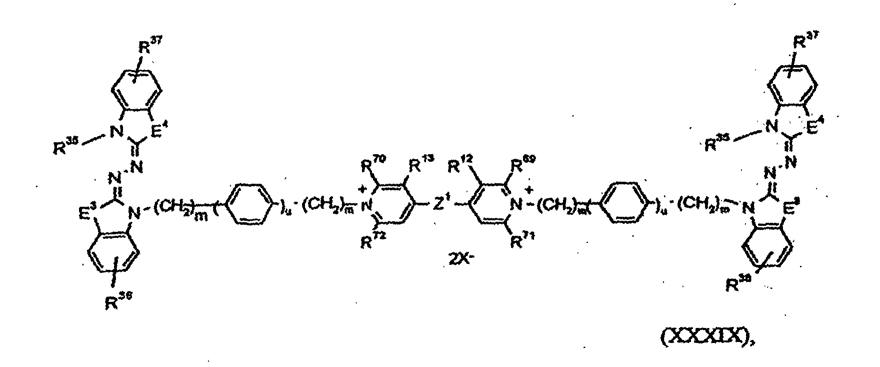 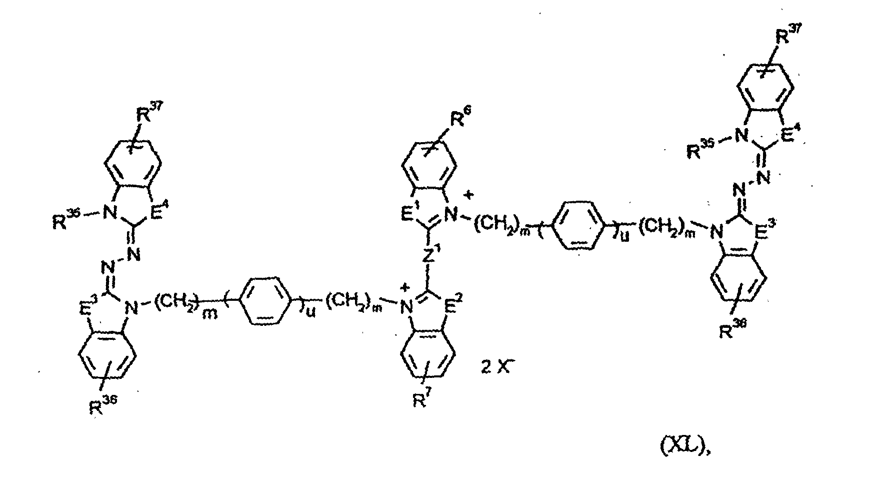 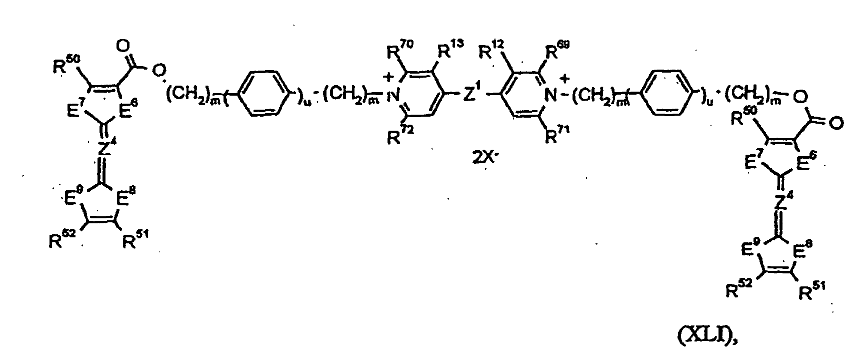 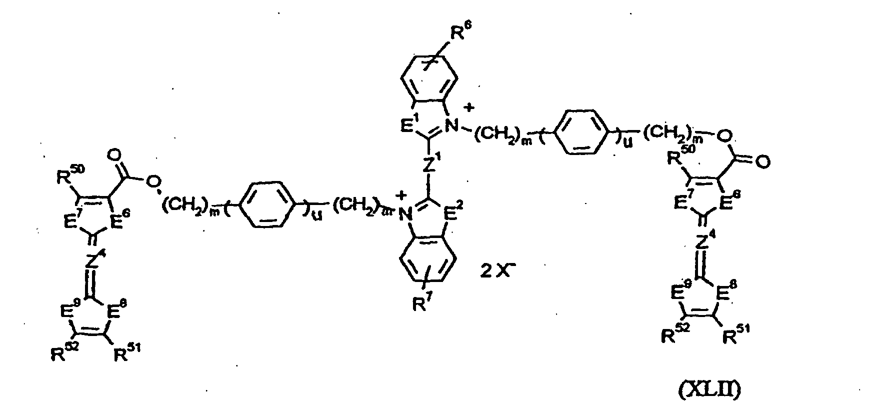 où
R³, R⁵, R³⁵ et R³⁹ représentent indépendamment les uns des autres méthyle, éthyle, propyle, butyle, pentyle, hexyle ou benzyle,
R⁶, R⁷ et R³⁶, R³⁷ sont identiques par paires et représentent l'hydrogène, méthyle, méthoxy, le chlore, cyano ou méthoxycarbonyle,
R¹² et R¹³ représentent l'hydrogène ou bien, quand Z¹ représente une liaison directe, conjointement un pont -CH=CH-,
R⁶⁹ à R⁷² sont identiques et représentent l'hydrogène ou méthyle,
E¹ et E² sont identiques et représentent O ou S,
Z¹ représente une liaison directe ou -CH=CH-,
R³², R⁴⁷ et R⁴⁸ représentent l'hydrogène,
E³ à E⁵ représentent indépendamment l'un de l'autre O, S ou NR⁵⁹, où E³ et E⁴ sont cependant identiques,
R²⁹ à R³¹ et R⁵⁹ représentent indépendamment les uns des autres méthyle, éthyle, propyle, butyle, pentyle, hexyle ou benzyle, où R²⁹ à R³¹ sont de préférence identiques,
R⁴⁰ et R⁴¹ sont identiques et représentent l'hydrogène, méthyle, éthyle, propyle, butyle ou phényle,
Z³ représente une liaison directe, -CH=CH- ou -N=N-,
R⁵⁰ à R⁵² représentent indépendamment les uns des autres l'hydrogène, méthyle, méthoxy, le chlore, cyano, méthoxycarbonyle, éthoxycarbonyle ou phényle, mais sont cependant de préférence identiques,
E⁶ à E⁹ sont identiques entre eux et représentent S, Se ou NR⁵⁹,
Z⁴ représente une double liaison directe, un pont =CH-CH= ou =N-N=,
m représente un nombre entier de 1 à 5,
u représente 0 ou 1 et
X⁻ représente un anion incolore inerte dans les conditions redox.

13. Procédé de production de substances d'électrochromes selon les revendications 10 à 12, **caractérisé en ce que** des composés de formule
OX₂-B-A (XLIII)
sont mis à réagir avec des composés de formule
RED₁ (XLIV)
ou **en ce que** des composés de formule
OX₂ (XLV)
sont mis à réagir avec des composés de formule
A-B-RED₁ (XLVI)
où
A représente un groupe partant comme le chlore, le brome, l'iode, OSO₂-alkyle, OSO₂-perfluoroalkyle ou OSO₂-aryle,
et OX₂, RED₁ et B possèdent la signification indiquée dans la revendication 10.

14. Liquide électrochrome contenant un système électrochrome selon au moins l'une des revendications 1 à 9 et au moins un solvant inerte.

15. dispositif électrochrome contenant un liquide électrochrome selon la revendication 14.

16. Dispositif électrochrome selon la revendication 15, **caractérisé en ce qu'**il est sous forme d'une cellule, comme par exemple une cellule solaire, sous forme d'une vitre, d'un miroir, d'un toit ouvrant ou d'un dispositif d'affichage.

17. Dispositif électrochrome selon les revendications 15 et 16, **caractérisé en ce qu'**il consiste en deux plaques de verre ou de matière plastique transparentes tournées l'une vers l'autre, parmi lesquelles éventuellement l'une est réfléchissante et dont les côtés tournés l'un vers l'autre sont munis d'un revêtement électriquement conducteur, entre lesquelles est contenu le liquide électrochrome.
